# EUROPÄISCHE PATENTSCHRIFT

(11) **EP 3 341 490 B1**
(45) Veröffentlichungstag und Bekanntmachung des Hinweises auf die Patenterteilung: **12.05.2021**
(21) Anmeldenummer: 15757235.5
(22) Anmeldetag: 28.08.2015
(51) Int. Cl.: C12P 23/00, C12N 15/63, C12P 5/00

(54) **VERFAHREN ZUR FERMENTATIVEN ALPHA-IONON PRODUKTION**
METHOD OF FERMENTATIVE ALPHA-IONONE PRODUCTION
PROCÉDÉ DE PRODUCTION D'ALPHA-IONONE PAR FERMENTATION

(43) Veröffentlichungstag der Anmeldung: 04.07.2018
(73) Patentinhaber: Phytowelt Greentechnologies GmbH, 41334 Nettetal (DE)
(72) Erfinder: JACH, Guido, 53639 Königswinter (DE); AZDOUFFAL, Sanae, 40591 Düsseldorf (DE); SCHULLEHNER, Katrin, 50823 Köln (DE); WELTERS, Peter, 41334 Nettetal (DE); GOERGEN, Angela, 50674 Köln (DE)
(74) Vertreter: Grünecker Patent- und Rechtsanwälte PartG mbB
(86) Internationale Anmeldenummer: PCT/EP2015/069751
(87) Internationale Veröffentlichungsnummer: WO 2017/036495

(56) Entgegenhaltungen:
- WO-A1-99/61399
- WO-A1-2005/087942
- WO-A1-2016/036915
- US-A1- 2009 216 039
- CUNNINGHAM JR F X ET AL: "A study in scarlet: Enzymes of ketocarotenoid biosynthesis in the flowers of Adonis aestivalis", PLANT JOURNAL FEBRUARY 2005 BLACKWELL PUBLISHING LTD GB, Bd. 41, Nr. 3, Februar 2005 (2005-02), Seiten 478-492, XP002751183, DOI: 10.1111/J.1365-313X.2004.02309.X
- CUNNINGHAM F X ET AL: "One ring or two? Determination of ring number in carotenoids by lycopene epsilon-cyclases", PROCEEDINGS OF THE NATIONAL ACADEMY OF SCIENCES, NATIONAL ACADEMY OF SCIENCES, US, Bd. 98, Nr. 5, 27. Februar 2001 (2001-02-27), Seiten 2905-2910, XP002220813, ISSN: 0027-8424, DOI: 10.1073/PNAS.051618398 in der Anmeldung erwähnt
- CUNNINGHAM F X ET AL: "MOLECULAR STRUCTURE AND ENZYMATIC FUNCTION OF LYCOPENE CYCLASE FROM THE CYANOBACTERIUM SYNECHOCOCCUS SP STRAIN PCC7942", THE PLANT CELL, AMERICAN SOCIETY OF PLANT BIOLOGISTS, US, Bd. 6, Nr. 8, 1. August 1994 (1994-08-01), Seiten 1107-1121, XP000872730, ISSN: 1040-4651, DOI: 10.1105/TPC.6.8.1107

## Beschreibung

### Technisches Gebiet der Erfindung

Die vorliegende Erfindung betrifft ein Verfahren zur Herstellung von enantiomerenreinem alpha-Ionon und ein Verfahren zur Herstellung von hochreinem epsilon-Carotin. Des Weiteren betrifft die Erfindung einen Mikroorganismus, welcher in dem erfindungsgemäßen Verfahren zur Herstellung von enantiomerenreinem alpha-Ionon oder in dem erfindungsgemäßen Verfahren zur Herstellung von hochreinem epsilon-Carotin kultiviert wird. Dieser Mikroorganismus enthält heterologe Nukleotidsequenzen, die die Enzyme Geranylgeranyl-Diphosphat-Synthase, Isopentenyl-Diphosphat-Isomerase (IPI), Phytoen Desaturase/Dehydrogenase (crtl), Phytoensynthase (crtB) und Lycopen-epsilon-Zyklase (EC) oder Geranylgeranyl-Diphosphat-Synthas, Isopentenyl-Diphosphat-Isomerase (IPI), Phytoen Desaturase/Dehydrogenase (crtl), Phytoensynthase (crtB), Lycopen-epsilon-Zyklase (EC) und Carotenoid-Cleavage-Dioxygenase (CCD1) kodieren.

### Hintergrund der Erfindung

Duftstoffe werden heutzutage in einer Vielzahl von Produkten verwendet wie z. B. Wasch- und Reinigungsmitteln, aber auch in zahllosen kosmetischen Haut-und Körperpflegeprodukten, Deodorants und Parfüms. Diese Duftstoffe müssen nicht nur in ausreichender Menge und kostengünstig hergestellt werden können, sondern auch rein vorliegen. Letzteres ist erforderlich, um unerwünschte Nebenwirkungen vermeiden zu können, aber auch um maximale Freiheit der Formulierung von Duftstoffgemischen zu haben.

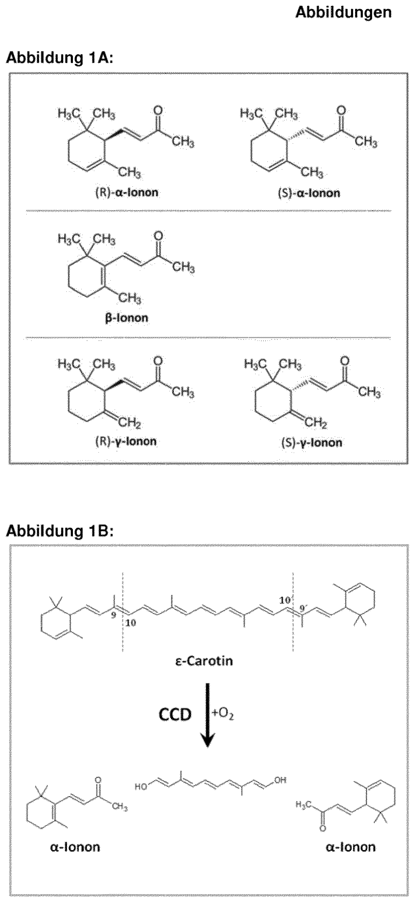

Die lonone sind eine zu den Terpenen gehörende Gruppe von weit verbreiteten Naturstoffen, die in vielen Pflanzen durch Umsetzung von Carotinoiden gebildet werden. Ionone werden in der Riechstoffindustrie in großen Mengen als Duftstoffe verwendet. Die Stoffgruppe umfasst die Einzelsubstanzen alpha-, beta- und gamma-Ionon, die sich hinsichtlich der Lage der Doppelbindung in der Ionon-Ringstruktur unterscheiden. Für alpha- und gamma-Ionon existieren zudem jeweils zwei Enantiomere: (R)- alpha-Ionon und (S)- alpha-Ionon bzw. (R)-gamma-Ionon und (S)-gamma-Ionon (Abbildung 1). Alle Einzelsubstanzen unterscheiden sich in ihrem Geruch. Dies gilt insbesondere auch für die Enantiomeren. So wird der Duft von (S)-alpha-Ionon als zedernholzartig/himbeerartig beschrieben, während das entsprechende R-Enantiomer einen fruchtig-blumigen Veilchenduft besitzt. Aufgrund dieser Eigenschaften ist (R)-alpha-Ionon für die Riechstoffindustrie besonders interessant.

In natürlichen Quellen liegen die lonone immer als Gemische unterschiedlicher Zusammensetzung vor. Die häufigsten Vertreter sind alpha- und β-Ionon, wobei β-Ionon jeweils das Hauptprodukt darstellt, während alpha-Ionon in geringerer Menge als Beimischung vorkommt. gamma-Ionon wird nur von wenigen Pflanzen gebildet. Um die Einzelsubstanzen in reiner Form aus der Natur zu gewinnen und für die industrielle Nutzung bereitzustellen, sind daher aufwändige und kostenintensive Anreicherungs- und Reinigungsschritte erforderlich. Dies gilt insbesondere für das industriell relevante, aber nur in Mindermengen vorkommende (R)-alpha-Ionon.

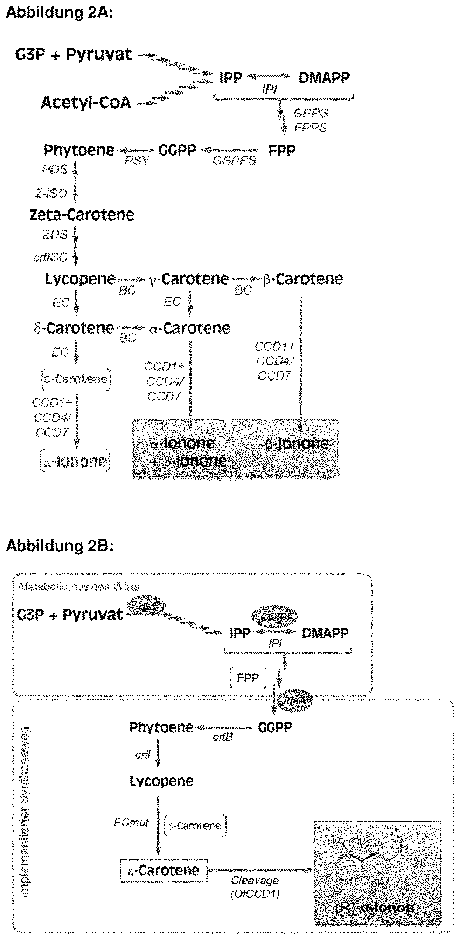

In Pflanzen erfolgt die Bildung der lonone durch einen mehrstufigen Syntheseweg: Zunächst wird das lineare Carotinoid Lycopen erzeugt, das dann durch die Aktivität unterschiedlicher Lycopen-Zyklasen in verschiedene weitere, mono- oder bizyklische Carotinoide umgesetzt wird (Abbildung 2A). Hauptprodukt ist zumeist β-Carotin. Die Ionon-Bildung erfolgt nachfolgend in einem weiteren Schritt durch oxidative Abspaltung aus den gebildeten Carotinoiden durch Carotenase-Enzyme, die auch als Carotinoid-spaltende Dioxygenasen (engl. carotenoid-cleavage-dioxygenases; CCD) bezeichnet werden (Abbildung 2A).

In der Pflanze führt die Umsetzung verschiedener Carotine durch Carotenasen (CCD) zur Bildung von alpha-Ionon (Abbildung 2A). Zumeist erfolgt eine Umsetzung von alpha-Carotin, was zu einem Gemisch aus alpha- und β-Ionon führt. Im Gegensatz hierzu erfolgt die ausschließliche Bildung von alpha-Ionon durch die CCD-katalysierte Spaltung von epsilon-Carotin oder seiner Vorstufe delta-Carotin. Zur gebildeten Gesamtmenge an alpha-Ionon trägt dieser Weg kaum bei, da delta-Carotin und epsilon-Carotin in den meisten Pflanzen nicht oder nur in Spuren gebildet werden.

Das alpha-Ionon kann auch chemisch synthetisiert werden. Ein Verfahren zur Synthese von alpha- und β-Ionon aus Citral wurde bereits 1893 entwickelt und patentiert. Dieses chemisch synthetisierte alpha-Ionon liegt als Racemat vor, enthält also beide unterschiedlich riechenden Enantiomere. Die Nutzbarkeit in der Riechstoffindustrie ist daher eingeschränkt. In neuerer Zeit sind auch enantioselektive Synthesemethoden für (S)-alpha-Ionon (Bovolenta et al., 2004) bzw. (R)-alpha-Ionon (Soorukram und Knochel, 2004) beschrieben worden. Die Enantiomerenreinheit des so erzeugten (R)-alpha-Ionons liegt bei 97 %, es sind also noch substanzielle Menge des S- Enantiomers enthalten. Die Produktausbeute beträgt 61 %.

Im Sinne einer nachhaltigen und umweltverträglichen Ionon-Produktion sind fermentative Produktionssysteme, insbesondere unter Verwendung rekombinanter Mikroorganismen zu bevorzugen.

Grundsätzlich sind rekombinante, delta-Carotin und epsilon-Carotin-produzierende Mikroorganismen als Basis für die Produktion von alpha-Ionon geeignet. Für eine effiziente alpha-Ionon-Produktion ist die Verwendung von delta-Carotin als Ausgangssubstanz aber wenig sinnvoll, da aus diesem monozyklischen Substrat nur ein Molekül Ionon pro Ausgangsmolekül erhalten werden kann. Eine Biosynthese unter Verwendung von epsilon-Carotin ist zu bevorzugen, da hiermit die doppelte Ausbeute an alpha-Ionon pro Ausgangsmolekül epsilon-Carotin erzielt werden kann.

Die bislang beschriebenen rekombinanten Systeme mit nachgewiesener Ionon-Synthese resultierten zumeist aus der biochemischen Charakterisierung verschiedener CCD1-Enzyme. Die natürlichen Abläufe wurden nachgeahmt, indem die zu testenden CCD1-Enzyme zusätzlich in rekombinante Bakterienstämme eingebracht wurden, in die zuvor bereits die Synthesegene für verschiedene Carotinoide implementiert worden waren (Misawa et al., 1990, Cunningham et al., 1996). Dabei wurde gezeigt, dass CCD1-Enzyme ein breites Spektrum an Substraten besitzen und diese Substrate mit unterschiedlicher Präferenz umsetzen. Bevorzugt wurden CCD1-Enzyme in Stämmen getestet, die Lycopen, beta-Carotin oder Zeaxanthin bereitstellen.

Carotinoide sind weit verbreitete lipophile Pigmente, die zur Klasse der Tetraterpene zählen. Die meisten Carotinoide lassen sich formal von dem azyklischen Lycopen ableiten und entstehen durch Zyklisierung der Endgruppen, Hydrierung oder Dehydrierung oder auch durch Einführung von Sauerstoff.

Ausgangsstoffe der Carotinoidsynthese sind die Isoprenderivate Isopentenyl-Diphosphat (IPP) und dessen Isomer Dimethyl-Allyl-Diphosphat (DMAPP), die je nach Wirtorganismus über den so genannten Methylerythritolphosphatweg (MEP-Weg) und/oder den so genannten Mevalonatweg (MVA-Weg) gebildet werden. In Pflanzen sind beide Synthesewege aktiv. Durch die Verknüpfung mehrerer IPP und DMAPP-Moleküle wird zunächst die wichtige Zwischenstufe Geranylgeranyl-Diphosphat (GGPP) gebildet. Durch die Kondensation zweier GGPP-Einheiten entsteht dann die erste Tetraterpenverbindung, das Phytoen. Das farblose Phytoen wird dann über wiederholte Desaturierung und Isomerisierung zum roten Lycopen umgesetzt, das eine zentrale Zwischenstufe darstellt, aus der durch unterschiedliche Zyklisierungsreaktionen die Carotinoide alpha-, beta-, gamma-, delta- und epsilon-Carotin gebildet werden. Eine Übersicht zeigt Abbildung 2a.

Carotinoid-Biosynthesewege wurden nicht nur für Pflanzen, sondern für verschiedene Mikroorganismen (Bakterien und Hefen) identifiziert und die entsprechenden Gene bzw. Gencluster isoliert. Bereits 1986 wurde von Perry und Mitarbeitern eine entsprechende bakterielle Genkaskade aus *Erwinia herbicola* zur Expression in *E. coli* kloniert (Perry et al, 1986). Die analoge Expressionskassette aus *Erwinia uredovora* wurde 1990 zum ersten Mal beschrieben (Misawa et al., 1990). Nachfolgend beschrieben Cunningham und Mitarbeiter rekombinante mikrobielle Systeme zur Synthese von Carotinoiden in *E. coli,* die die Carotinoid-Biosynthesegene der zwei zuvor benannten Erwinia-Arten verwendet, *E. uredovora* und *E. herbicola* (Cunningham et al., 1996).

Seitdem haben viele Forschergruppen das von Perry et al. (1986) beschriebene Plasmid als Basis verwendet, um die Funktionalität einzelner bakterieller oder pflanzlicher Enzyme der Carotinoid-Biosynthese über Komplementationsversuche aufzuklären (Cunningham et al., 1994, 1996). Dabei wurde immer die ursprüngliche Kassette aus *E. herbicola* mit dem ursprünglichen Promotor, Terminator und den Übergängen zwischen den einzelnen Genen einschließlich ursprünglicher Ribosomenbindungsstellen (Shine-Dalgarno-Sequenzen; SD) verwendet.

Kürzlich wurde von einem weiteren Gen-Cluster zur Carotinoid-Synthese berichtet, das in *E. coli* exprimiert wird. Die heterologe Expression der Gene aus *Cronobacter sakazakii* führt zu einer Gelbfärbung der Kolonien, die einzelnen Gene wurden als idi, crtE, crtX, crtY, crtl, crtB und crtZ identifiziert (Zhang et al., 2014). Diese wurden in unterschiedlichen Kombinationen mit optimierten SD-Sequenzen in den Zielvektor pWSK29 kloniert.

Bislang wurden verschiedene Cluster identifiziert und heterolog exprimiert; die Druckschriften berichten jedoch nicht über die Optimierung von Ausbeuten an Carotinen.

An der Synthese von epsilon-Carotin aus Lycopen und der Freisetzung von lononen aus Carotinoiden sind einige essentielle Enzyme beteiligt, die nachfolgend beschrieben werden.

Lycopen-epsilon-Zyklasen katalysieren die Bildung von alpha-Ionon-Ringstrukturen an den Enden des Lycopen-Moleküls, wobei zunächst das monozyklische delta-Carotin als Intermediat gebildet wird, welches dann von einigen Lycopen-epsilon-Zyklasen (EC) unter Ausbildung eines zweiten alpha-Iononrings zum epsilon-Carotin umgesetzt wird. Dem entsprechend werden zwei Klassen von Lycopen-epsilon-Zyklasen unterschieden: Eine Klasse kann nur einen Ring bilden und daher ausschließlich delta-Carotin synthetisieren. Hierzu gehört die epsilon-Zyklase aus *Arabidopsis thaliana* und die überwiegende Mehrzahl der bislang untersuchten und beschriebenen pflanzlichen EC-Enzyme. Die zweite EC-Klasse kann auch eine zweite Ringbildung am gleichen Molekül (bzw. der monozyklischen Zwischenstufe) durchführen und somit auch epsilon-Carotin produzieren. Hierzu gehört das EC-Enzym aus *Lactuca sativa* (Salat). Es produziert überwiegend epsilon-Carotin.

Die beschriebenen EC-Enzyme aus Zea *mays* (Mais) und *Adonis aestivalis* synthetisieren eine Mischung aus gleichen Anteilen delta-Carotin und epsilon-Carotin (Bai et al., 2009; Cunningham und Gantt, 2001).

Cunningham und Gantt (2001) konnten zeigen, dass der Austausch einer einzelnen Aminosäure zu einer Änderung des Produkts der enzymatischen Reaktion führt. Für das Enzym aus Salat (*Latuca sativa*) führte der Austausch von Histidin zu Leucin an Position 457 zur Bildung eines monozyklischen Produkts, während die komplementäre Mutation an entsprechender Position im EC-Enzym von *A.thaliana* (L448H) zu einem bizyklischen Produkt führte, d. h., dass die Bildung von epsilon-Carotin bevorzugt wird. Diese Arbeit zeigte auch die Einführung einer Hexapeptidsequenz aus der Salat-EC in das Arabidopsisenzym, die zu 4 Aminosäureaustauschen in diesem Enzym führte (A447S/L448H/Q451L/F452M). Dieses mutierte Enzym synthetisierte das bizyklische epsilon-Carotin als Hauptprodukt.

Neuere Arbeiten zeigen auch für die EC aus Mais, dass die Änderung der Aminosäuresequenz an einer Positionen (L461H) zu einer Erhöhung des Anteils an bizyklischem epsilon-Carotin auf 80% führt. Eine Mutation von Alanin zu Serin an Position 502 führte jedoch zu einem höheren Anteil des monozyklischen delta-Carotin (Bai et al., 2009).

Carotenasen sind pflanzliche Enzyme, die in der Lage sind, mono- und bizyklische Carotinoide im Bereich der linearen zentralen Molekülstruktur zu spalten. Die Reaktion erfolgt unter Verbrauch von O₂. Entsprechend des Reaktionsmechanismus werden die Enzyme daher auch als Carotinoid-spaltende Dioxygenasen (engl. carotenoid-cleavage-dioxygenases; CCD) bezeichnet. An welcher Stelle des Substratmoleküls die Spaltung erfolgt, wird durch das jeweilige CCD-Enzym bestimmt und ist eine wesentliche Enzymeigenschaft. Nur CCD-Enzyme, die ihre Carotinsubstrate zwischen den Positionen 9,10 und 9', 10' spalten können, sind in der Lage lonone freizusetzen.

Die Carotenoid-spaltende Dioxygenase 1 aus *A. thaliana* (AtCCD1) akzeptiert ebenso wie ihre Homologen aus Mais und Tomate ein breites Spektrum an linearen und zyklischen Carotinoid-Substraten und kann neben alpha- bzw. beta-Carotin auch Lycopen spalten (Vogel et al., 2008). Die Autoren zeigten für Mais-CCD1 auch die Spaltung von ζ-Carotin. Für die CCD1 aus *Osmanthus fragrans* (OfCCD1) wurde gezeigt, dass sie in vitro alpha- und β-Carotin umsetzt und dabei β- und alpha-Ionon produziert (Baldermann et al., 2010). Substratspezifischer ist die CCD aus *Daucus carota,* die kein Lycopen, Phytoene oder GGPP umsetzen kann, aber Zeaxanthin, β-Carotin und auch delta-Carotin (Yahyaa et al., 2013).

Wie oben beschrieben, stellt alpha-Ionon und insbesondere (R)-alpha-Ionon einen wichtigen Rohstoff für die Riechstoffindustrie dar. Die bisher vorhandenen Verfahren zur Herstellung von alpha-Ionon mittels der Isolation aus natürlichen Quellen oder der chemischen Synthese bieten nur unzureichenden Zugang zu diesem wichtigen Rohstoff in ausreichender Quantität und Reinheit. Des Weiteren ist vor dem Hintergrund einer umweltverträglichen und nachhaltigen Herstellung eine Alternative zur klassischen chemischen Synthese wünschenswert.

Es ist daher Aufgabe der vorliegenden Erfindung, alpha-Ionon und insbesondere (R)-alpha-Ionon mittels eines umweltverträglichen und nachhaltigen Verfahrens in ausreichender Quantität und Reinheit herzustellen.

### Beschreibung der Erfindung

Die oben formulierte Aufgabe wird durch die Bereitstellung eines Verfahrens zur Herstellung von enantiomerenreinem alpha-Ionon gelöst, umfassend das Kultivieren eines Mikroorganismus, der heterologe Nukleotidsequenzen enthält, die die folgenden Enzyme kodieren: Geranylgeranyl-Diphosphat-Synthase, Isopentenyl-Diphosphat-Isomerase (IPI), Phytoen-Desaturase/Dehydrogenase (crtl), Phytoensynthase (crtB), Lycopen-epsilon-Zyklase (EC) und Carotenoid-Cleavage-Dioxygenase (CCD1).

Die Anmeldung beschreibt eine Nukleinsäure welche eine Sequenz umfasst, die eine Lycopen-epsilon-Zyklase (EC) kodiert, die die Umsetzung von Lycopen zu epsilon-Carotin katalysiert, wobei die Lycopen-epsilon-Zyklase (EC) zu einer größeren epsilon-Carotin-Ausbeute führt als eine Referenz-Lycopen-epsilon-Zyklase (EC) mit einer Sequenz gemäß SEQ ID Nr. 26. Weiter beschreibt die Anmeldung die durch die Nukleinsäure kodierte Lycopen-epsilon-Zyklase (EC).

Weiterhin beschreibt die Anmeldung ein Plasmid, welches dadurch charakterisiert ist, dass es Nukleotidsequenzen umfasst, die die Enzyme Geranylgeranyl-Diphosphat-Synthase, Isopentenyl-Diphosphat-Isomerase (IPI), Phytoen-Desaturase/Dehydrogenase (crtl) und Phytoensynthase (crtB) kodieren, wobei die heterologe Expression des durch das Plasmid kodierten Lycopen-Biosynthesewegs zu einer erhöhten Lycopen-Ausbeute gegenüber der heterologen Expression des durch das Plasmid pAC-BETAipi-ΔcrtY (SEQ ID Nr. 28) kodierten Lycopen-Biosynthesewegs führt.

Außerdem trägt zur Lösung der Aufgabe der durch die Erfindung bereitgestellte Mikroorganismus bei, welcher heterologe Nukleotidsequenzen enthält, die die folgenden Enzyme kodieren: Geranylgeranyl-Diphosphat-Synthase, Isopentenyl-Diphosphat-Isomerase (IPI), Phytoen-Desaturase/Dehydrogenase (crtl), Phytoensynthase (crtB), und Lycopen-epsilon-Zyklase (EC), oder Geranylgeranyl-Diphosphat-Synthase, Isopentenyl-Diphosphat-Isomerase (IPI), Phytoen-Desaturase/Dehydrogenase (crtl),Phytoensynthase (crtB), Lycopen-epsilon-Zyklase (EC) und Carotenoid-Cleavage-Dioxygenase (CCD1).

Zur Lösung der Aufgabe trägt auch das durch die Erfindung bereitgestellte Verfahren zur Herstellung von hochreinem epsilon-Carotin aus Lycopen bei, umfassend das Kultivieren eines Mikroorganismus, der heterologe Nukleotidsequenzen enthält, die die folgenden Enzyme kodieren: Geranylgeranyl-Diphosphat-Synthase, Isopentenyl-Diphosphat-Isomerase (IPI), Phytoen-Desaturase/Dehydrogenase (crtl), Phytoensynthase (crtB), und Lycopen-epsilon-Zyklase (EC), wobei die Lycopen-epsilon-Zyklase (EC) eine der folgenden Mutationen oder Mutationskombinationen, bezogen auf die Referenzsequenz gemäß SEQ ID Nr:19, umfasst: ECmut9 (L404S), ECmut10 (A403S/L404T), ECmut3.3 (A403E/L404A/A445S) und ECmut3.2 (A403C/L404C/ A445S).

Schließlich beschreibt die Anmeldung ein Plasmid, welches dadurch charakterisiert ist, dass es Nukleotidsequenzen umfasst, die die folgenden Enzyme kodieren: 1-Desoxy-D-xylulose-5-phosphat-Synthase (DXS) und Isopentenyl-Pyrophosphat-Isomerase (CwIPI).

Die erfindungsgemäßen Mikroorganismen und Verfahren ermöglichen die effiziente fermentative Herstellung von Lycopen, mit deutlich verbesserten Ausbeuten gegenüber dem Stand der Technik, sowie von epsilon-Carotin, mit gegenüber dem Stand der Technik deutlich verbesserten Ausbeuten an epsilon-Carotin. Sowohl Lycopen als auch epsilon-Carotin sind Intermediate in der Herstellung von alpha-Ionon.

Die vorliegende Erfindung zeichnet sich also unter anderem durch eine gegenüber dem Stand der Technik verbesserte Herstellung der beiden Intermediate der alpha-Ionon-Biosynthese, Lycopen und epsilon-Carotin, aus (Abbildung 2B). Dieser Aspekt der vorliegenden Erfindung trägt maßgeblich dazu bei, alpha-Ionon und insbesondere (R)-alpha-Ionon mittels eines umweltverträglichen und nachhaltigen Verfahrens in ausreichender Quantität und Reinheit herzustellen.

Ein weiterer Vorteil der vorliegenden Erfindung gegenüber dem Stand der Technik ist die Bereitstellung von (R)-alpha-Ionon in enantiomerenreiner Form in ausreichender Quantität und Reinheit.

Außerdem sind die erfindungsgemäßen fermentativen Verfahren umweltverträglicher und nachhaltiger als die traditionell chemischen Syntheseverfahren des Stands der Technik.

### Kurze Beschreibung der Abbildungen

Abbildung 1A: Ionon-Strukturen inklusive der Struktur des R- und S-Enantiomers von alpha-Ionon
Abbildung 1B: Reaktionsschema der CCD-katalysierten lononbildung durch Carotin-Spaltung. Die Positionen der gespaltenen Doppelbindungen sind nummeriert. CCD = Carotenoid-cleavage-dioxygenase
Abbildung 2A: Ionon-Syntheseweg in Pflanzen. Ausgangsstoffe der Carotinoidsynthese sind die Isoprenderivate Isopentenyl-Diphosphat (IPP) und dessen Isomer Dimethyl-Allyl-Diphosphat (DMAPP), die in Pflanzen über den so genannten Methylerythritolphosphatweg (MEP-Weg) und/oder den so genannten Mevalonatweg (MVA-Weg) gebildet werden. Durch die Verknüpfung mehrerer IPP und DMAPP-Moleküle wird zunächst die wichtige Zwischenstufe Geranylgeranyl-Diphosphat (GGPP) gebildet. Durch die Kondensation zweier GGPP-Einheiten entsteht dann die erste Tetraterpenverbindung, das Phytoen. Pflanzen benötigen 4 verschiedene Enzyme, um Phytoen in Lycopen umzuwandeln, während im erfindungsgemäßen Syntheseweg nur das bakterielle Enzym crtl benötigt wird (Abbildung 2B). Im natürlichen pflanzlichen System sind sowohl das Enzym Lycopen-epsilion-Zyklase (EC) als auch Lycopen-beta-Zyklase (BC) enthalten. Es wird daher ein Gemisch aus alpha-, beta- und epsilon-Carotin gebildet, wobei epsilon-Carotin nur in einer geringen Zahl von Pflanzen in sehr kleinen Mengen nachgewiesen wurde. β-Carotin ist das Hauptprodukt. alpha-Carotin wird zumeist in geringen Mengen synthetisiert. Die Spaltung der Carotinoide zu den lononen erfolgt in zwei Stufen durch Kombinationen der Enzyme CCD1 und CCD4 bzw. CCD1 und CCD7. Entsprechend der vorliegenden Substratverteilung wird überwiegend β-Ionon gebildet. Das daneben in geringeren Mengen vorliegende alpha-Carotin wird zu gleichen Teilen in alpha- und beta-Ionon gespalten. alpha-Ionon liegt daher immer als mengenmäßig kleine Beimischung zum hauptsächlich erzeugten β-Ionon vor. Die Namen der erforderlichen Enzyme sind kursiv dargestellt und den entsprechenden Reaktionspfeilen zugeordnet. IPI: Isopentenyl Diphosphate Isomerase; GGPPS: Geranylgeranyl-Diphosphat-Synthase; PSY: Phytoen-Synthase; PDS: Phytoen-Desaturase; Z-ISO: zeta-Carotin-Isomerase; ZDS: zeta-Carotin-Desaturase; crtlSO: cis-Lycopen-Isomerase; EC: Lycopen-Epsilon-Cyclase; BC: Lycopen-Beta-Cyclase; CCD1: Carotinoid-Cleavage-Dioxygenasel (cytosolisch); CCD4: Carotinoid-Cleavage-Dioxygenase4 (plastidär); CCD7: Carotinoid-Cleavage-Dioxygenase7 (plastidär).
Abbildung 2B: Ein Beispiel des erfindungsgemäßen Ionon-Synthesewegs. Durch die Vermeidung der beta-Zyklase-Aktivität und die Verwendung einer mutierten Lycopen-epsilon-Zyklase wird ausschließlich epsilon-Carotin gebildet (die Zwischenstufe δ-Carotin ist maximal in Spuren nachweisbar). Für die Spaltung wird nur ein Enzym benötigt und es entsteht reines alpha-Ionon. Die Namen der eingesetzten Enzyme sind kursiv dargestellt und dem entsprechend Reaktionspfeil zugeordnet. dxs: Desoxy-D-xylulose-5-phosphat-Synthase; IPI: Isopentenyl Diphosphate Isomerase; CwIPI: Isopentenyl Diphosphate Isomerase aus *Curcuma wenyujin;* idsA: Geranylgeranyl-Diphosphat-Synthase; crtl: Phytoen-Desaturase/Dehydrogenase; crtB: Phytoen-Synthase; ECmut: mutierte Lycopen-Epsilon-Cyclase gemäß Erfindung; CCD1: Carotenoid-Cleavage-Dioxygenase (AtCCD1 oder OfCCD1). Die Anbindung des im mikrobiellen Wirt implementierten Syntheseweges an dessen Grundstoffwechsel ist gekennzeichnet.

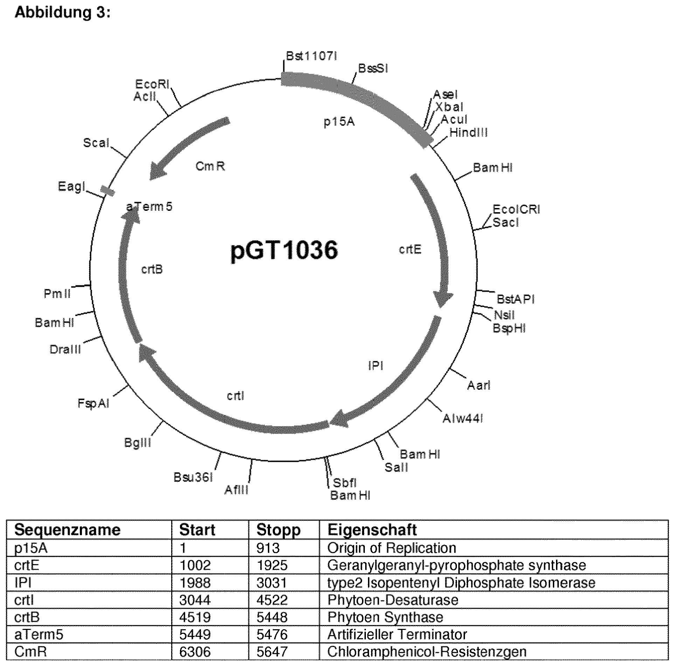

Abbildung 3: Plasmidkarte pGT1036 ("Lyc-Synthese" Plasmid, Expressionsplasmid). Die kodierenden Sequenzen der benannten Proteine sind als Pfeile dargestellt. Regulatorische DNA-Sequenzen sind als Box dargestellt. Die Positionen einmalig auftretender Restriktionsenzymschnittstellen sind angezeigt. Die exakten Positionen der benannten genetischen Elemente und deren Funktion sind in der Tabelle aufgelistet.

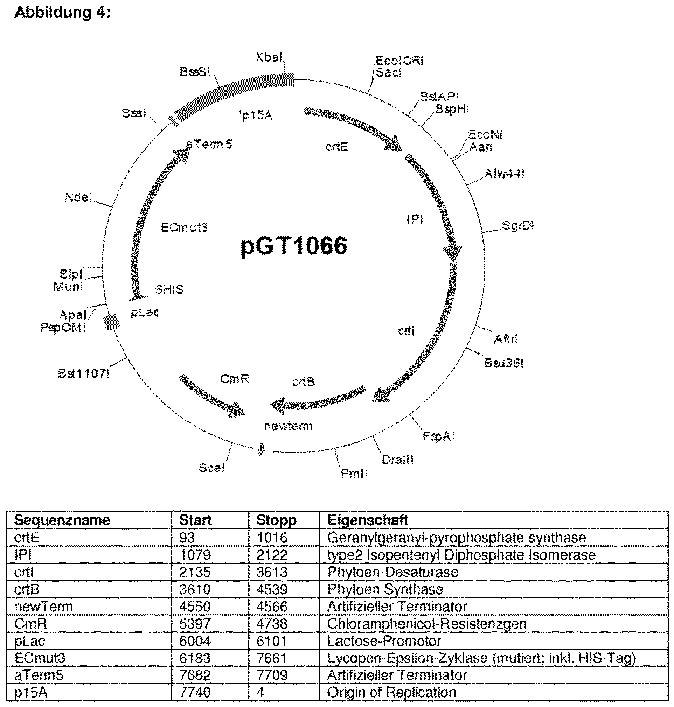

Abbildung 4: Plasmidkarte pGT1066 ("eCaro Synthese" Plasmid, Expressionsplasmid). Die kodierenden Sequenzen der benannten Proteine sind als Pfeile dargestellt. Regulatorische DNA-Sequenzen sind als Box dargestellt. Die Positionen einmalig auftretender Restriktionsenzymschnittstellen sind angezeigt. Die exakten Positionen der benannten genetischen Elemente und deren Funktion sind in der Tabelle aufgelistet.

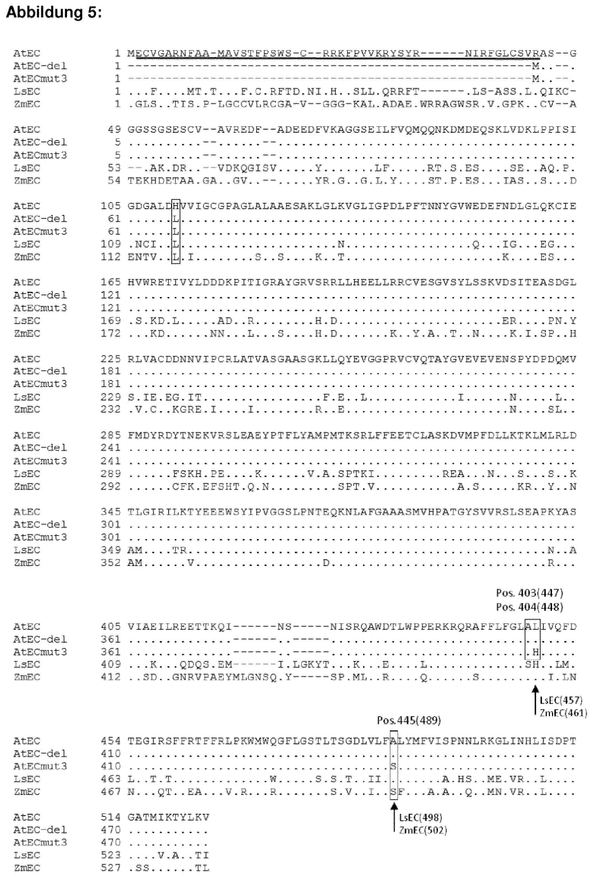

Abbildung 5: Homologievergleich von AtECmut3 und Lage der Punktmutationen. Vergleich der Datenbank-Proteinsequenz für die Lycopen-epsilon-Zyklase aus *A. thaliana* (AtEC) mit den erfindungsgemäßen Sequenzen AtEC-del und AtECmut3 und den homologen Enzymen aus Salat (LsEC) und Mais (ZmEC). Das im Wildtyp-Enzym AtEC detektierte Chloroplasten-Targetsignal (N-terminale 44 Aminosäuren) ist unterstrichen. AtEC-del ist die aus *A. thaliana* klonierte und N-terminal um 44 Aminosäuren verkürzte Proteinvariante, die die gemeinsame Basis der erzeugten erfindungsgemäßen Mutanten (AtECmut) darstellt. Die mutierten Positionen 403, 404 und 445 sind angegeben (Boxen). In Klammern sind die entsprechenden Positionsangaben für das Volllängen-Wildtyp-AtEC-Protein beigefügt. Die Positionen der für das Salat- bzw. Mais-Enzym beschriebenen Mutationen sind angezeigt.

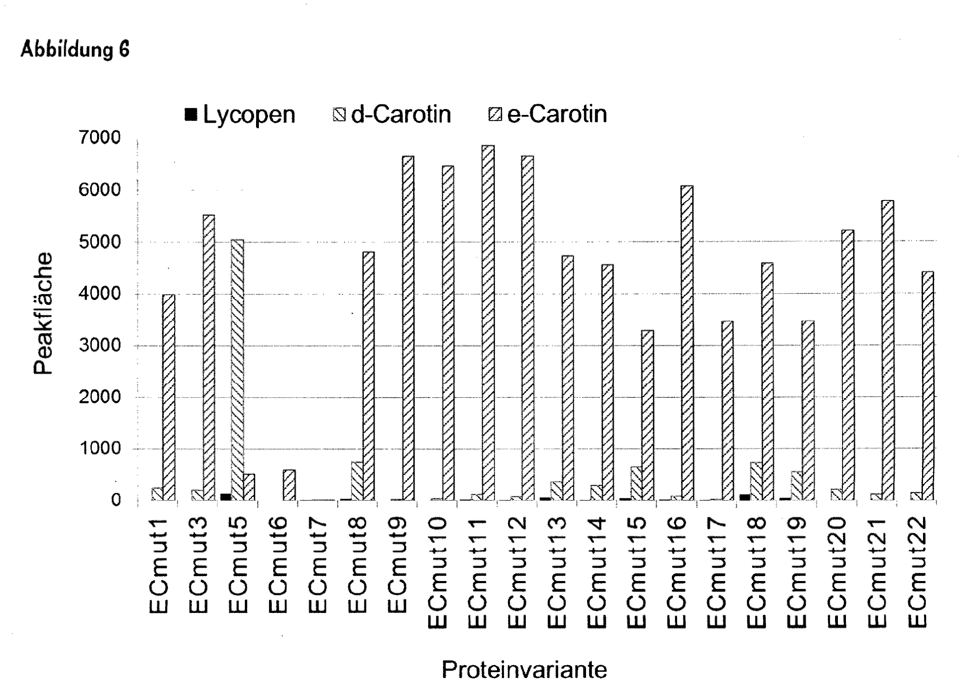

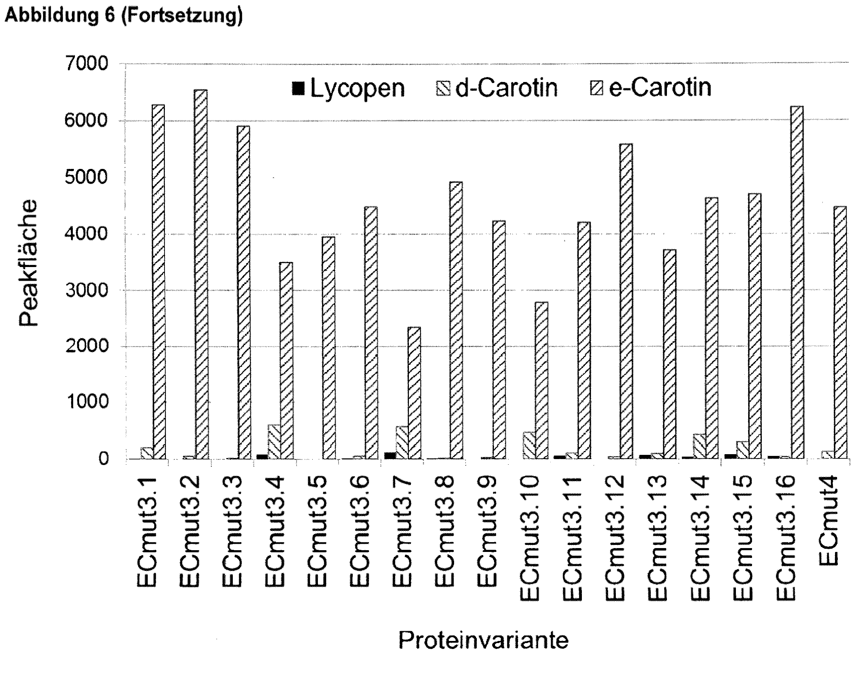

Abbildung 6: Quantitative HPLC-Analyse der AtECmut-Produktprofile. Messung der Ausbeuten an Lycopen, delta-Carotin und epsilon-Carotin für die verschiedenen erhaltenen Mutanten der Lycopen-epsilon-Zyklase (ECmut). Die entsprechenden Expressionvektoren wurden in *E. coli* TOP10 Zellen eingebracht und die erhaltenen Stämme bezüglich der synthetisierten Carotinoide per HPLC analysiert. Die Kultivierung der Zellen erfolgte für 24h bei 28°C in dYT-Medium (+Chloramphenicol und Ampicillin). Die gebildeten Carotinoide wurden mit Aceton quantitativ extrahiert und in das HPLC-Laufmittel überführt. Dargestellt sind die Absolutwerte der ermittelten Peakflächen für gleiche Zellzahlen der verschieden Stämme. Lycopen wurde von fast allen Stämmen nahezu vollständig umgesetzt; delta-Carotin wurde nur von einigen Stämmen nicht vollständig umgesetzt. Die meisten Stämme zeigen eine effiziente Produktion von epsilon-Carotin, das Ausgangsprodukt für die Umsetzung zu enantiomerenreinem alpha-Ionon ist. Die Variante ECmut1 entspricht der in der Literatur bereits beschriebenen Mutante (Cunningham & Gantt, 2001) und dient als Referenz. Die Mutanten ECmut 9, 10, 11, 12, 16, 21, 3.2, 3.3, 3.8 und 3.16 sind signifikant besser als die Referenz hinsichtlich Produktmenge und Produktreinheit.

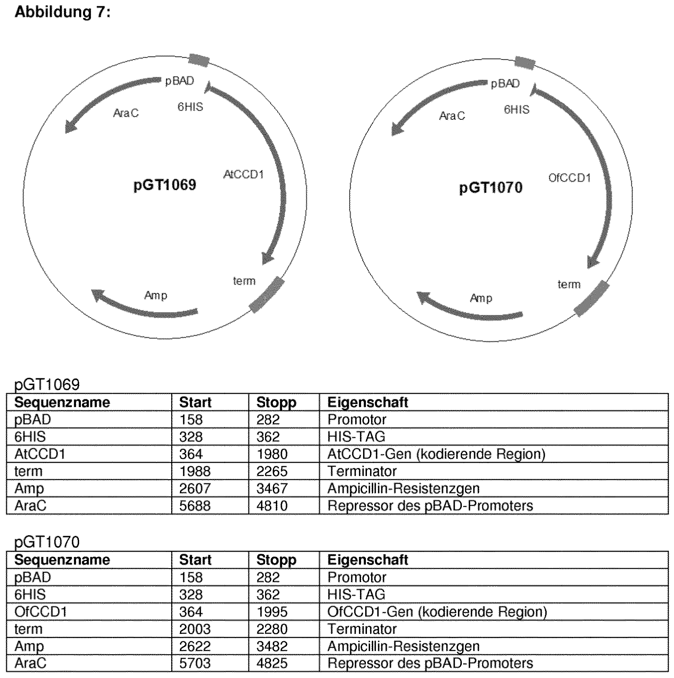

Abbildung 7: Plasmidkarten der Plasmide pGT1069 und pGT1070 (Expressionsplasmide für AtCCD1 und OfCCD1). Die kodierenden Sequenzen der benannten Proteine sind als Pfeile dargestellt. Regulatorische DNA-Sequenzen sind als Box dargestellt. Die exakten Positionen der benannten Elemente sind in den beiden Tabellen aufgelistet.

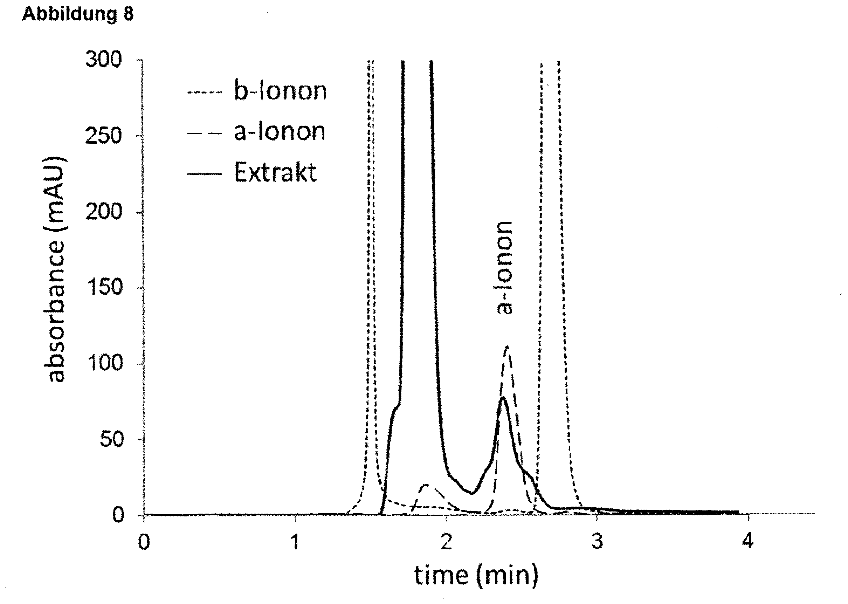

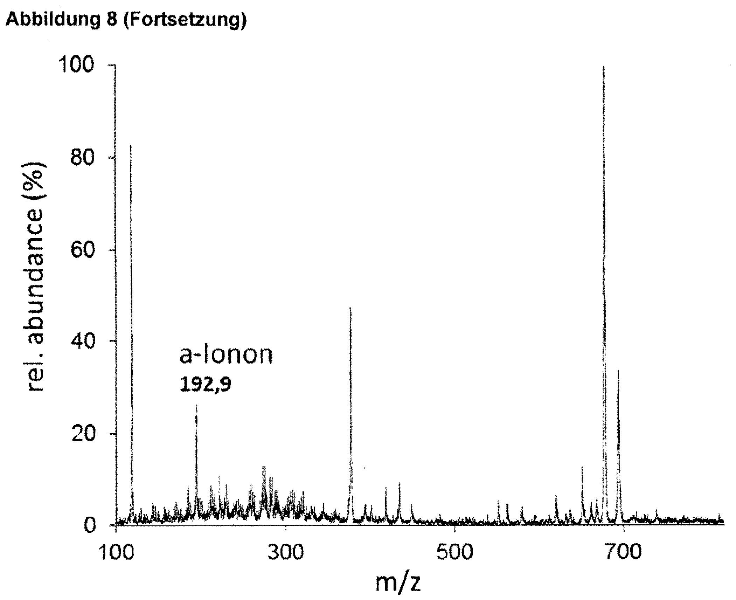

Abbildung 8: Nachweis der alpha-Ionon-Produktion. Dargestellt ist ein HPLC-Chromatogramm und ein LC-MS-Spektrum. Ein multitransgener *E. coli*-Stamm mit den Enzymen crtE, IPI, crtB, crtl, ECmut3, AtCCD1, wurde für 24Std. bei 28°C in LB-Medium unter Schütteln inkubiert und die Expression des AtCCD1-Enzym durch Zugabe von Arabinose (Endkonzentration: 0,1% (w/v)) für 4 Std. induziert. Die erhaltenen epsilon-Carotin-Abbauprodukte wurden im Anschluss nach Lyse der Zellen mit Diethylether extrahiert und dann mittels HPLC analysiert. Die Chromatogramme für die eingesetzten Ionon-Referenzsubstanzen und den erhaltenen Diethyletherextrakt sind dargestellt (gepunktete Linie: β-Ionon-Referenz, gestrichelte Linie: alpha-Ionon-Referenz; durchgezogene Linie: Chromatogramm des Extrakts). In gleicher Weise erzeugte Diethyletherextrakte wurden über LC-MS massenspektroskopisch vermessen. Die dem alpha-Ionon entsprechende Masse von 192,9 konnte eindeutig nachgewiesen werden.

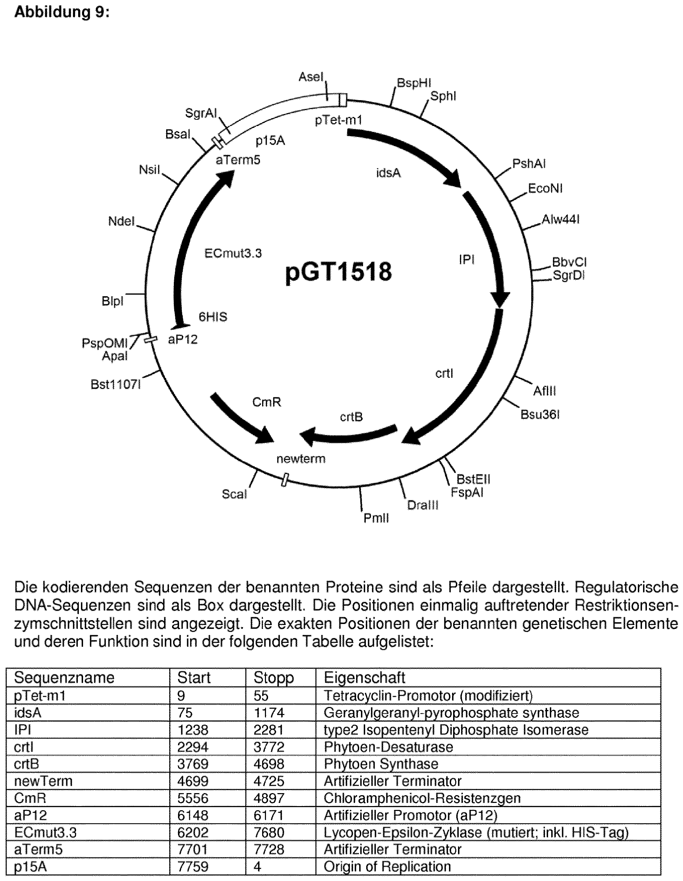

Abbildung 9: Plasmidkarte pGT1518 ("eCaro-Synthese" Plasmid, Expressionsplasmid). Dieses Plasmid kodiert für den erfindungsgemäßen Lycopen Biosyntheseweg (idsA, IPI, crtl und crtB) unter der Kontrolle des pTet-m1 Promoters und für die Lycopen-epsilon-Zyklase (EC) mit der Mutationskombination ECmut3.3.unter Kontrolle des aP12 Promotors. Die kodierenden Sequenzen der benannten Proteine sind als Pfeile dargestellt. Regulatorische DNA-Sequenzen sind als Box dargestellt. Die Positionen einmalig auftretender Restriktionsenzymschnittstellen sind angezeigt. Die exakten Positionen der benannten genetischen Elemente und deren Funktion sind in der Tabelle aufgelistet.

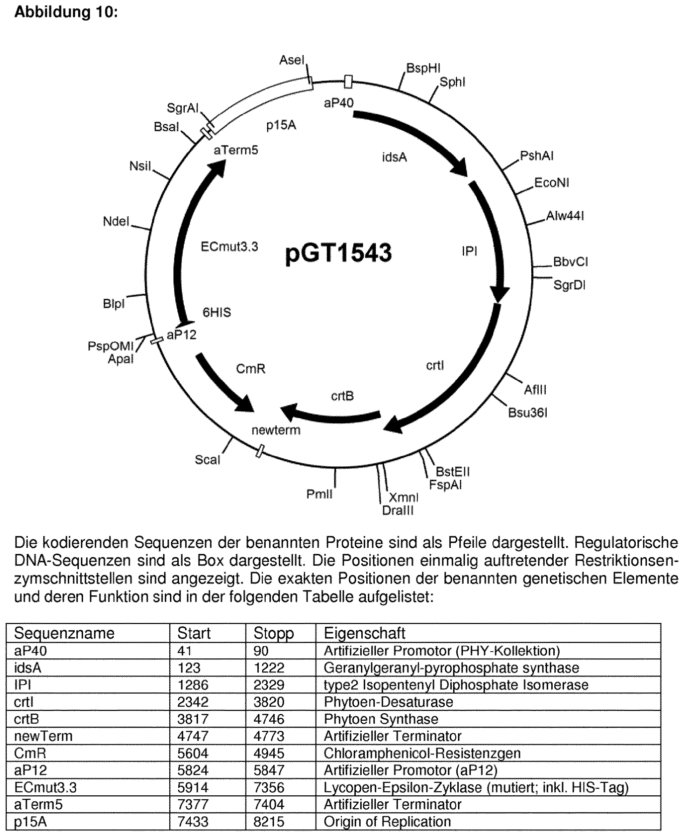

Abbildung 10: Plasmidkarte pGT1543 ("eCaro-Synthese" Plasmid, Expressionsplasmid). Dieses Plasmid kodiert für den erfindungsgemäßen Lycopen Biosyntheseweg (idsA, IPI, crtl und crtB) unter der Kontrolle des aP40 Promoters und für die Lycopen-epsilon-Zyklase (EC) mit der Mutationskombination ECmut3.3 unter der Kontrolle des aP12 Promotors. Die kodierenden Sequenzen der benannten Proteine sind als Pfeile dargestellt. Regulatorische DNA-Sequenzen sind als Box dargestellt. Die Positionen einmalig auftretender Restriktionsenzymschnittstellen sind angezeigt. Die exakten Positionen der benannten genetischen Elemente und deren Funktion sind in der Tabelle aufgelistet.

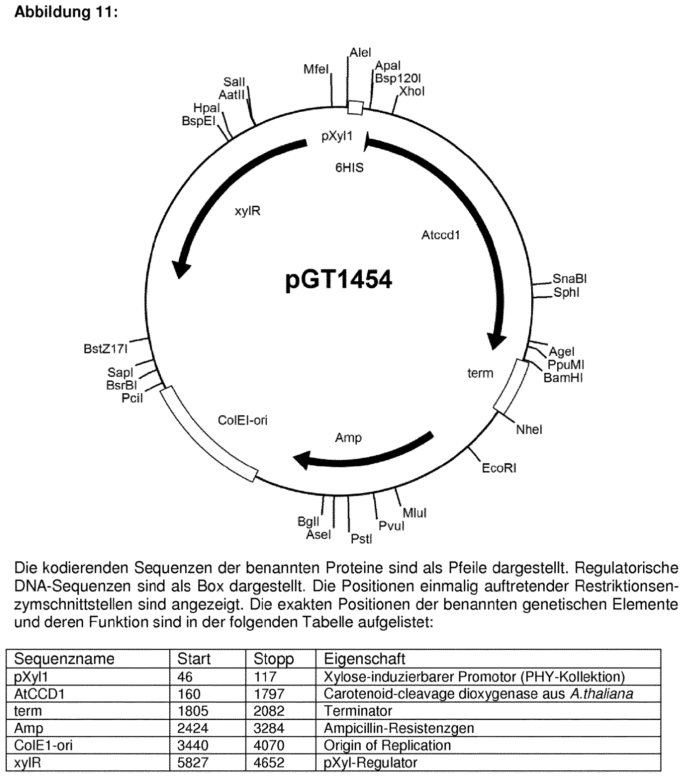

Abbildung 11: Plasmidkarte pGT1454 ("eCaro-Spaltung" Plasmid, Expressionsplasmid). Dieses Plasmid kodiert für Carotenoid-Cleavage-Dioxygenase (CCD1) aus Arabidopsis thaliana (AtCCD1). Die kodierenden Sequenzen der benannten Proteine sind als Pfeile dargestellt. Regulatorische DNA-Sequenzen sind als Box dargestellt. Die Positionen einmalig auftretender Restriktionsenzymschnittstellen sind angezeigt. Die exakten Positionen der benannten genetischen Elemente und deren Funktion sind in der Tabelle aufgelistet.

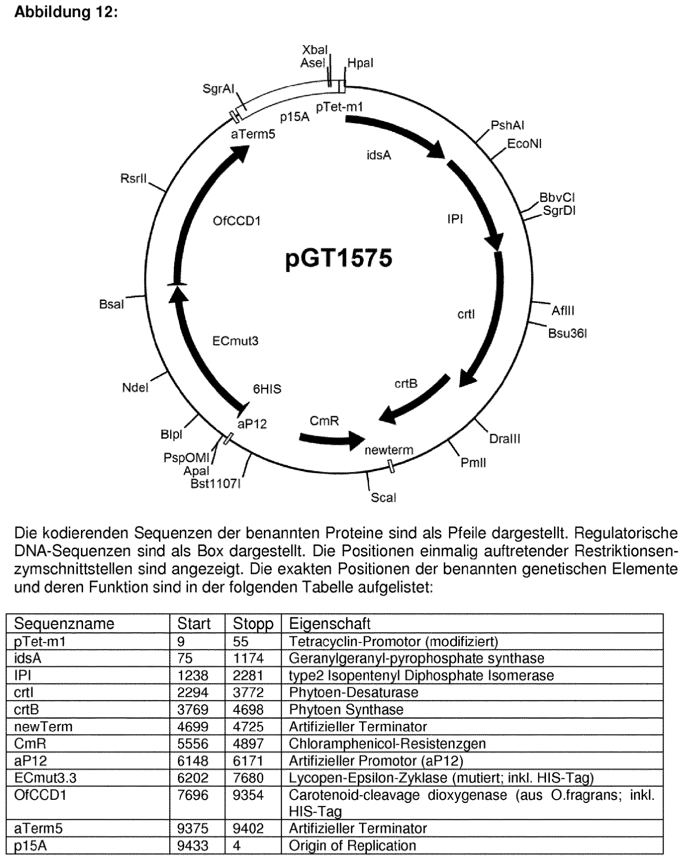

Abbildung 12: Plasmidkarte pGT1575 ("Ionon-Synthese" Plasmid, Expressionsplasmid). Dieses Plasmid kodiert für den erfindungsgemäßen Lycopen Biosyntheseweg (idsA, IPI, crtl und crtB) unter der Kontrolle des pTet-m1 Promotors und für die Lycopen-epsilon-Zyklase (EC) mit der Mutationskombination ECmut3.3 sowie für die Carotenoid-Cleavage-Dioxygenase (CCD1) aus Osmanthus fragrans (OfCCD1), beide unter der Kontrolle des aP12 Promotors. Die kodierenden Sequenzen der benannten Proteine sind als Pfeile dargestellt. Regulatorische DNA-Sequenzen sind als Box dargestellt. Die Positionen einmalig auftretender Restriktionsenzymschnittstellen sind angezeigt. Die exakten Positionen der benannten genetischen Elemente und deren Funktion sind in der Tabelle aufgelistet.

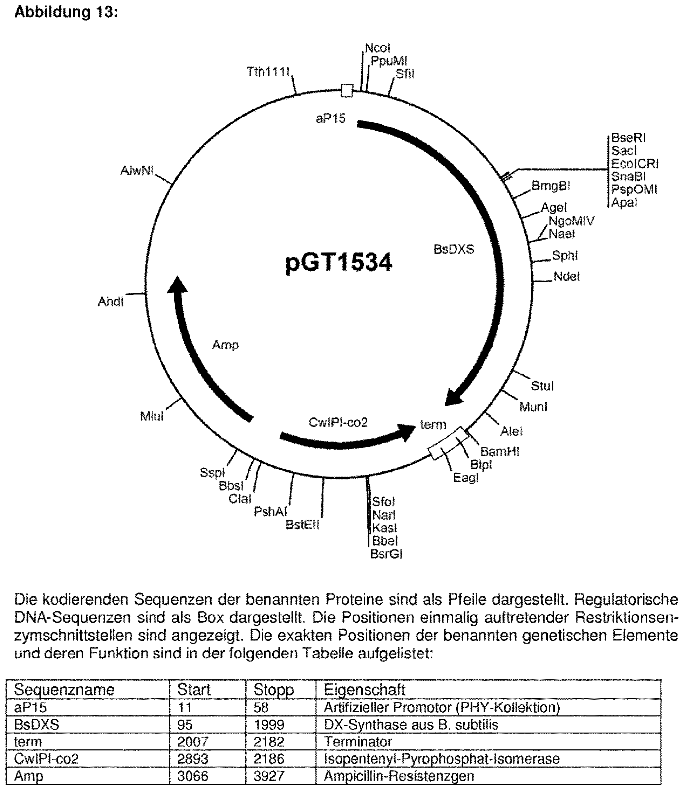

Abbildung 13: Plasmidkarte pGT1534 ("MEP-Weg" Plasmid, Expressionsplasmid). Dieses Plasmid kodiert für die 1-Desoxy-D-xylulose-5-Phosphat-Synthase (DXS) unter der Kontrolle des aP15 Promotors und die Isopentenyl-Diphosphat-Isomerase (CwIPI-co2), eine codonoptimierte Variante der Isopentenyl-Diphosphat-Isomerase (CwIPI) aus Curcuma wenyujin, unter der Kontrolle des pTet-m1 Promotors. Die kodierenden Sequenzen der benannten Proteine sind als Pfeile dargestellt. Regulatorische DNA-Sequenzen sind als Box dargestellt. Die Positionen einmalig auftretender Restriktionsenzymschnittstellen sind angezeigt. Die exakten Positionen der benannten genetischen Elemente und deren Funktion sind in der Tabelle aufgelistet.

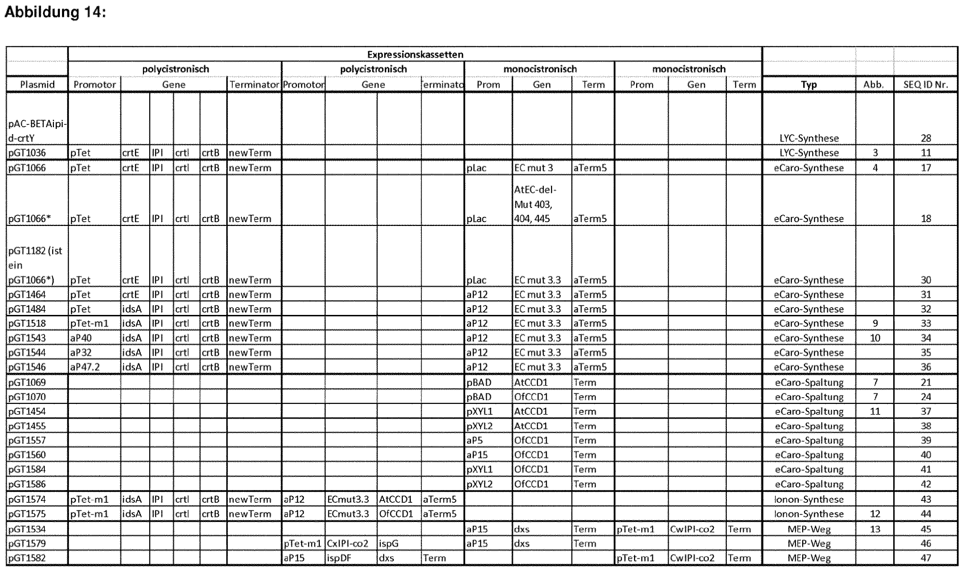

Abbildung 14: Die Tabelle zeigt eine Auswahl an Plasmiden, nämlich "Lyc-Synthese" Plasmide, "eCaro-Synthese" Plasmide, "eCaro-Spaltung" Plasmide, "Ionon-Synthese" Plasmide und "MEP-Weg" Plasmide. Die Tabelle zeigt die jeweiligen ExpressionsKassetten der erfindungsgemäßen Plasmide, die entweder polycistronisch oder monocistronisch organisiert sind. aP5, aP12, aP15, aP32, aP40 und aP47.2 bezeichnet die konstitutiven Promotoren. pTet: Tetracyclin-Promotor aus E. coli-Plasmid pBR332. pLac: Lac-Promotor; Promotorregion des genomischen E. coli Lac-Operon. pBAD: Arabinose induzierbarer Promotor; Promotorregion des genomischen E. coli Arabinose-Operon. pXyl: Xylose induzierbarer Promotor; regulatorische Sequenzen aus dem E.coli Xylose-Operon bestehend aus der bidirektionalen Promotorregion (cis-regulatorischen Sequenzen), die die polycistronischen Operons xylA/xylB und xylF/xylG/xylH/xylR, kontrolliert, wobei dessen Aktivität durch das xylR-Genprodukt des xylFGHR-Operons reguliert wird. pTet-m1: 12bp-Deletion in Promotor vor LYC-Operon; Promotoraktivität wird um den Faktor 2,8 gesteigert. pXyl0: synthetischer Xylose-induzierbarer Promotor. Entstanden durch direkte Verknüpfung des xylR-Gens mit den cis-regulatorischen Sequenzen (mittels Deletion der xylF-, xylG- und xylH-Gensequenzen). Basiskonstrukt. Induzierbarkeit: 25x; rel. Expressionsstärke (max): 2,5% des Referenzpromotors (pLac). pXyl1: Kombination von pXyl0 mit einer optimierten Ribosomen-Bindungsstelle (Shine-Dalgarno-Sequenz) zur effizienten Translation von Zielgenen. pXyl1 Promotor 3-4x aktiver als pXyl0 (max 10% der pLac-Aktivität). pXyl2: Basierend auf pXyl1 wurde die Sequenz der -10-Region (Bindungsstelle der RNA-Polymerase) des downstreamgerichteten Promotorelement modifiziert. Promotor 3-4x aktiver als pXyl0 (max 36% der pLac-Aktivität).

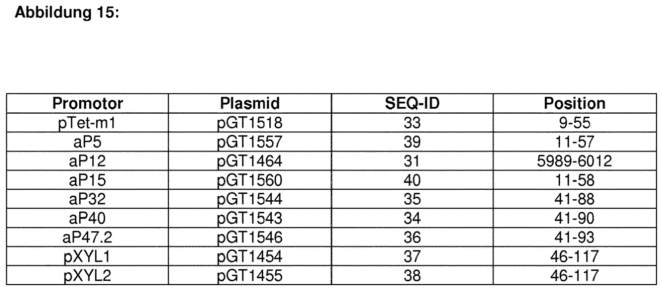

Abbildung 15: Die erfindungsgemäßen Promotoren.

### Detaillierte Beschreibung der Erfindung

Die vorliegende Erfindung erschöpft sich nicht in den hierin spezifisch aufgeführten Erzeugnissen und Verfahren, sondern stellt eine allgemeine technische Lehre zur Verfügung, die es dem Fachmann erlaubt, die hierin beschriebenen Vorteile zu erzielen. Die verwendete Terminologie soll die hier beschriebene allgemeine technische Lehre in keiner Weise einschränken, sondern dient lediglich der Beschreibung der spezifischen Ausführungsformen.

Die verwendeten EC-Klassifikationsnummern (EC-Nummern) klassifizieren Enzyme entsprechend der von ihnen katalysierten Reaktionen. Diese EC-Nummern werden von der *International Union of Biochemistry and Molecular Biology* (IUBMB) herausgegeben und können vom Fachmann im Internet recherchiert werden.

Die hierin verwendeten "accession numbers" (GenBank accession number - GenBank) dienen der eindeutigen Charakterisierung von Nukleotidsequenzen oder Aminosäuresequenzen und wurden der Internetseite des NCBI (*National Center for Biotechnology Information*) entnommen.

Der Begriff "AtEC", wie hierin verwendet, bezeichnet die *Arabidopsis thaliana* Lycopen-epsilon-Zyklase (EC) mit der GenBank accession number GenBank: AAL85102.1.

Der Begriff "LsEC", wie hierin verwendet, bezeichnet die *Lactuca sativa* Lycopen-epsilon-Zyklase (EC) mit der GenBank accession number GenBank: AAK07434.1.

Der Begriff "ZmEC", wie hierin verwendet, bezeichnet die Zea *mays* Lycopen-epsilon-Zyklase (EC) mit der GenBank accession number GenBank: ABU93262.1.

Der hierin verwendete Begriff "Lycopen" bezeichnet ein dem Fachmann bekanntes lineares Carotinoid, das dem Fachmann auch unter den Namen "Lycopin" und "Leukopin" oder "all-trans-Lycopen" bekannt ist. Diese Begriffe sind austauschbar.

Der Begriff "Lycopen-Biosyntheseweg", wie hierin verwendet, bezeichnet die Kombination aus den Enzymen Geranylgeranyl-Diphosphat-Synthase, Isopentenyl-Diphosphat-Isomerase (IPI), Phytoen-Desaturase/Dehydrogenase (crtl) und Phytoensynthase (crtB).

Der Begriff "AtECmut", wie hierin verwendet, bezeichnet die erfindungsgemäßen Mutanten der *Arabidopsis thaliana* Lycopen-epsilon-Zyklase, dabei kann sich der Begriff entweder auf das gesamte Protein oder nur die spezifische Mutation, welche als Ziffer dem Begriff angefügt ist (z. B. AtECmut3), beziehen. Welche Bedeutung der Begriff hat, ergibt sich für den Fachmann eindeutig aus dem jeweiligen Kontext. Der Begriff "AtECmut" wird hierin äquivalent mit dem Begriff "ECmut" verwendet.

Der Begriff "Ausbeute", wie hierin verwendet, bezeichnet die Menge eines hergestellten Stoffes bezogen auf ein festgelegtes Kulturvolumen (Flüssigkultur eines Mikroorganismus), beziehungsweise der aus einem festgelegten Kulturvolumen isolierten Trockenmasse oder bezogen auf eine andere Referenzgröße. Der Begriff "Menge", wie hierin verwendet, bezeichnet die Stoffmenge eines Stoffes oder eine andere Messgröße, deren Wert direkt abhängig ist von der Stoffmenge des Stoffes, z. B. die Peakfläche eines HPLC Absorptionschromatogramms.

Der Begriff "Sequenzidentität", wie hierin verwendet, bezeichnet die Übereinstimmung zweier Nukleotidsequenzen oder Aminosäuresequenzen, angegeben in Prozent, und ist abhängig von der Anzahl an identischen Positionen zwischen den beiden Sequenzen, wobei die Anzahl und die Länge an Lücken (engl. *gaps*), die eingeführt werden müssen, um ein optimales Sequenzalignment zu erhalten, berücksichtigt wird. Wie hierin verwendet, wird die Sequenzidentität nach dem BLAST-Algorithmus (Altschul et al 1990) bestimmt. Wie dem Fachmann bekannt, kann die Sequenzidentität nach dem BLAST-Algorithmus für Nukleotidsequenzen (blastn) oder Aminosäuresequenzen (blastp) einfach auf der NCBI Internetseite bestimmt werden (http://blast.ncbi.nlm.nih.gov/Blast.cgi).

Der Begriff "Geranylgeranyl-Diphosphat-Synthase", wie hierin verwendet, bezeichnet ein Enzym mit der EC-Nummer EC 2.5.1.29, welches die Kondensation von Farnesyl-Diphosphat und Isopentenyl-Diphosphat zu Geranylgeranyl-Diphosphat katalysiert. Bevorzugte Ausführungsformen sind die Geranylgeranyl-Diphosphat-Synthase crtE und idsA.

Der Begriff "1-Desoxy-D-xylulose-5-Phosphat-Synthase (DXS)", wie hierin verwendet, bezeichnet ein Enzym mit der EC-Nummer EC 2.2.1.7, welches die Kondensation von Pyruvat und Glycerinaldehyd-3-Phosphat zu 1-Desoxy-D-xylulose-5-Phosphat (DXP) katalysiert.

Der Begriff "Isopentenyl-Diphosphat-Isomerase (IPI)", wie hierin verwendet, bezeichnet ein Enzym mit der EC-Nummer EC 5.3.3.2, welches die Umlagerung von Isopentenyl-Diphosphat (IPP) zu Dimethylallyl-Diphosphat (DMAPP), bzw. die Gegenreaktion, katalysiert. Auch das Enzym CwIPI oder die codonoptimierte Variante CwIPI-co2 sind Isopentenyl-Diphosphat-Isomerasen mit einer enzymatischen Aktivität gemäß der EC-Nummer EC5.3.3.2.

Der Begriff "Phytoen-Desaturase/Dehydrogenase (crtl)", wie hierin verwendet, bezeichnet ein Enzym mit der EC-Nummer EC 1.3.99.31, welches die Desaturierung (Oxidation) von Phytoen zu all-trans-Lycopen katalysiert.

Der Begriff "Phytoensynthase (crtB)", wie hierin verwendet, bezeichnet ein Enzym mit der EC-Nummer EC 2.5.1.32, welches die Kondensation von zwei Molekülen Geranylgeranyl-Diphosphat zu Phytoen katalysiert.

### Lycopen-epsilon-Zyklase

Die Anmeldung beschreibt eine Nukleinsäure, die eine Lycopen-epsilon-Zyklase kodiert.

Die Nukleinsäure ist dadurch charakterisiert, dass sie eine Sequenz umfasst, die eine Lycopen-epsilon-Zyklase (EC) kodiert, die die Umsetzung von Lycopen zu epsilon-Carotin katalysiert, wobei die Lycopen-epsilon-Zyklase (EC) zu einer größeren epsilon-Carotin-Ausbeute führt als eine Referenz-Lycopen-epsilon-Zyklase mit einer Sequenz gemäß SEQ ID Nr. 26 (AtECmut1).

In einer bevorzugten Ausführungsform der Nukleinsäure, die mit jeder der vorherigen und nachfolgenden Ausführungsformen kombinierbar ist, führt die Lycopen-epsilon-Zyklase (EC) zu einer größeren epsilon-Carotin-Ausbeute, wobei die Lycopen-epsilon-Zyklase (EC) in einem Mikroorganismus exprimiert wird. Um die Lycopen-Ausbeute der Lycopen-epsilon-Zyklase (EC) mit der Referenz-Lycopen-epsilon-Zyklase vergleichen zu können, werden beide Zyklasen in dem gleichen Mikroorganismus unter gleichen Bedingungen exprimiert. Für die Expression der Lycopen-epsilon-Zyklase (EC) und der Referenz-Lycopen-epsilon-Zyklase in dem Mikroorganismus kann ein Plasmid, das die Lycopen-epsilon-Zyklase (EC) oder eine Referenz-Lycopen-epsilon-Zyklase kodiert, mittels Transformation in den Mikroorganismus eingebracht werden.

In einer bevorzugten Ausführungsform der Nukleinsäure, die mit jeder der vorherigen und nachfolgenden Ausführungsformen kombinierbar ist, weist die kodierte Lycopen-epsilon-Zyklase eine Sequenz mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 19 (AtEC-del) auf.

SEQ ID Nr. 19 (AtEC-del) bezeichnet die Sequenz der Lycopen-epsilon-Zyklase aus *Arabidopsis thaliana,* bei der die 44 N-terminalen Aminosäuren (exklusive des N-terminalen Methionins) der Wildtyp-Sequenz entfernt wurden. Dieses N-terminale Peptid ist ein Chloroplasten-Importsignal (Transitpeptid), welches in der Pflanze den Transport des neu synthetisierten Proteins in die Chloroplasten bewirkt. Die Positionsangaben der Aminosäuren der erfindungsgemäßen Mutationen der verschiedenen Lycopen-epsilon-Zyklase Varianten beziehen sich auf diese N-terminal trunkierte Version der Lycopen-epsilon-Zyklase aus *A. thaliana* (SEQ ID Nr. 19). Die entsprechenden Positionen in der Wildtyp-Sequenz der Lycopen-epsilon-Zyklase aus *A. thaliana* sind daher um 44 Positionen verschoben. So entspricht Position 403 in der trunkierten Version (SEQ ID Nr. 19) Position 447 in der Wildtyp-Sequenz (AAL85102.1), Position 404 entspricht Position 448 und Position 445 entspricht Position 489.

In einer weiteren Ausführungsform der Nukleinsäure, die mit jeder der vorherigen und nachfolgenden Ausführungsformen kombinierbar ist, weicht die Sequenz der kodierten Lycopen-epsilon-Zyklase mindestens an einer der Positionen 403, 404 und 445 von der Sequenz gemäß SEQ ID Nr. 19 (AtEC-del) ab.

In einer Ausführungsform der Nukleinsäure, die mit jeder der vorherigen und nachfolgenden Ausführungsformen kombinierbar ist, umfasst die kodierte Lycopen-epsilon-Zyklase eine der folgenden Mutationen oder Mutationskombinationen: ECmut2 (A445S), ECmut9 (L404S), ECmut3 (L404H/A445S), ECmut3.10 (A403C/A445S), ECmut3.12 (L404T/A445S), ECmut4 (A403S/L404H), ECmut5 (A403F /L404W), ECmut6 (A403G/L404G), ECmut7 (A403K/L404D), ECmut8 (A403W/L404R), ECmut10 (A403S/L404T), ECmut11 (A403F/L404S), ECmut12 (A403C/L404S), ECmut13 (A403I/L404T), ECmut14 (A403T/L404R), ECmut15 (A403F/L404R), ECmut16 (A403W/L404G), ECmut17 (A403C/A404C), ECmut18 (A403L/L404V), ECmut19 (A403K/L404R), ECmut20 (A403Y/L404K), ECmut21 (A403Q/L404K), ECmut22 (A403G/L404Q), ECmut3.1 (A403S/L404H/A445S), ECmut3.2 (A403C/L404C/A445S), ECmut3.3 (A403E/L404A/A445S), ECmut3.4 (A403W/L404R/A445S), ECmut3.5 (A403M/L404A/A445S), ECmut3.6 (A403N/L404T/A445S), ECmut3.7 (A403N/L404A/A445S), ECmut3.8 (A403H/L404S/A445S), ECmut3.9 (A403E/L404G/A445S), ECmut3.11 (A403K/L404G/A445S), ECmut3.13 (A403R/L404S/A445S), ECmut3.14 (A403G/L404R/A445S), ECmut3.15 (A403F/L404V/A445S) und ECmut3.16 (A403G/L404G/A445S).

In einer bevorzugten Ausführungsform der Nukleinsäure, die mit jeder der vorherigen und nachfolgenden Ausführungsformen kombinierbar ist, umfasst die kodierte Lycopen-epsilon-Zyklase eine der Mutationen oder Mutationskombinationen: ECmut16 (A403W/L404G), ECmut3.12 (L404T/A445S), ECmut3.3 (A403E/L404A/A445S), ECmut3.5 (A403M/L404A/A445S), ECmut3.8 (A403H/L404S/A445S), ECmut3.9 (A403E/L404G/A445S), ECmut3.16 (A403G/L404G/A445S), ECmut9 (L404S), ECmut10 (A403S/L404T) und ECmut3.2 (A403C/L404C/A445S).

In einer besonders bevorzugten Ausführungsform der Nukleinsäure, die mit jeder der vorherigen und nachfolgenden Ausführungsformen kombinierbar ist, umfasst die kodierte Lycopen-epsilon-Zyklase eine der folgenden Mutationen oder Mutationskombinationen: ECmut9 (L404S), ECmut10 (A403S/L404T) und ECmut3.2 (A403C/L404C/A445S).

In einer weiteren besonders bevorzugten Ausführungsform der Nukleinsäure, die mit jeder der vorherigen und nachfolgenden Ausführungsformen kombinierbar ist, besteht die kodierte Lycopen-epsilon-Zyklase aus einer Sequenz gemäß SEQ ID Nr. 19 und weist eine der oben genannten Mutationen oder Mutationskombinationen auf. Insbesondere bevorzugt sind dabei Ausführungsformen mit einer Mutationskombination, ausgewählt aus der Gruppe, bestehend aus ECmut16 (A403W/L404G), ECmut3.12 (L404T/A445S), ECmut3.3 (A403E/L404A/A445S), ECmut3.5 (A403M/L404A/A445S), ECmut3.8 (A403H/L404S/A445S), ECmut3.9 (A403E/L404G/A445S) und ECmut3.16 (A403G/L404G/A445S) und besonders bevorzugt sind Ausführungsformen mit einer Mutation oder Mutationskombination, ausgewählt aus der Gruppe, bestehend aus ECmut9 (L404S), ECmut10 (A403S/L404T) und ECmut3.2 (A403C/L404C/A445S).

Des Weiteren beschreibt die Anmeldung die Lycopen-epsilon-Zyklase selbst.

Die Lycopen-epsilon-Zyklase ist dadurch charakterisiert, dass sie von einer der oben beschriebenen Nukleinsäuren kodiert wird.

Die kodierte Lycopen-epsilon-Zyklase besteht aus einer Sequenz gemäß SEQ ID Nr. 19 und weist eine der erfindungsgemäßen Mutationen oder Mutationskombinationen auf. Insbesondere bevorzugt sind dabei Ausführungsformen mit einer Mutationskombination, ausgewählt aus der Gruppe, bestehend aus ECmut16 (A403W/L404G), ECmut3.12 (L404T/A445S), ECmut3.3 (A403E/L404A/A445S), ECmut3.5 (A403M/L404A/A445S), ECmut3.8 (A403H/L404S/A445S), ECmut3.9 (A403E/L404G/A445S) und ECmut3.16 (A403G/L404G/A445S) und besonders bevorzugt sind Ausführungsformen mit einer Mutation oder Mutationskombination, ausgewählt aus der Gruppe, bestehend aus ECmut9 (L404S), ECmut10 (A403S/L404T) und ECmut3.2 (A403C/L404C/A445S).

### Plasmide

Die Anmeldung beschreibt auch verschiedene Plasmide, die Nukleotidsequenzen umfassen, die die Komponenten der Lycopen, epsilon-Carotin und/oder alpha-Ionon Biosynthese kodieren. Besonders bevorzugte Plasmide sind in Abbildung 14 aufgeführt Die nachfolgend beschriebenen Plasmide und Expressionskassetten sind nicht von der beanspruchten Erfindung umfasst.

Zur Erfindung gehörig ist ein Plasmid, das Nukleotidsequenzen umfasst, die die Komponenten der Lycopen Biosynthese Geranylgeranyl-Diphosphat-Synthase, Isopentenyl-Diphosphat-Isomerase (IPI), Phytoen-Desaturase/Dehydrogenase (crtl) und Phytoensynthase (crtB) kodieren ("Lyc-Synthese" Plasmid). Zur Erfindung gehörig ist ferner ein Plasmid, das Nukleotidsequenzen umfasst, die die Komponenten der epsilon-Carotin Biosynthese Geranylgeranyl-Diphosphat-Synthase, Isopentenyl-Diphosphat-Isomerase (IPI), Phytoen-Desaturase/Dehydrogenase (crtl), Phytoensynthase (crtB) und Lycopen-epsilon-Zyklase (EC) kodieren ("eCaro-Synthese" Plasmid). Weiterhin zur Erfindung gehörig ist ein Plasmid, das Nukleotidsequenzen umfasst, die die Komponenten zur Spaltung von epsilon-Carotin zu alpha-Ionon Carotenoid-Cleavage-Dioxygenase (CCD1) kodieren ("eCaro-Spaltung" Plasmid). Zur Erfindung gehörig ist auch ein Plasmid, das Nukleotidsequenzen umfasst, die die Komponenten der alpha-Ionon Biosynthese Geranylgeranyl-Diphosphat-Synthase, Isopentenyl-Diphosphat-Isomerase (IPI), Phytoen-Desaturase/Dehydrogenase (crtl) Phytoensynthase (crtB), Lycopen-epsilon-Zyklase (EC) und Carotenoid-Cleavage-Dioxygenase (CCD1) kodiert ("Ionon Synthese" Plasmid). Ebenso zur Erfindung gehörig ist ein Plasmid, das Nukleotidsequenzen umfasst, die die Komponente zur Anbindung des Methylerythritolphosphatwegs (MEP-Weg) an die Lycopen, epsilon-Carotin und/oder alpha-Ionon Biosynthese, nämlich 1-Desoxy-D-xylulose-5-Phosphat-Synthase (DXS) kodieren ("MEP-Weg" Plasmid).

In einer bevorzugten Ausführungsform der Plasmide, die mit jeder der vorherigen und nachfolgenden Ausführungsformen kombinierbar ist, führt die heterologe Expression des durch das Plasmid kodierten Lycopen-Biosynthesewegs (Geranylgeranyl-Diphosphat-Synthase, Isopentenyl-Diphosphat-Isomerase (IPI), Phytoen-Desaturase/Dehydrogenase (crtl) und Phytoensynthase (crtB)) zu einer erhöhten Lycopen-Ausbeute in einem Mikroorganismus, vorzugsweise einem Bakterium. Ein bevorzugtes Bakterium ist *E. coli.* Besonders bevorzugt sind hierbei die *E. coli* Stämme, XL1-blue, TOP10, XL10 blue, DH5-alpha, JM109, C41, BL21gold (DE3) und W3110. Insbesondere kann der erfindungsgemäße Mikroorganismus der *E. coli* Stamm TOP10 sein. Besonders bevorzugt ist der E. coli Stamm BL21gold (DE3).

In einer bevorzugten Ausführungsform der Plasmide, die mit jeder der vorherigen und nachfolgenden Ausführungsformen kombinierbar ist, sind die Enzyme Geranylgeranyl-Diphosphat-Synthase, Isopentenyl-Diphosphat-Isomerase (IPI), Phytoen-Desaturase/Dehydrogenase (crtl) und Phytoensynthase (crtB) die entsprechenden Enzyme aus *Erwinia herbicola.*

In einer bevorzugten Ausführungsform der Plasmide, die mit jeder der vorherigen und nachfolgenden Ausführungsformen kombinierbar ist, stehen die durch das Plasmid kodierten Enzyme unter der Kontrolle eines induzierbaren Promotors. Insbesondere bevorzugt sind die induzierbaren Promotoren pTet, pBAD, pLac, und pXyl, die auch in Beispiel 10 und Abbildung 15 näher beschrieben sind. Besonders bevorzugt sind die induzierbaren Promotoren pTet-m1, pXyl0, pXyl1 und pXyl2 (Beispiel 10 und Abbildung 15).

In einer bevorzugten Ausführungsform der Plasmide, die mit jeder der vorherigen und nachfolgenden Ausführungsformen kombinierbar ist, stehen die durch das Plasmid kodierten Enzyme unter der Kontrolle eines konstitutiven Promotors. Insbesondere bevorzugt sind die konstitutiven Promotoren aP5, aP12, aP15, aP32 und aP47.2 (Beispiel 10 und Abbildung 15).

### "Lyc-Synthese" Plasmid

Das "Lyc-Synthese" Plasmid ist dadurch charakterisiert, dass es Nukleotidsequenzen umfasst, die die folgenden Enzyme kodieren: Geranylgeranyl-Diphosphat-Synthase, Isopentenyl-Diphosphat-Isomerase (IPI), Phytoen-Desaturase/Dehydrogenase (crtl) und Phytoensynthase (crtB), wobei die heterologe Expression des durch das Plasmid kodierten Lycopen-Biosynthesewegs zu einer erhöhten Lycopen-Ausbeute gegenüber der heterologen Expression des durch das Plasmid pAC-BETAipi-ΔcrtY (SEQ ID Nr. 28) kodierten Lycopen-Biosyntheseweg führt.

In einer weiteren Ausführungsform des Plasmids, die mit jeder der vorherigen und nachfolgenden Ausführungsformen kombinierbar ist, umfasst das Plasmid eine Sequenz, oder besteht vorzugsweise aus dieser Sequenz, die mindestens 80 % oder mindestens 85 %, 90 %, 91 %, 92 %, 93 %, 94 %, 95 %, 96 %, 97 %, 98 %, 99 % oder 100 % Sequenzidentität mit einer Referenzsequenz besitzt.

In einer weiteren Ausführungsform des Plasmids, die mit jeder der vorherigen und nachfolgenden Ausführungsformen kombinierbar ist, ist die Referenzsequenz eine Sequenz gemäß SEQ ID Nr. 28, wobei die Referenzsequenz relativ zu der Sequenz gemäß SEQ ID Nr. 28 (pAC-BETAipi-ΔcrtY) eine Deletion der Basen 984-1394 und 3432-4198 aufweist.

In einer weiteren Ausführungsform des Plasmids, die mit jeder der vorherigen und nachfolgenden Ausführungsformen kombinierbar ist, weist die Referenzsequenz relativ zu der Sequenz gemäß SEQ ID Nr. 28 (pAC-BETAipi-ΔcrtY) eine Deletion der Basen 984-1394, 3432-4198 und 6605-7242 auf.

In einer bevorzugten Ausführungsform des Plasmids, die mit jeder der vorherigen und nachfolgenden Ausführungsformen kombinierbar ist, ist die Referenzsequenz eine Sequenz gemäß SEQ ID Nr. 11 (pGT1036). Insbesondere kann die Sequenz des Plasmids auch eine Sequenz umfassen, die mit der Sequenz gemäß SEQ ID Nr. 11 identisch ist. In einer besonders bevorzugten Ausführungsform besteht das Plasmid aus einer Sequenz, die mit der Sequenz gemäß SEQ ID Nr. 11 identisch ist.

### "eCaro-Synthese" Plasmid

Das "eCaro-Synthese" Plasmid ist dadurch charakterisiert, dass es Nukleotidsequenzen umfasst, die die folgenden Enzyme kodieren: Geranylgeranyl-Diphosphat-Synthase, Isopentenyl-Diphosphat-Isomerase (IPI), Phytoen-Desaturase/Dehydrogenase (crtl) und Phytoensynthase (crtB) und Lycopen-epsilon-Zyklase (EC), wobei die heterologe Expression des durch das Plasmid kodierten Lycopen-Biosynthesewegs zu einer erhöhten Lycopen-Ausbeute gegenüber der heterologen Expression des durch das Plasmid pAC-BETAIPI-ΔcrtY (SEQ ID Nr. 28) kodierten Lycopen-Biosyntheseweg führt.

Das "eCaro-Synthese" Plasmid umfasst auch insbesondere sämtliche Ausführungsformen des "Lyc-Synthese" Plasmids und der Lycopen-epsilon-Zyklase (EC).

In einer bevorzugten Ausführungsform des erfindungsgemäßen "eCaro-Synthese" Plasmids, die mit jeder der vorherigen und nachfolgenden Ausführungsformen kombinierbar ist, ist die Referenzsequenz (charakterisiert unter dem Abschnitt "Lyc-Synthese" Plasmid) eine Sequenz gemäß SEQ ID Nr. 18 (pGT1066*, entspricht pGT1066 jedoch mit n, entsprechend a, t, c oder g, für die Nukleotide der Codons, die für Aminosäurepositionen 403, 404 und 445 des AtEC-del-Enzyms, kodieren). Insbesondere kann die Sequenz des Plasmids auch eine Sequenz umfassen, die mit der Sequenz gemäß SEQ ID Nr. 18 identisch ist. In einer besonders bevorzugten Ausführungsform besteht das Plasmid aus einer Sequenz, die mit der Sequenz gemäß SEQ ID Nr. 18 identisch ist.

In einer besonders bevorzugten Ausführungsform des "eCaro-Synthese" Plasmids, die mit jeder der vorherigen und nachfolgenden Ausführungsformen kombinierbar ist, ist die Referenzsequenz eine Sequenz gemäß SEQ ID Nr. 29 (pGT1066-AtEC-del), wobei die durch das Plasmid kodierte Lycopen-epsilon-Zyklase (EC) eine der folgenden Mutationen oder Mutationskombinationen umfasst, oder aufweist: ECmut2 (A445S), ECmut9 (L404S), ECmut3 (L404H/A445S), ECmut3.10 (A403C/A445S), ECmut3.12 (L404T/A445S), ECmut4 (A403S/L404H), ECmut5 (A403F /L404W), ECmut6 (A403G/L404G), ECmut7 (A403K/L404D), ECmut8 (A403W/L404R), ECmut10 (A403S/L404T), ECmut11 (A403F/L404S), ECmut12 (A403C/L404S), ECmut13 (A403I/L404T), ECmut14 (A403T/L404R), ECmut15 (A403F/L404R), ECmut16 (A403W/L404G), ECmut17 (A403C/A404C), ECmut18 (A403L/L404V), ECmut19 (A403K/L404R), ECmut20 (A403Y/L404K), ECmut21 (A403Q/L404K), ECmut22 (A403G/L404Q), ECmut3.1 (A403S/L404H/A445S), ECmut3.2 (A403C/L404C/A445S), ECmut3.3 (A403E/L404A/A445S), ECmut3.4 (A403W/L404R/A445S), ECmut3.5 (A403M/L404A/A445S), ECmut3.6 (A403N/L404T/A445S), ECmut3.7 (A403N/L404A/A445S), ECmut3.8 (A403H/L404S/A445S), ECmut3.9 (A403E/L404G/A445S), ECmut3.11 (A403K/L404G/A445S), ECmut3.13 (A403R/L404S/A445S), ECmut3.14 (A403G/L404R/A445S), ECmut3.15 (A403F/L404V/A445S) und ECmut3.16 (A403G/L404G/A445S). Besonders bevorzugte Mutationskombinationen sind ECmut16 (A403W/L404G), ECmut3.12 (L404T/A445S), ECmut3.3 (A403E/L404A/A445S), ECmut3.5 (A403M/L404A/A445S), ECmut3.8 (A403H/L404S/A445S), ECmut3.9 (A403E/L404G/A445S) und ECmut3.16 (A403G/L404G/A445S). Insbesonders bevorzugt sind die Mutationen oder Mutationskombinationen ECmut9 (L404S), ECmut10 (A403S/L404T) und ECmut3.2 (A403C/L404C/A445S).

In einer besonders bevorzugten Ausführungsform des "eCaro-Synthese" Plasmids, die mit jeder der vorherigen und nachfolgenden Ausführungsformen kombinierbar ist, besteht das Plasmid aus einer Sequenz gemäß SEQ ID Nr. 29 (pGT1066-AtEC-del), wobei die durch das Plasmid kodierte Lycopen-epsilon-Zyklase (EC) eine der folgenden Mutationen oder Mutationskombinationen aufweist: ECmut9 (L404S), ECmut10 (A403S/L404T), ECmut3.2 (A403C/L404C/A445S) und ECmut3.3 (A403E/L404A/A445S).

In einer besonders bevorzugten Ausführungsform des "eCaro-Synthese" Plasmids, die mit jeder der vorherigen und nachfolgenden Ausführungsformen kombinierbar ist, weist das Plasmid mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 30, 31, 32, 33, 34, 35 oder 36 auf.

### "eCaro-Spaltung" Plasmid

Das "eCaro-Spaltung" Plasmid ist dadurch charakterisiert, dass es eine Nukleotidsequenz umfasst, die das Enzym Carotenoid-Cleavage-Dioxygenase (CCD1) kodiert.

In einer bevorzugten Ausführungsform des "eCaro-Spaltung" Plasmids ist die Carotenoid-Cleavage-Dioxygenase (CCD1) eine Carotenoid-Cleavage-Dioxygenase (CCD1) aus Arabidopsis thaliana oder Osmanthus fragrans.

In einer bevorzugten Ausführungsform des "eCaro-Spaltung" Plasmids, die mit jeder der vrherigen und nachfolgenden Ausführungsformen kombinierbar ist, weist das Plasmid mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 21, 24, 37, 38, 39, 40, 41 oder 42 auf. Besonders bevorzugt sind hierbei die Sequenzen gemäß SEQ ID Nr. 37 und 41.

### "Ionon Synthese" Plasmid

Das "Ionon Synthese" Plasmid ist dadurch charakterisiert, dass es Nukleotidsequenzen umfasst, die die folgenden Enzyme kodieren: Geranylgeranyl-Diphosphat-Synthase, Isopentenyl-Diphosphat-Isomerase (IPI), Phytoen-Desaturase/Dehydrogenase (crtl) und Phytoensynthase (crtB) und Lycopen-epsilon-Zyklase (EC) und Carotenoid-Cleavage-Dioxygenase (CCD1), wobei die heterologe Expression des durch das Plasmid kodierten Lycopen-Biosynthesewegs zu einer erhöhten Lycopen-Ausbeute gegenüber der heterologen Expression des durch das Plasmid pAC-BETAIPI-ΔcrtY (SEQ ID Nr. 28) kodierten Lycopen-Biosyntheseweg führt.

Das "Ionon Synthese" Plasmid umfasst auch insbesondere sämtliche Ausführungsformen des "Lyc-Synthese" Plasmids, der erfindungsgemäßen Lycopen-epsilon-Zyklase (EC) und des "eCaro Synthese" Plasmids.

In einer bevorzugten Ausführungsform des "Ionon Synthese" Plasmids, die mit jeder der vorherigen und nachfolgenden Ausführungsformen kombinierbar ist, weist das Plasmid mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 43 oder 44 auf, wobei die Sequenz gemäß SEQ ID Nr. 44 besonders bevorzugt ist. Insbesondere bevorzugt ist ein Plasmid der eine Sequenz gemäß SEQ ID Nr. 44 aufweist.

### "MEP-Weg" Plasmid

Das "MEP-Weg" Plasmid ist dadurch charakterisiert, dass es Nukleotidsequenzen umfasst, die das folgende Enzym kodieren: 1-Desoxy-D-xylulose-5-Phosphat-Synthase (DXS).

In einer Ausführungsform des "MEP-Weg" Plasmids, die mit jeder der vorherigen und nachfolgenden Ausführungsformen kombinierbar ist, umfasst das Plasmid Nukleotidsequenzen, die die Isopentenyl-Diphosphat-Isomerase (CwIPI) aus Curcuma wenyujin kodieren. Insbesondere bevorzugt ist eine codonoptimierte synthetische Gensequenz der Isopentenyl-Diphosphat-Isomerase (CwIPI-co2). Die Isopentenyl-Diphosphat-Isomerase (CwIPI) ist per se für die Anbindung der Lycopen, epsilon-Carotin und/oder alpha-Ionon Biosynthese an den MEP-Weg nicht notwendig. In einer bevorzugten Ausführungsform des "MEP-Weg" Plasmids, die mit jeder der vorherigen und nachfolgenden Ausführungsformen kombinierbar ist, weist das "MEP-Weg" Plasmid mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 45, 46 oder 47 auf, wobei die Sequenz gemäß SEQ ID Nr. 45 besonders bevorzugt ist. Insbesondere bevorzugt ist ein Plasmid, das eine Sequenz gemäß SEQ ID Nr. 45 aufweist.

### Expressionskassetten

Ein weiterer Aspekt der Erfindung betrifft die Expressionskassetten, die der Fachmann den Abbildungen, insbesondere Abbildungen 3, 4, 7 und 9 bis 14, sowie dem Sequenzprotokoll entnehmen kann. Die Expressionskassetten können integriert im Genom des erfindungsgemäßen Mikroorganismus vorliegen. Die Expressionskassetten können mit dem Fachmann bekannten Verfahren in das Genom eines Mikroorganismus integriert werden, insbesondere mittels homologer Rekombination. In einer bevorzugten Ausführungsform können die Expressionskassetten in einem *E. coli* Stamm vorliegen, insbesondere in XL1-blue, TOP10, XL10 blue, DH5-alpha, JM109, C41, BL21gold (DE3) und W3110. Insbesondere kann der erfindungsgemäße Mikroorganismus der *E. coli* Stamm TOP10 sein. Besonders bevorzugt ist der E. coli Stamm BL21gold (DE3).

Die Expressionskassetten umfassen insbesondere die Expressionskassetten, wie sie in Abbildung 14 aufgelistet sind.

Insbesondere umfassen die Expressionskassetten, die bevorzugt im Genom eines Mikroorganismus wie *E. coli* vorliegen können, Expressionskassetten die mindestens 80 % oder mindestens 85 %, 90 %, 91 %, 92 %, 93 %, 94 %, 95 %, 96 %, 97 %, 98 %, 99 % oder 100 % Sequenzidentität mit den Expressionskassetten gemäß Abbildung 14 aufweisen.

In einer bevorzugten Ausführungsform der Expressionskassetten, die mit jeder der vorherigen und nachfolgenden Ausführungsformen kombinierbar ist, stehen die durch die Expressionskassetten kodierten Enzyme unter der Kontrolle eines konstitutiven Promotors. Insbesondere bevorzugt sind die konstitutiven Promotoren aP5, aP12, aP15, aP32 und aP47.2.

In einer bevorzugten Ausführungsform der Expressionskassetten, die mit jeder der vorherigen und nachfolgenden Ausführungsformen kombinierbar ist, stehen die durch die Expressionskassetten kodierten Enzyme unter der Kontrolle eines induzierbaren Promotors. Insbesondere bevorzugt sind die induzierbaren Promotoren pTet, pBAD, pLac, und pXyl, die auch in Beispiel 10 näher beschrieben sind. Besonders bevorzugt sind die induzierbaren Promotoren pTet-m1, pXyl0, pXyl1 und pXyl2 (Beispiel 10).

### Mikroorganismen

Der erfindungsgemäße Mikroorganismus ist dadurch charakterisiert, dass er in dem erfindungsgemäßen Verfahren zur Herstellung von enantiomerenreinem alpha-Ionon oder in dem erfindungsgemäßen Verfahren zur Herstellung von hochreinem epsilon-Carotin kultiviert wird, wobei er heterologe Nukleotidsequenzen enthält, die die folgenden Enzyme kodieren: Geranylgeranyl-Diphosphat-Synthase, Isopentenyl-Diphosphat-Isomerase (IPI), Phytoen-Desaturase/Dehydrogenase (crtl), Phytoensynthase (crtB) und Lycopen-epsilon-Zyklase (EC), wobei die Lycopen-epsilon-Zyklase (EC) eine der folgenden Mutationen oder Mutationskombinationen, bezogen auf die Referenzsequenz gemäß SEQ ID Nr:19, umfasst: ECmut9 (L404S), ECmut10 (A403S/ L404T), ECmut3.3 (A403E/L404A/A445S) und ECmut3.2 (A403C/L404C/A445S) oder Geranylgeranyl-Diphosphat-Synthase, Isopentenyl-Diphosphat-Isomerase (IPI), Phytoen-Desaturase/Dehydrogenase (crtl), Phytoensynthase (crtB), Lycopen-epsilon-Zyklase (EC) und Carotenoid-Cleavage-Dioxygenase (CCD1).

In einer bevorzugten Ausführungsform des erfindungsgemäßen Mikroorganismus, die mit jeder der vorherigen und nachfolgenden Ausführungsformen kombinierbar ist, sind die Enzyme auf einem oder mehreren Plasmiden kodiert. Insbesondere bevorzugte Ausführungsformen des erfindungsgemäßen Mikroorganismus enthalten ein oder mehrere der Plasmide. Insbesondere bevorzugt sind die "Lyc-Synthese", "eCaro-Synthese", "eCaro-Spaltung", "Ionon-Synthese" und "MEP-Weg" Plasmide.

In einer weiteren bevorzugten Ausführungsform des erfindungsgemäßen Mikroorganismus, die mit jeder der vorherigen und nachfolgenden Ausführungsformen kombinierbar ist, sind die Enzyme auf einer oder mehreren Expressionskassetten kodiert, die im Genom des Mikroorganismus integriert sind.

In einer bevorzugten Ausführungsform des erfindungsgemäßen Mikroorganismus, die mit jeder der vorherigen und nachfolgenden Ausführungsformen kombinierbar ist, sind die Enzyme Geranylgeranyl-Diphosphat-Synthase, Isopentenyl-Diphosphat-Isomerase (IPI), Phytoen-Desaturase/Dehydrogenase (crtl) und Phytoensynthase (crtB) die entsprechenden Enzyme aus *Erwinia herbicola.*

In einer bevorzugten Ausführungsform des erfindungsgemäßen Mikroorganismus, die mit jeder der vorherigen und nachfolgenden Ausführungsformen kombinierbar ist, liegen das oder die Plasmide als individuelle Strukturen in dem Mikroorganismus vor, oder sind in das Genom des Mikroorganismus integriert.

In einer bevorzugten Ausführungsform des erfindungsgemäßen Mikroorganismus, die mit jeder der vorherigen und nachfolgenden Ausführungsformen kombinierbar ist, steht die Expression der Carotenoid-Cleavage-Dioxygenase (CCD1) unter der transkriptionellen Kontrolle eines induzierbaren Promotors. In einer weiteren bevorzugten Ausführungsform, die mit jeder der vorherigen und nachfolgenden Ausführungsformen kombinierbar ist, ist der induzierbare Promotor der durch Arabinose induzierbare Promotor pBAD. Besonders bevorzugt sind weiterhin die konstitutiven und/oder induzierbaren Promotoren pXYL1, pXYL2, aP5 und aP15. In einer weiteren bevorzugten Ausführungsform des erfindungsgemäßen Mikroorganismus, die mit jeder der vorherigen und nachfolgenden Ausführungsformen kombinierbar ist, enthält der Mikroorganismus die erfindungsgemäße Nukleinsäure, die eine Lycopen-epsilon-Zyklase kodiert.

In einer weiteren bevorzugten Ausführungsform des erfindungsgemäßen Mikroorganismus, die mit jeder der vorherigen und nachfolgenden Ausführungsformen kombinierbar ist, spaltet die Carotenoid-Cleavage-Dioxygenase (CCD1) oxidativ die 9, 10- und 9', 10'-Doppelbindungen des epsilon-Carotin.

In einer besonders bevorzugten Ausführungsform des erfindungsgemäßen Mikroorganismus, die mit jeder der vorherigen und nachfolgenden Ausführungsformen kombinierbar ist, enthält der Mikroorganismus das "eCaro-Synthese" Plasmid, welches eine Sequenz umfasst, oder aus dieser besteht, die mindestens 80% oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 29 (pGT1066-AtEC-del) besitzt, wobei die durch das Plasmid kodierte Lycopen-epsilon-Zyklase (EC) eine der folgenden Mutationen oder Mutationskombinationen aufweist: ECmut16 (A403W/L404G), ECmut3.12 (L404T/A445S), ECmut3.3 (A403E/L404A/A445S), ECmut3.5 (A403M/L404A/A445S), ECmut3.8 (A403H/L404S/A445S), ECmut3.9 (A403E/L404G/A445S), ECmut3.16 (A403G/L404G/A445S), ECmut9 (L404S), ECmut10 (A403S/L404T) und ECmut3.2 (A403C/L404C/A445S). Besonders bevorzugt sind die Mutationen oder Mutationskombinationen ECmut9 (L404S), ECmut10 (A403S/L404T) und ECmut3.2 (A403C/L404C/A445S).

In einer besonders bevorzugten Ausführungsform des erfindungsgemäßen Mikroorganismus, die mit jeder der vorherigen und nachfolgenden Ausführungsformen kombinierbar ist, enthält der Mikroorganismus das "eCaro-Synthese" Plasmid, welches aus einer Sequenz gemäß SEQ ID Nr. 29 besteht, wobei die durch das Plasmid kodierte Lycopen-epsilon-Zyklase (EC) eine der folgenden Mutationen oder Mutationskombinationen aufweist: ECmut9 (L404S), ECmut10 (A403S/L404T) und ECmut3.2 (A403C/L404C/A445S).

In einer bevorzugten Ausführungsform des erfindungsgemäßen Mikroorganismus, die mit jeder der vorherigen und nachfolgenden Ausführungsformen kombinierbar ist, ist der Mikroorganismus ein *E. coli* Stamm. Besonders bevorzugt sind hierbei die *E. coli* Stämme, XL1-blue, TOP10, XL10 blue, DH5-alpha, JM109, C41, BL21gold (DE3) und W3110. Insbesondere kann der erfindungsgemäße Mikroorganismus der *E. coli* Stamm TOP10 sein. Besonders bevorzugt ist der E. coli Stamm BL21gold (DE3).

In einer weiteren bevorzugten Ausführungsform des erfindungsgemäßen Mikroorganismus, die mit jeder der vorherigen und nachfolgenden Ausführungsformen kombinierbar ist, enthält der Mikroorganismus das "eCaro-Synthese" Plasmid und das "eCaro-Spaltung" Plasmid, welches mindestens 80% oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 21 (pGT1069) oder gemäß SEQ ID Nr. 24 (pGT1070) hat. Besonders bevorzugte Ausführungsformen des Mikroorganismus enthalten das "eCaro-Synthese" Plasmid und das "eCaro-Sapltung" Plasmid mit einer Sequenz gemäß SEQ ID Nr. 21 (pGT1069) oder gemäß SEQ ID Nr. 24 (pGT1070), wobei das "eCaro-Synthese" Plasmid bevorzugterweise aus einer Sequenz gemäß SEQ ID Nr. 29 (pGT1066-AtEC-del) besteht, wobei die durch das Plasmid kodierte Lycopen-epsilon-Zyklase (EC) eine der folgenden Mutationen oder Mutationskombinationen aufweist: ECmut9 (L404S), ECmut10 (A403S/L404T) und ECmut3.2 (A403C/L404C/A445S).

### Verfahren zur Herstellung von hochreinem epsilon-Carotin

Das erfindungsgemäße Verfahren zur Herstellung von hochreinem epsilon-Carotin mit einem Gesamtcarotinoidgehalt von 97,7 bis 100% aus Lycopen umfasst das Kultivieren eines Mikroorganismus, der heterologe Nukleotidsequenzen enthält, die die folgenden Enzyme kodieren: Geranylgeranyl-Diphosphat-Synthase, Isopentenyl-Diphosphat-Isomerase (IPI), Phytoen-Desaturase/Dehydrogenase (crtl), Phytoensynthase (crtB) und Lycopen-epsilon-Zyklase (EC), wobei die Lycopen-epsilon-Zyklase (EC) eine der folgenden Mutationen oder Mutationskombinationen, bezogen auf die Referenzsequenz gemäß SEQ ID Nr:19, umfasst: ECmut9 (L404S), ECmut10 (A403S/L404T), ECmut3.3 (A403E/L404A/A445S) und ECmut3.2 (A403C/L404C/A445S).

In einer besonders bevorzugten Ausführungsform des erfindungsgemäßen Verfahrens zur Herstellung von hochreinem epsilon-Carotin aus Lycopen, die mit jeder der vorherigen und nachfolgenden Ausführungsformen kombinierbar ist, wird der erfindungsgemäße Mikroorganismus kultiviert.

In einer bevorzugten Ausführungsform des erfindungsgemäßen Verfahrens zur Herstellung von hochreinem epsilon-Carotin aus Lycopen, die mit jeder der vorherigen und nachfolgenden Ausführungsformen kombinierbar ist, ist die Geranylgeranyl-Diphosphat-Synthase die Geranylgeranyl-Diphosphat-Synthase crtE oder die Geranylgeranyl-Diphosphat-Synthase idsA.

In einer bevorzugten Ausführungsform des erfindungsgemäßen Verfahrens zur Herstellung von hochreinem epsilon-Carotin aus Lycopen, die mit jeder der vorherigen und nachfolgenden Ausführungsformen kombinierbar ist, ist die Lycopen-epsilon-Zyklase (EC) die Lycopen-epsilon-Zyklase (EC), die mindestens 80% oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 19 besitzt und mindestens an einer der Positionen 403,404 und 445 von der Sequenz gemäß SEQ ID Nr. 19 eine der folgenden Mutationen umfasst: ECmut9 (L404S), ECmut10 (A403S/L404T), ECmut3.3 (A403E/L404A/A445S) und ECmut3.2 (A403C/L404C/A445S).

In einer bevorzugten Ausführungsform des erfindungsgemäßen Verfahrens zur Herstellung von hochreinem epsilon-Carotin aus Lycopen, die mit jeder der vorherigen und nachfolgenden Ausführungsformen kombinierbar ist, sind die Enzyme auf einem oder mehreren Plasmiden kodiert. Diese Plasmide können als individuelle Strukturen in dem Mikroorganismus vorliegen oder in das Genom des Mikroorganismus integriert sein. Diese Enzyme können koexprimiert werden.

In einer bevorzugten Ausführungsform des erfindungsgemäßen Verfahrens zur Herstellung von hochreinem epsilon-Carotin aus Lycopen, die mit jeder der vorherigen und nachfolgenden Ausführungsformen kombinierbar ist, enthält der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 30, 31, 32, 33, 34, 35 oder 36.

In einer bevorzugten Ausführungsform des erfindungsgemäßen Verfahrens zur Herstellung von hochreinem epsilon-Carotin aus Lycopen, die mit jeder der vorherigen und nachfolgenden Ausführungsformen kombinierbar ist, enthält der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 45, 46 oder 47.

In dem erfindungsgemäßen Verfahren zur Herstellung von hochreinem epsilon-Carotin aus Lycopen wird der erfindungsgemäße Mikroorganismus kultiviert, welcher das "eCaro-Synthese" Plasmid enthält, welches eine Sequenz umfasst, oder aus dieser besteht, die mindestens 80% oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 29 (pGT1066-AtEC-del) besitzt, wobei die durch das Plasmid kodierte Lycopen-epsilon-Zyklase (EC) eine der folgenden Mutationen oder Mutationskombinationen umfasst, oder aufweist: ECmut9 (L404S), ECmut10 (A403S/L404T) und ECmut3.2 (A403C/L404C/A445S).

In einer besonders bevorzugten Ausführungsform des erfindungsgemäßen Verfahrens zur Herstellung von hochreinem epsilon-Carotin aus Lycopen, die mit jeder der vorherigen und nachfolgenden Ausführungsformen kombinierbar ist, wird der erfindungsgemäße Mikroorganismus kultiviert, der das "eCaro-Synthese" Plasmid enthält, welches aus einer Sequenz gemäß SEQ ID Nr. 29 (pGT1066-AtEC-del) besteht, wobei die durch das Plasmid kodierte Lycopen-epsilon-Zyklase (EC) eine der folgenden Mutationen oder Mutationskombinationen aufweist: ECmut9 (L404S), ECmut10 (A403S/L404T), ECmut3.2 (A403C/L404C/A445S) und ECmut3.3 (A403E/L404A/A445S).

In einer bevorzugten Ausführungsform des erfindungsgemäßen Verfahrens zur Herstellung von hochreinem epsilon-Carotin aus Lycopen, die mit jeder der vorherigen und nachfolgenden Ausführungsformen kombinierbar ist, ist der erfindungsgemäße Mikroorganismus *E. coli.* Besonders bevorzugt sind hierbei die *E. coli* Stämme, XL1-blue, TOP10, XL10 blue, DH5-alpha, JM109, C41, BL21gold (DE3) und W3110. Insbesondere kann der erfindungsgemäße Mikroorganismus der *E. coli* Stamm TOP10 sein. Besonders bevorzugt ist der E.coli Stamm BL21gold (DE3).

In einer bevorzugten Ausführungsform des Verfahrens zur Herstellung von hochreinem epsilon-Carotin aus Lycopen, die mit jeder der vorherigen und nachfolgenden Ausführungsformen kombinierbar ist, enthält der Mikroorganismus heterologe Nukleotidsequenzen, die das Enzym 1-Desoxy-D-xylulose-5-Phosphat-Synthase (DXS) kodieren.

In einer bevorzugten Ausführungsform des Verfahrens zur Herstellung von hochreinem epsilon-Carotin aus Lycopen, die mit jeder der vorherigen und nachfolgenden Ausführungsformen kombinierbar ist, enthält der Mikroorganismus heterologe Nukleotidsequenzen, die das Enzym Isopentenyl-Diphosphat-Isomerase (CwIPI) kodieren.

In einer besonders bevorzugten Ausführungsform des Verfahrens zur Herstellung von hochreinem epsilon-Carotin aus Lycopen, die mit jeder der vorherigen und nachfolgenden Ausführungsformen kombinierbar ist, enthält der Mikroorganismus ein Plasmid das mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 45, 46 oder 47 aufweist, wobei die Sequenz gemäß SEQ ID Nr. 45 besonders bevorzugt ist. Insbesondere bevorzugt ist ein Plasmid, das eine Sequenz gemäß SEQ ID Nr. 45 aufweist.

### Verfahren zur Herstellung von enantiomerenreinem alpha-Ionon

Das erfindungsgemäße Verfahren zur Herstellung von enantiomerenreinem alpha-Ionon umfasst das Kultivieren eines Mikroorganismus, der heterologe Nukleotidsequenzen enthält, die die folgenden Enzyme kodieren: Geranylgeranyl-Diphosphat-Synthase, Isopentenyl-Diphosphat-Isomerase (IPI), Phytoen-Desaturase/Dehydrogenase (crtl), Phytoensynthase (crtB), Lycopen-epsilon-Zyklase (EC) und Carotenoid-Cleavage-Dioxygenase (CCD1).

In einer bevorzugten Ausführungsform des erfindungsgemäßen Verfahrens zur Herstellung von enantiomerenreinem alpha-Ionon, die mit jeder der vorherigen und nachfolgenden Ausführungsformen kombinierbar ist, wird der erfindungsgemäße Mikroorganismus kultiviert.

In einer bevorzugten Ausführungsform des erfindungsgemäßen Verfahrens zur Herstellung von enantiomerenreinem alpha-Ionon, die mit jeder der vorherigen und nachfolgenden Ausführungsformen kombinierbar ist, wird (R)-alpha-Ionon hergestellt.

In einer bevorzugten Ausführungsform des erfindungsgemäßen Verfahrens zur Herstellung von enantiomerenreinem alpha-Ionon, die mit jeder der vorherigen und nachfolgenden Ausführungsformen kombinierbar ist, ist die Geranylgeranyl-Diphosphat-Synthase die Geranylgeranyl-Diphosphat-Synthase crtE oder die Geranylgeranyl-Diphosphat-Synthase idsA.

In einer bevorzugten Ausführungsform des erfindungsgemäßen Verfahrens zur Herstellung von enantiomerenreinem alpha-Ionon, die mit jeder der vorherigen und nachfolgenden Ausführungsformen kombinierbar ist, ist die Lycopen-epsilon-Zyklase (EC) die erfindungsgemäße Lycopen-epsilon-Zyklase (EC). Besonders bevorzugt sind dabei Ausführungsformen in denen die Lycopen-epsilon-Zyklase (EC) mindestens 80% oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 19 besitzt und mindestens an einer der Positionen 403,404 und 445 von der Sequenz gemäß SEQ ID Nr. 19 abweicht. Insbesondere bevorzugt sind Ausführungsformen in denen die erfindungsgemäße Lycopen-epsilon-Zyklase (EC) eine der folgenden Mutationen oder Mutationskombinationen umfasst: ECmut9 (L404S), ECmut10 (A403S/L404T), ECmut3.3 (A403E/L404A/A445S) und ECmut3.2 (A403C/L404C/A445S).

In einer bevorzugten Ausführungsform des erfindungsgemäßen Verfahrens zur Herstellung von enantiomerenreinem alpha-Ionon, die mit jeder der vorherigen und nachfolgenden Ausführungsformen kombinierbar ist, ist die Carotenoid-Cleavage-Dioxygenase (CCD1) eine Carotenoid-Cleavage-Dioxygenase (CCD1) aus Arabidopsis thaliana oder Osmanthus fragrans. In einer bevorzugten Ausführungsform des erfindungsgemäßen Verfahrens zur Herstellung von enantiomerenreinem alpha-Ionon, die mit jeder der vorherigen und nachfolgenden Ausführungsformen kombinierbar ist, sind die Enzyme auf einem oder mehreren Plasmid kodiert. Diese Plasmide können als individuelle Strukturen in dem Mikroorganismus vorliegen oder in das Genom des Mikroorganismus integriert sein. Diese Enzyme können koexprimiert werden.

In dem erfindungsgemäßen Verfahren zur Herstellung von enantiomerenreinem alpha-Ionon, vorzugsweise (R)-alpha-Ionon, das mit jeder der vorherigen und nachfolgenden Ausführungsformen kombinierbar ist, wird der erfindungsgemäße Mikroorganismus kultiviert, welcher das "eCaro-Synthese" Plasmid und das "eCaro-Spaltung" Plasmid enthält, wobei das erfindungsgemäße "eCaro-Synthese" Plasmid eine Sequenz umfasst, oder aus dieser besteht, die mindestens 80% oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 29 (pGT1066-AtEC-del) besitzt, wobei die durch das Plasmid kodierte Lycopen-epsilon-Zyklase (EC) eine der folgenden Mutationen oder Mutationskombinationen umfasst, oder aufweist: ECmut2 (A445S), ECmut9 (L404S), ECmut3 (L404H/A445S), ECmut3.10 (A403C/A445S), ECmut3.12 (L404T/A445S), ECmut4 (A403S/L404H), ECmut5 (A403F /L404W), ECmut6 (A403G/L404G), ECmut7 (A403K/L404D), ECmut8 (A403W/L404R), ECmut10 (A403S/L404T), ECmut11 (A403F/L404S), ECmut12 (A403C/L404S), ECmut13 (A403I/L404T), ECmut14 (A403T/L404R), ECmut15 (A403F/L404R), ECmut16 (A403W/L404G), ECmut17 (A403C/A404C), ECmut18 (A403L/L404V), ECmut19 (A403K/L404R), ECmut20 (A403Y/L404K), ECmut21 (A403Q/L404K), ECmut22 (A403G/L404Q), ECmut3.1 (A403S/L404H/A445S), ECmut3.2 (A403C/L404C/A445S), ECmut3.3 (A403E/L404A/A445S), ECmut3.4 (A403W/L404R/A445S), ECmut3.5 (A403M/L404A/A445S), ECmut3.6 (A403N/L404T/A445S), ECmut3.7 (A403N/L404A/A445S), ECmut3.8 (A403H/L404S/A445S), ECmut3.9 (A403E/L404G/A445S), ECmut3.11 (A403K/L404G/A445S), ECmut3.13 (A403R/L404S/A445S), ECmut3.14 (A403G/L404R/A445S), ECmut3.15 (A403F/L404V/A445S) und ECmut3.16 (A403G/L404G/A445S). Besonders bevorzugt sind die Mutationen oder Mutationskombinationen ECmut9 (L404S), ECmut10 (A403S/L404T) und ECmut3.2 (A403C/L404C/A445S). Das "eCaro-Spaltung" Plasmid ist bevorzugt ein Plasmid, welches mindestens 80% oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 21 (pGT1069) oder gemäß SEQ ID Nr. 24 (pGT1070) hat. Besonders bevorzugt ist ein weiteres Plasmid, welches eine Sequenz aufweist, die identisch ist mit einer Sequenz gemäß SEQ ID Nr. 21 (pGT1069) oder gemäß SEQ ID Nr. 24 (pGT1070).

In einer besonders bevorzugten Ausführungsform des erfindungsgemäßen Verfahrens zur Herstellung von enantiomerenreinem alpha-Ionon, vorzugsweise (R)-alpha-Ionon, die mit jeder der vorherigen und nachfolgenden Ausführungsformen kombinierbar ist, wird der erfindungsgemäße Mikroorganismus kultiviert, der das "eCaro-Synthese" Plasmid und das "eCaro-Spaltung" Plasmid enthält, wobei das "eCaro-Synthese" Plasmid aus einer Sequenz gemäß SEQ ID Nr. 29 (pGT1066-AtEC-del) besteht und die durch das erfindungsgemäße Plasmid kodierte Lycopen-epsilon-Zyklase (EC) eine der folgenden Mutationen oder Mutationskombinationen aufweist: ECmut9 (L404S), ECmut10 (A403S/L404T), ECmut3.2 (A403C/L404C/A445S) und ECmut3.3 (A403E/L404A/A445S).

In einer besonders bevorzugten Ausführungsform des erfindungsgemäßen Verfahrens zur Herstellung von enantiomerenreinem alpha-Ionon, vorzugsweise (R)-alpha-Ionon, die mit jeder der vorherigen und nachfolgenden Ausführungsformen kombinierbar ist, besteht das "eCaro-Spaltung" Plasmid aus einer Sequenz gemäß SEQ ID Nr. 21 (pGT1069) oder gemäß SEQ ID Nr. 24 (pGT1070).

In einer besonders bevorzugten Ausführungsform des erfindungsgemäßen Verfahrens zur Herstellung von enantiomerenreinem alpha-Ionon, vorzugsweise (R)-alpha-Ionon, die mit jeder der vorherigen und nachfolgenden Ausführungsformen kombinierbar ist, wird der erfindungsgemäße Mikroorganismus kultiviert, der das "eCaro-Synthese" Plasmid und das "eCaro-Spaltung" Plasmid enthält, wobei das "eCaro-Spaltung" Plasmid aus einer Sequenz gemäß SEQ ID Nr. 21 (pGT1069) oder gemäß SEQ ID Nr. 24 (pGT1070) besteht und wobei das "eCaro-Synthese" Plasmid aus einer Sequenz gemäß SEQ ID Nr. 29 (pGT1066-AtEC-del) besteht, wobei die durch das Plasmid kodierte Lycopen-epsilon-Zyklase (EC) eine der folgenden Mutationen oder Mutationskombinationen aufweist: ECmut9 (L404S), ECmut10 (A403S/L404T), ECmut3.2 (A403C/L404C/A445S) und ECmut3.3 (A403E/L404A/A445S).

In einer bevorzugten Ausführungsform des erfindungsgemäßen Verfahrens zur Herstellung von enantiomerenreinem alpha-Ionon, die mit jeder der vorherigen und nachfolgenden Ausführungsformen kombinierbar ist, enthält der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 30, 31, 32, 33, 34, 35 oder 36.

In einer bevorzugten Ausführungsform des erfindungsgemäßen Verfahrens zur Herstellung von enantiomerenreinem alpha-Ionon, die mit jeder der vorherigen und nachfolgenden Ausführungsformen kombinierbar ist, enthält der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 21, 24, 37, 38, 39, 40, 41 oder 42.

In einer bevorzugten Ausführungsform des erfindungsgemäßen Verfahrens zur Herstellung von enantiomerenreinem alpha-Ionon, die mit jeder der vorherigen und nachfolgenden Ausführungsformen kombinierbar ist, enthält der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 43 oder 44.

In einer bevorzugten Ausführungsform des erfindungsgemäßen Verfahrens zur Herstellung von enantiomerenreinem alpha-Ionon, die mit jeder der vorherigen und nachfolgenden Ausführungsformen kombinierbar ist, enthält der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 45, 46 oder 47.

In einer besonders bevorzugten Ausführungsform des erfindungsgemäßen Verfahrens zur Herstellung von enantiomerenreinem alpha-Ionon, die mit jeder der vorherigen und nachfolgenden Ausführungsformen kombinierbar ist, enthält der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 33 und ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 37. In einer ebenso bevorzugten Ausführungsform enthält der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 33 und ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 41. In einer weiteren besonders bevorzugten Ausführungsform enthält der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 44 und ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 45.

In einer besonders bevorzugten Ausführungsform des erfindungsgemäßen Verfahrens zur Herstellung von enantiomerenreinem alpha-Ionon, die mit jeder der vorherigen und nachfolgenden Ausführungsformen kombinierbar ist, enthält der Mikroorganismus ein Plasmid mit einer Sequenz gemäß SEQ ID Nr. 33 und ein Plasmid mit einer Sequenz gemäß SEQ ID Nr. 37, oder ein Plasmid mit einer Sequenz gemäß SEQ ID Nr. 33 und ein Plasmid mit einer Sequenz gemäß SEQ ID Nr. 41, oder ein Plasmid mit einer Sequenz gemäß SEQ ID Nr. 44 und ein Plasmid mit einer Sequenz gemäß SEQ ID Nr. 45.

In einer besonders bevorzugten Ausführungsform des erfindungsgemäßen Verfahrens zur Herstellung von enantiomerenreinem alpha-Ionon, die mit jeder der vorherigen und nachfolgenden Ausführungsformen kombinierbar ist, ist der Mikroorganismus ein *E. coli* Stamm. Besonders bevorzugt sind hierbei die *E. coli* Stämme, XL1-blue, TOP10, XL10 blue, DH5-alpha, JM109, C41, BL21gold (DE3) und W3110. Insbesondere kann der erfindungsgemäße Mikroorganismus der *E. coli* Stamm TOP10 sein. Insbesondere bevorzugt ist der E. coli Stamm BL21gold (DE3).

In einer bevorzugten Ausführungsform des Verfahrens zur Herstellung von enantiomerenreinem alpha-Ionon, die mit jeder der vorherigen und nachfolgenden Ausführungsformen kombinierbar ist, enthält der Mikroorganismus heterologe Nukleotidsequenzen, die das Enzym 1-Desoxy-D-xylulose-5-Phosphat-Synthase (DXS) kodieren.

In einer bevorzugten Ausführungsform des Verfahrens zur Herstellung von enantiomerenreinem alpha-Ionon, die mit jeder der vorherigen und nachfolgenden Ausführungsformen kombinierbar ist, enthält der Mikroorganismus heterologe Nukleotidsequenzen, die das Enzym Isopentenyl-Diphosphat-Isomerase (CwIPI) kodieren.

In einer besonders bevorzugten Ausführungsform des Verfahrens zur Herstellung von enantiomerenreinem alpha-Ionon, die mit jeder der vorherigen und nachfolgenden Ausführungsformen kombinierbar ist, enthält der Mikroorganismus ein Plasmid das mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 45, 46 oder 47 aufweist, wobei die Sequenz gemäß SEQ ID Nr. 45 besonders bevorzugt ist. Insbesondere bevorzugt ist ein Plasmid, das eine Sequenz gemäß SEQ ID Nr. 45 aufweist.

### Beispiele

### Beispiel 1: Optimierung eines Expressionsplasmids

Ausgangspunkt für die Optimierung des Expressionsvektors war das Plasmid pAC-BETAipi (Cunningham et al., 2007), welches Carotinoidgene aus *E. herbicola* trägt (crtE, IPI, crtB und crtl). Unter anderem wurden die folgenden Modifikationen an dem Plasmid pAC-BETAipi vorgenommen, um das Plasmid pGT1036 (SEQ ID Nr. 11) mittels dem Fachmann bekannten molekularbiologischen Standardverfahren (Sambrook J, Fritsch EF, Maniatis T. in: Molecular Cloning, A Laboratory Manual, 1989 (Nolan C, Ed.), Cold Spring Harbor Laboratory Press, Cold Spring Harbor, NY) herzustellen: Deletion 984-1394, Deletion 3432-5356 und Deletion 7761-8399. Eine Plasmidkarte des resultierenden Plasmids pGT1036 ist in Abbildung 3A dargestellt und Abbildung 3B führt die komplette Nukleinsäuresequenz von pGT1036 auf.

Die Analyse der Lycopen-Ausbeute wurde analog der in Beispiel 6 beschriebenen Analyse der epsilon-Carotin-Ausbeute durchgeführt. In Kürze: Die HPLC-Analytik der bakteriellen Carotinoid-Extrakte erfolgte mittels HP Series II 1090 Liquid Chromatograph (Agilent Technologies, Böblingen) mit ternärem Pumpensystem und Dioden- Array-Detektor. Zur Auftrennung wurde eine Zorbax SB-C18- Trennsäule (3,5 µm, 4,6x150 mm, Agilent Technologies, Böblingen) bei einer Säulentemperatur von 40 °C. Die Auftrennung der Carotinoide erfolgte zunächst über 2 min. isokratisch mit 20 % Ethylacetat (EtAc) in Acetonitril (AcN), danach über einen Gradienten von 20 % EtAc in AcN bis 50 % EtAc in AcN für 10 min. und anschließend für 3 min. isokratisch bei 50 % EtAc in AcN mit einer Flussrate von 1 ml min-1. Die Auswertung wurde mittels HP ChemStation for LC Version A.05.02 durchgeführt und erfolgte für Lycopen bei einer Wellenlange von 450 nm. Die HPLC-Bedingungen waren wie folgt: Säule - Zorbax C18 3,5 µm 150-4.6 (Agilent), Säulentemperatur - 40 °C, Laufmittel A-Acetonitril, Laufmittel B - Ethylacetat, Flußrate - 1 ml/min, und Gradient - 2 min isokratisch bei 20 %B, in 10 min auf 50 % B, 3 min isokratisch bei 50 % B.

Die Auswertung/Detektion erfolgte über Absorptionsmessung. Lycopen wurde bei einer Wellenlänge von 450 nm detektiert.

Zur Bestimmung der Lycopenmenge wird die Fläche der entsprechenden Peaks in den Chromatogrammen berechnet. Sie ist direkt proportional zur Substanzmenge. Zur Erstellung einer Eichkurve vermisst man steigende Mengen reiner Referenzsubstanzen in dieser Weise. Mit Hilfe dieser Eichkurve kann dann auch die Peakfläche auf die vorliegende Substanzmenge (in g) umgerechnet werden.

Die oben beschriebenen Veränderungen des Plasmids pAC-BETAipi führten zu einer signifikanten Steigerung der Lycopen-Ausbeute. Gegenüber dem Referenzplasmid pAC-BETAipi-ΔcrtY wies das Plasmid pGT1036 eine 4,2-fach erhöhte Lycopen-Ausbeute auf.

### Beispiel 2: Klonierung einer artifiziellen Terminatorsequenz aTerm5

Ausgehend von dem Expressionsplasmid pGJ2720 (Jach et al. 2006) wurde über PCR eine kurze DNA-Sequenz am 3'-Ende des Reportergens RFP (Red-Fluorescent-Protein) eingefügt, die aus einer Zufallssequenz von 18bp besteht, die wiederum von einer sich umgekehrt wiederholenden (inverted repeat) 10bp-Sequenz flankiert wird. Folgende Primer wurden für die PCR-Reaktion eingesetzt (N=Zufallsnukleotid):
SEQ ID Nr. 1: NNNNNNNNAACGGGATTTTTTGCTGAAAGGAGGAACTATATCC
SEQ ID Nr. 2: NNNNNNNNNNAACGGGCTTTGTTAGCAGCCGG

Die PCR-Reaktion (50µl Endvolumen) enthielt folgende, in bidest. Wasser gelösten Komponenten: 5ng pGJ2720-Plasmid (template), je 20 pmol der Primer P2750 und P2751, je 10 nmol der Nukleotide dATP, dCTP, dGTP, dTTP und 5µl Q5-Puffer(10x). Folgendes Programm wurde verwendet: 2 min 98°C, dann 30 Zyklen mit jeweils 30 sec 98°C, 30 sec 65°C und 90 sec 72°C, und abschließend 5min 72°C.

Nach Zugabe von 10 Units des Restriktionsenzyms Dpnl wurde der PCR-Ansatz dann für 1h bei 37°C inkubiert. Anschließend wurde das erhaltene PCR-Produkt, entsprechend der Herstellerangaben, über eine Säule gereinigt (PCR-Purification-Kit; Maschery und Nagel). Zur Phosphorylierung des 5'-Ende des PCR-Produkts wurde das Eluat (50µl) dann mit 2µl 10mM ATP und 1µl Polynukleotid-Kinase versetzt und für 15min bei 37°C und dann für 20min bei 65°C inkubiert. 5µl dieses Präparats wurden dann in einem Standard-Ligationsansatz (Sambrook et al.; Endvolumen 20µl) eingesetzt. Die Ligationsprodukte wurden dann über Standard-Transformationsmethoden in *E. coli*-Zellen eingebracht. Die Identifikation funktionaler Terminatorsequenzen erfolgt im Anschluss über die Analyse der Reportergenexpression der erhaltenen Klone. Eine Kollektion funktionaler Klone wurde zusammengestellt, die jeweilige Plasmid-DNA isoliert und die Sequenz der jeweils vorliegenden Terminatorsequenz über DNA-Sequenzierung ermittelt.

### Beispiel 3: Klonierung der A. thaliana Lycopen-epsilon-Zyklase (EC)

Es wurde eine *in-silico* Analyse der von dem *Arabidopsis thaliana* Gen At5g57030 kodierten Lycopen-epsilon-Zyklase (EC) durchgeführt, die zeigte, dass die ersten 44 Aminosäuren (exklusive des N-terminalen Methionins) der Proteinsequenz ein Chloroplasten-Lokalisationssignal (Transitpeptid) darstellen. Mittels PCR wurde die ermittelte kodierende Region für das reife Protein (AtEC-del, SEQ ID Nr. 19) aus *A. thaliana* cDNA amplifiziert, da die genomische Gensequenz mehrere Introns enthält und daher für die mikrobielle Expression des Enzyms ungeeignet ist. Anschließend wurde in das Expressionsplasmid pGJ2720 subkloniert und die erhaltene DNA-Sequenz verifiziert.

### Beispiel 4: Lycopen-epsilon-Zyklase (EC) Mutanten

Es wurde mittels molekularbiologischen Standardverfahren eine Lycopen-epsilon-Zyklase (EC) Expressionskassette generiert. Die generierte EC-Expressionskassette bestehend aus Lac-Promotor (pLac), der Sequenz, die für AtEC-del (SEQ ID Nr. 19) kodiert, und dem Terminator aTerm5 (siehe Beispiel2), wurde über eine PCR-Reaktion amplifiziert und in das erzeugte Plasmid pGT1036 (Abbildung 3A, SEQ ID Nr. 11) eingefügt. Abbildung 4 zeigt beispielhaft die Plasmidkarte und die Nukleotidsequenz für ein Expressionsplasmid enthaltend das Gen für ECmut3 (pGT1066, SEQ ID Nr. 17). Mit Hilfe der folgenden Oligonukleotidprimer wurden dann PCR-basiert gezielt Mutationen (L404H, A445S, L404H/A445S, A403S/L404H) oder Zufallsmutationen an den Positionen 403/404 und/oder 445 der AtEC-del-Aminosäuresequenz eingeführt (siehe Abbildung 5):
SEQ ID Nr. 3: GTCTTGCACACATAGTTCAATTCG
SEQ ID Nr. 4: CTATGTGTGCAAGACCAAAGAGAAAGAATGCTCTCTG
SEQ ID Nr. 5: CTCTTTTCTTTATACATGTTCGTCATTTCACC
SEQ ID Nr. 6: GTATAAAGAAAAGAGAACGAGATCTCCTG
SEQ ID Nr. 7: GTCTTTCACACATAGTTCAATTCGATACCG
SEQ ID Nr. 8: CTATGTGTGAAAGACCAAAGAGAAAGAATGCTC
SEQ ID Nr. 9: GCATTCTTTCTCTTTGGTCTTNNKNNKATAGTTCAATTCGATACCGAAGGC
SEQ ID Nr. 10: CCAAAGAGAAAGAATGCTCTCTG

Die PCR-Reaktionen (50µl Endvolumen) enthielten folgende, in bidest. Wasser gelösten Komponenten: 5ng pGJ2720-Plasmid (template), je 20 pmol einer der Primerkombinationen (SEQ ID Nr. 3/ SEQ ID Nr. 4, SEQ ID Nr. 5/ SEQ ID Nr. 6, SEQ ID Nr. 7/ SEQ ID Nr. 8 oder SEQ ID Nr. 9/ SEQ ID Nr. 10), je 10 nmol der Nukleotide dATP, dCTP, dGTP, dTTP und 5µl Q5-Puffer(10x). Folgendes Programm wurde verwendet: 2 min 98°C, dann 30 Zyklen mit jeweils 30 sec 98°C, 30 sec 60°C und 4min 72°C, und abschließend 5min 72°C. Nach Zugabe von 10 Units des Restriktionsenzyms Dpnl wurde der PCR-Ansatz dann für 1h bei 37°C inkubiert. Anschließend wurde das erhaltene PCR-Produkt entsprechend der Herstellerangaben über eine Säule gereinigt (PCR-Purification-Kit; Maschery und Nagel). Für die PCR-Produkte wurden LIC-Reaktionen (ligation-independent-cloning) durchgeführt und die Reaktionsprodukte über Standardmethoden in E. coli XL1-blue Zellen transformiert.

Das Screening der AtEC-del-Zufallsmutanten erfolgte durch ausplattieren der Transformanten aus Festmedium (LB + Chloramphenicol), Inkubation für 24 Std. bei 28°C und anschließender Auswahl von Kolonien mit der intensivsten Gelbfärbung aufgrund des epsilon-Carotinoidgehalts. Zur Bestimmung der erhaltenen Mutationen wurde die Plasmid-DNA der ausgewählten Klone isoliert und mittels DNA-Sequenzierung analysiert.

Die folgenden Mutanten wurden aufgrund ihrer intensiven Gelbfärbung ausgewählt:

### Einfachmutanten:

ECmut2 (A445S), ECmut9 (L404S)

### Doppelmutanten:

ECmut3 (L404H/A445S), ECmut3.10 (A403C/A445S), ECmut3.12 (L404T/A445S),
ECmut4 (A403S/L404H), ECmut5 (A403F /L404W), ECmut6 (A403G/L404G),
ECmut7 (A403K/L404D), ECmut8 (A403W/L404R), ECmut10 (A403S/L404T),
ECmut11 (A403F/L404S), ECmut12 (A403C/L404S), ECmut13 (A4031/L404T),
ECmut14 (A403T/L404R), ECmut15 (A403F/L404R), ECmut16 (A403W/L404G),
ECmut17 (A403C/A404C), ECmut18 (A403L/L404V), ECmut19 (A403K/L404R),
ECmut20 (A403Y/L404K), ECmut21 (A403Q/L404K), ECmut22 (A403G/L404Q),

### Dreifachmutanten:

ECmut3.1 (A403S/L404H/A445S), ECmut3.2 (A403C/L404C/A445S),
ECmut3.3 (A403E/L404A/A445S), ECmut3.4 (A403W/L404R/A445S),
ECmut3.5 (A403M/L404A/A445S), ECmut3.6 (A403N/L404T/A445S),
ECmut3.7 (A403N/L404A/A445S), ECmut3.8 (A403H/L404S/A445S),
ECmut3.9 (A403E/L404G/A445S), ECmut3.11 (A403K/L404G/A445S),
ECmut3.13 (A403R/L404S/A445S), ECmut3.14 (A403G/L404R/A445S),
ECmut3.15 (A403F/L404V/A445S), ECmut3.16 (A403G/L404G/A445S)

### Beispiel 5: Transformation von Wirtszellen

Alle Expressionsplasmide wurden mittels Transformation in *E*. *coli* TOP10 Zellen eingebracht. Die Transformation der Wirtszellen erfolgte nach Standardmethoden (Sambrook J, Fritsch EF, Maniatis T. in: Molecular Cloning, A Laboratory Manual, 1989 (Nolan C, Ed.), Cold Spring Harbor Laboratory Press, Cold Spring Harbor, NY).

### Beispiel 6: Nachweis von epsilon-Carotin

Die rekombinanten Stämme wurden bezüglich der synthetisierten Carotinoide per HPLC analysiert.

Die rekombinanten Stämme wurden erzeugt durch Transformation eines *E.coli* Stammes mit den verschiedenen Expressionsplasmiden, die außer crtE, IPI, crtl und crtB die Nukleinsäure für eine der verschiedenen Lycopen-Epsilon-Zyclase-Mutanten (ECmut) enthalten.

Die Kultivierung der rekombinanten Stämme erfolgte für 24h bei 28°C in dYT-Medium (+Chloramphenicol und Ampicillin). Die Zellen wurden dann über Zentrifugation (10min, 4000g) pelletiert, der Medienüberstand entfernt und die gebildeten Carotinoide aus dem Zellpellet mit Aceton quantitativ extrahiert. Die Extrakte wurden bis Trocknung unter Vakuum eingedampft und die resultierenden Carotinoidpellets in gleichen Volumina Acetonitril (1ml) gelöst und unmittelbar für die HPLC-Analyse eingesetzt.

Die HPLC-Analytik der bakteriellen Carotinoid-Extrakte erfolgte mittels HP Series II 1090 Liquid Chromatograph (Agilent Technologies, Böblingen) mit ternärem Pumpensystem und Dioden-Array-Detektor. Zur Auftrennung wurde eine Zorbax SB-C18- Trennsäule (3,5 µm, 4,6x150 mm, Agilent Technologies, Böblingen) bei einer Säulentemperatur von 40 °C. Die Auftrennung der Carotinoide erfolgte zunächst über 2 min. isokratisch mit 20 % Ethylacetat (EtAc) in Acetonitril (AcN), danach über einen Gradienten von 20 % EtAc in AcN bis 50 % EtAc in AcN für 10 min. und anschließend für 3 min. isokratisch bei 50 % EtAc in AcN mit einer Flussrate von 1 ml min-1.

Die Auswertung wurde mittels HP ChemStation for LC Version A.05.02 durchgeführt und erfolgte für alpha-,β-, delta-, und epsilon-Carotin bei einer Wellenlange von 450 nm.

Die HPLC-Analyse ergab, dass mit Ausnahme von ECmut5- alle generierten Mutanten die Ausgangsverbindung Lycopen praktisch vollständig umsetzen und epsilon-Carotin als Hauptprodukt bilden (Tabelle 1, Abbildung 6A und 6B). Die Variante ECmut1 entspricht der in der Literatur bereits beschriebenen Mutante (AtEC-L448H; Cunningham & Gantt, 2001) und diente als Referenz.

Die Mutanten ECmut9, -10, -11, -12, -16, -21, -3.2, -3.3, -3.5, -3.8, -3.9, -3.12 und -3.16 sind signifikant besser als die Referenz hinsichtlich Produktreinheit und Produktmenge. Der Anteil des durch die EC-Mutanten synthetisierten epsilon-Carotin am Gesamtcarotinoidgehalt der Zellen beträgt 97,7 bis 100% (siehe Tabelle 1), während für die Referenz (ECmut1) ein Anteil von 94,3% ermittelt wurde, der damit leicht über dem publizierten Referenzwert (92%; Cunningham et al., 2001) liegt.

Die besten Mutanten (ECmut9, -10, -3.2, -3.3, -3.5, -3.8, -3.9, -3.12) erbrachten epsilon-Carotingehalte von 99,3-100%. Das Verhältnis von epsilon-Carotin zu seiner Vorstufe delta-Carotin liegt für die genannten Mutanten im Bereich von 147:1 bis 492:1 und damit 3 bis 10fach höher als die besten bislang publizierten Mengenverhältnisse, die im Bereich von 10:1 bis 49:1 lagen (siehe Tabelle 1 und Cunningham et al., 2001, Bai et al. 2009). Für ECmut3.5 lag die delta-Carotinmenge unter der Nachweisgrenze, so dass aufgrund des Vollumsatzes hier kein Quotient bestimmt werden konnte bzw. dieser unendlich groß ist.

Überraschenderweise zeigte die Analyse, dass nicht nur die Reinheit des gebildeten epsilon-Carotin, sondern auch Produktmenge abhängig von der verwendeten EC-Mutante ist (Tabelle 1, Abbildung 6A und 6B).

**Tabelle 1: Vergleich der Carotinoidausbeuten bekannter Lycopen-epsilon-Zyclasen (EC) mit den erfindungsgemäßen Mutanten (ECmut)**

| | | **Carotinoidausbeuten (% der Gesamtmenge)** | | | | | | | |
|---|---|---|---|---|---|---|---|---|---|
| **Enzym** | **Mutation** | **Lyc** | **a-Caro** | **g-Caro** | **d-Caro** | **e-Caro** | **e-Caro/ d-Caro** | **e-Caro-Ausbeute** | **Ref.** |
| AtEC | - | 1 | | | 98 | 1 | 0,01 | | Cunningham 2001 |
| | - | 2 | 0 | 13,6 | 84,2 | 0,2 | 0,00 | | Bai 2009 |
| | A447S/L448H/Q451L/F452M | 0 | | | 2 | 98 | 49 | | Cunningham 2001 |
| | L448H | 0 | | | 8 | 92 | 11,5 | | Cunningham 2001 |
| | L448R | 0 | | | 8 | 92 | 11,5 | | Cunningham 2001 |
| | L448D | 37 | | | 56 | 8 | 0,14 | | Cunningham 2001 |
| | A447D | 1 | | | 98 | 1 | 0,01 | | Cunningham 2001 |
| LsEC | - | 3 | | | 8 | 90 | 11,25 | | Cunningham 2001 |
| | - | 6,3 | 12 | 4,2 | 7,1 | 70,3 | 9,90 | | Bai 2009 |
| | H457R | 3 | | | 6 | 91 | 15,17 | | Cunningham 2001 |
| | H457D | 22 | | | 18 | 60 | 3,33 | | Cunningham 2001 |
| | H457L | 17 | | | 73 | 10 | 0,14 | | Cunningham 2001 |
| AaEC | | 0 | | | 44 | 56 | 1,27 | | Cunningham 2001 |
| ZmEC | - | 5,5 | 3,4 | 9,3 | 42,6 | 39,2 | 0,92 | | Bai 2009 |
| | L461H | 4 | 9,5 | 5 | 5,4 | 76,1 | 14,09 | | Bai 2009 |
| | S502A | 2,9 | 0,2 | 11,8 | 80,6 | 4,5 | 0,06 | | Bai 2009 |
| *ECmut1 (Re* | *L448H* | *0* | | | *5,7* | *94,3* | *16,48* | *100* | |
| ECmut9 | L448S | 0 | | | 0,4 | 99,6 | 221,7 | 167 | |
| LCmnt10 | A447S/L448T | 0 | | | 0,6 | 99,4 | 170,1 | 162 | |
| ECmut3.12 | L448TA489S | 0 | | | 0,7 | 99,3 | 147,1 | 140 | |
| ECmut3.2 | A447C/L448C/A489S | 0 | | | 0,7 | 99,3 | 133,8 | 164 | |
| ECmut3.3 | A447E/L448A/A489S | 0 | | | 0,2 | 99,8 | 492,5 | 148 | |
| ECmut3.5 | A447M/L448A/A489S | 0 | | | 0 | 100 | nb | 99 | |
| ECmut3.8 | A447H/L448S/A489S | 0,2 | | | 0,2 | 99,6 | 410,7 | 124 | |
| ECmut3.9 | A447E/L448G/A489S | 0 | | | 0,5 | 99,5 | 184,3 | 106 | |
| ECmut3.16 | A447G/L448G/A489S | 0,8 | | | 0,6 | 98,6 | 152,2 | 156 | |

Die ersten beiden Spalten benennen das Enzym bzw. die Enzymmutanten und die jeweiligen Aminosäureaustausche. Zur besseren Vergleichbarkeit mit den Literaturdaten sind hier die Mutationen der erfindungsgemäßen ECmut-Enzyme entsprechend des Volllängenenzyms benannt. Die Positionen 447, 448 und 489 des wildtyp *A.thaliana* Enzyms Lycopen-epsilon-Zyklase (AtEC) entsprechen den Positionen 403,404 und 445 in der erfindungsgemäßen Mutante AtEC-del (SEQ ID Nr. 19)(siehe Abbildung 5). Die beschriebenen Carotinoidausbeuten in Prozent für Lyc, a-Caro, g-Caro, d-Caro, e-Caro stellen den prozentualen Anteil des jeweiligen Carotinoids an der Gesamtmenge der aufgeführten Carotinoide dar. Die beschriebene e-Carotin-Ausbeute stellt das Verhältnis, ausgedrückt in Prozent, zwischen der Menge an gebildetem epsilon-Carotin der Referenzmutante ECmut1 (L448H) und der jeweiligen erfindungsgemäßen EC-Mutante dar, wobei der Referenzwert (ECmut1 (L448H)) als 100 % festgelegt wurde.

Lyc=Lycopen, a-Caro=alpha-Carotin, g-Caro=gamma-Carotin, d-Caro=delta-Carotin, e-Caro=epsilon-Carotin.; At = *Arabidopsis thaliana,* Ls =*Latuca sativa,* Zm= Zea *mays, EC*=Lycopen-epsilon-Zyklase.

### Beispiel 7: Verfahren zur Gewinnung von alpha-Ionon

Zur Produktion von alpha-Ionon in Schüttelkolbenkulturen wurden die erfindungsgemäßen Expressionsplasmide (z. B. pGT1066 kodierend für ECmut3 und ein CCD1-Expressionsplasmid pGT1069 oder pGT1070) zunächst über Standardtransformationsprotokolle gemeinsam in E. *coli*-TOP10 eingebracht und diese dann unter selektiven Bedingungen (Selektion mit Chloramphenicol (25mg/L) und Ampicillin (100mg/L)) auf Agarplatten mit LB-Medium angezogen (Inkubation 24h bei 28-30°C). Zur Produktion des Substrats epsilon-Carotin wurde Flüssigmedium (dYT + Chloramphenicol (25mg/L) und Ampicillin (100mg/L)) mit einer Einzelkolonie von den erhaltenen Platten beimpft und die Kultur für 24h bei 28-30°C unter Schütteln (200rpm) inkubiert. Anschließend wurde die Expression der Carotinoid Cleavage Dioxygenase (CCD) und somit die Umsetzung des gebildeten epsilon-Carotins zu alpha-Ionon durch Zugabe des Induktionsmediums (dYT + 0,5% Arabinose + Chloramphenicol (25mg/ltr) und Ampicillin (100mg/ltr)) gestartet. Es wurde 1/5 des Originalvolumens zugesetzt. Die Kultur wurde dann für weitere 4h bei 28°C inkubiert. Zur Extraktion des gebildeten alpha-Ionons wurden die Bakterienzellen über Zentrifugation (10min; 5000rpm) abgetrennt, anschließend lysiert und das Lysat mit Diethylether ausgeschüttelt.

### Beispiel 8: Nachweis von alpha-lonon

Das gebildete epsilon-Carotin wurde quantitativ umgesetzt, was bereits makroskopisch anhand der Entfärbung der Zellen erkennbar war. Zur Extraktion des gebildeten alpha-lonons wurden die Bakterienzellen mittels Zentrifugation (10min; 5000g) abgetrennt, anschließend lysiert und das Lysat mit Diethylether ausgeschüttelt, wie bereits in Beispiel 7 beschrieben. Die erhaltenen Präparate wurden per HPLC und LC-MS analysiert (Abbildung 8). Die HPLC-Analytik der bakteriellen Carotinoid-Extrakte erfolgte mittels HP Series II 1090 Liquid Chromatograph (Agilent Technologies, Böblingen) mit ternärem Pumpensystem und Dioden- Array-Detektor. Zur Auftrennung wurde eine Zorbax SB-C18- Trennsäule (3,5 µm, 4,6x150 mm, Agilent Technologies, Böblingen) bei einer Säulentemperatur von 40 °C. Die Auftrennung der Carotinoide erfolgte zunächst über 2 min. isokratisch mit 20 % Ethylacetat (EtAc) in Acetonitril (AcN), danach über einen Gradienten von 20 % EtAc in AcN bis 50 % EtAc in AcN für 10 min. und anschließend für 3 min. isokratisch bei 50 % EtAc in AcN mit einer Flussrate von 1 ml min-1. Die Auswertung wurde mittels HP ChemStation for LC Version A.05.02 durchgeführt und erfolgte für alpha-,β -, delta - und epsilon-Carotin bei einer Wellenlange von 450 nm und für alpha- und β- Ionon bei 280 nm.

### Beispiel 9: Analyse der Enantiomer-Verteilung

Die Analyse des fermentativ erzeugten alpha-lonons hinsichtlich der Enantiomer-Verteilung/Reinheit erfolgte mittels GC-Massenspektroskopie. Zur Vorbereitung wurden die Diethyletherextrakte (siehe Beispiel 8) bis zur Trockene eingedampft, um den Diethylether zu entfernen, und die erhaltene Trockensubstanz in Acetonitril gelöst. Diese Probe wurde dann unverdünnt zur GC-Massenspektrometrie eingesetzt.

### Bestimmung der Enantiomerenverteilung:

Hierzu wurde enantioselektive Gaschromatografie/Massenspektrometrie (GC/MS) wie folgt durchgeführt: Die Massenspektren wurden an einem Gaschromatografen Varian 3800 (Varian, Darmstadt), der an ein Massenspektrometer Saturn 2000 (Varian, Darmstadt) gekoppelt war, erzeugt. Zur Bestimmung der Enantiomerenverteilung von alpha-lonon wurden Massenspektren im Cl-Modus mit einer lonisierungsenergie von 70 eV aufgenommen. Folgende Kapillarsäule wurde verwendet: BGB174, 30 m × 0,25 mm Innendurchmesser (ID), 0,25 µm Filmdicke, Phenomenex. Die folgenden Bedingungen für die GC/MS wurden verwendet:
- Probenaufgabe: on column, 40°C, 1µl Injektionsvolumen
- Trägergas: Helium, Flussgeschwindigkeit 35 cm/s
- Massenspektrometer: Ionenfalle Saturn 2000-2000R, Varian, Darmstadt
- Temperaturprogramm: Temperaturgradient 70-220°C mit: 0min 70°C, 4°C/min Anstieg, 5min 220°C

### Bestimmung des β-Ionongehalts:

Hierzu wurde semiquantitative Gaschromatografie/Massenspektrometrie (GC/MS) an einem Gaschromatografen Varian 3800 (Varian, Darmstadt), der an ein Massenspektrometer Saturn 2000 (Varian, Darmstadt) gekoppelt war, durchgeführt. Zur semiquantitativen Bestimmung von beta-Ionon wurden Massenspektren im El-Modus mit einer lonisierungsenergie von 70 eV aufgenommen. Folgende Kapillarsäule wurde verwendet: FFAP, 30 m × 0,25 mm Innendurchmesser (ID), 0,25 µm Filmdicke, Phenomenex.

### Die Bedingungen für die GC/MS waren wie folgt:

- Probenaufgabe: on column, 40°C (Injektionsvolumen: 1 µl)
- Trägergas: Helium, Flussgeschwindigkeit 35 cm/s
- Massenspektrometer: lonenfalle Saturn 2000-2000R, Varian, Darmstadt
- Temperaturprogramm: Temperaturgradient 40-240°C mit: 1min 40°C, 6°C/min Anstieg, 5min 240°C

### Ergebnisse:

Für die alpha-Ionon-Probe wurde eine Enantiomerenverteilung von 100% [R] zu 0% [S] ermittelt. Die Probe enthält enantiomerenreines (R)-alpha-Ionon.

Der Gehalt an beta-Ionon lag unter der Nachweisgrenze (< 2 µg/L). Die Probe enthält reines alpha-Ionon.

### Beispiel 10: Promotoren

Durch die Wahl der Promotoren kann die Synthese der Zwischenprodukte (Lycopen, epsilon-Carotin) und des Endprodukts (alpha-Ionon) aufeinander abgestimmt werden ("fine tuning"). Hierfür könnten indizierbare oder konstitutive Promotoren zum Einsatz kommen. Abhängig von der Konstruktion eines Mikroorganismus mit vielen, freien Plasmiden oder der Integration jeweils einer Expressionskassette in das mikrobielle Genom sind unterschiedliche Promotorstärken wünschenswert.

### Promotoren aus dem Stand der Technik:

- pTet, Tetracyclin-Promotor aus E. coli-Plasmid pBR332 (), konstitutiv
- pLac: Lac-Promotor; Promotorregion des genomischen E. coli Lac-Operon
- pBAD: Arabinose induzierbarer Promotor; Promotorregion des genomischen E. coli Arabinose-Operon
- pXyl Promotor: Xylose induzierbarer Promotor; regulatorische Sequenzen aus dem E.coli Xylose-Operon bestehend aus der bidirektionalen Promotorregion (cis-regulatorischen Sequenzen), die die polycistronischen Operons xylA/xylB und xylF/xylG/xylH/xylR, kontrolliert, wobei dessen Aktivität durch das xylR-Genprodukt des xylFGHR-Operons reguliert wird.

### Erfindungsgemäße induzierbare Promotoren:

- pTet-m1: 12bp-Deletion in Promotor vor LYC-Operon, Promotoraktivität wird um den Faktor 2,8 gesteigert
- pXyl0: synthetischer Xylose-induzierbarer Promotor. Entstanden durch direkte Verknüpfung des xylR-Gens mit den cis-regulatorischen Sequenzen (mittels Deletion der xylF-, xylG- und xylH-Gensequenzen). Basiskonstrukt. Induzierbarkeit: 25x; rel. Expressionsstärke (max): 2,5% des Referenzpromotors (pLac)
- pXyl1: Kombination von pXyl0 mit einer optimierten Ribosomen-Bindungsstelle (Shine-Dalgarno-Sequenz) zur effizienten Translation von Zielgenen.

### Promotor 3-4x aktiver als pXyl0 (max 10% der pLac-Aktivität)

- pXyl2: Basierend auf pXyl1 wurde die Sequenz der -10-Region (Bindungsstelle der RNA-Polymerase) des downstream-gerichteten Promotorelement modifiziert. Promotor 3-4x aktiver als pXyl0 (max 36% der pLac-Aktivität)

### Erfindungsgemäße konstitutive Promotoren:

Die verwendeten Promotoren stammen aus einer Kollektion konstitutiv exprimierender Promotoren, die über einen PCR-basierten Ansatz erzeugt worden ist. Ein Promotor-loses RFP-Reportergenkonstrukt (pGJ2720del) diente hierbei als template. Über einen invers-PCR-Ansatz wird die gesamte Plasmidsequenz mit einer proof-reading Polymerase amplifiziert, wobei das DNA-Fragment um die in den Oligonukleotidprimern enthalten zusätzlichen Sequenzen (Extensions) verlängert wird. Primer1 (-10-Primer) bindet an die template-DNA im Bereich der Ribosomenbindungsstelle vor dem Reportergen. Seine Extension besteht aus 9 Zufallsbasen gefolgt von der Sequenz TATAAT und weiteren 6 Zufallsbasen. Primer2 bindet (in reverser Orientierung) unmittelbar vor der Bindungsstelle von Primer1. Die Primer2-Extension (-35-Primer) besteht aus 9 Zufallsbasen, gefolgt von der Sequenz TGTCAA und weiteren 6 Zufallsbasen. Primer1 und 2 weisen annealing-Temperaturen von 60°C auf. Die Primer wurden mit Enzyme Polynukleotid-Kinase (New England Biolabs) entsprechend der Herstellerangaben phosphoryliert und dann zur Amplifikation des templates mit folgendem PCR-Programm verwendet: 2min 98°C, gefolgt von 30 Zyklen mit 45s 98°C, 30s 60°C und 2min 72°C. Das erhaltene PCR-Fragment wurde elektrophoretisch auf einem Agarosegel aufgetrennt und die DNA-Bande aus dem Gel isoliert (PCR and Gelextraktion-Kit, Machery&Nagel). Mit Hilfe des Enzyms T4-DNA-Ligase wurde eine Auto-Ligation der isolierten DNA-Fragmente durchgeführt. Die Ligationsprodukte wurden mittels Standard-Transformationsmethoden in E.coli XL1 blue Zellen transformiert und rekombinante Zellen auf selektiven Medien angezogen. Die Selektion der erhaltenen funktionalen Promotoren erfolgte makroskopisch anhand der RFP-Reportergenexpression (Rot-Färbung) und in Vergleich mit einem entsprechenden Mikroorganismus, der das RFP-Reportergen unter Kontrolle des maximal induzierten pLac-Promotors exprimiert.. Klone unterschiedlicher Expressionshöhe wurden ausgewählt, die Plasmid-DNA isoliert und die jeweils enthaltene Promotorsequenz mittels DNA-Sequenzierung bestimmt. Die Benennung erfolgt nach dem Schema aPxx entsprechend der Klonauswahl. Die Promotornummer korreliert nicht mit der Expressionsstärke.
- aP12: Aktivität: 35% des pLac-Promotors (induziert)
- aP15: Aktivität: 39% des pLac-Promotors (induziert)
- aP32: Aktivität: 51% des pLac-Promotors (induziert)
- aP47.2: Aktivität: 180% des pLac-Promotors (induziert)

### Beispiel 11: Carotinoidausbeute

Der Carotinoid-produzierende E. coli-Stamm wird in flüssigem dYT-Medium für 18-48Std bei 28°C angezogen. Im Photometer wird die Zelldichte der erhaltenen Kulturen (=OD600) durch Messung der Absorption bei 600nm bestimmt. Gegebenenfalls wird die Kultur mit dYT-Medium geeignet verdünnt (i.a. 1:10), um Extinktions-Messwerte im Bereich von 0,1 bis 0,8 zu erhalten. Anhand der Ergebnisse werden die Kulturen auf OD600/ml = 4 eingestellt (Verdünnung mit frischem Medium). Die Zellen aus 1ml dieser Kulturen werden durch Zentrifugation (1min, 13000rpm) pelletiert und der Medienüberstand entfernt. Das Zellpellet wird in 1ml Aceton resuspendiert und der Ansatz für 30min bei RT und im Dunkeln inkubiert, wodurch die Carotinoide extrahiert werden. Der Ansatz wird dann zur Abtrennung der Zellen/des Zellschrotts zentrifugiert (2min, 13000rpm) und der Überstand überführt. Sollte das Pellet noch Carotinoide enthalten (Färbung noch sichtbar) wird die Extraktion wiederholt und die Überstände der Extraktionen vereint. Die Carotinoidkonzentrationen der Extrakte werden photometrisch bestimmt (in g/L), indem die Absorptionsspektren (gegen Aceton als Blindwert) aufgenommen und die gemessenen Extinktionen bei 474 nm (Lycopen) bzw. 442 nm (e-Carotin) anhand der spezifischen Extinktionskoeffizienten (Lycopen: 3450 (L*g⁻¹*cm⁻¹); e-Carotin:2900 (L*g⁻¹*cm⁻¹)) umgerechnet werden. Das Trockengewicht der extrahierten Zellmasse wird über folgende, empirisch ermittelte Formel aus den gemessenen Zelldichten berechnet: TGw (g/L) = 0,35 x OD600. Zur Beurteilung der Carotinoidsyntheseleistung wird die Carotinoidmenge pro Biomasse (mg Carotinoid/g TGw) bestimmt.

**Tabelle 2:**

| **Plasmid** | **Änderung** | **Rel. Ausbeute* Carotinoid** |
|---|---|---|
| pAC-BETAipi-d-crtY | - | 1,0x |
| pGT1036** | 1. Deletion Basen 984-1394 (Bildung neuer crtE-Shine-Dalgarno-Sequenz) | 4,2x |
| | 2. Deletion Basen 3432-4198 | |
| | 3. Insertion der Sequenz GGAGGTACAAC an dieser Stelle und Modifikation 3418 - 3432 (Bildung neuer crtl-Shine-Dalgarno) | |
| | 4. Deletion Basen 6605-7242 | |
| | 5. Insertion Terminatorsequenz | |
| pGT1066 | Einbau pLac:ECmutX.x Kassette | 4,2x |
| pGT1182 | =pGT1066 mit ECmut3.3 | 4,2x |
| pGT1464^{***} | Ersatz der Basen 5183-6146 durch aP12-Sequenz | 8,0x |
| | (->Austausch pLac-Promotor vor ECmut3.3 gegen PHY-Promotor aP12) | |
| pGT1484*** | Ersatz der Basen 96-1015 durch idsA-Sequenz (->Austausch crtE gegen idsA) | 10,0x |
| pGT1518**** | Deletion der Basen 123-140 (17bp) in pTet-Promotor (->pTet-m1 (Aktivität 2,8x höher!) ) | 11,8x |
| pGT1543**** | Deletion der Basen 8669-141 und Insertion des aP30-Promotors | 12,5x |

| | | |
|---|---|---|
| * bezogen auf gleiche Biomassemengen ** Positionsangaben bezogen auf pAC-BETAipi-d-crtY *** Positionsangaben bezogen auf pGT1182 **** Positionsangaben bezogen auf pGT1484 | | |

**Tabelle 3:**

| **Plasmidkombination** | **Relative alpha-Ionon-Ausbeute** |
|---|---|
| pGT1182 / pGT1454 | 1x |
| pGT1464 / pGT1454 | 1,6x |
| pGT1484 / pGT1454 | 1,8x |
| pGT1518 / pGT1454 | 2,4x |
| pGT1518 / pGT1584 | 3,0x |
| pGT1575 / pGT1534 | 4,8x |

### Literaturverzeichnis

Bai L, Kim E-H, DellaPenna D, Brutnell TP (2009) Novel lycopene epsilon cyclase activities in maize revealed through perturbation of carotenoid biosynthesis. The Plant Journal 59: 588-599.
Baldermann S, Kato M, Kurosawa M, Kurobayashi Y, Fujita A, Fleischmann P, Watanabe N (2010) Functional characterization of a carotenoid cleavage dioxygenase 1 and its relation to the carotenoid accumulation and volatile emission during the floral development of Osmanthus fragrans Lour. Journal of Experimental Botany 61: 2967-2977.
Bovolenta M, Castronovo F, Vadalà A, Zanoni G, Vidari G (2004) A Simple and Efficient Highly Enantioselective Synthesis of α-Ionone and α-Damascone. The Journal of Organic Chemistry 69: 8959-8962.
Cunningham FX, Gantt E (2001) One ring or two? Determination of ring number in carotenoids by lycopene ?-cyclases. Proceedings of the National Academy of Sciences 98: 2905-2910.
Cunningham FX, Gantt E (2007) A portfolio of plasmids for identification and analysis of carotenoid pathway enzymes: Adonis aestivalis as a case study. Photosynthesis Research 92: 245-259.
Cunningham FX, Pogson B, Sun Z, McDonald KA, DellaPenna D, Gantt E (1996) Functional analysis of the beta and epsilon lycopene cyclase enzymes of Arabidopsis reveals a mechanism for control of cyclic carotenoid formation. The Plant Cell Online 8: 1613-1626.
Cunningham FX, Sun Z, Chamovitz D, Hirschberg J, Gantt E (1994) Molecular structure and enzymatic function of lycopene cyclase from the cyanobacterium Synechococcus sp strain PCC7942. The Plant Cell Online 6: 1107-1121.
Jach G, Pesch M, Richter K., Frings S and Uhrig J (2006) An improved mRFP1 adds red to bimolecular fluorescence complementation. Nature Methods 3 No8: 597-600
Misawa N, Nakagawa M, Kobayashi K, Yamano S, Izawa Y, Nakamura K, Harashima K (1990) Elucidation of the Erwinia uredovora carotenoid biosynthetic pathway by functional analysis of gene products expressed in Escherichia coli. Journal of Bacteriology 172: 6704-6712.
Perry KL, Simonitch TA, Harrison-Lavoie KJ, Liu ST (1986) Cloning and regulation of Erwinia herbicola pigment genes. Journal of Bacteriology 168: 607-612.
Sambrook J, Fritsch EF, Maniatis T. in: Molecular Cloning, A Laboratory Manual, 1989 (Nolan C, Ed.), Cold Spring Harbor Laboratory Press, Cold Spring Harbor, NY
Soorukram D, Knochel P (2004) Enantioselective Synthesis of α-Ionone Derivatives Using an Anti SN2' Substitution of Functionalized Zinc Organometallics. Organic Letters 6: 2409-2411.
Vogel JT, Tan B-C, McCarty DR, Klee HJ (2008) The Carotenoid Cleavage Dioxygenase 1 Enzyme Has Broad Substrate Specificity, Cleaving Multiple Carotenoids at Two Different Bond Positions. Journal of Biological Chemistry 283: 11364-11373.
Yahyaa M, Bar E, Dubey NK, Meir A, Davidovich-Rikanati R, Hirschberg J, Aly R, Tholl D, Simon PW, Tadmor Y, Lewinsohn E, Ibdah M (2013) Formation of Norisoprenoid Flavor Compounds in Carrot (Daucus carota L.) Roots: Characterization of a Cyclic-Specific Carotenoid Cleavage Dioxygenase 1 Gene. Journal of Agricultural and Food Chemistry 61: 12244-12252.
Zhang W, Hu X, Wang L, Wang X (2014) Reconstruction of the Carotenoid Biosynthetic Pathway of Cronobacter sakazakii BAA894 in Escherichia coli. PLoS ONE 9: e86739.

## Patentansprüche

1. Verfahren zur Herstellung von enantiomerenreinem alpha-Ionon, umfassend das Kultivieren eines Mikroorganismus, der heterologe Nukleotidsequenzen enthält, die die folgenden Enzyme kodieren:
a. Geranylgeranyl-Diphosphat-Synthase,
b. Isopentenyl-Diphosphat-Isomerase (ipi),
c. Phytoen-Desaturase/Dehydrogenase (crtl),
d. Phytoensynthase (crtB),
e. Lycopen-epsilon-Zyklase (EC) und
f. Carotenoid-Cleavage-Dioxygenase (CCD1).

2. Verfahren gemäß Anspruch 1, wobei die Geranylgeranyl-Diphosphat-Synthase die Geranylgeranyl-Diphosphat-Synthase crtE oder die Geranylgeranyl-Diphosphat-Synthase idsA ist.

3. Verfahren gemäß Anspruch 1 oder 2, wobei die Lycopen-epsilon-Zyklase (EC) mindestens 80% Sequenzidentität mit einer Sequenz gemäß SEQ ID Nr. 19 besitzt und mindestens an einer der Positionen 403, 404 und 445 von der Sequenz gemäß SEQ ID Nr. 19 abweicht.

4. Verfahren gemäß einem der Ansprüche 1 bis 3, wobei Lycopen-epsilon-Zyklase (EC) eine der folgenden Mutationen oder Mutationskombinationen umfasst: ECmut9 (L404S), ECmut10 (A403S/L404T), ECmut3.3 (A403E/L404A/A445S) und ECmut3.2 (A403C/L404C/A445S).

5. Verfahren gemäß einem der Ansprüche 1 bis 4, wobei die Carotenoid-Cleavage-Dioxygenase (CCD1) eine Carotenoid-Cleavage-Dioxygenase (CCD1) aus Arabidopsis thaliana oder Osmanthus fragrans ist.

6. Verfahren gemäß einem der Ansprüche 1 bis 5, wobei die Enzyme auf einem oder mehreren Plasmiden kodiert sind, wobei das oder die Plasmide als individuelle Strukturen in dem Mikroorganismus vorliegen oder in das Genom des Mikroorganismus integriert sind.

7. Verfahren gemäß einem der Ansprüche 1 bis 6, wobei die kodierten Enzyme koexprimiert werden.

8. Verfahren gemäß einem der Ansprüche 1 bis 7, wobei der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 30, 31, 32, 33, 34 , 35 oder 36 enthält.

9. Verfahren gemäß einem der Ansprüche 1 bis 8, wobei der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 21, 24, ,37, 38, 39, 40 ,41 oder 42 enthält.

10. Verfahren gemäß einem der Ansprüche 1 bis 9, wobei der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 43 oder 44 enthält.

11. Verfahren gemäß einem der Ansprüche 1 bis 10, wobei der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 45, 46 oder 47 enthält.

12. Verfahren gemäß einem der Ansprüche 1 bis 11, wobei der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 33 und ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr.37 enthält, oder
wobei der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 33 und ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 41 enthält, oder
wobei der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 44 und ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 45 enthält.

13. Verfahren zur Herstellung von hochreinem epsilon-Carotin mit einem Gesamtcarotinoidgehalt von 97,7 bis 100% aus Lycopen, umfassend das Kultivieren eines Mikroorganismus, der heterologe Nukleotidsequenzen enthält, die die folgenden Enzyme kodieren:
a. Geranylgeranyl-Diphosphat-Synthase,
b. Isopentenyl-Diphosphat-Isomerase (ipi),
c. Phytoen-Desaturase/Dehydrogenase (crtl),
d. Phytoensynthase (crtB), und
e. Lycopen-epsilon-Zyklase (EC),
wobei die Lycopen-epsilon-Zyklase (EC) eine der folgenden Mutationen oder Mutationskombinationen, bezogen auf die Referenzsequenz gemäß SEQ ID Nr:19, umfasst: ECmut9 (L404S), ECmut10 (A403S/L404T), ECmut3.3 (A403E/L404A/A445S) und ECmut3.2 (A403C/L404C/A445S).

14. Verfahren gemäß Anspruch 13, wobei die Geranylgeranyl-Diphosphat-Synthase die Geranylgeranyl-Diphosphat-Synthase crtE oder die Geranylgeranyl-Diphosphat-Synthase idsA ist.

15. Verfahren gemäß Anspruch 13 oder 14, wobei die Lycopen-epsilon-Zyklase (EC) mindestens 80% Sequenzidentität mit einer Sequenz gemäß SEQ ID Nr. 19 besitzt und mindestens an einer der Positionen 403, 404 und 445 von der Sequenz gemäß SEQ ID Nr. 19 abweicht.

16. Verfahren gemäß einem der Ansprüche 13 bis 15, wobei die Lycopen-epsilon-Zyklase (EC) der Sequenz gemäß SEQ ID Nr. 19 eine der folgenden Mutationen oder Mutationskombinationen umfasst: ECmut9 (L404S), ECmut10 (A403S/L404T), ECmut3.3 (A403E/L404A/A445S) und ECmut3.2 (A403C/L404C/A445S).

17. Verfahren gemäß einem der Ansprüche 13 bis 16, wobei die Enzyme auf einem oder mehreren Plasmiden kodiert sind, wobei das oder die Plasmide als individuelle Strukturen in dem Mikroorganismus vorliegen oder in das Genom des Mikroorganismus integriert sind.

18. Verfahren gemäß einem der Ansprüche 13 bis 17, wobei die kodierten Enzyme koexprimiert werden.

19. Verfahren gemäß einem der Ansprüche 13 bis 18, wobei der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 30, 31, 32, 33, 34, 35 oder 36 enthält.

20. Verfahren gemäß einem der Ansprüche 13 bis 19, wobei der Mikroorganismus ein Plasmid mit mindestens 80 % oder mindestens 85 %, 90 %, 91 %, 92 %, 93 %, 94 %, 95 %, 96 %, 97 %, 98 %, 99 % oder 100 % Sequenzidentität mit einer Sequenz gemäß SEQ ID Nr. 45, 46 oder 47 enthält.

21. Mikroorganismus gemäß dem in dem Verfahren gemäß einem der Ansprüche 1 bis 12 oder 13 bis 20 kultivierten Mikroorganismus.

## Claims

1. A method of producing enantiomerically pure alpha-ionone comprising culturing a microorganism that contains heterologous nucleotide sequences that encode the following enzymes:
a. geranylgeranyl-diphosphate-synthase,
b. isopentenyl-diphosphate-isomerase (ipi),
c. phytoene-desaturase/dehydrogenase (crtl),
d. phytoene synthase (crtB),
e. lycopene-epsilon-cyclase (EC) and
f. carotenoid-cleavage-dioxygenase (CCD1).

2. The method according to claim 1, wherein the geranylgeranyl-diphosphate-synthase is the geranylgeranyl-diphosphate-synthase crtE or the geranylgeranyl-diphosphate-synthase idsA.

3. The method according to claim 1 or 2, wherein the lycopene-epsilon-cyclase (EC) has at least 80% sequence identity with a sequence according to SEQ ID NO. 19 and deviates at least at one position of 403, 404 and 445 from the sequence according to SEQ ID NO. 19.

4. The method according to one of claims 1 to 3, wherein the lycopene-epsilon-cyclase (EC) comprises one of the following mutations or mutation combinations: ECmut9 (L404S), ECmut10 (A403S/L404T), ECmut3.3 (A403E/L404A/A445S) and ECmut3.2 (A403C/L404C/A445S).

5. The method according to one of claims 1 to 4, wherein the carotenoid-cleavage-dioxygenase (CCD1) is a carotenoid-cleavage-dioxygenase (CCD1) from *Arabidopsis thaliana* or *Osmanthus fragans.*

6. The method according to one of claims 1 to 5, wherein the enzymes are encoded on one or multiple plasmids, wherein the one or the multiple plasmids are present in the microorganism as individual structures or integrated into the genome of the microorganism.

7. The method according to one of claims 1 to 6, wherein the encoded enzymes are co-expressed.

8. The method according to one of claims 1 to 7, wherein the microorganism contains a plasmid with at least 80% or at least 85%, 90%, 91%, 92%, 93%, 94%, 95%, 96%, 97%, 98%, 99% for 100% sequence identity with a sequence according to SEQ ID NO. 30, 31, 32, 33, 34, 35 or 36.

9. The method according to one of claims 1 to 8, wherein the microorganism contains a plasmid with at least 80% or at least 85%, 90%, 91%, 92%, 93%, 94%, 95%, 96%, 97%, 98%, 99% or 100% sequence identity with a sequence according to SEQ ID NO. 21, 24, 37, 38, 39, 40, 41 or 42.

10. The method according to one of claims 1 to 9, wherein the microorganism contains a plasmid with at least 80% or at least 85%, 90%, 91%, 92%, 93%, 94%, 95%, 96%, 97%, 98%, 99% or 100% sequence identity with a sequence according to SEQ ID NO. 43 or 44.

11. The method according to one of claims 1 to 10, wherein the microorganism contains a plasmid with at least 80% or at least 85%, 90%, 91%, 92%, 93%, 94%, 95%, 96%, 97%, 98%, 99% or 100% sequence identity with a sequence according to SEQ ID NO. 45, 46 or 47.

12. The method according to one of claims 1 to 11, wherein the microorganism contains a plasmid with at least 80% or at least 85%, 90%, 91%, 92%, 93%, 94%, 95%, 96%, 97%, 98%, 99% or 100% sequence identity with a sequence according to SEQ ID NO. 33 and a plasmid with at least 80% or at least 85%, 90%, 91%, 92%, 93%, 94%, 95%, 96%, 97%, 98%, 99% or 100% sequence identity with a sequence according to SEQ ID NO. 37, or
wherein the microorganism contains a plasmid with at least 80% or at least 85%, 90%, 91%, 92%, 93%, 94%, 95%, 96%, 97%, 98%, 99% or 100% sequence identity with a sequence according to SEQ ID NO. 33 and a plasmid with at least 80% or at least 85%, 90%, 91%, 92%, 93%, 94%, 95%, 96%, 97%, 98%, 99% or 100% sequence identity with a sequence according to SEQ ID NO. 41, or
wherein the microorganism contains a plasmid with at least 80% or at least 85%, 90%, 91%, 92%, 93%, 94%, 95%, 96%, 97%, 98%, 99% or 100% sequence identity with a sequence according to SEQ ID NO. 44 and a plasmid with at least 80% or at least 85%, 90%, 91%, 92%, 93%, 94%, 95%, 96%, 97%, 98%, 99% or 100% sequence identity with a sequence according to SEQ ID NO. 45.

13. A method of producing highly pure epsilon-carotene with a total carotenoid content of 97.7 to 100% from lycopene, comprising culturing a microorganism that contains heterologous nucleotide sequences that encode the following enzymes:
a. geranylgeranyl-diphosphate-synthase,
b. isopentenyl-diphosphate-isomerase (ipi),
c. phytoene-desaturase/dehydrogenase (crtl)
d. phytoene synthase (crtB), and
e. lycopene-epsilon-cyclase (EC),
wherein the lycopene-epsilon-cyclase (EC) comprises one of the following mutations or mutation combinations relative to the reference sequence according to SEQ ID NO. 19: ECmut9 (L404S), ECmut10 (A403S/L404T), ECmut3.3 (A403E/L404A/A445S) and ECmut3.2 (A403C/L404C/A445S).

14. The method according to claim 13, wherein the geranylgeranyl-diphosphate-synthase is the geranylgeranyl-diphosphate synthase crtE or the geranylgeranyl-diphosphate-synthase idsA.

15. The method according to claims 13 or 14, wherein the lycopene-epsilon-cyclase (EC) has at least 80% sequence identity with a sequence according to SEQ ID NO. 19 and deviates at least at one of the positions 403, 404 and 445 from the sequence according to SEQ ID NO. 19.

16. The method according to one of claims 13 to 15, wherein the lycopene-epsilon-cyclase (EC) with a sequence according to SEQ ID NO. 19 comprises one of the following mutations or mutation combinations: ECmut9 (L404S), ECmut10 (A403S/L404T), ECmut3.3 (A403E/L404A/A445S) and ECmut3.2 (A403C/L404C/A445S).

17. The method according to one of claims 13 to 16, wherein the enzymes are encoded on one or multiple plasmids, wherein the one or the multiple plasmids are present in the microorganism as individual structures or integrated into the genome of the microorganism.

18. The method according to one of claims 13 to 17, wherein the encoded enzymes are co-expressed.

19. The method according to one of claims 13 to 18, wherein the microorganism contains a plasmid with at least 80% or at least 85%, 90%, 91%, 92%, 93%, 94%, 95%, 96%, 97%, 98%, 99% or 100% sequence identity with a sequence according to SEQ ID NO. 30, 31, 32, 33, 34, 35 or 36.

20. The method according to one of claims 13 to 19, wherein the microorganism contains a plasmid with at least 80% or at least 85%, 90%, 91%, 92%, 93%, 94%, 95%, 96%, 97%, 98%, 99% or 100% sequence identity with a sequence according to SEQ ID NO. 45, 46 of 47.

21. A microorganism according to the microorganism that is cultured in the method according to one of claims 1 to 12 or 13 to 20.

## Revendications

1. Procédé de production d'alpha-ionone énantiomériquement pure, comprenant la culture d'un micro-organisme contenant des séquences nucléotidiques hétérologues qui codent pour les enzymes ci-dessous :
a. Géranylgéranyle diphosphate synthase,
b. Isopentényle diphosphate isomérase (ipi),
c. Phytoène désaturase/déshydrogénase (crtl),
d. Phytoène synthase (crtB),
e. Lycopène epsilon cyclase (EC) et
f. Dioxygénase de clivage des caroténoïdes (CCD1).

2. Procédé selon la revendication 1, dans lequel la géranylgéranyle diphosphate synthase est la géranylgéranyle diphosphate synthase crtE ou la géranylgéranyle diphosphate synthase idsA.

3. Procédé selon la revendication 1 ou 2, dans lequel la lycopène epsilon cyclase (EC) présente au moins 80 % d'identité de séquence avec une séquence selon SEQ ID N°19 et diffère de la séquence selon SEQ ID N°19 au moins au niveau de l'une des positions 403, 404 et 445.

4. Procédé selon l'une quelconque des revendications 1 à 3, dans lequel la lycopène epsilon cyclase (EC) comprend l'une des mutations ou combinaisons de mutations ci-dessous: ECmut9 (L404S), ECmut10 (A403S/L404T), ECmut3.3 (A403E/L404A)/A445S) et ECmut3.2 (A403C/L404C/A445S).

5. Procédé selon l'une quelconque des revendications 1 à 4, dans lequel la dioxygénase de clivage des caroténoïdes (CCD1) est une dioxygénase de clivage des caroténoïdes (CCD1) issue d'Arabidopsis thaliana ou d'Osmanthus fragrans.

6. Procédé selon l'une quelconque des revendications 1 à 5, dans lequel les enzymes sont codées sur un ou plusieurs plasmide(s), dans lequel le ou les plasmide(s) est/sont présent(s) dans le microorganisme sous forme de structure(s) individuelle(s) ou est/sont intégré(s) dans le génome du microorganisme.

7. Procédé selon l'une quelconque des revendications 1 à 6, dans lequel les enzymes codées sont co-exprimées.

8. Procédé selon l'une quelconque des revendications 1 à 7, dans lequel le microorganisme contient un plasmide présentant au moins 80 % ou au moins 85 %, 90 %, 91 %, 92 %, 93 %, 94 %, 95 %, 96 %, 97 %, 98 %, 99 % ou 100 % d'identité de séquence avec une séquence selon SEQ ID N°30, 31, 32, 33, 34, 35 ou 36.

9. Procédé selon l'une quelconque des revendications 1 à 8, dans lequel le microorganisme contient un plasmide présentant au moins 80 % ou au moins 85 %, 90 %, 91 %, 92 %, 93 %, 94 %, 95 %, 96 %, 97 %, 98 %, 99 % ou 100 % d'identité de séquence avec une séquence selon SEQ ID N°21, 24, 37, 38, 39, 40, 41 ou 42.

10. Procédé selon l'une quelconque des revendications 1 à 9, dans lequel le microorganisme contient un plasmide présentant au moins 80 % ou au moins 85 %, 90 %, 91 %, 92 %, 93 %, 94 %, 95 %, 96 %, 97 %, 98 %, 99 % ou 100 % d'identité de séquence avec une séquence selon SEQ ID N°43 ou 44.

11. Procédé selon l'une quelconque des revendications 1 à 10, dans lequel le microorganisme contient un plasmide présentant au moins 80 % ou au moins 85 %, 90 %, 91 %, 92 %, 93 %, 94 %, 95 %, 96 %, 97 %, 98 %, 99 % ou 100 % d'identité de séquence avec une séquence selon SEQ ID N°45, 46 ou 47.

12. Procédé selon l'une quelconque des revendications 1 à 11, dans lequel le microorganisme contient un plasmide présentant au moins 80 % ou au moins 85 %, 90 %, 91 %, 92 %, 93 %, 94 %, 95 %, 96 %, 97 %, 98 %, 99 % ou 100 % d'identité de séquence avec une séquence selon SEQ ID N°33 et un plasmide présentant au moins 80 % ou au moins 85 %, 90 %, 91 %, 92 %, 93 %, 94 %, 95 %, 96 %, 97 %, 98%, 99% ou 100% d'identité de séquence avec une séquence selon SEQ ID N°37, ou
dans lequel le microorganisme contient un plasmide présentant au moins 80 % ou au moins 85 %, 90 %, 91 %, 92 %, 93 %, 94 %, 95 %, 96 %, 97 %, 98 %, 99 % ou 100 % d'identité de séquence avec une séquence selon SEQ ID N°33 et un plasmide présentant au moins 80 % ou au moins 85 %, 90 %, 91 %, 92 %, 93 %, 94 %, 95 %, 96 %, 97 %, 98 %, 99 % ou 100 % d'identité de séquence avec une séquence selon SEQ ID N°41, ou
dans lequel le microorganisme contient un plasmide présentant au moins 80 % ou au moins 85 %, 90 %, 91 %, 92 %, 93 %, 94 %, 95 %, 96 %, 97 %, 98 %, 99 % ou 100 % d'identité de séquence avec une séquence selon SEQ ID N°44 et un plasmide présentant au moins 80 % ou au moins 85 %, 90 %, 91 %, 92 %, 93 %, 94 %, 95 %, 96 %, 97 %, 98 %, 99 % ou 100 % d'identité de séquence avec une séquence selon SEQ ID N°45.

13. Procédé de production d'epsilon-carotène de haute pureté présentant une teneur totale en caroténoïdes comprise entre 97,7 et 100% à partir de lycopène, comprenant la culture d'un micro-organisme contenant des séquences nucléotidiques hétérologues qui codent pour les enzymes ci-dessous :
a. Géranylgéranyle diphosphate synthase,
b. Isopentényle diphosphate isomérase (ipi),
c. Phytoène désaturase/déshydrogénase (crtl),
d. Phytoène synthase (crtB), et
e. Lycopène epsilon cyclase (EC),
dans lequel la lycopène epsilon cyclase (EC) comprend l'une des mutations ou combinaisons de mutations ci-dessous, par rapport à la séquence de référence selon SEQ ID N°19 : ECmut9 (L404S), ECmut10 (A403S/L404T), ECmut3.3 (A403E/L404A/A445S) et ECmut3.2 (A403C/L404C/A445S).

14. Procédé selon la revendication 13, dans lequel la géranylgéranyle diphosphate synthase est la géranylgéranyle diphosphate synthase crtE ou la géranylgéranyle diphosphate synthase idsA.

15. Procédé selon la revendication 13 ou 14, dans lequel la lycopène epsilon cyclase (EC) présente au moins 80 % d'identité de séquence avec une séquence selon SEQ ID N°19 et diffère de la séquence selon SEQ ID N°19 au moins au niveau de l'une des positions 403, 404 et 445.

16. Procédé selon l'une quelconque des revendications 13 à 15, dans lequel la lycopène epsilon cyclase (EC) de la séquence selon SEQ ID N°19 comprend l'une des mutations ou combinaisons de mutations ci-dessous : ECmut9 (L404S), ECmut10 (A403S/L404T), ECmut3.3 (A403E/L404A/A445S) et ECmut3.2 (A403C/L404C/A445S).

17. Procédé selon l'une quelconque des revendications 13 à 16, dans lequel les enzymes sont codées sur un ou plusieurs plasmide(s), dans lequel le ou les plasmide(s) est/sont présent(s) dans le microorganisme sous forme de structure(s) individuelle(s) ou est/sont intégré(s) dans le génome du microorganisme.

18. Procédé selon l'une quelconque des revendications 13 à 17, dans lequel les enzymes codées sont co-exprimées.

19. Procédé selon l'une quelconque des revendications 13 à 18, dans lequel le microorganisme contient un plasmide présentant au moins 80 % ou au moins 85 %, 90 %, 91 %, 92 %, 93 %, 94 %, 95 %, 96 %, 97 %, 98 %, 99 % ou 100 % d'identité de séquence avec une séquence selon SEQ ID N°30, 31, 32, 33, 34, 35 ou 36.

20. Procédé selon l'une quelconque des revendications 13 à 19, dans lequel le microorganisme contient un plasmide présentant au moins 80 % ou au moins 85 %, 90 %, 91 %, 92 %, 93 %, 94 %, 95 %, 96 %, 97 %, 98 %, 99 % ou 100 % d'identité de séquence avec une séquence selon SEQ ID N°45, 46 ou 47.

21. Microorganisme selon le microorganisme cultivé conformément au procédé selon l'une quelconque des revendications 1 à 12 ou 13 à 20.


### SEQUENZPROTOKOLL

<110> Phytowelt GreenTechnologies GmbH
<120> Verfahren zur fermentativen alpha-Ionon Produktion
<130> PCT95229HV268pau
<140> noch nicht bekannt
   <141> 2014-08-29
<150> DE102014217346.9
   <151> 2014-08-29
<160> 47
<170> BiSSAP 1.3
<210> 1
   <211> 43
   <212> DNA
   <213> Artifizielle Sequenz
<220>
   <223> Primer
<220>
   <221> misc_feature
   <222> 1..8
   <223> /note="n ist a, c, g, or t"
<400> 1
   nnnnnnnnaa cgggattttt tgctgaaagg aggaactata tcc 43
<210> 2
   <211> 32
   <212> DNA
   <213> Artifizielle Sequenz
<220>
   <223> Primer
<220>
   <221> misc_feature
   <222> 1..10
   <223> /note="n ist a, c, g, or t"
<400> 2
   nnnnnnnnnn aacgggcttt gttagcagcc gg 32
<210> 3
   <211> 24
   <212> DNA
   <213> Artifizielle sequenz
<220>
   <223> Primer
<400> 3
   gtcttgcaca catagttcaa ttcg 24
<210> 4
   <211> 37
   <212> DNA
   <213> Artifizielle Sequenz
<220>
   <223> Primer
<400> 4
   ctatgtgtgc aagaccaaag agaaagaatg ctctctg 37
<210> 5
   <211> 32
   <212> DNA
   <213> Artifizielle Sequenz
<220>
   <223> Primer
<400> 5
   ctcttttctt tatacatgtt cgtcatttca cc 32
<210> 6
   <211> 29
   <212> DNA
   <213> Artifizielle Sequenz
<220>
   <223> Primer
<400> 6
   gtataaagaa aagagaacga gatctcctg 29
<210> 7
   <211> 30
   <212> DNA
   <213> Artifizielle Sequenz
<220>
   <223> Primer
<400> 7
   gtctttcaca catagttcaa ttcgataccg 30
<210> 8
   <211> 33
   <212> DNA
   <213> Artifizielle Sequenz
<220>
   <223> Primer
<400> 8
   ctatgtgtga aagaccaaag agaaagaatg ctc 33
<210> 9
   <211> 51
   <212> DNA
   <213> Artifizielle Sequenz
<220>
   <223> Primer
<220>
   <221> misc_feature
   <222> 22..23
   <223> /note="n ist a, c, g, or t"
<220>
   <221> misc_feature
   <222> 25..26
   <223> /note="n ist a, c, g, or t"
<400> 9
   gcattctttc tctttggtct tnnknnkata gttcaattcg ataccgaagg c 51
<210> 10
   <211> 23
   <212> DNA
   <213> Artifizielle Sequenz
<220>
   <223> Primer
<400> 10
   ccaaagagaa agaatgctct ctg 23
<210> 11
   <211> 6667
   <212> DNA
   <213> Artifizielle Sequenz
<220>
   <223> Nukleotid-Sequenz von pGT1036
<400> 11
   gcgctagcgg agtgtatact ggcttactat gttggcactg atgagggtgt cagtgaagtg 60
   cttcatgtgg caggagaaaa aaggctgcac cggtgcgtca gcagaatatg tgatacagga 120
   tatattccgc ttcctcgctc actgactcgc tacgctcggt cgttcgactg cggcgagcgg 180
   aaatggctta cgaacggggc ggagatttcc tggaagatgc caggaagata cttaacaggg 240
   aagtgagagg gccgcggcaa agccgttttt ccataggctc cgcccccctg acaagcatca 300
   cgaaatctga cgctcaaatc agtggtggcg aaacccgaca ggactataaa gataccaggc 360
   gtttccccct ggcggctccc tcgtgcgctc tcctgttcct gcctttcggt ttaccggtgt 420
   cattccgctg ttatggccgc gtttgtctca ttccacgcct gacactcagt tccgggtagg 480
   cagttcgctc caagctggac tgtatgcacg aaccccccgt tcagtccgac cgctgcgcct 540
   tatccggtaa ctatcgtctt gagtccaacc cggaaagaca tgcaaaagca ccactggcag 600
   cagccactgg taattgattt agaggagtta gtcttgaagt catgcgccgg ttaaggctaa 660
   actgaaagga caagttttgg tgactgcgct cctccaagcc agttacctcg gttcaaagag 720
   ttggtagctc agagaacctt cgaaaaaccg ccctgcaagg cggttttttc gttttcagag 780
   caagagatta cgcgcagacc aaaacgatct caagaagatc atcttattaa tcagataaaa 840
   tatttctaga tttcagtgca atttatctct tcaaatgtag cacctgaagt cagccccata 900
   cgatataagt tgtaattctc atgtttgaca gcttatcatc gataagcttt aatgcggtag 960
   tttatcacag ttaaattgct aacgcagtca ggaaccttgc aatggtgagt ggcagtaaag 1020
   cgggcgtttc gcctcatcgc gaaatagaag taatgagaca atccattgac gatcacctgg 1080
   ctggcctgtt acctgaaacc gacagccagg atatcgtcag ccttgcgatg cgtgaaggcg 1140
   tcatggcacc cggtaaacgg atccgtccgc tgctgatgct gctggccgcc cgcgacctcc 1200
   gctaccaggg cagtatgcct acgctgctcg atctcgcctg cgccgttgaa ctgacccata 1260
   ccgcgtcgct gatgctcgac gacatgccct gcatggacaa cgccgagctg cgccgcggtc 1320
   agcccactac ccacaaaaaa tttggtgaga gcgtggcgat ccttgcctcc gttgggctgc 1380
   tctctaaagc ctttggtctg atcgccgcca ccggcgatct gccgggggag aggcgtgccc 1440
   aggcggtcaa cgagctctct accgccgtgg gcgtgcaggg cctggtactg gggcagtttc 1500
   gcgatcttaa cgatgccgcc ctcgaccgta cccctgacgc tatcctcagc accaaccacc 1560
   tcaagaccgg cattctgttc agcgcgatgc tgcagatcgt cgccattgct tccgcctcgt 1620
   cgccgagcac gcgagagacg ctgcacgcct tcgccctcga cttcggccag gcgtttcaac 1680
   tgctggacga tctgcgtgac gatcacccgg aaaccggtaa agatcgcaat aaggacgcgg 1740
   gaaaatcgac gctggtcaac cggctgggcg cagacgcggc ccggcaaaag ctgcgcgagc 1800
   atattgattc cgccgacaaa cacctcactt ttgcctgtcc gcagggcggc gccatccgac 1860
   agtttatgca tctgtggttt ggccatcacc ttgccgactg gtcaccggtc atgaaaatcg 1920
   cctgataccg cccttttggg ttcaagcagt acataacgat ggaaccacat tacaggagta 1980
   gtgatgaatg aaggacgagc gccttgttca gcgtaagaac gatcatctgg atatcgttct 2040
   cgacccccgt cgcgccgtaa ctcaggctag cgcaggtttt gagcgctggc gctttaccca 2100
   ctgcgccctg ccagagctga attttagcga catcacgctg gaaaccacct tcctgaatcg 2160
   gcagctacag gctccgctgc tgatcagctc catgaccggc ggcgttgagc gctcgcgcca 2220
   tatcaaccgc cacctcgccg aggcggcgca ggtgctaaaa attgcgatgg gggtgggctc 2280
   ccagcgcgtc gccattgaga gcgacgcggg cttagggctg gataaaaccc tgcggcagct 2340
   ggctccggac gtgccgctgc tggcgaacct cggcgcggcg cagctgaccg gcagaaaagg 2400
   tattgattac gcccgacggg ccgtggagat gatcgaggcg gatgcgctga ttgtgcacct 2460
   aaacccgctg caggaggcgc tacagcccgg cggcgatcgc gactggcgcg gacggctggc 2520
   ggctattgaa actctggtcc gcgagctgcc cgttccgctg gtggtgaaag aggtgggagc 2580
   cggtatctcc cgaaccgtgg ccgggcagct gatcgatgcc ggcgttaccg tgattgacgt 2640
   cgcgggcgcg ggcggcacca gctgggccgc cgttgaaggc gagcgggcgg ccaccgagca 2700
   gcagcgcagc gtggccaacg tctttgccga ctgggggatc cccaccgctg aggcgctggt 2760
   tgacattgcc gaggcctggc cgcagatgcc ccttattgcc tcgggcggga ttaaaaacgg 2820
   cgtcgacgcg gcgaaagcgc tgcggctcgg cgcgtgcatg gtagggcagg ccgccgccgt 2880
   gctcggcagc gcaggcgtct ccacggagaa ggtgatcgat cacttcaacg tgattattga 2940
   gcagctgcgg gtggcctgct tctgcaccgg cagccgcagc ctgagcgatc taaagcaggc 3000
   tgatatccgc tatgttcgtg atacgccata aggaggtaca accatgaaga aaaccgttgt 3060
   gattggcgca ggctttggtg gcctggcgct ggcgattcgc ctgcaggcgg cagggatccc 3120
   aaccgtactg ctggagcagc gggacaagcc cggcggtcgg gcctacgtct ggcatgacca 3180
   gggctttacc tttgacgccg ggccgacggt gatcaccgat cctaccgcgc ttgaggcgct 3240
   gttcaccctg gccggcaggc gcatggagga ttacgtcagg ctgctgccgg taaaaccctt 3300
   ctaccgactc tgctgggagt ccgggaagac cctcgactat gctaacgaca gcgccgagct 3360
   tgaggcgcag attacccagt tcaacccccg cgacgtcgag ggctaccggc gctttctggc 3420
   ttactcccag gcggtattcc aggagggata tttgcgcctc ggcagcgtgc cgttcctctc 3480
   ttttcgcgac atgctgcgcg ccgggccgca gctgcttaag ctccaggcgt ggcagagcgt 3540
   ctaccagtcg gtttcgcgct ttattgagga tgagcatctg cggcaggcct tctcgttcca 3600
   ctccctgctg gtaggcggca accccttcac cacctcgtcc atctacaccc tgatccacgc 3660
   ccttgagcgg gagtgggggg tctggttccc tgagggcggc accggggcgc tggtgaacgg 3720
   catggtgaag ctgtttaccg atctgggcgg ggagatcgaa ctcaacgccc gggtcgaaga 3780
   gctggtggtg gccgataacc gcgtaagcca ggtccggctg gcggatggtc ggatctttga 3840
   caccgacgcc gtagcctcga acgctgacgt ggtgaacacc tataaaaagc tgctcggcca 3900
   ccatccggtg gggcagaagc gggcggcagc gctggagcgc aagagcatga gcaactcgct 3960
   gtttgtgctc tacttcggcc tgaaccagcc tcattcccag ctggcgcacc ataccatctg 4020
   ttttggtccc cgctaccggg agctgatcga cgagatcttt accggcagcg cgctggcgga 4080
   tgacttctcg ctctacctgc actcgccctg cgtgaccgat ccctcgctcg cgcctcccgg 4140
   ctgcgccagc ttctacgtgc tggccccggt gccgcatctt ggcaacgcgc cgctggactg 4200
   ggcgcaggag gggccgaagc tgcgcgaccg catctttgac taccttgaag agcgctatat 4260
   gcccggcctg cgtagccagc tggtgaccca gcggatcttt accccggcag acttccacga 4320
   cacgctggat gcgcatctgg gatcggcctt ctccatcgag ccgctgctga cccaaagcgc 4380
   ctggttccgc ccgcacaacc gcgacagcga cattgccaac ctctacctgg tgggcgcagg 4440
   tactcaccct ggggcgggca ttcctggcgt agtggcctcg gcgaaagcca ccgccagcct 4500
   gatgattgag gatctgcaat gagccaaccg ccgctgcttg accacgccac gcagaccatg 4560
   gccaacggct cgaaaagttt tgccaccgct gcgaagctgt tcgacccggc cacccgccgt 4620
   agcgtgctga tgctctacac ctggtgccgc cactgcgatg acgtcattga cgaccagacc 4680
   cacggcttcg ccagcgaggc cgcggcggag gaggaggcca cccagcgcct ggcccggctg 4740
   cgcacgctga ccctggcggc gtttgaaggg gccgagatgc aggatccggc cttcgctgcc 4800
   tttcaggagg tggcgctgac ccacggtatt acgccccgca tggcgctcga tcacctcgac 4860
   ggctttgcga tggacgtggc tcagacccgc tatgtcacct ttgaggatac gctgcgctac 4920
   tgctatcacg tggcgggcgt ggtgggtctg atgatggcca gggtgatggg cgtgcgggat 4980
   gagcgggtgc tggatcgcgc ctgcgatctg gggctggcct tccagctgac gaatatcgcc 5040
   cgggatatta ttgacgatgc ggctattgac cgctgctatc tgcccgccga gtggctgcag 5100
   gatgccgggc tgaccccgga gaactatgcc gcgcgggaga atcgggccgc gctggcgcgg 5160
   gtggcggagc ggcttattga tgccgcagag ccgtactaca tctcctccca ggccgggcta 5220
   cacgatctgc cgccgcgctg cgcctgggcg atcgccaccg cccgcagcgt ctaccgggag 5280
   atcggtatta aggtaaaagc ggcgggaggc agcgcctggg atcgccgcca gcacaccagc 5340
   aaaggtgaaa aaattgccat gctgatggcg gcaccggggc aggttattcg ggcgaagacg 5400
   acgagggtga cgccgcgtcc ggccggtctt tggcagcgtc ccgtttagcc cgttctgttt 5460
   aagaacggga ttttttgcgc ttgtttcggc gtgggtatgg tggcaggccc cgtggccggg 5520
   ggactgttgg gcgccatctc cttggggtcg aatttgcttt cgaatttctg ccattcatcc 5580
   gcttattatc acttattcag gcgtagcacc aggcgtttaa gggcaccaat aactgcctta 5640
   aaaaaattac gccccgccct gccactcatc gcagtactgt tgtaattcat taagcattct 5700
   gccgacatgg aagccatcac agacggcatg atgaacctga atcgccagcg gcatcagcac 5760
   cttgtcgcct tgcgtataat atttgcccat ggtgaaaacg ggggcgaaga agttgtccat 5820
   attggccacg tttaaatcaa aactggtgaa actcacccag ggattggctg agacgaaaaa 5880
   catattctca ataaaccctt tagggaaata ggccaggttt tcaccgtaac acgccacatc 5940
   ttgcgaatat atgtgtagaa actgccggaa atcgtcgtgg tattcactcc agagcgatga 6000
   aaacgtttca gtttgctcat ggaaaacggt gtaacaaggg tgaacactat cccatatcac 6060
   cagctcaccg tctttcattg ccatacggaa ttccggatga gcattcatca ggcgggcaag 6120
   aatgtgaata aaggccggat aaaacttgtg cttatttttc tttacggtct ttaaaaaggc 6180
   cgtaatatcc agctgaacgg tctggttata ggtacattga gcaactgact gaaatgcctc 6240
   aaaatgttct ttacgatgcc attgggatat atcaacggtg gtatatccag tgattttttt 6300
   ctccatttta gcttccttag ctcctgaaaa tctcgataac tcaaaaaata cgcccggtag 6360
   tgatcttatt tcattatggt gaaagttgga acctcttacg tgccgatcaa cgtctcattt      6420
   tcgccaaaag ttggcccagg gcttcccggt atcaacaggg acaccaggat ttatttattc      6480
   tgcgaagtga tcttccgtca caggtattta ttcggcgcaa agtgcgtcgg gtgatgctgc      6540
   caacttactg atttagtgta tgatggtgtt tttgaggtgc tccagtggct tctgtttcta      6600
   tcagctgtcc ctcctgttca gctactgacg gggtggtgcg taacggcaaa agcaccgccg       6660
   gacatca                                                                6667
<210> 12
   <211> 307
   <212> PRT
   <213> Erwinia herbicola
<220>
   <223> crtE
<400> 12
   Met Val Ser Gly Ser Lys Ala Gly Val Ser Pro His Arg Glu Ile Glu
   1               5                   10                  15
   Val Met Arg Gln Ser Ile Asp Asp His Leu Ala Gly Leu Leu Pro Glu
               20                  25                  30
   Thr Asp Ser Gln Asp Ile Val Ser Leu Ala Met Arg Glu Gly Val Met
           35                  40                  45
   Ala Pro Gly Lys Arg Ile Arg Pro Leu Leu Met Leu Leu Ala Ala Arg
       50                  55                  60
   Asp Leu Arg Tyr Gln Gly Ser Met Pro Thr Leu Leu Asp Leu Ala Cys
   65                  70                  75                  80
   Ala Val Glu Leu Thr His Thr Ala Ser Leu Met Leu Asp Asp Met Pro
                   85                  90                  95
   Cys Met Asp Asn Ala Glu Leu Arg Arg Gly Gln Pro Thr Thr His Lys
               100                 105                 110
   Lys Phe Gly Glu Ser Val Ala Ile Leu Ala Ser Val Gly Leu Leu Ser
           115                 120                 125
   Lys Ala Phe Gly Leu Ile Ala Ala Thr Gly Asp Leu Pro Gly Glu Arg
       130                 135                 140
   Arg Ala Gln Ala Val Asn Glu Leu Ser Thr Ala Val Gly Val Gln Gly
   145                 150                 155                 160
   Leu Val Leu Gly Gln Phe Arg Asp Leu Asn Asp Ala Ala Leu Asp Arg
                   165                 170                 175
   Thr Pro Asp Ala Ile Leu Ser Thr Asn His Leu Lys Thr Gly Ile Leu
               180                 185                 190
   Phe Ser Ala Met Leu Gln Ile Val Ala Ile Ala Ser Ala Ser Ser Pro
           195                 200                 205
   Ser Thr Arg Glu Thr Leu His Ala Phe Ala Leu Asp Phe Gly Gln Ala
       210                 215                 220
   Phe Gln Leu Leu Asp Asp Leu Arg Asp Asp His Pro Glu Thr Gly Lys
   225                 230                 235                 240
   Asp Arg Asn Lys Asp Ala Gly Lys Ser Thr Leu Val Asn Arg Leu Gly
                   245                 250                 255
   Ala Asp Ala Ala Arg Gln Lys Leu Arg Glu His Ile Asp Ser Ala Asp
               260                 265                 270
   Lys His Leu Thr Phe Ala Cys Pro Gln Gly Gly Ala Ile Arg Gln Phe
           275                 280                 285
   Met His Leu Trp Phe Gly His His Leu Ala Asp Trp Ser Pro Val Met
       290                 295                 300
   Lys Ile Ala
   305
<210> 13
   <211> 347
   <212> PRT
   <213> Erwinia herbicola
<220>
   <223> IPI
<400> 13
   Met Lys Asp Glu Arg Leu Val Gln Arg Lys Asn Asp His Leu Asp Ile
   1 5 10 15
   Val Leu Asp Pro Arg Arg Ala Val Thr Gln Ala Ser Ala Gly Phe Glu
   20 25 30
   Arg Trp Arg Phe Thr His Cys Ala Leu Pro Glu Leu Asn Phe Ser Asp
   35 40 45
   Ile Thr Leu Glu Thr Thr Phe Leu Asn Arg Gln Leu Gln Ala Pro Leu
   50 55 60
   Leu Ile Ser Ser Met Thr Gly Gly Val Glu Arg Ser Arg His Ile Asn
   65 70 75 80
   Arg His Leu Ala Glu Ala Ala Gln Val Leu Lys Ile Ala Met Gly Val
   85 90 95
   Gly Ser Gln Arg Val Ala Ile Glu Ser Asp Ala Gly Leu Gly Leu Asp
   100 105 110
   Lys Thr Leu Arg Gln Leu Ala Pro Asp Val Pro Leu Leu Ala Asn Leu
   115 120 125
   Gly Ala Ala Gln Leu Thr Gly Arg Lys Gly Ile Asp Tyr Ala Arg Arg
   130 135 140
   Ala Val Glu Met Ile Glu Ala Asp Ala Leu Ile Val His Leu Asn Pro
   145 150 155 160
   Leu Gln Glu Ala Leu Gln Pro Gly Gly Asp Arg Asp Trp Arg Gly Arg
   165 170 175
   Leu Ala Ala Ile Glu Thr Leu Val Arg Glu Leu Pro Val Pro Leu Val
   180 185 190
   Val Lys Glu Val Gly Ala Gly Ile Ser Arg Thr Val Ala Gly Gln Leu
   195 200 205
   Ile Asp Ala Gly Val Thr Val Ile Asp Val Ala Gly Ala Gly Gly Thr
   210 215 220
   Ser Trp Ala Ala Val Glu Gly Glu Arg Ala Ala Thr Glu Gln Gln Arg
   225 230 235 240
   Ser Val Ala Asn Val Phe Ala Asp Trp Gly Ile Pro Thr Ala Glu Ala
   245 250 255
   Leu Val Asp Ile Ala Glu Ala Trp Pro Gln Met Pro Leu Ile Ala Ser
   260 265 270
   Gly Gly Ile Lys Asn Gly Val Asp Ala Ala Lys Ala Leu Arg Leu Gly
   275 280 285
   Ala Cys Met Val Gly Gln Ala Ala Ala Val Leu Gly Ser Ala Gly Val
   290 295 300
   Ser Thr Glu Lys Val Ile Asp His Phe Asn Val Ile Ile Glu Gln Leu
   305 310 315 320
   Arg Val Ala Cys Phe Cys Thr Gly Ser Arg Ser Leu Ser Asp Leu Lys
   325 330 335
   Gln Ala Asp Ile Arg Tyr Val Arg Asp Thr Pro
   340 345
<210> 14
   <211> 492
   <212> PRT
   <213> Erwinia herbicola
<220>
   <223> crtI
<400> 14
   Met Lys Lys Thr Val Val Ile Gly Ala Gly Phe Gly Gly Leu Ala Leu
   1               5                   10                  15
   Ala Ile Arg Leu Gln Ala Ala Gly Ile Pro Thr Val Leu Leu Glu Gln
               20                  25                  30
   Arg Asp Lys Pro Gly Gly Arg Ala Tyr Val Trp His Asp Gln Gly Phe
           35                  40                  45
   Thr Phe Asp Ala Gly Pro Thr Val Ile Thr Asp Pro Thr Ala Leu Glu
       50                  55                  60
   Ala Leu Phe Thr Leu Ala Gly Arg Arg Met Glu Asp Tyr Val Arg Leu
   65                  70                  75                  80
   Leu Pro Val Lys Pro Phe Tyr Arg Leu Cys Trp Glu Ser Gly Lys Thr
                   85                  90                  95
   Leu Asp Tyr Ala Asn Asp Ser Ala Glu Leu Glu Ala Gln Ile Thr Gln
               100                 105                 110
   Phe Asn Pro Arg Asp Val Glu Gly Tyr Arg Arg Phe Leu Ala Tyr Ser
           115                 120                 125
   Gln Ala Val Phe Gln Glu Gly Tyr Leu Arg Leu Gly Ser Val Pro Phe
       130                 135                 140
   Leu Ser Phe Arg Asp Met Leu Arg Ala Gly Pro Gln Leu Leu Lys Leu
   145                 150                 155                 160
   Gln Ala Trp Gln Ser Val Tyr Gln Ser Val Ser Arg Phe Ile Glu Asp
                   165                 170                 175
   Glu His Leu Arg Gln Ala Phe Ser Phe His Ser Leu Leu Val Gly Gly
               180                 185                 190
   Asn Pro Phe Thr Thr Ser Ser Ile Tyr Thr Leu Ile His Ala Leu Glu
           195                 200                 205
   Arg Glu Trp Gly Val Trp Phe Pro Glu Gly Gly Thr Gly Ala Leu Val
       210                 215                 220
   Asn Gly Met Val Lys Leu Phe Thr Asp Leu Gly Gly Glu Ile Glu Leu
   225                 230                 235                 240
   Asn Ala Arg Val Glu Glu Leu Val Val Ala Asp Asn Arg Val Ser Gln
                   245                 250                 255
   Val Arg Leu Ala Asp Gly Arg Ile Phe Asp Thr Asp Ala Val Ala Ser
               260                 265                 270
   Asn Ala Asp Val Val Asn Thr Tyr Lys Lys Leu Leu Gly His His Pro
           275                 280                 285
   Val Gly Gln Lys Arg Ala Ala Ala Leu Glu Arg Lys Ser Met Ser Asn
       290                 295                 300
   Ser Leu Phe Val Leu Tyr Phe Gly Leu Asn Gln Pro His Ser Gln Leu
   305                 310                 315                 320
   Ala His His Thr Ile Cys Phe Gly Pro Arg Tyr Arg Glu Leu Ile Asp
                   325                 330                 335
   Glu Ile Phe Thr Gly Ser Ala Leu Ala Asp Asp Phe Ser Leu Tyr Leu
               340                 345                 350
   His Ser Pro Cys Val Thr Asp Pro Ser Leu Ala Pro Pro Gly Cys Ala
           355                 360                 365
   Ser Phe Tyr Val Leu Ala Pro Val Pro His Leu Gly Asn Ala Pro Leu
       370                 375                 380
   Asp Trp Ala Gln Glu Gly Pro Lys Leu Arg Asp Arg Ile Phe Asp Tyr
   385                 390                 395                 400
   Leu Glu Glu Arg Tyr Met Pro Gly Leu Arg Ser Gln Leu Val Thr Gln
                   405                 410                 415
   Arg Ile Phe Thr Pro Ala Asp Phe His Asp Thr Leu Asp Ala His Leu
               420                 425                 430
   Gly Ser Ala Phe Ser Ile Glu Pro Leu Leu Thr Gln Ser Ala Trp Phe
           435                 440                 445
   Arg Pro His Asn Arg Asp Ser Asp Ile Ala Asn Leu Tyr Leu Val Gly
       450                 455                 460
   Ala Gly Thr His Pro Gly Ala Gly Ile Pro Gly Val Val Ala Ser Ala
   465                 470                 475                 480
   Lys Ala Thr Ala Ser Leu Met Ile Glu Asp Leu Gln
                   485                 490
<210> 15
   <211> 309
   <212> PRT
   <213> Erwinia herbicola
<220>
   <223> crtB
<400> 15
   Met Ser Gln Pro Pro Leu Leu Asp His Ala Thr Gln Thr Met Ala Asn
   1               5                   10                  15
   Gly Ser Lys Ser Phe Ala Thr Ala Ala Lys Leu Phe Asp Pro Ala Thr
               20                  25                  30
   Arg Arg Ser Val Leu Met Leu Tyr Thr Trp Cys Arg His Cys Asp Asp
           35                  40                  45
   Val Ile Asp Asp Gln Thr His Gly Phe Ala Ser Glu Ala Ala Ala Glu
       50                  55                  60
   Glu Glu Ala Thr Gln Arg Leu Ala Arg Leu Arg Thr Leu Thr Leu Ala
   65                  70                  75                  80
   Ala Phe Glu Gly Ala Glu Met Gln Asp Pro Ala Phe Ala Ala Phe Gln
                   85                  90                  95
   Glu Val Ala Leu Thr His Gly Ile Thr Pro Arg Met Ala Leu Asp His
               100                 105                 110
   Leu Asp Gly Phe Ala Met Asp Val Ala Gln Thr Arg Tyr Val Thr Phe
           115                 120                 125
   Glu Asp Thr Leu Arg Tyr Cys Tyr His Val Ala Gly Val Val Gly Leu
       130                 135                 140
   Met Met Ala Arg Val Met Gly Val Arg Asp Glu Arg Val Leu Asp Arg
   145                 150                 155                 160
   Ala Cys Asp Leu Gly Leu Ala Phe Gln Leu Thr Asn Ile Ala Arg Asp
                   165                 170                 175
   Ile Ile Asp Asp Ala Ala Ile Asp Arg Cys Tyr Leu Pro Ala Glu Trp
               180                 185                 190
   Leu Gln Asp Ala Gly Leu Thr Pro Glu Asn Tyr Ala Ala Arg Glu Asn
           195                 200                 205
   Arg Ala Ala Leu Ala Arg Val Ala Glu Arg Leu Ile Asp Ala Ala Glu
       210                 215                 220
   Pro Tyr Tyr Ile Ser Ser Gln Ala Gly Leu His Asp Leu Pro Pro Arg
   225                 230                 235                 240
   Cys Ala Trp Ala Ile Ala Thr Ala Arg Ser Val Tyr Arg Glu Ile Gly
                   245                 250                 255
   Ile Lys Val Lys Ala Ala Gly Gly Ser Ala Trp Asp Arg Arg Gln His
               260                 265                 270
   Thr Ser Lys Gly Glu Lys Ile Ala Met Leu Met Ala Ala Pro Gly Gln
           275                 280                 285
   Val Ile Arg Ala Lys Thr Thr Arg Val Thr Pro Arg Pro Ala Gly Leu
       290                 295                 300
   Trp Gln Arg Pro Val
   305
<210> 16
   <211> 219
   <212> PRT
   <213> Escherichia coli
<220>
   <223> CmR
<400> 16
   Met Glu Lys Lys Ile Thr Gly Tyr Thr Thr Val Asp Ile Ser Gln Trp
   1               5                   10                  15
   His Arg Lys Glu His Phe Glu Ala Phe Gln Ser Val Ala Gln Cys Thr
               20                  25                  30
   Tyr Asn Gln Thr Val Gln Leu Asp Ile Thr Ala Phe Leu Lys Thr Val
           35                  40                  45
   Lys Lys Asn Lys His Lys Phe Tyr Pro Ala Phe Ile His Ile Leu Ala
       50                  55                  60
   Arg Leu Met Asn Ala His Pro Glu Phe Arg Met Ala Met Lys Asp Gly
   65                  70                  75                  80
   Glu Leu Val Ile Trp Asp Ser Val His Pro Cys Tyr Thr Val Phe His
                   85                  90                  95
   Glu Gln Thr Glu Thr Phe Ser Ser Leu Trp Ser Glu Tyr His Asp Asp
               100                 105                 110
   Phe Arg Gln Phe Leu His Ile Tyr Ser Gln Asp Val Ala Cys Tyr Gly
           115                 120                 125
   Glu Asn Leu Ala Tyr Phe Pro Lys Gly Phe Ile Glu Asn Met Phe Phe
       130                 135                 140
   Val Ser Ala Asn Pro Trp Val Ser Phe Thr Ser Phe Asp Leu Asn Val
   145                 150                 155                 160
   Ala Asn Met Asp Asn Phe Phe Ala Pro Val Phe Thr Met Gly Lys Tyr
                   165                 170                 175
   Tyr Thr Gln Gly Asp Lys Val Leu Met Pro Leu Ala Ile Gln Val His
               180                 185                 190
   His Ala Val Cys Asp Gly Phe His Val Gly Arg Met Leu Asn Glu Leu
           195                 200                 205
   Gln Gln Tyr Cys Asp Glu Trp Gln Gly Gly Ala
       210                 215
<210> 17
   <211> 8632
   <212> DNA
   <213> Artifizielle Sequenz
<220>
   <223> Nukleotid-Sequenz von pGT1066 (ECmut3)
<400> 17
<210> 18
   <211> 8632
   <212> DNA
   <213> Artifizielle Sequenz
<220>
   <223> Nukleotid-Sequenz von pGT1066*
<220>
   <221> misc_feature
   <222> 7425..7430
   <223> /note="n ist a, c, g, or t"
<220>
   <221> misc_feature
   <222> 7551..7553
   <223> /note="n ist a, c, g, or t"
<400> 18
   ttgtaattct catgtttgac agcttatcat cgataagctt taatgcggta gtttatcaca      60
   gttaaattgc taacgcagtc aggaaccttg caatggtgag tggcagtaaa gcgggcgttt     120
   cgcctcatcg cgaaatagaa gtaatgagac aatccattga cgatcacctg gctggcctgt     180
   tacctgaaac cgacagccag gatatcgtca gccttgcgat gcgtgaaggc gtcatggcac     240
   ccggtaaacg gatccgtccg ctgctgatgc tgctggccgc ccgcgacctc cgctaccagg     300
   gcagtatgcc tacgctgctc gatctcgcct gcgccgttga actgacccat accgcgtcgc     360
   tgatgctcga cgacatgccc tgcatggaca acgccgagct gcgccgcggt cagcccacta     420
   cccacaaaaa atttggtgag agcgtggcga tccttgcctc cgttgggctg ctctctaaag     480
   cctttggtct gatcgccgcc accggcgatc tgccggggga gaggcgtgcc caggcggtca     540
   acgagctctc taccgccgtg ggcgtgcagg gcctggtact ggggcagttt cgcgatctta     600
   acgatgccgc cctcgaccgt acccctgacg ctatcctcag caccaaccac ctcaagaccg     660
   gcattctgtt cagcgcgatg ctgcagatcg tcgccattgc ttccgcctcg tcgccgagca     720
   cgcgagagac gctgcacgcc ttcgccctcg acttcggcca ggcgtttcaa ctgctggacg     780
   atctgcgtga cgatcacccg gaaaccggta aagatcgcaa taaggacgcg ggaaaatcga     840
   cgctggtcaa ccggctgggc gcagacgcgg cccggcaaaa gctgcgcgag catattgatt     900
   ccgccgacaa acacctcact tttgcctgtc cgcagggcgg cgccatccga cagtttatgc     960
   atctgtggtt tggccatcac cttgccgact ggtcaccggt catgaaaatc gcctgatacc    1020
   gcccttttgg gttcaagcag tacataacga tggaaccaca ttacaggagt agtgatgaat    1080
   gaaggacgag cgccttgttc agcgtaagaa cgatcatctg gatatcgttc tcgacccccg 1140
   tcgcgccgta actcaggcta gcgcaggttt tgagcgctgg cgctttaccc actgcgccct 1200
   gccagagctg aattttagcg acatcacgct ggaaaccacc ttcctgaatc ggcagctaca 1260
   ggctccgctg ctgatcagct ccatgaccgg cggcgttgag cgctcgcgcc atatcaaccg 1320
   ccacctcgcc gaggcggcgc aggtgctaaa aattgcgatg ggggtgggct cccagcgcgt 1380
   cgccattgag agcgacgcgg gcttagggct ggataaaacc ctgcggcagc tggctccgga 1440
   cgtgccgctg ctggcgaacc tcggcgcggc gcagctgacc ggcagaaaag gtattgatta 1500
   cgcccgacgg gccgtggaga tgatcgaggc ggatgcgctg attgtgcacc taaacccgct 1560
   gcaggaggcg ctacagcccg gcggcgatcg cgactggcgc ggacggctgg cggctattga 1620
   aactctggtc cgcgagctgc ccgttccgct ggtggtgaaa gaggtgggag ccggtatctc 1680
   ccgaaccgtg gccgggcagc tgatcgatgc cggcgttacc gtgattgacg tcgcgggcgc 1740
   gggcggcacc agctgggccg ccgttgaagg cgagcgggcg gccaccgagc agcagcgcag 1800
   cgtggccaac gtctttgccg actgggggat ccccaccgct gaggcgctgg ttgacattgc 1860
   cgaggcctgg ccgcagatgc cccttattgc ctcgggcggg attaaaaacg gcgtcgacgc 1920
   ggcgaaagcg ctgcggctcg gcgcgtgcat ggtagggcag gccgccgccg tgctcggcag 1980
   cgcaggcgtc tccacggaga aggtgatcga tcacttcaac gtgattattg agcagctgcg 2040
   ggtggcctgc ttctgcaccg gcagccgcag cctgagcgat ctaaagcagg ctgatatccg 2100
   ctatgttcgt gatacgccat aaggaggtac aaccatgaag aaaaccgttg tgattggcgc 2160
   aggctttggt ggcctggcgc tggcgattcg cctgcaggcg gcagggatcc caaccgtact 2220
   gctggagcag cgggacaagc ccggcggtcg ggcctacgtc tggcatgacc agggctttac 2280
   ctttgacgcc gggccgacgg tgatcaccga tcctaccgcg cttgaggcgc tgttcaccct 2340
   ggccggcagg cgcatggagg attacgtcag gctgctgccg gtaaaaccct tctaccgact 2400
   ctgctgggag tccgggaaga ccctcgacta tgctaacgac agcgccgagc ttgaggcgca 2460
   gattacccag ttcaaccccc gcgacgtcga gggctaccgg cgctttctgg cttactccca 2520
   ggcggtattc caggagggat atttgcgcct cggcagcgtg ccgttcctct cttttcgcga 2580
   catgctgcgc gccgggccgc agctgcttaa gctccaggcg tggcagagcg tctaccagtc 2640
   ggtttcgcgc tttattgagg atgagcatct gcggcaggcc ttctcgttcc actccctgct 2700
   ggtaggcggc aaccccttca ccacctcgtc catctacacc ctgatccacg cccttgagcg 2760
   ggagtggggg gtctggttcc ctgagggcgg caccggggcg ctggtgaacg gcatggtgaa 2820
   gctgtttacc gatctgggcg gggagatcga actcaacgcc cgggtcgaag agctggtggt 2880
   ggccgataac cgcgtaagcc aggtccggct ggcggatggt cggatctttg acaccgacgc 2940
   cgtagcctcg aacgctgacg tggtgaacac ctataaaaag ctgctcggcc accatccggt 3000
   ggggcagaag cgggcggcag cgctggagcg caagagcatg agcaactcgc tgtttgtgct 3060
   ctacttcggc ctgaaccagc ctcattccca gctggcgcac cataccatct gttttggtcc 3120
   ccgctaccgg gagctgatcg acgagatctt taccggcagc gcgctggcgg atgacttctc 3180
   gctctacctg cactcgccct gcgtgaccga tccctcgctc gcgcctcccg gctgcgccag 3240
   cttctacgtg ctggccccgg tgccgcatct tggcaacgcg ccgctggact gggcgcagga 3300
   ggggccgaag ctgcgcgacc gcatctttga ctaccttgaa gagcgctata tgcccggcct 3360
   gcgtagccag ctggtgaccc agcggatctt taccccggca gacttccacg acacgctgga 3420
   tgcgcatctg ggatcggcct tctccatcga gccgctgctg acccaaagcg cctggttccg 3480
   cccgcacaac cgcgacagcg acattgccaa cctctacctg gtgggcgcag gtactcaccc 3540
   tggggcgggc attcctggcg tagtggcctc ggcgaaagcc accgccagcc tgatgattga 3600
   ggatctgcaa tgagccaacc gccgctgctt gaccacgcca cgcagaccat ggccaacggc 3660
   tcgaaaagtt ttgccaccgc tgcgaagctg ttcgacccgg ccacccgccg tagcgtgctg 3720
   atgctctaca cctggtgccg ccactgcgat gacgtcattg acgaccagac ccacggcttc 3780
   gccagcgagg ccgcggcgga ggaggaggcc acccagcgcc tggcccggct gcgcacgctg 3840
   accctggcgg cgtttgaagg ggccgagatg caggatccgg ccttcgctgc ctttcaggag 3900
   gtggcgctga cccacggtat tacgccccgc atggcgctcg atcacctcga cggctttgcg 3960
   atggacgtgg ctcagacccg ctatgtcacc tttgaggata cgctgcgcta ctgctatcac 4020
   gtggcgggcg tggtgggtct gatgatggcc agggtgatgg gcgtgcggga tgagcgggtg 4080
   ctggatcgcg cctgcgatct ggggctggcc ttccagctga cgaatatcgc ccgggatatt 4140
   attgacgatg cggctattga ccgctgctat ctgcccgccg agtggctgca ggatgccggg 4200
   ctgaccccgg agaactatgc cgcgcgggag aatcgggccg cgctggcgcg ggtggcggag 4260
   cggcttattg atgccgcaga gccgtactac atctcctccc aggccgggct acacgatctg 4320
   ccgccgcgct gcgcctgggc gatcgccacc gcccgcagcg tctaccggga gatcggtatt 4380
   aaggtaaaag cggcgggagg cagcgcctgg gatcgccgcc agcacaccag caaaggtgaa 4440
   aaaattgcca tgctgatggc ggcaccgggg caggttattc gggcgaagac gacgagggtg 4500
   acgccgcgtc cggccggtct ttggcagcgt cccgtttaga ccgactccaa acgagtcggt 4560
   ttttttgcgc ttgtttcggc gtgggtatgg tggcaggccc cgtggccggg ggactgttgg 4620
   gcgccatctc cttggggtcg aatttgcttt cgaatttctg ccattcatcc gcttattatc 4680
   acttattcag gcgtagcaac caggcgttta agggcaccaa taactgcctt aaaaaaatta 4740
   cgccccgccc tgccactcat cgcagtactg ttgtaattca ttaagcattc tgccgacatg 4800
   gaagccatca cagacggcat gatgaacctg aatcgccagc ggcatcagca ccttgtcgcc 4860
   ttgcgtataa tatttgccca tggtgaaaac gggggcgaag aagttgtcca tattggccac 4920
   gtttaaatca aaactggtga aactcaccca gggattggct gagacgaaaa acatattctc 4980
   aataaaccct ttagggaaat aggccaggtt ttcaccgtaa cacgccacat cttgcgaata 5040
   tatgtgtaga aactgccgga aatcgtcgtg gtattcactc cagagcgatg aaaacgtttc 5100
   agtttgctca tggaaaacgg tgtaacaagg gtgaacacta tcccatatca ccagctcacc 5160
   gtctttcatt gccatacgga attccggatg agcattcatc aggcgggcaa gaatgtgaat 5220
   aaaggccgga taaaacttgt gcttattttt ctttacggtc tttaaaaagg ccgtaatatc 5280
   cagctgaacg gtctggttat aggtacattg agcaactgac tgaaatgcct caaaatgttc 5340
   tttacgatgc cattgggata tatcaacggt ggtatatcca gtgatttttt tctccatttt 5400
   agcttcctta gctcctgaaa atctcgataa ctcaaaaaat acgcccggta gtgatcttat 5460
   ttcattatgg tgaaagttgg aacctcttac gtgccgatca acgtctcatt ttcgccaaaa 5520
   gttggcccag ggcttcccgg tatcaacagg gacaccagga tttatttatt ctgcgaagtg 5580
   atcttccgtc acaggtattt attcggcgca aagtgcgtcg ggtgatgctg ccaacttact 5640
   gatttagtgt atgatggtgt ttttgaggtg ctccagtggc ttctgtttct atcagctgtc 5700
   cctcctgttc agctactgac ggggtggtgc gtaacggcaa aagcaccgcc ggacatcagc 5760
   gctagcggag tgtatacact ccgctatcgc tacgtgactg ggtcatggct gcgccccgac 5820
   acccgccaac acccgctgac gcgccctgac gggcttgtct gctcccggca tccgcttaca 5880
   gacaagctgt gaccgtctcc gggagctgca tgtgtcagag gttttcaccg tcatcaccga 5940
   aacgcgcgag gcagctggca cgacaggttt cccgactgga aagcgggcag tgagcgcaac 6000
   gcaattaatg tgagttagct cactcattag gcaccccagg ctttacactt tatgcttccg 6060
   gctcgtatgt tgtgtggaat tgtgagcgga taacaatttc acacaggaaa cagctatgac 6120
   catgattacg ccaagctcta gctagaaata attttgttta actttaagaa ggagatatac 6180
   ccatgacaca gagggcccac catcaccatc accattccat ggctagcggc ggcggaagtt 6240
   ccggtagtga gagttgtgta gcggtgagag aagatttcgc tgacgaagaa gattttgtga 6300
   aagctggtgg ttctgagatt ctatttgttc aaatgcagca gaacaaagat atggatgaac 6360
   agtctaagct tgttgataag ttgcctccta tatcaattgg tgatggtgct ttggatctag 6420
   tggttattgg ttgtggtcct gctggtttag ccttggctgc agaatcagct aagcttggat 6480
   taaaagttgg actcattggt ccagatcttc cttttactaa caattacggt gtttgggaag 6540
   atgaattcaa tgatcttggg ctgcaaaaat gtattgagca tgtttggaga gagactattg 6600
   tgtatctgga tgatgacaag cctattacca ttggccgtgc ttatggaaga gttagtcgac 6660
   gtttgctcca tgaggagctt ttgaggaggt gtgtcgagtc aggtgtctcg taccttagct 6720
   cgaaagttga cagcataaca gaagcttctg atggccttag acttgttgct tgtgacgaca 6780
   ataacgtcat tccctgcagg cttgccactg ttgcttctgg agcagcttcg ggaaagctct 6840
   tgcaatacga agttggtgga cctagagtct gtgtgcaaac tgcatacggc gtggaggttg 6900
   aggtggaaaa tagtccatat gatccagatc aaatggtttt catggattac agagattata 6960
   ctaacgagaa agttcggagc ttagaagctg agtatccaac gtttctgtac gccatgccta 7020
   tgacaaagtc aagactcttc ttcgaggaga catgtttggc ctcaaaagat gtcatgccct 7080
   ttgatttgct aaaaacgaag ctcatgttaa gattagatac actcggaatt cgaattctaa 7140
   agacttacga agaggagtgg tcctatatcc cagttggtgg ttccttgcca aacaccgaac 7200
   aaaagaatct cgcctttggt gctgccgcta gcatggtaca tcccgcaaca ggctattcag 7260
   ttgtgagatc tttgtctgaa gctccaaaat atgcatcagt catcgcagag atactaagag 7320
   aagagactac caaacagatc aacagtaata tttcaagaca agcttgggat actttatggc 7380
   caccagaaag gaaaagacag agagcattct ttctctttgg tcttnnnnnn atagttcaat 7440
   tcgataccga aggcattaga agcttcttcc gtactttctt ccgccttcca aaatggatgt 7500
   ggcaagggtt tctaggatca acattaacat caggagatct cgttctcttt nnnttataca 7560
   tgttcgtcat ttcaccaaac aatttgagaa aaggtctcat caatcatctc atctctgatc 7620
   caaccggagc aaccatgata aaaacctatc tcaaagtatg aggatccggc tgctaacaaa 7680
   gcccgttctg tttaagaacg ggattttttg ctgaaaggag gaactatatc cggccggatt 7740
   actggcttac tatgttggca ctgatgaggg tgtcagtgaa gtgcttcatg tggcaggaga 7800
   aaaaaggctg caccggtgcg tcagcagaat atgtgataca ggatatattc cgcttcctcg 7860
   ctcactgact cgctacgctc ggtcgttcga ctgcggcgag cggaaatggc ttacgaacgg 7920
   ggcggagatt tcctggaaga tgccaggaag atacttaaca gggaagtgag agggccgcgg 7980
   caaagccgtt tttccatagg ctccgccccc ctgacaagca tcacgaaatc tgacgctcaa 8040
   atcagtggtg gcgaaacccg acaggactat aaagatacca ggcgtttccc cctggcggct 8100
   ccctcgtgcg ctctcctgtt cctgcctttc ggtttaccgg tgtcattccg ctgttatggc 8160
   cgcgtttgtc tcattccacg cctgacactc agttccgggt aggcagttcg ctccaagctg 8220
   gactgtatgc acgaaccccc cgttcagtcc gaccgctgcg ccttatccgg taactatcgt 8280
   cttgagtcca acccggaaag acatgcaaaa gcaccactgg cagcagccac tggtaattga 8340
   tttagaggag ttagtcttga agtcatgcgc cggttaaggc taaactgaaa ggacaagttt 8400
   tggtgactgc gctcctccaa gccagttacc tcggttcaaa gagttggtag ctcagagaac 8460
   cttcgaaaaa ccgccctgca aggcggtttt ttcgttttca gagcaagaga ttacgcgcag 8520
   accaaaacga tctcaagaag atcatcttat taatcagata aaatatttct agatttcagt 8580
   gcaatttatc tcttcaaatg tagcacctga agtcagcccc atacgatata ag 8632
<210> 19
   <211> 480
   <212> PRT
   <213> Artifizielle Sequenz
<220>
   <223> Proteinvariante AtEC-del
<400> 19
   Met Ala Ser Gly Gly Gly Ser Ser Gly Ser Glu Ser Cys Val Ala Val
   1 5 10 15
   Arg Glu Asp Phe Ala Asp Glu Glu Asp Phe Val Lys Ala Gly Gly Ser
   20 25 30
   Glu Ile Leu Phe Val Gln Met Gln Gln Asn Lys Asp Met Asp Glu Gln
   35 40 45
   Ser Lys Leu Val Asp Lys Leu Pro Pro Ile Ser Ile Gly Asp Gly Ala
   50 55 60
   Leu Asp Leu Val Val Ile Gly Cys Gly Pro Ala Gly Leu Ala Leu Ala
   65 70 75 80
   Ala Glu Ser Ala Lys Leu Gly Leu Lys Val Gly Leu Ile Gly Pro Asp
   85 90 95
   Leu Pro Phe Thr Asn Asn Tyr Gly Val Trp Glu Asp Glu Phe Asn Asp
   100 105 110
   Leu Gly Leu Gln Lys Cys Ile Glu His Val Trp Arg Glu Thr Ile Val
   115 120 125
   Tyr Leu Asp Asp Asp Lys Pro Ile Thr Ile Gly Arg Ala Tyr Gly Arg
   130 135 140
   Val Ser Arg Arg Leu Leu His Glu Glu Leu Leu Arg Arg Cys Val Glu
   145 150 155 160
   Ser Gly Val Ser Tyr Leu Ser Ser Lys Val Asp Ser Ile Thr Glu Ala
   165 170 175
   Ser Asp Gly Leu Arg Leu Val Ala Cys Asp Asp Asn Asn Val Ile Pro
   180 185 190
   Cys Arg Leu Ala Thr Val Ala Ser Gly Ala Ala Ser Gly Lys Leu Leu
   195 200 205
   Gln Tyr Glu Val Gly Gly Pro Arg Val Cys Val Gln Thr Ala Tyr Gly
   210 215 220
   Val Glu Val Glu Val Glu Asn Ser Pro Tyr Asp Pro Asp Gln Met Val
   225 230 235 240
   Phe Met Asp Tyr Arg Asp Tyr Thr Asn Glu Lys Val Arg Ser Leu Glu
   245 250 255
   Ala Glu Tyr Pro Thr Phe Leu Tyr Ala Met Pro Met Thr Lys Ser Arg
   260 265 270
   Leu Phe Phe Glu Glu Thr Cys Leu Ala Ser Lys Asp Val Met Pro Phe
   275 280 285
   Asp Leu Leu Lys Thr Lys Leu Met Leu Arg Leu Asp Thr Leu Gly Ile
   290 295 300
   Arg Ile Leu Lys Thr Tyr Glu Glu Glu Trp Ser Tyr Ile Pro Val Gly
   305 310 315 320
   Gly Ser Leu Pro Asn Thr Glu Gln Lys Asn Leu Ala Phe Gly Ala Ala
   325 330 335
   Ala Ser Met Val His Pro Ala Thr Gly Tyr Ser Val Val Arg Ser Leu
   340 345 350
   Ser Glu Ala Pro Lys Tyr Ala Ser Val Ile Ala Glu Ile Leu Arg Glu
   355 360 365
   Glu Thr Thr Lys Gln Ile Asn Ser Asn Ile Ser Arg Gln Ala Trp Asp
   370 375 380
   Thr Leu Trp Pro Pro Glu Arg Lys Arg Gln Arg Ala Phe Phe Leu Phe
   385 390 395 400
   Gly Leu Ala Leu Ile Val Gln Phe Asp Thr Glu Gly Ile Arg Ser Phe
   405 410 415
   Phe Arg Thr Phe Phe Arg Leu Pro Lys Trp Met Trp Gln Gly Phe Leu
   420 425 430
   Gly Ser Thr Leu Thr Ser Gly Asp Leu Val Leu Phe Ala Leu Tyr Met
   435 440 445
   Phe Val Ile Ser Pro Asn Asn Leu Arg Lys Gly Leu Ile Asn His Leu
   450 455 460
   Ile Ser Asp Pro Thr Gly Ala Thr Met Ile Lys Thr Tyr Leu Lys Val
   465 470 475 480
<210> 20
   <211> 480
   <212> PRT
   <213> Artifizielle Sequenz
<220>
   <223> Proteinvariante AtECmut3
<400> 20
   Met Ala Ser Gly Gly Gly Ser Ser Gly Ser Glu Ser Cys Val Ala Val
   1               5                   10                  15
   Arg Glu Asp Phe Ala Asp Glu Glu Asp Phe Val Lys Ala Gly Gly Ser
               20                  25                  30
   Glu Ile Leu Phe Val Gln Met Gln Gln Asn Lys Asp Met Asp Glu Gln
           35                  40                  45
   Ser Lys Leu Val Asp Lys Leu Pro Pro Ile Ser Ile Gly Asp Gly Ala
       50                  55                  60
   Leu Asp Leu Val Val Ile Gly Cys Gly Pro Ala Gly Leu Ala Leu Ala
   65                  70                  75                  80
   Ala Glu Ser Ala Lys Leu Gly Leu Lys Val Gly Leu Ile Gly Pro Asp
                   85                  90                  95
   Leu Pro Phe Thr Asn Asn Tyr Gly Val Trp Glu Asp Glu Phe Asn Asp
               100                 105                 110
   Leu Gly Leu Gln Lys Cys Ile Glu His Val Trp Arg Glu Thr Ile Val
           115                 120                 125
   Tyr Leu Asp Asp Asp Lys Pro Ile Thr Ile Gly Arg Ala Tyr Gly Arg
       130                 135                 140
   Val Ser Arg Arg Leu Leu His Glu Glu Leu Leu Arg Arg Cys Val Glu
   145                 150                 155                 160
   Ser Gly Val Ser Tyr Leu Ser Ser Lys Val Asp Ser Ile Thr Glu Ala
                   165                 170                 175
   Ser Asp Gly Leu Arg Leu Val Ala Cys Asp Asp Asn Asn Val Ile Pro
               180                 185                 190
   Cys Arg Leu Ala Thr Val Ala Ser Gly Ala Ala Ser Gly Lys Leu Leu
           195                 200                 205
   Gln Tyr Glu Val Gly Gly Pro Arg Val Cys Val Gln Thr Ala Tyr Gly
       210                 215                 220
   Val Glu Val Glu Val Glu Asn Ser Pro Tyr Asp Pro Asp Gln Met Val
   225                 230                 235                 240
   Phe Met Asp Tyr Arg Asp Tyr Thr Asn Glu Lys Val Arg Ser Leu Glu
                   245                 250                 255
   Ala Glu Tyr Pro Thr Phe Leu Tyr Ala Met Pro Met Thr Lys Ser Arg
               260                 265                 270
   Leu Phe Phe Glu Glu Thr Cys Leu Ala Ser Lys Asp Val Met Pro Phe
           275                 280                 285
   Asp Leu Leu Lys Thr Lys Leu Met Leu Arg Leu Asp Thr Leu Gly Ile
       290                 295                 300
   Arg Ile Leu Lys Thr Tyr Glu Glu Glu Trp Ser Tyr Ile Pro Val Gly
   305                 310                 315                 320
   Gly Ser Leu Pro Asn Thr Glu Gln Lys Asn Leu Ala Phe Gly Ala Ala
                   325                 330                 335
   Ala Ser Met Val His Pro Ala Thr Gly Tyr Ser Val Val Arg Ser Leu
                   340                 345                 350
   Ser Glu Ala Pro Lys Tyr Ala Ser Val Ile Ala Glu Ile Leu Arg Glu
               355                 360                 365
   Glu Thr Thr Lys Gln Ile Asn Ser Asn Ile Ser Arg Gln Ala Trp Asp
       370                 375                 380
   Thr Leu Trp Pro Pro Glu Arg Lys Arg Gln Arg Ala Phe Phe Leu Phe
   385                 390                 395                 400
   Gly Leu Ala His Ile Val Gln Phe Asp Thr Glu Gly Ile Arg Ser Phe
                   405                 410                 415
   Phe Arg Thr Phe Phe Arg Leu Pro Lys Trp Met Trp Gln Gly Phe Leu
               420                 425                 430
   Gly Ser Thr Leu Thr Ser Gly Asp Leu Val Leu Phe Ser Leu Tyr Met
           435                 440                 445
   Phe Val Ile Ser Pro Asn Asn Leu Arg Lys Gly Leu Ile Asn His Leu
       450                 455                 460
   Ile Ser Asp Pro Thr Gly Ala Thr Met Ile Lys Thr Tyr Leu Lys Val
   465                 470                 475                 480
<210> 21
   <211> 5717
   <212> DNA
   <213> Artifizielle Sequenz
<220>
   <223> Nukleotid-Sequenz von pGT1069
<400> 21
   aaaccaattg tccatattgc atcagacatt gccgtcactg cgtcttttac tggctcttct      60
   cgctaaccaa accggtaacc ccgcttatta aaagcattct gtaacaaagc gggaccaaag     120
   ccatgacaaa aacgcgtaac aaaagtgtct ataatcacgg cagaaaagtc cacattgatt     180
   atttgcacgg cgtcacactt tgctatgcca tagcattttt atccataaga ttagctgatc     240
   ctacctgacg ctttttatcg caactctcta ctgtttctcc atacccgttt aaataatttt     300
   gtttaacttt aagaaggaga tatacccatg acacagaggg cccaccatca ccatcaccat     360
   tccatggcgg agaaactcag tgatggcagc agcatcatct cagtccatcc tagaccctcc     420
   aagggtttct cctcgaagct tctcgatctt ctcgagagac ttgttgtcaa gctcatgcac     480
   gatgcttctc tccctctcca ctacctctca ggcaacttcg ctcccatccg tgatgaaact     540
   cctcccgtca aggatctccc cgtccatgga tttcttcccg aatgcttgaa tggtgaattt     600
   gtgagggttg gtccaaaccc caagtttgat gctgtcgctg gatatcactg gtttgatgga     660
   gatgggatga ttcatggggt acgcatcaaa gatgggaaag ctacttatgt ttctcgatat     720
   gttaagacat cacgtcttaa gcaggaagag ttcttcggag ctgccaaatt catgaagatt     780
   ggtgacctta aggggttttt cggattgcta atggtcaatg tccaacagct gagaacgaag     840
   ctcaaaatat tggacaacac ttatggaaat ggaactgcca atacagcact cgtatatcac     900
   catggaaaac ttctagcatt acaggaggca gataagccgt acgtcatcaa agttttggaa     960
   gatggagacc tgcaaactct tggtataata gattatgaca agagattgac ccactccttc    1020
   actgctcacc caaaagttga cccggttacg ggtgaaatgt ttacattcgg ctattcgcat 1080
   acgccacctt atctcacata cagagttatc tcgaaagatg gcattatgca tgacccagtc 1140
   ccaattacta tatcagagcc tatcatgatg catgattttg ctattactga gacttatgca 1200
   atcttcatgg atcttcctat gcacttcagg ccaaaggaaa tggtgaaaga gaagaaaatg 1260
   atatactcat ttgatcccac aaaaaaggct cgttttggtg ttcttccacg ctatgccaag 1320
   gatgaactta tgattagatg gtttgagctt cccaactgct ttattttcca caacgccaat 1380
   gcttgggaag aagaggatga agtcgtcctc atcacttgtc gtcttgagaa tccagatctt 1440
   gacatggtca gtgggaaagt gaaagaaaaa ctcgaaaatt ttggcaacga actgtacgaa 1500
   atgagattca acatgaaaac gggctcagct tctcaaaaaa aactatccgc atctgcggtt 1560
   gatttcccca gaatcaatga gtgctacacc ggaaagaaac agagatacgt atatggaaca 1620
   attctggaca gtatcgcaaa ggttaccgga atcatcaagt ttgatctgca tgcagaagct 1680
   gagacaggga aaagaatgct ggaagtagga ggtaatatca aaggaatata tgacctggga 1740
   gaaggcagat atggttcaga ggctatctat gttccgcgtg agacagcaga agaagacgac 1800
   ggttacttga tattctttgt tcatgatgaa aacacaggga aatcatgcgt gactgtgata 1860
   gacgcaaaaa caatgtcggc tgaaccggtg gcagtggtgg agctgccgca cagggtccca 1920
   tatggcttcc atgccttgtt tgttacagag gaacaactcc aggaacaaac tcttatataa 1980
   ggatccggct gctaacaaag cccgaaagga agctgagttg gctgctgcca ccgctgagca 2040
   ataactagca taaccccttg gggcctctaa acgggtcttg aggggttttt tgctgaaagg 2100
   aggaactata tccggccgga tatccacagg acgggtgtgg tcgccatgat cgcgtagtcg 2160
   atagtggctc caagtagcga agcgagcagg actgggcggc ggccaaagcg gtcggacagt 2220
   gctccgagaa cgggtgcgca tagaaattgc atcaacgcat atagcgctag cagcacgcca 2280
   tagtgactgg cgatgctgtc ggaatggacg atatcccgca agaggcccgg cagtaccggc 2340
   ataaccaagc ctatgcctac agcatccagg gtgacggtgc cgaggatgac gatgagcgca 2400
   ttgttagatt tcatacacgg tgcctgactg cgttagcaat ttaactgtga taaactaccg 2460
   cattaaagct tatcgatgat aagctgtcaa acatgagaat tcttgaagac gaaagggcct 2520
   cgtgatacgc ctatttttat aggttaatgt catgcatgag acaataaccc tgataaatgc 2580
   ttcaataata ttgaaaaagg aagagtatga gtattcaaca tttccgtgtc gcccttattc 2640
   ccttttttgc ggcattttgc cttcctgttt ttgctcaccc agaaacgctg gtgaaagtaa 2700
   aagatgctga agatcagttg ggtgcacgag tgggttacat cgaactggat ctcaacagcg 2760
   gtaagatcct tgagagtttt cgccccgaag aacgttttcc aatgatgagc acttttaaag 2820
   ttctgctatg tggcgcggta ttatcccgtg ttgacgccgg gcaagagcaa ctcggtcgcc 2880
   gcatacacta ttctcagaat gacttggttg acgcgtcacc agtcacagaa aagcatctta 2940
   cggatggcat gacagtaaga gaattatgca gtgctgccat aaccatgagt gataacactg 3000
   cggccaactt acttctgaca acgatcggag gaccgaagga gctaaccgct tttttgcaca 3060
   acatggggga tcatgtaact cgccttgatc gttgggaacc ggagctgaat gaagccatac 3120
   caaacgacga gcgtgacacc acgatgcctg cagcaatggc aacaacgttg cgcaaactat 3180
   taactggcga actacttact ctagcttccc ggcaacaatt aatagactgg atggaggcgg 3240
   ataaagttgc aggaccactt ctgcgctcgg cccttccggc tggctggttt attgctgata 3300
   aatctggagc cggtgagcgt gggtctcgcg gtatcattgc agcactgggg ccagatggta 3360
   agccctcccg tatcgtagtt atctacacga cggggagtca ggcaactatg gatgaacgaa 3420
   atagacagat cgctgagata ggtgcctcac tgattaagca ttggtaactg tcagaccaag 3480
   tttactcata tatactttag attgatttaa aacttcattt ttaatttaaa aggatctagg 3540
   tgaagatcct ttttgataat ctcatgcatg accaaaatcc cttaacgtga gttttcgttc 3600
   cactgagcgt cagaccccgt agaaaagatc aaaggatctt cttgagatcc tttttttctg 3660
   cgcgtaatct gctgcttgca aacaaaaaaa ccaccgctac cagcggtggt ttgtttgccg 3720
   gatcaagagc taccaactct ttttccgaag gtaactggct tcagcagagc gcagatacca 3780
   aatactgtcc ttctagtgta gccgtagtta ggccaccact tcaagaactc tgtagcaccg 3840
   cctacatacc tcgctctgct aatcctgtta ccagtggctg ctgccagtgg cgataagtcg 3900
   tgtcttaccg ggttggactc aagacgatag ttaccggata aggcgcagcg gtcgggctga 3960
   acggggggtt cgtgcacaca gcccagcttg gagcgaacga cctacaccga actgagatac 4020
   ctacagcgtg agctatgaga aagcgccacg cttcccgaag ggagaaaggc ggacaggtat 4080
   ccggtaagcg gcagggtcgg aacaggagag cgcacgaggg agcttccagg gggaaacgcc 4140
   tggtatcttt atagtcctgt cgggtttcgc cacctctgac ttgagcgtcg atttttgtga 4200
   tgctcgtcag gggggcggag cctatggaaa aacgccagca acgcggcctt tttacggttc 4260
   ctggcctttt gctggccttt tgctcacatg ttctttcctg cgttatcccc tgattctgtg 4320
   gataaccgta ttaccgcctt tgagtgagct gataccgctc gccgcagccg aacgaccgag 4380
   cgcagcgagt cagtgagcga ggaagcggaa gagcgcctga tgcggtattt tctccttacg 4440
   catctgtgcg gtatttcaca ccgcatatat ggtgcactct cagtacaatc tgctctgatg 4500
   ccgcatagtt aagccagtat acactccgct atcgctacgt gactgggtca tggctgcgcc 4560
   ccgacacccg ccaacacccg ctgacgcgcc ctgacgggct tgtctgctcc cggcatccgc 4620
   ttacagacaa gctgtgaccg tctccgggag ctgcatgtgt cagaggtttt caccgtcatc 4680
   accgaaacgc gcgaggcagc tggcacgaca ggtttcccga ctggaatgtg cctgtcaaat 4740
   ggacgaagca gggattctgc aaaccctatg ctactccgtc aagccgtcaa ttgtctgatt 4800
   cgttaccaat tatgacaact tgacggctac atcattcact ttttcttcac aaccggcacg     4860
   gaactcgctc gggctggccc cggtgcattt tttaaatacc cgcgagaaat agagttgatc     4920
   gtcaaaacca acattgcgac cgacggtggc gataggcatc cgggtggtgc tcaaaagcag     4980
   cttcgcctgg ctgatacgtt ggtcctcgcg ccagcttaag acgctaatcc ctaactgctg     5040
   gcggaaaaga tgtgacagac gcgacggcga caagcaaaca tgctgtgcga cgctggcgat     5100
   atcaaaattg ctgtctgcca ggtgatcgct gatgtactga caagcctcgc gtacccgatt     5160
   atccatcggt ggatggagcg actcgttaat cgcttccatg cgccgcagta acaattgctc     5220
   aagcagattt atcgccagca gctccgaata gcgcccttcc ccttgcccgg cgttaatgat     5280
   ttgcccaaac aggtcgctga aatgcggctg gtgcgcttca tccgggcgaa agaaccccgt     5340
   attggcaaat attgacggcc agttaagcca ttcatgccag taggcgcgcg gacgaaagta     5400
   aacccactgg tgataccatt cgcgagcctc cggatgacga ccgtagtgat gaatctctcc     5460
   tggcgggaac agcaaaatat cacccggtcg gcaaacaaat tctcgtccct gatttttcac     5520
   caccccctga ccgcgaatgg tgagattgag aatataacct ttcattccca gcggtcggtc     5580
   gataaaaaaa tcgagataac cgttggcctc aatcggcgtt aaacccgcca ccagatgggc     5640
   attaaacgag tatcccggca gcaggggatc attttgcgct tcagccatac ttttcatact     5700
   cccgccattc agagaag                                                    5717
<210> 22
   <211> 11
   <212> PRT
   <213> Artifizielle Sequenz
<220>
   <223> 6His
<400> 22
   Met Thr Gln Arg Ala His His His His His His
   1               5                   10
<210> 23
   <211> 538
   <212> PRT
   <213> Arabidopsis thaliana
<400> 23
   Met Ala Glu Lys Leu Ser Asp Gly Ser Ser Ile Ile Ser Val His Pro
   1               5                   10                  15
   Arg Pro Ser Lys Gly Phe Ser Ser Lys Leu Leu Asp Leu Leu Glu Arg
               20                  25                  30
   Leu Val Val Lys Leu Met His Asp Ala Ser Leu Pro Leu His Tyr Leu
           35                  40                  45
   Ser Gly Asn Phe Ala Pro Ile Arg Asp Glu Thr Pro Pro Val Lys Asp
       50                  55                  60
   Leu Pro Val His Gly Phe Leu Pro Glu Cys Leu Asn Gly Glu Phe Val
   65                  70                  75                  80
   Arg Val Gly Pro Asn Pro Lys Phe Asp Ala Val Ala Gly Tyr His Trp
                   85                  90                  95
   Phe Asp Gly Asp Gly Met Ile His Gly Val Arg Ile Lys Asp Gly Lys
               100                 105                 110
   Ala Thr Tyr Val Ser Arg Tyr Val Lys Thr Ser Arg Leu Lys Gln Glu
           115                 120                 125
   Glu Phe Phe Gly Ala Ala Lys Phe Met Lys Ile Gly Asp Leu Lys Gly
       130                 135                 140
   Phe Phe Gly Leu Leu Met Val Asn Val Gln Gln Leu Arg Thr Lys Leu
   145                 150                 155                 160
   Lys Ile Leu Asp Asn Thr Tyr Gly Asn Gly Thr Ala Asn Thr Ala Leu
                   165                 170                 175
   Val Tyr His His Gly Lys Leu Leu Ala Leu Gln Glu Ala Asp Lys Pro
               180                 185                 190
   Tyr Val Ile Lys Val Leu Glu Asp Gly Asp Leu Gln Thr Leu Gly Ile
           195                 200                 205
   Ile Asp Tyr Asp Lys Arg Leu Thr His Ser Phe Thr Ala His Pro Lys
       210                 215                 220
   Val Asp Pro Val Thr Gly Glu Met Phe Thr Phe Gly Tyr Ser His Thr
   225                 230                 235                 240
   Pro Pro Tyr Leu Thr Tyr Arg Val Ile Ser Lys Asp Gly Ile Met His
                   245                 250                 255
   Asp Pro Val Pro Ile Thr Ile Ser Glu Pro Ile Met Met His Asp Phe
               260                 265                 270
   Ala Ile Thr Glu Thr Tyr Ala Ile Phe Met Asp Leu Pro Met His Phe
           275                 280                 285
   Arg Pro Lys Glu Met Val Lys Glu Lys Lys Met Ile Tyr Ser Phe Asp
       290                 295                 300
   Pro Thr Lys Lys Ala Arg Phe Gly Val Leu Pro Arg Tyr Ala Lys Asp
   305                 310                 315                 320
   Glu Leu Met Ile Arg Trp Phe Glu Leu Pro Asn Cys Phe Ile Phe His
                   325                 330                 335
   Asn Ala Asn Ala Trp Glu Glu Glu Asp Glu Val Val Leu Ile Thr Cys
               340                 345                 350
   Arg Leu Glu Asn Pro Asp Leu Asp Met Val Ser Gly Lys Val Lys Glu
           355                 360                 365
   Lys Leu Glu Asn Phe Gly Asn Glu Leu Tyr Glu Met Arg Phe Asn Met
       370                 375                 380
   Lys Thr Gly Ser Ala Ser Gln Lys Lys Leu Ser Ala Ser Ala Val Asp
   385                 390                 395                 400
   Phe Pro Arg Ile Asn Glu Cys Tyr Thr Gly Lys Lys Gln Arg Tyr Val
                   405                 410                 415
   Tyr Gly Thr Ile Leu Asp Ser Ile Ala Lys Val Thr Gly Ile Ile Lys
               420                 425                 430
   Phe Asp Leu His Ala Glu Ala Glu Thr Gly Lys Arg Met Leu Glu Val
           435                 440                 445
   Gly Gly Asn Ile Lys Gly Ile Tyr Asp Leu Gly Glu Gly Arg Tyr Gly
       450                 455                 460
   Ser Glu Ala Ile Tyr Val Pro Arg Glu Thr Ala Glu Glu Asp Asp Gly
   465                 470                 475                 480
   Tyr Leu Ile Phe Phe Val His Asp Glu Asn Thr Gly Lys Ser Cys Val
                   485                 490                 495
   Thr Val Ile Asp Ala Lys Thr Met Ser Ala Glu Pro Val Ala Val Val
               500                 505                 510
   Glu Leu Pro His Arg Val Pro Tyr Gly Phe His Ala Leu Phe Val Thr
           515                 520                 525
   Glu Glu Gln Leu Gln Glu Gln Thr Leu Ile
       530                 535
<210> 24
   <211> 5732
   <212> DNA
   <213> Artifizielle Sequenz
<220>
   <223> Nukleotid-Sequenz von pGT1070
<400> 24
   aaaccaattg tccatattgc atcagacatt gccgtcactg cgtcttttac tggctcttct      60
   cgctaaccaa accggtaacc ccgcttatta aaagcattct gtaacaaagc gggaccaaag     120
   ccatgacaaa aacgcgtaac aaaagtgtct ataatcacgg cagaaaagtc cacattgatt     180
   atttgcacgg cgtcacactt tgctatgcca tagcattttt atccataaga ttagctgatc     240
   ctacctgacg ctttttatcg caactctcta ctgtttctcc atacccgttt aaataatttt     300
   gtttaacttt aagaaggaga tatacccatg acacagaggg cccaccatca ccatcaccat     360
   tccatgggta tgcagggtga agatgcacag cgtaccggta atattgttgc agttaaaccg     420
   aaaccgagcc agggtctgac cagcaaagca attgattggc tggaatggct gtttgtgaaa     480
   atgatgcatg atagcaaaca gccgctgcat tatctgagcg gtaattttgc accggttgat     540
   gaaacccctc cgctgaaaga tctgccggtt accggtcatc tgccggaatg tctgaatggt     600
   gaatttgttc gtgttggtcc gaatccgaaa tttgcaagca ttgcaggtta tcattggttt     660
   gatggtgatg gtatgattca tggcatgcgc attaaagatg gtaaagcaac ctatgttagc     720
   cgttatgttc agaccagccg tctgaaacaa gaggaattct ttggtcgtgc catgttcatg     780
   aaaatcggtg atctgaaagg tatgtttggt ctgctgatgg ttaatatgca gatgctgcgt     840
   gcaaaactga aagttctgga tattagctat ggtattggca ccgcaaatac cgcactggtt     900
   tatcatcatg gtaaactgct ggcactgagc gaagcagata aaccgtatgc aattaaagtg     960
   ctggaagatg gtgatctgca gaccattggc ctgctggatt atgataaacg tctggcacat    1020
   agctttaccg cacatccgaa agttgatccg tttaccggtg agatgtttac ctttggttat    1080
   agccataccc ctccgtatgt tacctatcgt gttattagca aagatggtgc aatgaatgat    1140
   ccggttccga ttaccgttag cggtccgatc atgatgcacg attttgcaat taccgaaaac    1200
   tacgccatct ttatggatct gccgctgtat ttcaaaccga aagaaatggt gaaagacaag    1260
   aaattcatct ttagcttcga tgccacccag aaagcacgtt ttggtattct gcctcgttat    1320
   gccaagaatg agctgctgat taaatggttt gaactgccga actgcttcat ctttcataat    1380
   gcaaatgcat gggaagaggg tgatgaagtt gttctgatta cctgtcgtct ggaaaatccg    1440
   gatctggata tggtgaatag caccgttaaa gaacgtctgg acaactttaa gaacgagctg    1500
   tatgaaatgc gcttcaatct gcagaatggt ctggcaagcc agaaaaaact gagcgttagc    1560
   gcagttgatt ttccgcgtgt taatgaaagc tataccaccc gtaaacagcg ttatgtttat    1620
   ggcaccaccc tggataagat tgccaaagtt accggcatca tcaaattcga tctgcatgcc    1680
   gaaccggaaa ccggtaaaga gaagctggaa ctgggtggta atgtgaaagg catttttgat    1740
   ctgggtccgg gtcgttttgg ttcagaagca gtttttgttc cgcgtcatcc gggtattacc    1800
   agcgaagagg atgatggtta tctgatcttc tttgtgcacg atgaaaacac cggcaaaagc 1860
   gcagttaatg ttattgatgc aaaaaccatg agccctgatc cggtggcagt tgtggaactg 1920
   cctaaacgtg ttccgtatgg ttttcatgca ttttttgtta ccgaagatca gctgcaagaa 1980
   caggccaaag tttaaggatc cggctgctaa caaagcccga aaggaagctg agttggctgc 2040
   tgccaccgct gagcaataac tagcataacc ccttggggcc tctaaacggg tcttgagggg 2100
   ttttttgctg aaaggaggaa ctatatccgg ccggatatcc acaggacggg tgtggtcgcc 2160
   atgatcgcgt agtcgatagt ggctccaagt agcgaagcga gcaggactgg gcggcggcca 2220
   aagcggtcgg acagtgctcc gagaacgggt gcgcatagaa attgcatcaa cgcatatagc 2280
   gctagcagca cgccatagtg actggcgatg ctgtcggaat ggacgatatc ccgcaagagg 2340
   cccggcagta ccggcataac caagcctatg cctacagcat ccagggtgac ggtgccgagg 2400
   atgacgatga gcgcattgtt agatttcata cacggtgcct gactgcgtta gcaatttaac 2460
   tgtgataaac taccgcatta aagcttatcg atgataagct gtcaaacatg agaattcttg 2520
   aagacgaaag ggcctcgtga tacgcctatt tttataggtt aatgtcatgc atgagacaat 2580
   aaccctgata aatgcttcaa taatattgaa aaaggaagag tatgagtatt caacatttcc 2640
   gtgtcgccct tattcccttt tttgcggcat tttgccttcc tgtttttgct cacccagaaa 2700
   cgctggtgaa agtaaaagat gctgaagatc agttgggtgc acgagtgggt tacatcgaac 2760
   tggatctcaa cagcggtaag atccttgaga gttttcgccc cgaagaacgt tttccaatga 2820
   tgagcacttt taaagttctg ctatgtggcg cggtattatc ccgtgttgac gccgggcaag 2880
   agcaactcgg tcgccgcata cactattctc agaatgactt ggttgacgcg tcaccagtca 2940
   cagaaaagca tcttacggat ggcatgacag taagagaatt atgcagtgct gccataacca 3000
   tgagtgataa cactgcggcc aacttacttc tgacaacgat cggaggaccg aaggagctaa 3060
   ccgctttttt gcacaacatg ggggatcatg taactcgcct tgatcgttgg gaaccggagc 3120
   tgaatgaagc cataccaaac gacgagcgtg acaccacgat gcctgcagca atggcaacaa 3180
   cgttgcgcaa actattaact ggcgaactac ttactctagc ttcccggcaa caattaatag 3240
   actggatgga ggcggataaa gttgcaggac cacttctgcg ctcggccctt ccggctggct 3300
   ggtttattgc tgataaatct ggagccggtg agcgtgggtc tcgcggtatc attgcagcac 3360
   tggggccaga tggtaagccc tcccgtatcg tagttatcta cacgacgggg agtcaggcaa 3420
   ctatggatga acgaaataga cagatcgctg agataggtgc ctcactgatt aagcattggt 3480
   aactgtcaga ccaagtttac tcatatatac tttagattga tttaaaactt catttttaat 3540
   ttaaaaggat ctaggtgaag atcctttttg ataatctcat gcatgaccaa aatcccttaa 3600
   cgtgagtttt cgttccactg agcgtcagac cccgtagaaa agatcaaagg atcttcttga 3660
   gatccttttt ttctgcgcgt aatctgctgc ttgcaaacaa aaaaaccacc gctaccagcg 3720
   gtggtttgtt tgccggatca agagctacca actctttttc cgaaggtaac tggcttcagc 3780
   agagcgcaga taccaaatac tgtccttcta gtgtagccgt agttaggcca ccacttcaag 3840
   aactctgtag caccgcctac atacctcgct ctgctaatcc tgttaccagt ggctgctgcc 3900
   agtggcgata agtcgtgtct taccgggttg gactcaagac gatagttacc ggataaggcg 3960
   cagcggtcgg gctgaacggg gggttcgtgc acacagccca gcttggagcg aacgacctac 4020
   accgaactga gatacctaca gcgtgagcta tgagaaagcg ccacgcttcc cgaagggaga 4080
   aaggcggaca ggtatccggt aagcggcagg gtcggaacag gagagcgcac gagggagctt 4140
   ccagggggaa acgcctggta tctttatagt cctgtcgggt ttcgccacct ctgacttgag 4200
   cgtcgatttt tgtgatgctc gtcagggggg cggagcctat ggaaaaacgc cagcaacgcg 4260
   gcctttttac ggttcctggc cttttgctgg ccttttgctc acatgttctt tcctgcgtta 4320
   tcccctgatt ctgtggataa ccgtattacc gcctttgagt gagctgatac cgctcgccgc 4380
   agccgaacga ccgagcgcag cgagtcagtg agcgaggaag cggaagagcg cctgatgcgg 4440
   tattttctcc ttacgcatct gtgcggtatt tcacaccgca tatatggtgc actctcagta 4500
   caatctgctc tgatgccgca tagttaagcc agtatacact ccgctatcgc tacgtgactg 4560
   ggtcatggct gcgccccgac acccgccaac acccgctgac gcgccctgac gggcttgtct 4620
   gctcccggca tccgcttaca gacaagctgt gaccgtctcc gggagctgca tgtgtcagag 4680
   gttttcaccg tcatcaccga aacgcgcgag gcagctggca cgacaggttt cccgactgga 4740
   atgtgcctgt caaatggacg aagcagggat tctgcaaacc ctatgctact ccgtcaagcc 4800
   gtcaattgtc tgattcgtta ccaattatga caacttgacg gctacatcat tcactttttc 4860
   ttcacaaccg gcacggaact cgctcgggct ggccccggtg cattttttaa atacccgcga 4920
   gaaatagagt tgatcgtcaa aaccaacatt gcgaccgacg gtggcgatag gcatccgggt 4980
   ggtgctcaaa agcagcttcg cctggctgat acgttggtcc tcgcgccagc ttaagacgct 5040
   aatccctaac tgctggcgga aaagatgtga cagacgcgac ggcgacaagc aaacatgctg 5100
   tgcgacgctg gcgatatcaa aattgctgtc tgccaggtga tcgctgatgt actgacaagc 5160
   ctcgcgtacc cgattatcca tcggtggatg gagcgactcg ttaatcgctt ccatgcgccg 5220
   cagtaacaat tgctcaagca gatttatcgc cagcagctcc gaatagcgcc cttccccttg 5280
   cccggcgtta atgatttgcc caaacaggtc gctgaaatgc ggctggtgcg cttcatccgg 5340
   gcgaaagaac cccgtattgg caaatattga cggccagtta agccattcat gccagtaggc 5400
   gcgcggacga aagtaaaccc actggtgata ccattcgcga gcctccggat gacgaccgta 5460
   gtgatgaatc tctcctggcg ggaacagcaa aatatcaccc ggtcggcaaa caaattctcg 5520
   tccctgattt ttcaccaccc cctgaccgcg aatggtgaga ttgagaatat aacctttcat 5580
   tcccagcggt cggtcgataa aaaaatcgag ataaccgttg gcctcaatcg gcgttaaacc      5640
   cgccaccaga tgggcattaa acgagtatcc cggcagcagg ggatcatttt gcgcttcagc      5700
   catacttttc atactcccgc cattcagaga ag                                    5732
<210> 25
   <211> 543
   <212> PRT
   <213> Osmanthus fragrans
<400> 25
   Met Gly Met Gln Gly Glu Asp Ala Gln Arg Thr Gly Asn Ile Val Ala
   1               5                   10                  15
   Val Lys Pro Lys Pro Ser Gln Gly Leu Thr Ser Lys Ala Ile Asp Trp
               20                  25                  30
   Leu Glu Trp Leu Phe Val Lys Met Met His Asp Ser Lys Gln Pro Leu
           35                  40                  45
   His Tyr Leu Ser Gly Asn Phe Ala Pro Val Asp Glu Thr Pro Pro Leu
       50                  55                  60
   Lys Asp Leu Pro Val Thr Gly His Leu Pro Glu Cys Leu Asn Gly Glu
   65                  70                  75                  80
   Phe Val Arg Val Gly Pro Asn Pro Lys Phe Ala Ser Ile Ala Gly Tyr
                   85                  90                  95
   His Trp Phe Asp Gly Asp Gly Met Ile His Gly Met Arg Ile Lys Asp
               100                 105                 110
   Gly Lys Ala Thr Tyr Val Ser Arg Tyr Val Gln Thr Ser Arg Leu Lys
           115                 120                 125
   Gln Glu Glu Phe Phe Gly Arg Ala Met Phe Met Lys Ile Gly Asp Leu
       130                 135                 140
   Lys Gly Met Phe Gly Leu Leu Met Val Asn Met Gln Met Leu Arg Ala
   145                 150                 155                 160
   Lys Leu Lys Val Leu Asp Ile Ser Tyr Gly Ile Gly Thr Ala Asn Thr
                   165                 170                 175
   Ala Leu Val Tyr His His Gly Lys Leu Leu Ala Leu Ser Glu Ala Asp
               180                 185                 190
   Lys Pro Tyr Ala Ile Lys Val Leu Glu Asp Gly Asp Leu Gln Thr Ile
           195                 200                 205
   Gly Leu Leu Asp Tyr Asp Lys Arg Leu Ala His Ser Phe Thr Ala His
       210                 215                 220
   Pro Lys Val Asp Pro Phe Thr Gly Glu Met Phe Thr Phe Gly Tyr Ser
   225                 230                 235                 240
   His Thr Pro Pro Tyr Val Thr Tyr Arg Val Ile Ser Lys Asp Gly Ala
                   245                 250                 255
   Met Asn Asp Pro Val Pro Ile Thr Val Ser Gly Pro Ile Met Met His
               260                 265                 270
   Asp Phe Ala Ile Thr Glu Asn Tyr Ala Ile Phe Met Asp Leu Pro Leu
           275                 280                 285
   Tyr Phe Lys Pro Lys Glu Met Val Lys Asp Lys Lys Phe Ile Phe Ser
       290                 295                 300
   Phe Asp Ala Thr Gln Lys Ala Arg Phe Gly Ile Leu Pro Arg Tyr Ala
   305                 310                 315                 320
   Lys Asn Glu Leu Leu Ile Lys Trp Phe Glu Leu Pro Asn Cys Phe Ile
                   325                 330                 335
   Phe His Asn Ala Asn Ala Trp Glu Glu Gly Asp Glu Val Val Leu Ile
               340                 345                 350
   Thr Cys Arg Leu Glu Asn Pro Asp Leu Asp Met Val Asn Ser Thr Val
           355                 360                 365
   Lys Glu Arg Leu Asp Asn Phe Lys Asn Glu Leu Tyr Glu Met Arg Phe
       370                 375                 380
   Asn Leu Gln Asn Gly Leu Ala Ser Gln Lys Lys Leu Ser Val Ser Ala
   385                 390                 395                 400
   Val Asp Phe Pro Arg Val Asn Glu Ser Tyr Thr Thr Arg Lys Gln Arg
                   405                 410                 415
   Tyr Val Tyr Gly Thr Thr Leu Asp Lys Ile Ala Lys Val Thr Gly Ile
               420                 425                 430
   Ile Lys Phe Asp Leu His Ala Glu Pro Glu Thr Gly Lys Glu Lys Leu
           435                 440                 445
   Glu Leu Gly Gly Asn Val Lys Gly Ile Phe Asp Leu Gly Pro Gly Arg
       450                 455                 460
   Phe Gly Ser Glu Ala Val Phe Val Pro Arg His Pro Gly Ile Thr Ser
   465                 470                 475                 480
   Glu Glu Asp Asp Gly Tyr Leu Ile Phe Phe Val His Asp Glu Asn Thr
                   485                 490                 495
   Gly Lys Ser Ala Val Asn Val Ile Asp Ala Lys Thr Met Ser Pro Asp
               500                 505                 510
   Pro Val Ala Val Val Glu Leu Pro Lys Arg Val Pro Tyr Gly Phe His
           515                 520                 525
   Ala Phe Phe Val Thr Glu Asp Gln Leu Gln Glu Gln Ala Lys Val
       530                 535                 540
<210> 26
   <211> 480
   <212> PRT
   <213> Artifizielle Sequenz
<220>
   <223> AtECmutl
<400> 26
   Met Ala Ser Gly Gly Gly Ser Ser Gly Ser Glu Ser Cys Val Ala Val
   1               5                   10                  15
   Arg Glu Asp Phe Ala Asp Glu Glu Asp Phe Val Lys Ala Gly Gly Ser
               20                  25                  30
   Glu Ile Leu Phe Val Gln Met Gln Gln Asn Lys Asp Met Asp Glu Gln
           35                  40                  45
   Ser Lys Leu Val Asp Lys Leu Pro Pro Ile Ser Ile Gly Asp Gly Ala
       50                  55                  60
   Leu Asp Leu Val Val Ile Gly Cys Gly Pro Ala Gly Leu Ala Leu Ala
   65                  70                  75                  80
   Ala Glu Ser Ala Lys Leu Gly Leu Lys Val Gly Leu Ile Gly Pro Asp
                   85                  90                  95
   Leu Pro Phe Thr Asn Asn Tyr Gly Val Trp Glu Asp Glu Phe Asn Asp
               100                 105                 110
   Leu Gly Leu Gln Lys Cys Ile Glu His Val Trp Arg Glu Thr Ile Val
           115                 120                 125
   Tyr Leu Asp Asp Asp Lys Pro Ile Thr Ile Gly Arg Ala Tyr Gly Arg
       130                 135                 140
   Val Ser Arg Arg Leu Leu His Glu Glu Leu Leu Arg Arg Cys Val Glu
   145                 150                 155                 160
   Ser Gly Val Ser Tyr Leu Ser Ser Lys Val Asp Ser Ile Thr Glu Ala
                   165                 170                 175
   Ser Asp Gly Leu Arg Leu Val Ala Cys Asp Asp Asn Asn Val Ile Pro
               180                 185                 190
   Cys Arg Leu Ala Thr Val Ala Ser Gly Ala Ala Ser Gly Lys Leu Leu
           195                 200                 205
   Gln Tyr Glu Val Gly Gly Pro Arg Val Cys Val Gln Thr Ala Tyr Gly
       210                 215                 220
   Val Glu Val Glu Val Glu Asn Ser Pro Tyr Asp Pro Asp Gln Met Val
   225                 230                 235                 240
   Phe Met Asp Tyr Arg Asp Tyr Thr Asn Glu Lys Val Arg Ser Leu Glu
                   245                 250                 255
   Ala Glu Tyr Pro Thr Phe Leu Tyr Ala Met Pro Met Thr Lys Ser Arg
               260                 265                 270
   Leu Phe Phe Glu Glu Thr Cys Leu Ala Ser Lys Asp Val Met Pro Phe
           275                 280                 285
   Asp Leu Leu Lys Thr Lys Leu Met Leu Arg Leu Asp Thr Leu Gly Ile
       290                 295                 300
   Arg Ile Leu Lys Thr Tyr Glu Glu Glu Trp Ser Tyr Ile Pro Val Gly
   305                 310                 315                 320
   Gly Ser Leu Pro Asn Thr Glu Gln Lys Asn Leu Ala Phe Gly Ala Ala
                   325                 330                 335
   Ala Ser Met Val His Pro Ala Thr Gly Tyr Ser Val Val Arg Ser Leu
               340                 345                 350
   Ser Glu Ala Pro Lys Tyr Ala Ser Val Ile Ala Glu Ile Leu Arg Glu
           355                 360                 365
   Glu Thr Thr Lys Gln Ile Asn Ser Asn Ile Ser Arg Gln Ala Trp Asp
       370                 375                 380
   Thr Leu Trp Pro Pro Glu Arg Lys Arg Gln Arg Ala Phe Phe Leu Phe
   385                 390                 395                 400
   Gly Leu Ala His Ile Val Gln Phe Asp Thr Glu Gly Ile Arg Ser Phe
                   405                 410                 415
   Phe Arg Thr Phe Phe Arg Leu Pro Lys Trp Met Trp Gln Gly Phe Leu
               420                 425                 430
   Gly Ser Thr Leu Thr Ser Gly Asp Leu Val Leu Phe Ala Leu Tyr Met
           435                 440                 445
   Phe Val Ile Ser Pro Asn Asn Leu Arg Lys Gly Leu Ile Asn His Leu
       450                 455                 460
   Ile Ser Asp Pro Thr Gly Ala Thr Met Ile Lys Thr Tyr Leu Lys Val
   465                 470                 475                 480
<210> 27
   <211> 9599
   <212> DNA
   <213> Artifizielle Sequenz
<220>
   <223> pAC-BETAipi
<400> 27
   gagtgtatac tggcttacta tgttggcact gatgagggtg tcagtgaagt gcttcatgtg 60
   gcaggagaaa aaaggctgca ccggtgcgtc agcagaatat gtgatacagg atatattccg 120
   cttcctcgct cactgactcg ctacgctcgg tcgttcgact gcggcgagcg gaaatggctt 180
   acgaacgggg cggagatttc ctggaagatg ccaggaagat acttaacagg gaagtgagag 240
   ggccgcggca aagccgtttt tccataggct ccgcccccct gacaagcatc acgaaatctg 300
   acgctcaaat cagtggtggc gaaacccgac aggactataa agataccagg cgtttccccc 360
   tggcggctcc ctcgtgcgct ctcctgttcc tgcctttcgg tttaccggtg tcattccgct 420
   gttatggccg cgtttgtctc attccacgcc tgacactcag ttccgggtag gcagttcgct 480
   ccaagctgga ctgtatgcac gaaccccccg ttcagtccga ccgctgcgcc ttatccggta 540
   actatcgtct tgagtccaac ccggaaagac atgcaaaagc accactggca gcagccactg 600
   gtaattgatt tagaggagtt agtcttgaag tcatgcgccg gttaaggcta aactgaaagg 660
   acaagttttg gtgactgcgc tcctccaagc cagttacctc ggttcaaaga gttggtagct 720
   cagagaacct tcgaaaaacc gccctgcaag gcggtttttt cgttttcaga gcaagagatt 780
   acgcgcagac caaaacgatc tcaagaagat catcttatta atcagataaa atatttctag 840
   atttcagtgc aatttatctc ttcaaatgta gcacctgaag tcagccccat acgatataag 900
   ttgtaattct catgtttgac agcttatcat cgataagctt taatgcggta gtttatcaca 960
   gttaaattgc taacgcagtc aggcaccgtg tatgaaatct aacaatgcgc tcatcgtcat 1020
   cctcggcacc gtcaccctgg atgctgtagg cataggcttg gttatgccgg tactgccggg 1080
   cctcttgcgg gatatcgtcc attccgacag catcgccagt cactatggcg tgctgctagc 1140
   gctatatgcg ttgatgcaat ttctatgcgc acccgttctc ggagcactgt ccgaccgctt 1200
   tggccgccgc ccagtcctgc tcgcttcgct acttggagcc actatcgact acgcgatcat 1260
   ggcgaccaca cccgtcctgt ggatctaaag gcacagcgtc tcatgcttcg cacaatgtaa 1320
   aactgcttca gaacctggcg agagctatcc gcgcggtcta cggttaactg atactaaaag 1380
   acaattcagc gggtaacctt gcaatggtga gtggcagtaa agcgggcgtt tcgcctcatc 1440
   gcgaaataga agtaatgaga caatccattg acgatcacct ggctggcctg ttacctgaaa 1500
   ccgacagcca ggatatcgtc agccttgcga tgcgtgaagg cgtcatggca cccggtaaac 1560
   ggatccgtcc gctgctgatg ctgctggccg cccgcgacct ccgctaccag ggcagtatgc 1620
   ctacgctgct cgatctcgcc tgcgccgttg aactgaccca taccgcgtcg ctgatgctcg 1680
   acgacatgcc ctgcatggac aacgccgagc tgcgccgcgg tcagcccact acccacaaaa 1740
   aatttggtga gagcgtggcg atccttgcct ccgttgggct gctctctaaa gcctttggtc 1800
   tgatcgccgc caccggcgat ctgccggggg agaggcgtgc ccaggcggtc aacgagctct 1860
   ctaccgccgt gggcgtgcag ggcctggtac tggggcagtt tcgcgatctt aacgatgccg 1920
   ccctcgaccg tacccctgac gctatcctca gcaccaacca cctcaagacc ggcattctgt 1980
   tcagcgcgat gctgcagatc gtcgccattg cttccgcctc gtcgccgagc acgcgagaga 2040
   cgctgcacgc cttcgccctc gacttcggcc aggcgtttca actgctggac gatctgcgtg 2100
   acgatcaccc ggaaaccggt aaagatcgca ataaggacgc gggaaaatcg acgctggtca 2160
   accggctggg cgcagacgcg gcccggcaaa agctgcgcga gcatattgat tccgccgaca 2220
   aacacctcac ttttgcctgt ccgcagggcg gcgccatccg acagtttatg catctgtggt 2280
   ttggccatca ccttgccgac tggtcaccgg tcatgaaaat cgcctgatac cgcccttttg 2340
   ggttcaagca gtacataacg atggaaccac attacaggag tagtgatgaa tgaaggacga 2400
   gcgccttgtt cagcgtaaga acgatcatct ggatatcgtt ctcgaccccc gtcgcgccgt 2460
   aactcaggct agcgcaggtt ttgagcgctg gcgctttacc cactgcgccc tgccagagct 2520
   gaattttagc gacatcacgc tggaaaccac cttcctgaat cggcagctac aggctccgct 2580
   gctgatcagc tccatgaccg gcggcgttga gcgctcgcgc catatcaacc gccacctcgc 2640
   cgaggcggcg caggtgctaa aaattgcgat gggggtgggc tcccagcgcg tcgccattga 2700
   gagcgacgcg ggcttagggc tggataaaac cctgcggcag ctggctccgg acgtgccgct 2760
   gctggcgaac ctcggcgcgg cgcagctgac cggcagaaaa ggtattgatt acgcccgacg 2820
   ggccgtggag atgatcgagg cggatgcgct gattgtgcac ctaaacccgc tgcaggaggc 2880
   gctacagccc ggcggcgatc gcgactggcg cggacggctg gcggctattg aaactctggt 2940
   ccgcgagctg cccgttccgc tggtggtgaa agaggtggga gccggtatct cccgaaccgt 3000
   ggccgggcag ctgatcgatg ccggcgttac cgtgattgac gtcgcgggcg cgggcggcac 3060
   cagctgggcc gccgttgaag gcgagcgggc ggccaccgag cagcagcgca gcgtggccaa 3120
   cgtctttgcc gactggggga tccccaccgc tgaggcgctg gttgacattg ccgaggcctg 3180
   gccgcagatg ccccttattg cctcgggcgg gattaaaaac ggcgtcgacg cggcgaaagc 3240
   gctgcggctc ggcgcgtgca tggtagggca ggccgccgcc gtgctcggca gcgcaggcgt 3300
   ctccacggag aaggtgatcg atcacttcaa cgtgattatt gagcagctgc gggtggcctg 3360
   cttctgcacc ggcagccgca gcctgagcga tctaaagcag gctgatatcc gctatgtgcg 3420
   ggatacgcca tgagccattt tgccattgtg gcaccgccgc tctacagtca tgcggtggcg 3480
   ctgcatgccc tggcgctgga gatggcccaa cgcggccacc ggtaattgat ttagaggagt 3540
   tagtcttgaa gtcatgcgcc ggttaaggct aaactgaaag gacaagtttt ggtgactgcg 3600
   ctcctccaag ccagttacct cggttcaaag agttggtagc tcagagaacc ttcgaaaaac 3660
   cgccctgcaa ggcggttttt tcgttttcag agcaagagat tacgcgcaga ccaaaacgat 3720
   ctcaagaaga tcatcttatt aatcagataa aatatttcta gatttcagtg caatttatct 3780
   cttcaaatgt agcacctgaa gtcagcccca tacgatataa gttgtaattc tcatgtttga 3840
   cagcttatca tcgataagct cgaccagccg cgctacgttg ccgaggctaa tctggtgatc 3900
   acccacggcg gtctcaatac cgtactggat gcgctggctg ccgcgacgcc ggtgctggcg 3960
   gtgccactct ctttcgacca gcccgccgtg gctgcccggc tggtctataa cgggctgggt 4020
   cgccgggtat cgcgctttgc cagacagcag acgctggcgg atgagattgc ccaactgctg 4080
   ggggatgaga cgctgcatca gcgtctggcg acggcccgcc agcagcttaa cgacgccggg 4140
   ggcacgcccc gtgcggcgac cctgattgaa caggccatag cagggagtga gagcgtatcg 4200
   tgagggatct gattttagtc ggcggcggcc tggccaacgg gctgatcgcc tggcgtctgc 4260
   gccagcgcta cccgcagctt aacctgctgc tgatcgaggc cggggagcag cccggcggga 4320
   accatacctg gtcattccat gaagacgatc tgactcccgg gcagcacgcc tggctggccc 4380
   cgctggtggc ccacgcctgg ccgggctatg aggtgcagtt tcccgatctt cgccgtcgcc 4440
   tcgcgcgcgg ctactactcc attacctcag agcgctttgc cgaggccctg catcaggcgc 4500
   tgggggagaa catctggcta aactgttcgg tgagcgaggt gttacccaat agcgtgcgcc 4560
   ttgccaacgg tgaggcgctg cttgccggag cggtgattga cggacgcggc gtgaccgcca 4620
   gttcggcgat gcaaaccggc tatcagctct ttcttggtca gcagtggcgg ctgacacagc 4680
   cccacggcct gaccgtaccg atcctgatgg atgccacggt ggcgcagcag cagggctatc 4740
   gctttgtcta cacgctgccg ctctccgccg acacgctgct gatcgaggat acgcgctacg 4800
   ccaatgtccc gcagcgtgat gataatgccc tacgccagac ggttaccgac tatgctcaca 4860
   gcaaagggtg gcagctggcc cagcttgaac gcgaggagac cggctgtctg ccgattaccc 4920
   tggcgggtga catccaggct ctgtgggccg atgcgccggg cgtgccgcgc tcgggaatgc 4980
   gggctgggct atttcaccct accactggct attcgctgcc gctggcggtg gcccttgccg 5040
   acgcgattgc cgacagcccg cggctgggca gcgttccgct ctatcagctc acccggcagt 5100
   ttgccgaacg ccactggcgc aggcagggat tcttccgcct gctgaaccgg atgcttttcc 5160
   tggccgggcg cgaggagaac cgctggcggg tgatgcagcg cttttatggg ctgccggagc 5220
   ccaccgtaga gcgcttttac gccggtcggc tctctctctt tgataaggcc cgcattttga 5280
   cgggcaagcc accggttccg ctgggcgaag cctggcgggc ggcgctgaac cattttcctg 5340
   acagacgaga taaaggatga aaaaaaccgt tgtgattggc gcaggctttg gtggcctggc 5400
   gctggcgatt cgcctgcagg cggcagggat cccaaccgta ctgctggagc agcgggacaa 5460
   gcccggcggt cgggcctacg tctggcatga ccagggcttt acctttgacg ccgggccgac 5520
   ggtgatcacc gatcctaccg cgcttgaggc gctgttcacc ctggccggca ggcgcatgga 5580
   ggattacgtc aggctgctgc cggtaaaacc cttctaccga ctctgctggg agtccgggaa 5640
   gaccctcgac tatgctaacg acagcgccga gcttgaggcg cagattaccc agttcaaccc 5700
   ccgcgacgtc gagggctacc ggcgctttct ggcttactcc caggcggtat tccaggaggg 5760
   atatttgcgc ctcggcagcg tgccgttcct ctcttttcgc gacatgctgc gcgccgggcc 5820
   gcagctgctt aagctccagg cgtggcagag cgtctaccag tcggtttcgc gctttattga 5880
   ggatgagcat ctgcggcagg ccttctcgtt ccactccctg ctggtaggcg gcaacccctt 5940
   caccacctcg tccatctaca ccctgatcca cgcccttgag cgggagtggg gggtctggtt 6000
   ccctgagggc ggcaccgggg cgctggtgaa cggcatggtg aagctgttta ccgatctggg 6060
   cggggagatc gaactcaacg cccgggtcga agagctggtg gtggccgata accgcgtaag 6120
   ccaggtccgg ctggcggatg gtcggatctt tgacaccgac gccgtagcct cgaacgctga 6180
   cgtggtgaac acctataaaa agctgctcgg ccaccatccg gtggggcaga agcgggcggc 6240
   agcgctggag cgcaagagca tgagcaactc gctgtttgtg ctctacttcg gcctgaacca 6300
   gcctcattcc cagctggcgc accataccat ctgttttggt ccccgctacc gggagctgat 6360
   cgacgagatc tttaccggca gcgcgctggc ggatgacttc tcgctctacc tgcactcgcc 6420
   ctgcgtgacc gatccctcgc tcgcgcctcc cggctgcgcc agcttctacg tgctggcccc 6480
   ggtgccgcat cttggcaacg cgccgctgga ctgggcgcag gaggggccga agctgcgcga 6540
   ccgcatcttt gactaccttg aagagcgcta tatgcccggc ctgcgtagcc agctggtgac 6600
   ccagcggatc tttaccccgg cagacttcca cgacacgctg gatgcgcatc tgggatcggc 6660
   cttctccatc gagccgctgc tgacccaaag cgcctggttc cgcccgcaca accgcgacag 6720
   cgacattgcc aacctctacc tggtgggcgc aggtactcac cctggggcgg gcattcctgg 6780
   cgtagtggcc tcggcgaaag ccaccgccag cctgatgatt gaggatctgc aatgagccaa 6840
   ccgccgctgc ttgaccacgc cacgcagacc atggccaacg gctcgaaaag ttttgccacc 6900
   gctgcgaagc tgttcgaccc ggccacccgc cgtagcgtgc tgatgctcta cacctggtgc 6960
   cgccactgcg atgacgtcat tgacgaccag acccacggct tcgccagcga ggccgcggcg 7020
   gaggaggagg ccacccagcg cctggcccgg ctgcgcacgc tgaccctggc ggcgtttgaa 7080
   ggggccgaga tgcaggatcc ggccttcgct gcctttcagg aggtggcgct gacccacggt 7140
   attacgcccc gcatggcgct cgatcacctc gacggctttg cgatggacgt ggctcagacc 7200
   cgctatgtca cctttgagga tacgctgcgc tactgctatc acgtggcggg cgtggtgggt 7260
   ctgatgatgg ccagggtgat gggcgtgcgg gatgagcggg tgctggatcg cgcctgcgat 7320
   ctggggctgg ccttccagct gacgaatatc gcccgggata ttattgacga tgcggctatt 7380
   gaccgctgct atctgcccgc cgagtggctg caggatgccg ggctgacccc ggagaactat 7440
   gccgcgcggg agaatcgggc cgcgctggcg cgggtggcgg agcggcttat tgatgccgca 7500
   gagccgtact acatctcctc ccaggccggg ctacacgatc tgccgccgcg ctgcgcctgg 7560
   gcgatcgcca ccgcccgcag cgtctaccgg gagatcggta ttaaggtaaa agcggcggga 7620
   ggcagcgcct gggatcgccg ccagcacacc agcaaaggtg aaaaaattgc catgctgatg 7680
   gcggcaccgg ggcaggttat tcgggcgaag acgacgaggg tgacgccgcg tccggccggt 7740
   ctttggcagc gtcccgttta ggcgggcggc catgacgttc acgcaggatc gcctgtaggt 7800
   cggcaggctt gcgggcgtaa ataaaaccga aggagacgca gccctcccgg ccgcgcaccg 7860
   cgtggtgcag gcggtgggcg acgtagagcc gcttcaggta gccccggcgc gggatcccgc 7920
   ctcgccgtgc tgtccggtct caacctgatc cgccagaatc gagccaacgg gatcggccag 7980
   cacgaattcg gtatgcggag agtgtttggc aaaccatgcc tgcaagccac ccagcgtgcc 8040
   gccggagcca acgcctacta ccacggcatc aaccctgcct gccagctggt cgaacagctc 8100
   cggtgcggtg gtggtggcgt gcgccagcgg gttggccggg ttagagaact gatcaatata 8160
   gtaagcaccc ggcgtctcct ctgccaggcg gcgggcgtag tcctgatagt actccgggtg 8220
   gccctttgtc acgtcggagc gggtcaggcg cacatcaaca cccagcgcac gcaggtggta 8280
   gatcctctac gccggacgca tcgtggccgg catcaccggc gccacaggtg cggttgctgg 8340
   cgcctatatc gccgacatca ccgatgggga agatcgggct cgccacttcg ggctcatgag 8400
   cgcttgtttc ggcgtgggta tggtggcagg ccccgtggcc gggggactgt tgggcgccat 8460
   ctccttgggg tcgaatttgc tttcgaattt ctgccattca tccgcttatt atcacttatt 8520
   caggcgtagc accaggcgtt taagggcacc aataactgcc ttaaaaaaat tacgccccgc 8580
   cctgccactc atcgcagtac tgttgtaatt cattaagcat tctgccgaca tggaagccat 8640
   cacagacggc atgatgaacc tgaatcgcca gcggcatcag caccttgtcg ccttgcgtat 8700
   aatatttgcc catggtgaaa acgggggcga agaagttgtc catattggcc acgtttaaat 8760
   caaaactggt gaaactcacc cagggattgg ctgagacgaa aaacatattc tcaataaacc 8820
   ctttagggaa ataggccagg ttttcaccgt aacacgccac atcttgcgaa tatatgtgta 8880
   gaaactgccg gaaatcgtcg tggtattcac tccagagcga tgaaaacgtt tcagtttgct 8940
   catggaaaac ggtgtaacaa gggtgaacac tatcccatat caccagctca ccgtctttca 9000
   ttgccatacg gaattccgga tgagcattca tcaggcgggc aagaatgtga ataaaggccg 9060
   gataaaactt gtgcttattt ttctttacgg tctttaaaaa ggccgtaata tccagctgaa 9120
   cggtctggtt ataggtacat tgagcaactg actgaaatgc ctcaaaatgt tctttacgat 9180
   gccattggga tatatcaacg gtggtatatc cagtgatttt tttctccatt ttagcttcct 9240
   tagctcctga aaatctcgat aactcaaaaa atacgcccgg tagtgatctt atttcattat 9300
   ggtgaaagtt ggaacctctt acgtgccgat caacgtctca ttttcgccaa aagttggccc 9360
   agggcttccc ggtatcaaca gggacaccag gatttattta ttctgcgaag tgatcttccg 9420
   tcacaggtat ttattcggcg caaagtgcgt cgggtgatgc tgccaactta ctgatttagt 9480
   gtatgatggt gtttttgagg tgctccagtg gcttctgttt ctatcagctg tccctcctgt 9540
   tcagctactg acggggtggt gcgtaacggc aaaagcaccg ccggacatca gcgctagcg 9599
<210> 28
   <211> 8442
   <212> DNA
   <213> Artifizielle Sequenz
<220>
   <223> pAC-BETAipi-del-crtY
<400> 28
   gagtgtatac tggcttacta tgttggcact gatgagggtg tcagtgaagt gcttcatgtg 60
   gcaggagaaa aaaggctgca ccggtgcgtc agcagaatat gtgatacagg atatattccg 120
   cttcctcgct cactgactcg ctacgctcgg tcgttcgact gcggcgagcg gaaatggctt 180
   acgaacgggg cggagatttc ctggaagatg ccaggaagat acttaacagg gaagtgagag 240
   ggccgcggca aagccgtttt tccataggct ccgcccccct gacaagcatc acgaaatctg 300
   acgctcaaat cagtggtggc gaaacccgac aggactataa agataccagg cgtttccccc 360
   tggcggctcc ctcgtgcgct ctcctgttcc tgcctttcgg tttaccggtg tcattccgct       420
   gttatggccg cgtttgtctc attccacgcc tgacactcag ttccgggtag gcagttcgct       480
   ccaagctgga ctgtatgcac gaaccccccg ttcagtccga ccgctgcgcc ttatccggta       540
   actatcgtct tgagtccaac ccggaaagac atgcaaaagc accactggca gcagccactg       600
   gtaattgatt tagaggagtt agtcttgaag tcatgcgccg gttaaggcta aactgaaagg       660
   acaagttttg gtgactgcgc tcctccaagc cagttacctc ggttcaaaga gttggtagct       720
   cagagaacct tcgaaaaacc gccctgcaag gcggtttttt cgttttcaga gcaagagatt       780
   acgcgcagac caaaacgatc tcaagaagat catcttatta atcagataaa atatttctag       840
   atttcagtgc aatttatctc ttcaaatgta gcacctgaag tcagccccat acgatataag       900
   ttgtaattct catgtttgac agcttatcat cgataagctt taatgcggta gtttatcaca       960
   gttaaattgc taacgcagtc aggcaccgtg tatgaaatct aacaatgcgc tcatcgtcat      1020
   cctcggcacc gtcaccctgg atgctgtagg cataggcttg gttatgccgg tactgccggg      1080
   cctcttgcgg gatatcgtcc attccgacag catcgccagt cactatggcg tgctgctagc      1140
   gctatatgcg ttgatgcaat ttctatgcgc acccgttctc ggagcactgt ccgaccgctt      1200
   tggccgccgc ccagtcctgc tcgcttcgct acttggagcc actatcgact acgcgatcat      1260
   ggcgaccaca cccgtcctgt ggatctaaag gcacagcgtc tcatgcttcg cacaatgtaa      1320
   aactgcttca gaacctggcg agagctatcc gcgcggtcta cggttaactg atactaaaag      1380
   acaattcagc gggtaacctt gcaatggtga gtggcagtaa agcgggcgtt tcgcctcatc      1440
   gcgaaataga agtaatgaga caatccattg acgatcacct ggctggcctg ttacctgaaa      1500
   ccgacagcca ggatatcgtc agccttgcga tgcgtgaagg cgtcatggca cccggtaaac      1560
   ggatccgtcc gctgctgatg ctgctggccg cccgcgacct ccgctaccag ggcagtatgc      1620
   ctacgctgct cgatctcgcc tgcgccgttg aactgaccca taccgcgtcg ctgatgctcg      1680
   acgacatgcc ctgcatggac aacgccgagc tgcgccgcgg tcagcccact acccacaaaa      1740
   aatttggtga gagcgtggcg atccttgcct ccgttgggct gctctctaaa gcctttggtc      1800
   tgatcgccgc caccggcgat ctgccggggg agaggcgtgc ccaggcggtc aacgagctct      1860
   ctaccgccgt gggcgtgcag ggcctggtac tggggcagtt tcgcgatctt aacgatgccg      1920
   ccctcgaccg tacccctgac gctatcctca gcaccaacca cctcaagacc ggcattctgt      1980
   tcagcgcgat gctgcagatc gtcgccattg cttccgcctc gtcgccgagc acgcgagaga      2040
   cgctgcacgc cttcgccctc gacttcggcc aggcgtttca actgctggac gatctgcgtg      2100
   acgatcaccc ggaaaccggt aaagatcgca ataaggacgc gggaaaatcg acgctggtca      2160
   accggctggg cgcagacgcg gcccggcaaa agctgcgcga gcatattgat tccgccgaca      2220
   aacacctcac ttttgcctgt ccgcagggcg gcgccatccg acagtttatg catctgtggt 2280
   ttggccatca ccttgccgac tggtcaccgg tcatgaaaat cgcctgatac cgcccttttg 2340
   ggttcaagca gtacataacg atggaaccac attacaggag tagtgatgaa tgaaggacga 2400
   gcgccttgtt cagcgtaaga acgatcatct ggatatcgtt ctcgaccccc gtcgcgccgt 2460
   aactcaggct agcgcaggtt ttgagcgctg gcgctttacc cactgcgccc tgccagagct 2520
   gaattttagc gacatcacgc tggaaaccac cttcctgaat cggcagctac aggctccgct 2580
   gctgatcagc tccatgaccg gcggcgttga gcgctcgcgc catatcaacc gccacctcgc 2640
   cgaggcggcg caggtgctaa aaattgcgat gggggtgggc tcccagcgcg tcgccattga 2700
   gagcgacgcg ggcttagggc tggataaaac cctgcggcag ctggctccgg acgtgccgct 2760
   gctggcgaac ctcggcgcgg cgcagctgac cggcagaaaa ggtattgatt acgcccgacg 2820
   ggccgtggag atgatcgagg cggatgcgct gattgtgcac ctaaacccgc tgcaggaggc 2880
   gctacagccc ggcggcgatc gcgactggcg cggacggctg gcggctattg aaactctggt 2940
   ccgcgagctg cccgttccgc tggtggtgaa agaggtggga gccggtatct cccgaaccgt 3000
   ggccgggcag ctgatcgatg ccggcgttac cgtgattgac gtcgcgggcg cgggcggcac 3060
   cagctgggcc gccgttgaag gcgagcgggc ggccaccgag cagcagcgca gcgtggccaa 3120
   cgtctttgcc gactggggga tccccaccgc tgaggcgctg gttgacattg ccgaggcctg 3180
   gccgcagatg ccccttattg cctcgggcgg gattaaaaac ggcgtcgacg cggcgaaagc 3240
   gctgcggctc ggcgcgtgca tggtagggca ggccgccgcc gtgctcggca gcgcaggcgt 3300
   ctccacggag aaggtgatcg atcacttcaa cgtgattatt gagcagctgc gggtggcctg 3360
   cttctgcacc ggcagccgca gcctgagcga tctaaagcag gctgatatcc gctatgtgcg 3420
   ggatacgcca tgagccattt tgccattgtg gcaccgccgc tctacagtca tgcggtggcg 3480
   ctgcatgccc tggcgctgga gatggcccaa cgcggccacc ggtaattgat ttagaggagt 3540
   tagtcttgaa gtcatgcgcc ggttaaggct aaactgaaag gacaagtttt ggtgactgcg 3600
   ctcctccaag ccagttacct cggttcaaag agttggtagc tcagagaacc ttcgaaaaac 3660
   cgccctgcaa ggcggttttt tcgttttcag agcaagagat tacgcgcaga ccaaaacgat 3720
   ctcaagaaga tcatcttatt aatcagataa aatatttcta gatttcagtg caatttatct 3780
   cttcaaatgt agcacctgaa gtcagcccca tacgatataa gttgtaattc tcatgtttga 3840
   cagcttatca tcgataagct cgaccagccg cgctacgttg ccgaggctaa tctggtgatc 3900
   acccacggcg gtctcaatac cgtactggat gcgctggctg ccgcgacgcc ggtgctggcg 3960
   gtgccactct ctttcgacca gcccgccgtg gctgcccggc tggtctataa cgggctgggt 4020
   cgccgggtat cgcgctttgc cagacagcag acgctggcgg atgagattgc ccaactgctg 4080
   ggggatgaga cgctgcatca gcgtctggcg acggcccgcc agcagcttaa cgacgccggg 4140
   ggcacgcccc gtgcggcgac cctgattgaa caggccatag cagggagtga gagcgtatca      4200
   tgaaaaaaac cgttgtgatt ggcgcaggct ttggtggcct ggcgctggcg attcgcctgc      4260
   aggcggcagg gatcccaacc gtactgctgg agcagcggga caagcccggc ggtcgggcct      4320
   acgtctggca tgaccagggc tttacctttg acgccgggcc gacggtgatc accgatccta      4380
   ccgcgcttga ggcgctgttc accctggccg gcaggcgcat ggaggattac gtcaggctgc      4440
   tgccggtaaa acccttctac cgactctgct gggagtccgg gaagaccctc gactatgcta      4500
   acgacagcgc cgagcttgag gcgcagatta cccagttcaa cccccgcgac gtcgagggct      4560
   accggcgctt tctggcttac tcccaggcgg tattccagga gggatatttg cgcctcggca      4620
   gcgtgccgtt cctctctttt cgcgacatgc tgcgcgccgg gccgcagctg cttaagctcc      4680
   aggcgtggca gagcgtctac cagtcggttt cgcgctttat tgaggatgag catctgcggc      4740
   aggccttctc gttccactcc ctgctggtag gcggcaaccc cttcaccacc tcgtccatct      4800
   acaccctgat ccacgccctt gagcgggagt ggggggtctg gttccctgag ggcggcaccg      4860
   gggcgctggt gaacggcatg gtgaagctgt ttaccgatct gggcggggag atcgaactca      4920
   acgcccgggt cgaagagctg gtggtggccg ataaccgcgt aagccaggtc cggctggcgg      4980
   atggtcggat ctttgacacc gacgccgtag cctcgaacgc tgacgtggtg aacacctata      5040
   aaaagctgct cggccaccat ccggtggggc agaagcgggc ggcagcgctg gagcgcaaga      5100
   gcatgagcaa ctcgctgttt gtgctctact tcggcctgaa ccagcctcat tcccagctgg      5160
   cgcaccatac catctgtttt ggtccccgct accgggagct gatcgacgag atctttaccg      5220
   gcagcgcgct ggcggatgac ttctcgctct acctgcactc gccctgcgtg accgatccct      5280
   cgctcgcgcc tcccggctgc gccagcttct acgtgctggc cccggtgccg catcttggca      5340
   acgcgccgct ggactgggcg caggaggggc cgaagctgcg cgaccgcatc tttgactacc      5400
   ttgaagagcg ctatatgccc ggcctgcgta gccagctggt gacccagcgg atctttaccc      5460
   cggcagactt ccacgacacg ctggatgcgc atctgggatc ggccttctcc atcgagccgc      5520
   tgctgaccca aagcgcctgg ttccgcccgc acaaccgcga cagcgacatt gccaacctct      5580
   acctggtggg cgcaggtact caccctgggg cgggcattcc tggcgtagtg gcctcggcga      5640
   aagccaccgc cagcctgatg attgaggatc tgcaatgagc caaccgccgc tgcttgacca      5700
   cgccacgcag accatggcca acggctcgaa aagttttgcc accgctgcga agctgttcga      5760
   cccggccacc cgccgtagcg tgctgatgct ctacacctgg tgccgccact gcgatgacgt      5820
   cattgacgac cagacccacg gcttcgccag cgaggccgcg gcggaggagg aggccaccca      5880
   gcgcctggcc cggctgcgca cgctgaccct ggcggcgttt gaaggggccg agatgcagga      5940
   tccggccttc gctgcctttc aggaggtggc gctgacccac ggtattacgc cccgcatggc      6000
   gctcgatcac ctcgacggct ttgcgatgga cgtggctcag acccgctatg tcacctttga 6060
   ggatacgctg cgctactgct atcacgtggc gggcgtggtg ggtctgatga tggccagggt 6120
   gatgggcgtg cgggatgagc gggtgctgga tcgcgcctgc gatctggggc tggccttcca 6180
   gctgacgaat atcgcccggg atattattga cgatgcggct attgaccgct gctatctgcc 6240
   cgccgagtgg ctgcaggatg ccgggctgac cccggagaac tatgccgcgc gggagaatcg 6300
   ggccgcgctg gcgcgggtgg cggagcggct tattgatgcc gcagagccgt actacatctc 6360
   ctcccaggcc gggctacacg atctgccgcc gcgctgcgcc tgggcgatcg ccaccgcccg 6420
   cagcgtctac cgggagatcg gtattaaggt aaaagcggcg ggaggcagcg cctgggatcg 6480
   ccgccagcac accagcaaag gtgaaaaaat tgccatgctg atggcggcac cggggcaggt 6540
   tattcgggcg aagacgacga gggtgacgcc gcgtccggcc ggtctttggc agcgtcccgt 6600
   ttaggcgggc ggccatgacg ttcacgcagg atcgcctgta ggtcggcagg cttgcgggcg 6660
   taaataaaac cgaaggagac gcagccctcc cggccgcgca ccgcgtggtg caggcggtgg 6720
   gcgacgtaga gccgcttcag gtagccccgg cgcgggatcc cgcctcgccg tgctgtccgg 6780
   tctcaacctg atccgccaga atcgagccaa cgggatcggc cagcacgaat tcggtatgcg 6840
   gagagtgttt ggcaaaccat gcctgcaagc cacccagcgt gccgccggag ccaacgccta 6900
   ctaccacggc atcaaccctg cctgccagct ggtcgaacag ctccggtgcg gtggtggtgg 6960
   cgtgcgccag cgggttggcc gggttagaga actgatcaat atagtaagca cccggcgtct 7020
   cctctgccag gcggcgggcg tagtcctgat agtactccgg gtggcccttt gtcacgtcgg 7080
   agcgggtcag gcgcacatca acacccagcg cacgcaggtg gtagatcctc tacgccggac 7140
   gcatcgtggc cggcatcacc ggcgccacag gtgcggttgc tggcgcctat atcgccgaca 7200
   tcaccgatgg ggaagatcgg gctcgccact tcgggctcat gagcgcttgt ttcggcgtgg 7260
   gtatggtggc aggccccgtg gccgggggac tgttgggcgc catctccttg gggtcgaatt 7320
   tgctttcgaa tttctgccat tcatccgctt attatcactt attcaggcgt agcaccaggc 7380
   gtttaagggc accaataact gccttaaaaa aattacgccc cgccctgcca ctcatcgcag 7440
   tactgttgta attcattaag cattctgccg acatggaagc catcacagac ggcatgatga 7500
   acctgaatcg ccagcggcat cagcaccttg tcgccttgcg tataatattt gcccatggtg 7560
   aaaacggggg cgaagaagtt gtccatattg gccacgttta aatcaaaact ggtgaaactc 7620
   acccagggat tggctgagac gaaaaacata ttctcaataa accctttagg gaaataggcc 7680
   aggttttcac cgtaacacgc cacatcttgc gaatatatgt gtagaaactg ccggaaatcg 7740
   tcgtggtatt cactccagag cgatgaaaac gtttcagttt gctcatggaa aacggtgtaa 7800
   caagggtgaa cactatccca tatcaccagc tcaccgtctt tcattgccat acggaattcc 7860
   ggatgagcat tcatcaggcg ggcaagaatg tgaataaagg ccggataaaa cttgtgctta 7920
   tttttcttta cggtctttaa aaaggccgta atatccagct gaacggtctg gttataggta 7980
   cattgagcaa ctgactgaaa tgcctcaaaa tgttctttac gatgccattg ggatatatca 8040
   acggtggtat atccagtgat ttttttctcc attttagctt ccttagctcc tgaaaatctc 8100
   gataactcaa aaaatacgcc cggtagtgat cttatttcat tatggtgaaa gttggaacct 8160
   cttacgtgcc gatcaacgtc tcattttcgc caaaagttgg cccagggctt cccggtatca 8220
   acagggacac caggatttat ttattctgcg aagtgatctt ccgtcacagg tatttattcg 8280
   gcgcaaagtg cgtcgggtga tgctgccaac ttactgattt agtgtatgat ggtgtttttg 8340
   aggtgctcca gtggcttctg tttctatcag ctgtccctcc tgttcagcta ctgacggggt 8400
   ggtgcgtaac ggcaaaagca ccgccggaca tcagcgctag cg 8442
<210> 29
   <211> 8632
   <212> DNA
   <213> Artifizielle Sequenz
<220>
   <223> pGT1066-AtEC-del
<400> 29
   ttgtaattct catgtttgac agcttatcat cgataagctt taatgcggta gtttatcaca      60
   gttaaattgc taacgcagtc aggaaccttg caatggtgag tggcagtaaa gcgggcgttt     120
   cgcctcatcg cgaaatagaa gtaatgagac aatccattga cgatcacctg gctggcctgt     180
   tacctgaaac cgacagccag gatatcgtca gccttgcgat gcgtgaaggc gtcatggcac     240
   ccggtaaacg gatccgtccg ctgctgatgc tgctggccgc ccgcgacctc cgctaccagg     300
   gcagtatgcc tacgctgctc gatctcgcct gcgccgttga actgacccat accgcgtcgc     360
   tgatgctcga cgacatgccc tgcatggaca acgccgagct gcgccgcggt cagcccacta     420
   cccacaaaaa atttggtgag agcgtggcga tccttgcctc cgttgggctg ctctctaaag     480
   cctttggtct gatcgccgcc accggcgatc tgccggggga gaggcgtgcc caggcggtca     540
   acgagctctc taccgccgtg ggcgtgcagg gcctggtact ggggcagttt cgcgatctta     600
   acgatgccgc cctcgaccgt acccctgacg ctatcctcag caccaaccac ctcaagaccg     660
   gcattctgtt cagcgcgatg ctgcagatcg tcgccattgc ttccgcctcg tcgccgagca     720
   cgcgagagac gctgcacgcc ttcgccctcg acttcggcca ggcgtttcaa ctgctggacg     780
   atctgcgtga cgatcacccg gaaaccggta aagatcgcaa taaggacgcg ggaaaatcga     840
   cgctggtcaa ccggctgggc gcagacgcgg cccggcaaaa gctgcgcgag catattgatt     900
   ccgccgacaa acacctcact tttgcctgtc cgcagggcgg cgccatccga cagtttatgc     960
   atctgtggtt tggccatcac cttgccgact ggtcaccggt catgaaaatc gcctgatacc    1020
   gcccttttgg gttcaagcag tacataacga tggaaccaca ttacaggagt agtgatgaat 1080
   gaaggacgag cgccttgttc agcgtaagaa cgatcatctg gatatcgttc tcgacccccg 1140
   tcgcgccgta actcaggcta gcgcaggttt tgagcgctgg cgctttaccc actgcgccct 1200
   gccagagctg aattttagcg acatcacgct ggaaaccacc ttcctgaatc ggcagctaca 1260
   ggctccgctg ctgatcagct ccatgaccgg cggcgttgag cgctcgcgcc atatcaaccg 1320
   ccacctcgcc gaggcggcgc aggtgctaaa aattgcgatg ggggtgggct cccagcgcgt 1380
   cgccattgag agcgacgcgg gcttagggct ggataaaacc ctgcggcagc tggctccgga 1440
   cgtgccgctg ctggcgaacc tcggcgcggc gcagctgacc ggcagaaaag gtattgatta 1500
   cgcccgacgg gccgtggaga tgatcgaggc ggatgcgctg attgtgcacc taaacccgct 1560
   gcaggaggcg ctacagcccg gcggcgatcg cgactggcgc ggacggctgg cggctattga 1620
   aactctggtc cgcgagctgc ccgttccgct ggtggtgaaa gaggtgggag ccggtatctc 1680
   ccgaaccgtg gccgggcagc tgatcgatgc cggcgttacc gtgattgacg tcgcgggcgc 1740
   gggcggcacc agctgggccg ccgttgaagg cgagcgggcg gccaccgagc agcagcgcag 1800
   cgtggccaac gtctttgccg actgggggat ccccaccgct gaggcgctgg ttgacattgc 1860
   cgaggcctgg ccgcagatgc cccttattgc ctcgggcggg attaaaaacg gcgtcgacgc 1920
   ggcgaaagcg ctgcggctcg gcgcgtgcat ggtagggcag gccgccgccg tgctcggcag 1980
   cgcaggcgtc tccacggaga aggtgatcga tcacttcaac gtgattattg agcagctgcg 2040
   ggtggcctgc ttctgcaccg gcagccgcag cctgagcgat ctaaagcagg ctgatatccg 2100
   ctatgttcgt gatacgccat aaggaggtac aaccatgaag aaaaccgttg tgattggcgc 2160
   aggctttggt ggcctggcgc tggcgattcg cctgcaggcg gcagggatcc caaccgtact 2220
   gctggagcag cgggacaagc ccggcggtcg ggcctacgtc tggcatgacc agggctttac 2280
   ctttgacgcc gggccgacgg tgatcaccga tcctaccgcg cttgaggcgc tgttcaccct 2340
   ggccggcagg cgcatggagg attacgtcag gctgctgccg gtaaaaccct tctaccgact 2400
   ctgctgggag tccgggaaga ccctcgacta tgctaacgac agcgccgagc ttgaggcgca 2460
   gattacccag ttcaaccccc gcgacgtcga gggctaccgg cgctttctgg cttactccca 2520
   ggcggtattc caggagggat atttgcgcct cggcagcgtg ccgttcctct cttttcgcga 2580
   catgctgcgc gccgggccgc agctgcttaa gctccaggcg tggcagagcg tctaccagtc 2640
   ggtttcgcgc tttattgagg atgagcatct gcggcaggcc ttctcgttcc actccctgct 2700
   ggtaggcggc aaccccttca ccacctcgtc catctacacc ctgatccacg cccttgagcg 2760
   ggagtggggg gtctggttcc ctgagggcgg caccggggcg ctggtgaacg gcatggtgaa 2820
   gctgtttacc gatctgggcg gggagatcga actcaacgcc cgggtcgaag agctggtggt 2880
   ggccgataac cgcgtaagcc aggtccggct ggcggatggt cggatctttg acaccgacgc 2940
   cgtagcctcg aacgctgacg tggtgaacac ctataaaaag ctgctcggcc accatccggt 3000
   ggggcagaag cgggcggcag cgctggagcg caagagcatg agcaactcgc tgtttgtgct 3060
   ctacttcggc ctgaaccagc ctcattccca gctggcgcac cataccatct gttttggtcc 3120
   ccgctaccgg gagctgatcg acgagatctt taccggcagc gcgctggcgg atgacttctc 3180
   gctctacctg cactcgccct gcgtgaccga tccctcgctc gcgcctcccg gctgcgccag 3240
   cttctacgtg ctggccccgg tgccgcatct tggcaacgcg ccgctggact gggcgcagga 3300
   ggggccgaag ctgcgcgacc gcatctttga ctaccttgaa gagcgctata tgcccggcct 3360
   gcgtagccag ctggtgaccc agcggatctt taccccggca gacttccacg acacgctgga 3420
   tgcgcatctg ggatcggcct tctccatcga gccgctgctg acccaaagcg cctggttccg 3480
   cccgcacaac cgcgacagcg acattgccaa cctctacctg gtgggcgcag gtactcaccc 3540
   tggggcgggc attcctggcg tagtggcctc ggcgaaagcc accgccagcc tgatgattga 3600
   ggatctgcaa tgagccaacc gccgctgctt gaccacgcca cgcagaccat ggccaacggc 3660
   tcgaaaagtt ttgccaccgc tgcgaagctg ttcgacccgg ccacccgccg tagcgtgctg 3720
   atgctctaca cctggtgccg ccactgcgat gacgtcattg acgaccagac ccacggcttc 3780
   gccagcgagg ccgcggcgga ggaggaggcc acccagcgcc tggcccggct gcgcacgctg 3840
   accctggcgg cgtttgaagg ggccgagatg caggatccgg ccttcgctgc ctttcaggag 3900
   gtggcgctga cccacggtat tacgccccgc atggcgctcg atcacctcga cggctttgcg 3960
   atggacgtgg ctcagacccg ctatgtcacc tttgaggata cgctgcgcta ctgctatcac 4020
   gtggcgggcg tggtgggtct gatgatggcc agggtgatgg gcgtgcggga tgagcgggtg 4080
   ctggatcgcg cctgcgatct ggggctggcc ttccagctga cgaatatcgc ccgggatatt 4140
   attgacgatg cggctattga ccgctgctat ctgcccgccg agtggctgca ggatgccggg 4200
   ctgaccccgg agaactatgc cgcgcgggag aatcgggccg cgctggcgcg ggtggcggag 4260
   cggcttattg atgccgcaga gccgtactac atctcctccc aggccgggct acacgatctg 4320
   ccgccgcgct gcgcctgggc gatcgccacc gcccgcagcg tctaccggga gatcggtatt 4380
   aaggtaaaag cggcgggagg cagcgcctgg gatcgccgcc agcacaccag caaaggtgaa 4440
   aaaattgcca tgctgatggc ggcaccgggg caggttattc gggcgaagac gacgagggtg 4500
   acgccgcgtc cggccggtct ttggcagcgt cccgtttaga ccgactccaa acgagtcggt 4560
   ttttttgcgc ttgtttcggc gtgggtatgg tggcaggccc cgtggccggg ggactgttgg 4620
   gcgccatctc cttggggtcg aatttgcttt cgaatttctg ccattcatcc gcttattatc 4680
   acttattcag gcgtagcaac caggcgttta agggcaccaa taactgcctt aaaaaaatta 4740
   cgccccgccc tgccactcat cgcagtactg ttgtaattca ttaagcattc tgccgacatg 4800
   gaagccatca cagacggcat gatgaacctg aatcgccagc ggcatcagca ccttgtcgcc 4860
   ttgcgtataa tatttgccca tggtgaaaac gggggcgaag aagttgtcca tattggccac 4920
   gtttaaatca aaactggtga aactcaccca gggattggct gagacgaaaa acatattctc 4980
   aataaaccct ttagggaaat aggccaggtt ttcaccgtaa cacgccacat cttgcgaata 5040
   tatgtgtaga aactgccgga aatcgtcgtg gtattcactc cagagcgatg aaaacgtttc 5100
   agtttgctca tggaaaacgg tgtaacaagg gtgaacacta tcccatatca ccagctcacc 5160
   gtctttcatt gccatacgga attccggatg agcattcatc aggcgggcaa gaatgtgaat 5220
   aaaggccgga taaaacttgt gcttattttt ctttacggtc tttaaaaagg ccgtaatatc 5280
   cagctgaacg gtctggttat aggtacattg agcaactgac tgaaatgcct caaaatgttc 5340
   tttacgatgc cattgggata tatcaacggt ggtatatcca gtgatttttt tctccatttt 5400
   agcttcctta gctcctgaaa atctcgataa ctcaaaaaat acgcccggta gtgatcttat 5460
   ttcattatgg tgaaagttgg aacctcttac gtgccgatca acgtctcatt ttcgccaaaa 5520
   gttggcccag ggcttcccgg tatcaacagg gacaccagga tttatttatt ctgcgaagtg 5580
   atcttccgtc acaggtattt attcggcgca aagtgcgtcg ggtgatgctg ccaacttact 5640
   gatttagtgt atgatggtgt ttttgaggtg ctccagtggc ttctgtttct atcagctgtc 5700
   cctcctgttc agctactgac ggggtggtgc gtaacggcaa aagcaccgcc ggacatcagc 5760
   gctagcggag tgtatacact ccgctatcgc tacgtgactg ggtcatggct gcgccccgac 5820
   acccgccaac acccgctgac gcgccctgac gggcttgtct gctcccggca tccgcttaca 5880
   gacaagctgt gaccgtctcc gggagctgca tgtgtcagag gttttcaccg tcatcaccga 5940
   aacgcgcgag gcagctggca cgacaggttt cccgactgga aagcgggcag tgagcgcaac 6000
   gcaattaatg tgagttagct cactcattag gcaccccagg ctttacactt tatgcttccg 6060
   gctcgtatgt tgtgtggaat tgtgagcgga taacaatttc acacaggaaa cagctatgac 6120
   catgattacg ccaagctcta gctagaaata attttgttta actttaagaa ggagatatac 6180
   ccatgacaca gagggcccac catcaccatc accattccat ggctagcggc ggcggaagtt 6240
   ccggtagtga gagttgtgta gcggtgagag aagatttcgc tgacgaagaa gattttgtga 6300
   aagctggtgg ttctgagatt ctatttgttc aaatgcagca gaacaaagat atggatgaac 6360
   agtctaagct tgttgataag ttgcctccta tatcaattgg tgatggtgct ttggatctag 6420
   tggttattgg ttgtggtcct gctggtttag ccttggctgc agaatcagct aagcttggat 6480
   taaaagttgg actcattggt ccagatcttc cttttactaa caattacggt gtttgggaag 6540
   atgaattcaa tgatcttggg ctgcaaaaat gtattgagca tgtttggaga gagactattg 6600
   tgtatctgga tgatgacaag cctattacca ttggccgtgc ttatggaaga gttagtcgac 6660
   gtttgctcca tgaggagctt ttgaggaggt gtgtcgagtc aggtgtctcg taccttagct 6720
   cgaaagttga cagcataaca gaagcttctg atggccttag acttgttgct tgtgacgaca 6780
   ataacgtcat tccctgcagg cttgccactg ttgcttctgg agcagcttcg ggaaagctct 6840
   tgcaatacga agttggtgga cctagagtct gtgtgcaaac tgcatacggc gtggaggttg 6900
   aggtggaaaa tagtccatat gatccagatc aaatggtttt catggattac agagattata 6960
   ctaacgagaa agttcggagc ttagaagctg agtatccaac gtttctgtac gccatgccta 7020
   tgacaaagtc aagactcttc ttcgaggaga catgtttggc ctcaaaagat gtcatgccct 7080
   ttgatttgct aaaaacgaag ctcatgttaa gattagatac actcggaatt cgaattctaa 7140
   agacttacga agaggagtgg tcctatatcc cagttggtgg ttccttgcca aacaccgaac 7200
   aaaagaatct cgcctttggt gctgccgcta gcatggtaca tcccgcaaca ggctattcag 7260
   ttgtgagatc tttgtctgaa gctccaaaat atgcatcagt catcgcagag atactaagag 7320
   aagagactac caaacagatc aacagtaata tttcaagaca agcttgggat actttatggc 7380
   caccagaaag gaaaagacag agagcattct ttctctttgg tcttgcactc atagttcaat 7440
   tcgataccga aggcattaga agcttcttcc gtactttctt ccgccttcca aaatggatgt 7500
   ggcaagggtt tctaggatca acattaacat caggagatct cgttctcttt gctttataca 7560
   tgttcgtcat ttcaccaaac aatttgagaa aaggtctcat caatcatctc atctctgatc 7620
   caaccggagc aaccatgata aaaacctatc tcaaagtatg aggatccggc tgctaacaaa 7680
   gcccgttctg tttaagaacg ggattttttg ctgaaaggag gaactatatc cggccggatt 7740
   actggcttac tatgttggca ctgatgaggg tgtcagtgaa gtgcttcatg tggcaggaga 7800
   aaaaaggctg caccggtgcg tcagcagaat atgtgataca ggatatattc cgcttcctcg 7860
   ctcactgact cgctacgctc ggtcgttcga ctgcggcgag cggaaatggc ttacgaacgg 7920
   ggcggagatt tcctggaaga tgccaggaag atacttaaca gggaagtgag agggccgcgg 7980
   caaagccgtt tttccatagg ctccgccccc ctgacaagca tcacgaaatc tgacgctcaa 8040
   atcagtggtg gcgaaacccg acaggactat aaagatacca ggcgtttccc cctggcggct 8100
   ccctcgtgcg ctctcctgtt cctgcctttc ggtttaccgg tgtcattccg ctgttatggc 8160
   cgcgtttgtc tcattccacg cctgacactc agttccgggt aggcagttcg ctccaagctg 8220
   gactgtatgc acgaaccccc cgttcagtcc gaccgctgcg ccttatccgg taactatcgt 8280
   cttgagtcca acccggaaag acatgcaaaa gcaccactgg cagcagccac tggtaattga 8340
   tttagaggag ttagtcttga agtcatgcgc cggttaaggc taaactgaaa ggacaagttt 8400
   tggtgactgc gctcctccaa gccagttacc tcggttcaaa gagttggtag ctcagagaac 8460
   cttcgaaaaa ccgccctgca aggcggtttt ttcgttttca gagcaagaga ttacgcgcag 8520
   accaaaacga tctcaagaag atcatcttat taatcagata aaatatttct agatttcagt 8580
   gcaatttatc tcttcaaatg tagcacctga agtcagcccc atacgatata ag 8632
<210> 30
   <211> 8632
   <212> DNA
   <213> Artifizielle Sequenz
<220>
   <223> Plasmid pGT1182
<400> 30
   ttgtaattct catgtttgac agcttatcat cgataagctt taatgcggta gtttatcaca      60
   gttaaattgc taacgcagtc aggaaccttg caatggtgag tggcagtaaa gcgggcgttt     120
   cgcctcatcg cgaaatagaa gtaatgagac aatccattga cgatcacctg gctggcctgt     180
   tacctgaaac cgacagccag gatatcgtca gccttgcgat gcgtgaaggc gtcatggcac     240
   ccggtaaacg gatccgtccg ctgctgatgc tgctggccgc ccgcgacctc cgctaccagg     300
   gcagtatgcc tacgctgctc gatctcgcct gcgccgttga actgacccat accgcgtcgc     360
   tgatgctcga cgacatgccc tgcatggaca acgccgagct gcgccgcggt cagcccacta     420
   cccacaaaaa atttggtgag agcgtggcga tccttgcctc cgttgggctg ctctctaaag     480
   cctttggtct gatcgccgcc accggcgatc tgccggggga gaggcgtgcc caggcggtca     540
   acgagctctc taccgccgtg ggcgtgcagg gcctggtact ggggcagttt cgcgatctta     600
   acgatgccgc cctcgaccgt acccctgacg ctatcctcag caccaaccac ctcaagaccg     660
   gcattctgtt cagcgcgatg ctgcagatcg tcgccattgc ttccgcctcg tcgccgagca     720
   cgcgagagac gctgcacgcc ttcgccctcg acttcggcca ggcgtttcaa ctgctggacg     780
   atctgcgtga cgatcacccg gaaaccggta aagatcgcaa taaggacgcg ggaaaatcga     840
   cgctggtcaa ccggctgggc gcagacgcgg cccggcaaaa gctgcgcgag catattgatt     900
   ccgccgacaa acacctcact tttgcctgtc cgcagggcgg cgccatccga cagtttatgc     960
   atctgtggtt tggccatcac cttgccgact ggtcaccggt catgaaaatc gcctgatacc    1020
   gcccttttgg gttcaagcag tacataacga tggaaccaca ttacaggagt agtgatgaat    1080
   gaaggacgag cgccttgttc agcgtaagaa cgatcatctg gatatcgttc tcgacccccg    1140
   tcgcgccgta actcaggcta gcgcaggttt tgagcgctgg cgctttaccc actgcgccct    1200
   gccagagctg aattttagcg acatcacgct ggaaaccacc ttcctgaatc ggcagctaca    1260
   ggctccgctg ctgatcagct ccatgaccgg cggcgttgag cgctcgcgcc atatcaaccg    1320
   ccacctcgcc gaggcggcgc aggtgctaaa aattgcgatg ggggtgggct cccagcgcgt     1380
   cgccattgag agcgacgcgg gcttagggct ggataaaacc ctgcggcagc tggctccgga    1440
   cgtgccgctg ctggcgaacc tcggcgcggc gcagctgacc ggcagaaaag gtattgatta    1500
   cgcccgacgg gccgtggaga tgatcgaggc ggatgcgctg attgtgcacc taaacccgct    1560
   gcaggaggcg ctacagcccg gcggcgatcg cgactggcgc ggacggctgg cggctattga 1620
   aactctggtc cgcgagctgc ccgttccgct ggtggtgaaa gaggtgggag ccggtatctc 1680
   ccgaaccgtg gccgggcagc tgatcgatgc cggcgttacc gtgattgacg tcgcgggcgc 1740
   gggcggcacc agctgggccg ccgttgaagg cgagcgggcg gccaccgagc agcagcgcag 1800
   cgtggccaac gtctttgccg actgggggat ccccaccgct gaggcgctgg ttgacattgc 1860
   cgaggcctgg ccgcagatgc cccttattgc ctcgggcggg attaaaaacg gcgtcgacgc 1920
   ggcgaaagcg ctgcggctcg gcgcgtgcat ggtagggcag gccgccgccg tgctcggcag 1980
   cgcaggcgtc tccacggaga aggtgatcga tcacttcaac gtgattattg agcagctgcg 2040
   ggtggcctgc ttctgcaccg gcagccgcag cctgagcgat ctaaagcagg ctgatatccg 2100
   ctatgttcgt gatacgccat aaggaggtac aaccatgaag aaaaccgttg tgattggcgc 2160
   aggctttggt ggcctggcgc tggcgattcg cctgcaggcg gcagggatcc caaccgtact 2220
   gctggagcag cgggacaagc ccggcggtcg ggcctacgtc tggcatgacc agggctttac 2280
   ctttgacgcc gggccgacgg tgatcaccga tcctaccgcg cttgaggcgc tgttcaccct 2340
   ggccggcagg cgcatggagg attacgtcag gctgctgccg gtaaaaccct tctaccgact 2400
   ctgctgggag tccgggaaga ccctcgacta tgctaacgac agcgccgagc ttgaggcgca 2460
   gattacccag ttcaaccccc gcgacgtcga gggctaccgg cgctttctgg cttactccca 2520
   ggcggtattc caggagggat atttgcgcct cggcagcgtg ccgttcctct cttttcgcga 2580
   catgctgcgc gccgggccgc agctgcttaa gctccaggcg tggcagagcg tctaccagtc 2640
   ggtttcgcgc tttattgagg atgagcatct gcggcaggcc ttctcgttcc actccctgct    2700
   ggtaggcggc aaccccttca ccacctcgtc catctacacc ctgatccacg cccttgagcg    2760
   ggagtggggg gtctggttcc ctgagggcgg caccggggcg ctggtgaacg gcatggtgaa 2820
   gctgtttacc gatctgggcg gggagatcga actcaacgcc cgggtcgaag agctggtggt 2880
   ggccgataac cgcgtaagcc aggtccggct ggcggatggt cggatctttg acaccgacgc 2940
   cgtagcctcg aacgctgacg tggtgaacac ctataaaaag ctgctcggcc accatccggt 3000
   ggggcagaag cgggcggcag cgctggagcg caagagcatg agcaactcgc tgtttgtgct 3060
   ctacttcggc ctgaaccagc ctcattccca gctggcgcac cataccatct gttttggtcc 3120
   ccgctaccgg gagctgatcg acgagatctt taccggcagc gcgctggcgg atgacttctc 3180
   gctctacctg cactcgccct gcgtgaccga tccctcgctc gcgcctcccg gctgcgccag 3240
   cttctacgtg ctggccccgg tgccgcatct tggcaacgcg ccgctggact gggcgcagga 3300
   ggggccgaag ctgcgcgacc gcatctttga ctaccttgaa gagcgctata tgcccggcct 3360
   gcgtagccag ctggtgaccc agcggatctt taccccggca gacttccacg acacgctgga 3420
   tgcgcatctg ggatcggcct tctccatcga gccgctgctg acccaaagcg cctggttccg 3480
   cccgcacaac cgcgacagcg acattgccaa cctctacctg gtgggcgcag gtactcaccc 3540
   tggggcgggc attcctggcg tagtggcctc ggcgaaagcc accgccagcc tgatgattga 3600
   ggatctgcaa tgagccaacc gccgctgctt gaccacgcca cgcagaccat ggccaacggc 3660
   tcgaaaagtt ttgccaccgc tgcgaagctg ttcgacccgg ccacccgccg tagcgtgctg 3720
   atgctctaca cctggtgccg ccactgcgat gacgtcattg acgaccagac ccacggcttc 3780
   gccagcgagg ccgcggcgga ggaggaggcc acccagcgcc tggcccggct gcgcacgctg 3840
   accctggcgg cgtttgaagg ggccgagatg caggatccgg ccttcgctgc ctttcaggag 3900
   gtggcgctga cccacggtat tacgccccgc atggcgctcg atcacctcga cggctttgcg 3960
   atggacgtgg ctcagacccg ctatgtcacc tttgaggata cgctgcgcta ctgctatcac 4020
   gtggcgggcg tggtgggtct gatgatggcc agggtgatgg gcgtgcggga tgagcgggtg 4080
   ctggatcgcg cctgcgatct ggggctggcc ttccagctga cgaatatcgc ccgggatatt 4140
   attgacgatg cggctattga ccgctgctat ctgcccgccg agtggctgca ggatgccggg 4200
   ctgaccccgg agaactatgc cgcgcgggag aatcgggccg cgctggcgcg ggtggcggag 4260
   cggcttattg atgccgcaga gccgtactac atctcctccc aggccgggct acacgatctg 4320
   ccgccgcgct gcgcctgggc gatcgccacc gcccgcagcg tctaccggga gatcggtatt 4380
   aaggtaaaag cggcgggagg cagcgcctgg gatcgccgcc agcacaccag caaaggtgaa 4440
   aaaattgcca tgctgatggc ggcaccgggg caggttattc gggcgaagac gacgagggtg 4500
   acgccgcgtc cggccggtct ttggcagcgt cccgtttaga ccgactccaa acgagtcggt 4560
   ttttttgcgc ttgtttcggc gtgggtatgg tggcaggccc cgtggccggg ggactgttgg 4620
   gcgccatctc cttggggtcg aatttgcttt cgaatttctg ccattcatcc gcttattatc 4680
   acttattcag gcgtagcaac caggcgttta agggcaccaa taactgcctt aaaaaaatta 4740
   cgccccgccc tgccactcat cgcagtactg ttgtaattca ttaagcattc tgccgacatg 4800
   gaagccatca cagacggcat gatgaacctg aatcgccagc ggcatcagca ccttgtcgcc 4860
   ttgcgtataa tatttgccca tggtgaaaac gggggcgaag aagttgtcca tattggccac 4920
   gtttaaatca aaactggtga aactcaccca gggattggct gagacgaaaa acatattctc 4980
   aataaaccct ttagggaaat aggccaggtt ttcaccgtaa cacgccacat cttgcgaata 5040
   tatgtgtaga aactgccgga aatcgtcgtg gtattcactc cagagcgatg aaaacgtttc 5100
   agtttgctca tggaaaacgg tgtaacaagg gtgaacacta tcccatatca ccagctcacc 5160
   gtctttcatt gccatacgga attccggatg agcattcatc aggcgggcaa gaatgtgaat 5220
   aaaggccgga taaaacttgt gcttattttt ctttacggtc tttaaaaagg ccgtaatatc 5280
   cagctgaacg gtctggttat aggtacattg agcaactgac tgaaatgcct caaaatgttc 5340
   tttacgatgc cattgggata tatcaacggt ggtatatcca gtgatttttt tctccatttt 5400
   agcttcctta gctcctgaaa atctcgataa ctcaaaaaat acgcccggta gtgatcttat 5460
   ttcattatgg tgaaagttgg aacctcttac gtgccgatca acgtctcatt ttcgccaaaa 5520
   gttggcccag ggcttcccgg tatcaacagg gacaccagga tttatttatt ctgcgaagtg 5580
   atcttccgtc acaggtattt attcggcgca aagtgcgtcg ggtgatgctg ccaacttact 5640
   gatttagtgt atgatggtgt ttttgaggtg ctccagtggc ttctgtttct atcagctgtc 5700
   cctcctgttc agctactgac ggggtggtgc gtaacggcaa aagcaccgcc ggacatcagc 5760
   gctagcggag tgtatacact ccgctatcgc tacgtgactg ggtcatggct gcgccccgac 5820
   acccgccaac acccgctgac gcgccctgac gggcttgtct gctcccggca tccgcttaca 5880
   gacaagctgt gaccgtctcc gggagctgca tgtgtcagag gttttcaccg tcatcaccga 5940
   aacgcgcgag gcagctggca cgacaggttt cccgactgga aagcgggcag tgagcgcaac 6000
   gcaattaatg tgagttagct cactcattag gcaccccagg ctttacactt tatgcttccg 6060
   gctcgtatgt tgtgtggaat tgtgagcgga taacaatttc acacaggaaa cagctatgac 6120
   catgattacg ccaagctcta gctagaaata attttgttta actttaagaa ggagatatac 6180
   ccatgacaca gagggcccac catcaccatc accattccat ggctagcggc ggcggaagtt 6240
   ccggtagtga gagttgtgta gcggtgagag aagatttcgc tgacgaagaa gattttgtga 6300
   aagctggtgg ttctgagatt ctatttgttc aaatgcagca gaacaaagat atggatgaac 6360
   agtctaagct tgttgataag ttgcctccta tatcaattgg tgatggtgct ttggatctag 6420
   tggttattgg ttgtggtcct gctggtttag ccttggctgc agaatcagct aagcttggat 6480
   taaaagttgg actcattggt ccagatcttc cttttactaa caattacggt gtttgggaag 6540
   atgaattcaa tgatcttggg ctgcaaaaat gtattgagca tgtttggaga gagactattg 6600
   tgtatctgga tgatgacaag cctattacca ttggccgtgc ttatggaaga gttagtcgac 6660
   gtttgctcca tgaggagctt ttgaggaggt gtgtcgagtc aggtgtctcg taccttagct 6720
   cgaaagttga cagcataaca gaagcttctg atggccttag acttgttgct tgtgacgaca 6780
   ataacgtcat tccctgcagg cttgccactg ttgcttctgg agcagcttcg ggaaagctct 6840
   tgcaatacga agttggtgga cctagagtct gtgtgcaaac tgcatacggc gtggaggttg 6900
   aggtggaaaa tagtccatat gatccagatc aaatggtttt catggattac agagattata 6960
   ctaacgagaa agttcggagc ttagaagctg agtatccaac gtttctgtac gccatgccta 7020
   tgacaaagtc aagactcttc ttcgaggaga catgtttggc ctcaaaagat gtcatgccct 7080
   ttgatttgct aaaaacgaag ctcatgttaa gattagatac actcggaatt cgaattctaa 7140
   agacttacga agaggagtgg tcctatatcc cagttggtgg ttccttgcca aacaccgaac 7200
   aaaagaatct cgcctttggt gctgccgcta gcatggtaca tcccgcaaca ggctattcag     7260
   ttgtgagatc tttgtctgaa gctccaaaat atgcatcagt catcgcagag atactaagag     7320
   aagagactac caaacagatc aacagtaata tttcaagaca agcttgggat actttatggc     7380
   caccagaaag gaaaagacag agagcattct ttctctttgg tcttgaggct atagttcaat     7440
   tcgataccga aggcattaga agcttcttcc gtactttctt ccgccttcca aaatggatgt     7500
   ggcaagggtt tctaggatca acattaacat caggagatct cgttctcttt tctttataca     7560
   tgttcgtcat ttcaccaaac aatttgagaa aaggtctcat caatcatctc atctctgatc     7620
   caaccggagc aaccatgata aaaacctatc tcaaagtatg aggatccggc tgctaacaaa     7680
   gcccgttctg tttaagaacg ggattttttg ctgaaaggag gaactatatc cggccggatt      7740
   actggcttac tatgttggca ctgatgaggg tgtcagtgaa gtgcttcatg tggcaggaga     7800
   aaaaaggctg caccggtgcg tcagcagaat atgtgataca ggatatattc cgcttcctcg     7860
   ctcactgact cgctacgctc ggtcgttcga ctgcggcgag cggaaatggc ttacgaacgg     7920
   ggcggagatt tcctggaaga tgccaggaag atacttaaca gggaagtgag agggccgcgg     7980
   caaagccgtt tttccatagg ctccgccccc ctgacaagca tcacgaaatc tgacgctcaa     8040
   atcagtggtg gcgaaacccg acaggactat aaagatacca ggcgtttccc cctggcggct     8100
   ccctcgtgcg ctctcctgtt cctgcctttc ggtttaccgg tgtcattccg ctgttatggc     8160
   cgcgtttgtc tcattccacg cctgacactc agttccgggt aggcagttcg ctccaagctg     8220
   gactgtatgc acgaaccccc cgttcagtcc gaccgctgcg ccttatccgg taactatcgt     8280
   cttgagtcca acccggaaag acatgcaaaa gcaccactgg cagcagccac tggtaattga     8340
   tttagaggag ttagtcttga agtcatgcgc cggttaaggc taaactgaaa ggacaagttt     8400
   tggtgactgc gctcctccaa gccagttacc tcggttcaaa gagttggtag ctcagagaac     8460
   cttcgaaaaa ccgccctgca aggcggtttt ttcgttttca gagcaagaga ttacgcgcag     8520
   accaaaacga tctcaagaag atcatcttat taatcagata aaatatttct agatttcagt     8580
   gcaatttatc tcttcaaatg tagcacctga agtcagcccc atacgatata ag             8632
<210> 31
   <211> 8492
   <212> DNA
   <213> Artifizielle Sequenz
<220>
   <223> Plasmid pGT1464
<400> 31
   ttgtaattct catgtttgac agcttatcat cgataagctt taatgcggta gtttatcaca       60
   gttaaattgc taacgcagtc aggaaccttg caatggtgag tggcagtaaa gcgggcgttt      120
   cgcctcatcg cgaaatagaa gtaatgagac aatccattga cgatcacctg gctggcctgt      180
   tacctgaaac cgacagccag gatatcgtca gccttgcgat gcgtgaaggc gtcatggcac 240
   ccggtaaacg gatccgtccg ctgctgatgc tgctggccgc ccgcgacctc cgctaccagg 300
   gcagtatgcc tacgctgctc gatctcgcct gcgccgttga actgacccat accgcgtcgc 360
   tgatgctcga cgacatgccc tgcatggaca acgccgagct gcgccgcggt cagcccacta 420
   cccacaaaaa atttggtgag agcgtggcga tccttgcctc cgttgggctg ctctctaaag 480
   cctttggtct gatcgccgcc accggcgatc tgccggggga gaggcgtgcc caggcggtca 540
   acgagctctc taccgccgtg ggcgtgcagg gcctggtact ggggcagttt cgcgatctta 600
   acgatgccgc cctcgaccgt acccctgacg ctatcctcag caccaaccac ctcaagaccg 660
   gcattctgtt cagcgcgatg ctgcagatcg tcgccattgc ttccgcctcg tcgccgagca 720
   cgcgagagac gctgcacgcc ttcgccctcg acttcggcca ggcgtttcaa ctgctggacg 780
   atctgcgtga cgatcacccg gaaaccggta aagatcgcaa taaggacgcg ggaaaatcga 840
   cgctggtcaa ccggctgggc gcagacgcgg cccggcaaaa gctgcgcgag catattgatt 900
   ccgccgacaa acacctcact tttgcctgtc cgcagggcgg cgccatccga cagtttatgc 960
   atctgtggtt tggccatcac cttgccgact ggtcaccggt catgaaaatc gcctgatacc 1020
   gcccttttgg gttcaagcag tacataacga tggaaccaca ttacaggagt agtgatgaat 1080
   gaaggacgag cgccttgttc agcgtaagaa cgatcatctg gatatcgttc tcgacccccg 1140
   tcgcgccgta actcaggcta gcgcaggttt tgagcgctgg cgctttaccc actgcgccct 1200
   gccagagctg aattttagcg acatcacgct ggaaaccacc ttcctgaatc ggcagctaca 1260
   ggctccgctg ctgatcagct ccatgaccgg cggcgttgag cgctcgcgcc atatcaaccg 1320
   ccacctcgcc gaggcggcgc aggtgctaaa aattgcgatg ggggtgggct cccagcgcgt 1380
   cgccattgag agcgacgcgg gcttagggct ggataaaacc ctgcggcagc tggctccgga 1440
   cgtgccgctg ctggcgaacc tcggcgcggc gcagctgacc ggcagaaaag gtattgatta 1500
   cgcccgacgg gccgtggaga tgatcgaggc ggatgcgctg attgtgcacc taaacccgct 1560
   gcaggaggcg ctacagcccg gcggcgatcg cgactggcgc ggacggctgg cggctattga 1620
   aactctggtc cgcgagctgc ccgttccgct ggtggtgaaa gaggtgggag ccggtatctc 1680
   ccgaaccgtg gccgggcagc tgatcgatgc cggcgttacc gtgattgacg tcgcgggcgc 1740
   gggcggcacc agctgggccg ccgttgaagg cgagcgggcg gccaccgagc agcagcgcag 1800
   cgtggccaac gtctttgccg actgggggat ccccaccgct gaggcgctgg ttgacattgc 1860
   cgaggcctgg ccgcagatgc cccttattgc ctcgggcggg attaaaaacg gcgtcgacgc 1920
   ggcgaaagcg ctgcggctcg gcgcgtgcat ggtagggcag gccgccgccg tgctcggcag 1980
   cgcaggcgtc tccacggaga aggtgatcga tcacttcaac gtgattattg agcagctgcg 2040
   ggtggcctgc ttctgcaccg gcagccgcag cctgagcgat ctaaagcagg ctgatatccg 2100
   ctatgttcgt gatacgccat aaggaggtac aaccatgaag aaaaccgttg tgattggcgc 2160
   aggctttggt ggcctggcgc tggcgattcg cctgcaggcg gcagggatcc caaccgtact 2220
   gctggagcag cgggacaagc ccggcggtcg ggcctacgtc tggcatgacc agggctttac 2280
   ctttgacgcc gggccgacgg tgatcaccga tcctaccgcg cttgaggcgc tgttcaccct 2340
   ggccggcagg cgcatggagg attacgtcag gctgctgccg gtaaaaccct tctaccgact 2400
   ctgctgggag tccgggaaga ccctcgacta tgctaacgac agcgccgagc ttgaggcgca 2460
   gattacccag ttcaaccccc gcgacgtcga gggctaccgg cgctttctgg cttactccca 2520
   ggcggtattc caggagggat atttgcgcct cggcagcgtg ccgttcctct cttttcgcga 2580
   catgctgcgc gccgggccgc agctgcttaa gctccaggcg tggcagagcg tctaccagtc 2640
   ggtttcgcgc tttattgagg atgagcatct gcggcaggcc ttctcgttcc actccctgct     2700
   ggtaggcggc aaccccttca ccacctcgtc catctacacc ctgatccacg cccttgagcg     2760
   ggagtggggg gtctggttcc ctgagggcgg caccggggcg ctggtgaacg gcatggtgaa 2820
   gctgtttacc gatctgggcg gggagatcga actcaacgcc cgggtcgaag agctggtggt 2880
   ggccgataac cgcgtaagcc aggtccggct ggcggatggt cggatctttg acaccgacgc 2940
   cgtagcctcg aacgctgacg tggtgaacac ctataaaaag ctgctcggcc accatccggt 3000
   ggggcagaag cgggcggcag cgctggagcg caagagcatg agcaactcgc tgtttgtgct 3060
   ctacttcggc ctgaaccagc ctcattccca gctggcgcac cataccatct gttttggtcc 3120
   ccgctaccgg gagctgatcg acgagatctt taccggcagc gcgctggcgg atgacttctc 3180
   gctctacctg cactcgccct gcgtgaccga tccctcgctc gcgcctcccg gctgcgccag 3240
   cttctacgtg ctggccccgg tgccgcatct tggcaacgcg ccgctggact gggcgcagga 3300
   ggggccgaag ctgcgcgacc gcatctttga ctaccttgaa gagcgctata tgcccggcct 3360
   gcgtagccag ctggtgaccc agcggatctt taccccggca gacttccacg acacgctgga 3420
   tgcgcatctg ggatcggcct tctccatcga gccgctgctg acccaaagcg cctggttccg 3480
   cccgcacaac cgcgacagcg acattgccaa cctctacctg gtgggcgcag gtactcaccc 3540
   tggggcgggc attcctggcg tagtggcctc ggcgaaagcc accgccagcc tgatgattga 3600
   ggatctgcaa tgagccaacc gccgctgctt gaccacgcca cgcagaccat ggccaacggc 3660
   tcgaaaagtt ttgccaccgc tgcgaagctg ttcgacccgg ccacccgccg tagcgtgctg 3720
   atgctctaca cctggtgccg ccactgcgat gacgtcattg acgaccagac ccacggcttc 3780
   gccagcgagg ccgcggcgga ggaggaggcc acccagcgcc tggcccggct gcgcacgctg 3840
   accctggcgg cgtttgaagg ggccgagatg caggatccgg ccttcgctgc ctttcaggag 3900
   gtggcgctga cccacggtat tacgccccgc atggcgctcg atcacctcga cggctttgcg 3960
   atggacgtgg ctcagacccg ctatgtcacc tttgaggata cgctgcgcta ctgctatcac 4020
   gtggcgggcg tggtgggtct gatgatggcc agggtgatgg gcgtgcggga tgagcgggtg 4080
   ctggatcgcg cctgcgatct ggggctggcc ttccagctga cgaatatcgc ccgggatatt 4140
   attgacgatg cggctattga ccgctgctat ctgcccgccg agtggctgca ggatgccggg 4200
   ctgaccccgg agaactatgc cgcgcgggag aatcgggccg cgctggcgcg ggtggcggag 4260
   cggcttattg atgccgcaga gccgtactac atctcctccc aggccgggct acacgatctg 4320
   ccgccgcgct gcgcctgggc gatcgccacc gcccgcagcg tctaccggga gatcggtatt 4380
   aaggtaaaag cggcgggagg cagcgcctgg gatcgccgcc agcacaccag caaaggtgaa 4440
   aaaattgcca tgctgatggc ggcaccgggg caggttattc gggcgaagac gacgagggtg 4500
   acgccgcgtc cggccggtct ttggcagcgt cccgtttaga ccgactccaa acgagtcggt 4560
   ttttttgcgc ttgtttcggc gtgggtatgg tggcaggccc cgtggccggg ggactgttgg 4620
   gcgccatctc cttggggtcg aatttgcttt cgaatttctg ccattcatcc gcttattatc 4680
   acttattcag gcgtagcaac caggcgttta agggcaccaa taactgcctt aaaaaaatta 4740
   cgccccgccc tgccactcat cgcagtactg ttgtaattca ttaagcattc tgccgacatg 4800
   gaagccatca cagacggcat gatgaacctg aatcgccagc ggcatcagca ccttgtcgcc 4860
   ttgcgtataa tatttgccca tggtgaaaac gggggcgaag aagttgtcca tattggccac 4920
   gtttaaatca aaactggtga aactcaccca gggattggct gagacgaaaa acatattctc 4980
   aataaaccct ttagggaaat aggccaggtt ttcaccgtaa cacgccacat cttgcgaata 5040
   tatgtgtaga aactgccgga aatcgtcgtg gtattcactc cagagcgatg aaaacgtttc 5100
   agtttgctca tggaaaacgg tgtaacaagg gtgaacacta tcccatatca ccagctcacc 5160
   gtctttcatt gccatacgga attccggatg agcattcatc aggcgggcaa gaatgtgaat 5220
   aaaggccgga taaaacttgt gcttattttt ctttacggtc tttaaaaagg ccgtaatatc 5280
   cagctgaacg gtctggttat aggtacattg agcaactgac tgaaatgcct caaaatgttc 5340
   tttacgatgc cattgggata tatcaacggt ggtatatcca gtgatttttt tctccatttt 5400
   agcttcctta gctcctgaaa atctcgataa ctcaaaaaat acgcccggta gtgatcttat 5460
   ttcattatgg tgaaagttgg aacctcttac gtgccgatca acgtctcatt ttcgccaaaa 5520
   gttggcccag ggcttcccgg tatcaacagg gacaccagga tttatttatt ctgcgaagtg 5580
   atcttccgtc acaggtattt attcggcgca aagtgcgtcg ggtgatgctg ccaacttact 5640
   gatttagtgt atgatggtgt ttttgaggtg ctccagtggc ttctgtttct atcagctgtc 5700
   cctcctgttc agctactgac ggggtggtgc gtaacggcaa aagcaccgcc ggacatcagc 5760
   gctagcggag tgtatacact ccgctatcgc tacgtgactg ggtcatggct gcgccccgac 5820
   acccgccaac acccgctgac gcgccctgac gggcttgtct gctcccggca tccgcttaca 5880
   gacaagctgt gaccgtctcc gggagctgca tgtgtcagag gttttcaccg tcatcaccga 5940
   aacgcgcgag gcagctggca cgacaggttt cccgactgga aaagcgaatt ggtataatgc 6000
   cctcgtaata attttgttta actttaagaa ggagatatac ccatgacaca gagggcccac 6060
   catcaccatc accattccat ggctagcggc ggcggaagtt ccggtagtga gagttgtgta 6120
   gcggtgagag aagatttcgc tgacgaagaa gattttgtga aagctggtgg ttctgagatt 6180
   ctatttgttc aaatgcagca gaacaaagat atggatgaac agtctaagct tgttgataag 6240
   ttgcctccta tatcaattgg tgatggtgct ttggatctag tggttattgg ttgtggtcct 6300
   gctggtttag ccttggctgc agaatcagct aagcttggat taaaagttgg actcattggt 6360
   ccagatcttc cttttactaa caattacggt gtttgggaag atgaattcaa tgatcttggg 6420
   ctgcaaaaat gtattgagca tgtttggaga gagactattg tgtatctgga tgatgacaag 6480
   cctattacca ttggccgtgc ttatggaaga gttagtcgac gtttgctcca tgaggagctt 6540
   ttgaggaggt gtgtcgagtc aggtgtctcg taccttagct cgaaagttga cagcataaca 6600
   gaagcttctg atggccttag acttgttgct tgtgacgaca ataacgtcat tccctgcagg 6660
   cttgccactg ttgcttctgg agcagcttcg ggaaagctct tgcaatacga agttggtgga 6720
   cctagagtct gtgtgcaaac tgcatacggc gtggaggttg aggtggaaaa tagtccatat 6780
   gatccagatc aaatggtttt catggattac agagattata ctaacgagaa agttcggagc 6840
   ttagaagctg agtatccaac gtttctgtac gccatgccta tgacaaagtc aagactcttc 6900
   ttcgaggaga catgtttggc ctcaaaagat gtcatgccct ttgatttgct aaaaacgaag 6960
   ctcatgttaa gattagatac actcggaatt cgaattctaa agacttacga agaggagtgg 7020
   tcctatatcc cagttggtgg ttccttgcca aacaccgaac aaaagaatct cgcctttggt 7080
   gctgccgcta gcatggtaca tcccgcaaca ggctattcag ttgtgagatc tttgtctgaa 7140
   gctccaaaat atgcatcagt catcgcagag atactaagag aagagactac caaacagatc 7200
   aacagtaata tttcaagaca agcttgggat actttatggc caccagaaag gaaaagacag 7260
   agagcattct ttctctttgg tcttgaggct atagttcaat tcgataccga aggcattaga 7320
   agcttcttcc gtactttctt ccgccttcca aaatggatgt ggcaagggtt tctaggatca 7380
   acattaacat caggagatct cgttctcttt tctttataca tgttcgtcat ttcaccaaac 7440
   aatttgagaa aaggtctcat caatcatctc atctctgatc caaccggagc aaccatgata 7500
   aaaacctatc tcaaagtatg aggatccggc tgctaacaaa gcccgttctg tttaagaacg 7560
   ggattttttg ctgaaaggag gaactatatc cggccggatt actggcttac tatgttggca 7620
   ctgatgaggg tgtcagtgaa gtgcttcatg tggcaggaga aaaaaggctg caccggtgcg 7680
   tcagcagaat atgtgataca ggatatattc cgcttcctcg ctcactgact cgctacgctc 7740
   ggtcgttcga ctgcggcgag cggaaatggc ttacgaacgg ggcggagatt tcctggaaga      7800
   tgccaggaag atacttaaca gggaagtgag agggccgcgg caaagccgtt tttccatagg      7860
   ctccgccccc ctgacaagca tcacgaaatc tgacgctcaa atcagtggtg gcgaaacccg      7920
   acaggactat aaagatacca ggcgtttccc cctggcggct ccctcgtgcg ctctcctgtt      7980
   cctgcctttc ggtttaccgg tgtcattccg ctgttatggc cgcgtttgtc tcattccacg      8040
   cctgacactc agttccgggt aggcagttcg ctccaagctg gactgtatgc acgaaccccc      8100
   cgttcagtcc gaccgctgcg ccttatccgg taactatcgt cttgagtcca acccggaaag      8160
   acatgcaaaa gcaccactgg cagcagccac tggtaattga tttagaggag ttagtcttga      8220
   agtcatgcgc cggttaaggc taaactgaaa ggacaagttt tggtgactgc gctcctccaa      8280
   gccagttacc tcggttcaaa gagttggtag ctcagagaac cttcgaaaaa ccgccctgca      8340
   aggcggtttt ttcgttttca gagcaagaga ttacgcgcag accaaaacga tctcaagaag      8400
   atcatcttat taatcagata aaatatttct agatttcagt gcaatttatc tcttcaaatg      8460
   tagcacctga agtcagcccc atacgatata ag                                    8492
<210> 32
   <211> 8669
   <212> DNA
   <213> Artifizielle Sequenz
<220>
   <223> Plasmid pGT1484
<400> 32
   atatttctag atttcagtgc aatttatctc ttcaaatgta gcacctgaag tcagccccat      60
   acgatataag ttgtaattct catgtttgac agcttatcat cgataagctt taatgcggta     120
   gtttatcaca gttaaattgc taacgcagtc aggaaccttg caatgagcag tttcgatgcc     180
   catgaccttg acctcgacaa atttccggag gtcgtgcgag atcgtttgac gcagttcctc     240
   gatgctcaag agctaacaat tgctgatatc ggcgctcctg tcacagatgc tgtggcccat     300
   cttcgcagtt tcgtgctcaa tggaggaaag cgaatccgtc ctctttatgc gtgggctggt     360
   ttcctggcgg cgcaaggcca taagaattct tctgaaaaac ttgagtccgt ccttgacgcc     420
   gcagcgagtc tcgaattcat ccaggcttgt gccttgattc atgacgatat tatcgattct     480
   tctgataccc ggcgcggagc ccctacagtt caccgggctg tggaagctga tcaccgcgcc      540
   aataatttcg aaggcgatcc tgagcacttt ggcgtttcag tctcgatttt ggctggcgat     600
   atggcattgg tgtgggcaga agacatgctg caggattccg gtttgagtgc agaggcattg     660
   gcccgcacga gggatgcttg gcgtggcatg cgtactgagg ttattggcgg ccagctgctt     720
   gatatttatc ttgagtcgca cgccaacgag tcggtggagc ttgcggattc tgtcaaccgc     780
   ttcaaaacgg ccgcttacac gattgcgcgc ccattgcacc tgggcgcctc cattgctggc 840
   ggttcgccgc agcttatcga cgcgctcctc cactacggcc acgacatcgg cattgcattc 900
   cagttgaggg atgatctgct tggtgtgttt ggtgatcctg ctatcaccgg caaaccagct 960
   ggagacgata tccgtgaagg caagcgcact gttcttcttg cgctcgctct acaacgcgct 1020
   gataagcaat ctcctgaagc tgcaacggcc attcgcgcag gtgttggaaa ggtgacttca 1080
   ccagaagata ttgctgtcat tacagagcat attcgagcta ctggtgctga agaagaagtt 1140
   gagcagcgaa tttcccagct gactgaatcc ggtttggctc acctcgatga tgtagacatc 1200
   cctgatgagg tgcgcgcaca gttgcgggca ctggctatcc gctcaaccga acgtcggatg 1260
   taataccgcc cttttgggtt caagcagtac ataacgatgg aaccacatta caggagtagt 1320
   gatgaatgaa ggacgagcgc cttgttcagc gtaagaacga tcatctggat atcgttctcg 1380
   acccccgtcg cgccgtaact caggctagcg caggttttga gcgctggcgc tttacccact 1440
   gcgccctgcc agagctgaat tttagcgaca tcacgctgga aaccaccttc ctgaatcggc 1500
   agctacaggc tccgctgctg atcagctcca tgaccggcgg cgttgagcgc tcgcgccata 1560
   tcaaccgcca cctcgccgag gcggcgcagg tgctaaaaat tgcgatgggg gtgggctccc 1620
   agcgcgtcgc cattgagagc gacgcgggct tagggctgga taaaaccctg cggcagctgg 1680
   ctccggacgt gccgctgctg gcgaacctcg gcgcggcgca gctgaccggc agaaaaggta 1740
   ttgattacgc ccgacgggcc gtggagatga tcgaggcgga tgcgctgatt gtgcacctaa 1800
   acccgctgca ggaggcgcta cagcccggcg gcgatcgcga ctggcgcgga cggctggcgg 1860
   ctattgaaac tctggtccgc gagctgcccg ttccgctggt ggtgaaagag gtgggagccg 1920
   gtatctcccg aaccgtggcc gggcagctga tcgatgccgg cgttaccgtg attgacgtcg 1980
   cgggcgcggg cggcaccagc tgggccgccg ttgaaggcga gcgggcggcc accgagcagc 2040
   agcgcagcgt ggccaacgtc tttgccgact gggggatccc caccgctgag gcgctggttg 2100
   acattgccga ggcctggccg cagatgcccc ttattgcctc gggcgggatt aaaaacggcg 2160
   tcgacgcggc gaaagcgctg cggctcggcg cgtgcatggt agggcaggcc gccgccgtgc 2220
   tcggcagcgc aggcgtctcc acggagaagg tgatcgatca cttcaacgtg attattgagc 2280
   agctgcgggt ggcctgcttc tgcaccggca gccgcagcct gagcgatcta aagcaggctg 2340
   atatccgcta tgttcgtgat acgccataag gaggtacaac catgaagaaa accgttgtga 2400
   ttggcgcagg ctttggtggc ctggcgctgg cgattcgcct gcaggcggca gggatcccaa 2460
   ccgtactgct ggagcagcgg gacaagcccg gcggtcgggc ctacgtctgg catgaccagg 2520
   gctttacctt tgacgccggg ccgacggtga tcaccgatcc taccgcgctt gaggcgctgt 2580
   tcaccctggc cggcaggcgc atggaggatt acgtcaggct gctgccggta aaacccttct 2640
   accgactctg ctgggagtcc gggaagaccc tcgactatgc taacgacagc gccgagcttg 2700
   aggcgcagat tacccagttc aacccccgcg acgtcgaggg ctaccggcgc tttctggctt 2760
   actcccaggc ggtattccag gagggatatt tgcgcctcgg cagcgtgccg ttcctctctt 2820
   ttcgcgacat gctgcgcgcc gggccgcagc tgcttaagct ccaggcgtgg cagagcgtct 2880
   accagtcggt ttcgcgcttt attgaggatg agcatctgcg gcaggccttc tcgttccact 2940
   ccctgctggt aggcggcaac cccttcacca cctcgtccat ctacaccctg atccacgccc 3000
   ttgagcggga gtggggggtc tggttccctg agggcggcac cggggcgctg gtgaacggca 3060
   tggtgaagct gtttaccgat ctgggcgggg agatcgaact caacgcccgg gtcgaagagc 3120
   tggtggtggc cgataaccgc gtaagccagg tccggctggc ggatggtcgg atctttgaca 3180
   ccgacgccgt agcctcgaac gctgacgtgg tgaacaccta taaaaagctg ctcggccacc 3240
   atccggtggg gcagaagcgg gcggcagcgc tggagcgcaa gagcatgagc aactcgctgt 3300
   ttgtgctcta cttcggcctg aaccagcctc attcccagct ggcgcaccat accatctgtt 3360
   ttggtccccg ctaccgggag ctgatcgacg agatctttac cggcagcgcg ctggcggatg 3420
   acttctcgct ctacctgcac tcgccctgcg tgaccgatcc ctcgctcgcg cctcccggct 3480
   gcgccagctt ctacgtgctg gccccggtgc cgcatcttgg caacgcgccg ctggactggg 3540
   cgcaggaggg gccgaagctg cgcgaccgca tctttgacta ccttgaagag cgctatatgc 3600
   ccggcctgcg tagccagctg gtgacccagc ggatctttac cccggcagac ttccacgaca 3660
   cgctggatgc gcatctggga tcggccttct ccatcgagcc gctgctgacc caaagcgcct 3720
   ggttccgccc gcacaaccgc gacagcgaca ttgccaacct ctacctggtg ggcgcaggta 3780
   ctcaccctgg ggcgggcatt cctggcgtag tggcctcggc gaaagccacc gccagcctga 3840
   tgattgagga tctgcaatga gccaaccgcc gctgcttgac cacgccacgc agaccatggc 3900
   caacggctcg aaaagttttg ccaccgctgc gaagctgttc gacccggcca cccgccgtag 3960
   cgtgctgatg ctctacacct ggtgccgcca ctgcgatgac gtcattgacg accagaccca 4020
   cggcttcgcc agcgaggccg cggcggagga ggaggccacc cagcgcctgg cccggctgcg 4080
   cacgctgacc ctggcggcgt ttgaaggggc cgagatgcag gatccggcct tcgctgcctt 4140
   tcaggaggtg gcgctgaccc acggtattac gccccgcatg gcgctcgatc acctcgacgg 4200
   ctttgcgatg gacgtggctc agacccgcta tgtcaccttt gaggatacgc tgcgctactg 4260
   ctatcacgtg gcgggcgtgg tgggtctgat gatggccagg gtgatgggcg tgcgggatga 4320
   gcgggtgctg gatcgcgcct gcgatctggg gctggccttc cagctgacga atatcgcccg 4380
   ggatattatt gacgatgcgg ctattgaccg ctgctatctg cccgccgagt ggctgcagga 4440
   tgccgggctg accccggaga actatgccgc gcgggagaat cgggccgcgc tggcgcgggt 4500
   ggcggagcgg cttattgatg ccgcagagcc gtactacatc tcctcccagg ccgggctaca 4560
   cgatctgccg ccgcgctgcg cctgggcgat cgccaccgcc cgcagcgtct accgggagat 4620
   cggtattaag gtaaaagcgg cgggaggcag cgcctgggat cgccgccagc acaccagcaa 4680
   aggtgaaaaa attgccatgc tgatggcggc accggggcag gttattcggg cgaagacgac 4740
   gagggtgacg ccgcgtccgg ccggtctttg gcagcgtccc gtttagaccg actccaaacg      4800
   agtcggtttt tttgcgcttg tttcggcgtg ggtatggtgg caggccccgt ggccggggga      4860
   ctgttgggcg ccatctcctt ggggtcgaat ttgctttcga atttctgcca ttcatccgct 4920
   tattatcact tattcaggcg tagcaaccag gcgtttaagg gcaccaataa ctgccttaaa 4980
   aaaattacgc cccgccctgc cactcatcgc agtactgttg taattcatta agcattctgc 5040
   cgacatggaa gccatcacag acggcatgat gaacctgaat cgccagcggc atcagcacct 5100
   tgtcgccttg cgtataatat ttgcccatgg tgaaaacggg ggcgaagaag ttgtccatat 5160
   tggccacgtt taaatcaaaa ctggtgaaac tcacccaggg attggctgag acgaaaaaca 5220
   tattctcaat aaacccttta gggaaatagg ccaggttttc accgtaacac gccacatctt 5280
   gcgaatatat gtgtagaaac tgccggaaat cgtcgtggta ttcactccag agcgatgaaa 5340
   acgtttcagt ttgctcatgg aaaacggtgt aacaagggtg aacactatcc catatcacca 5400
   gctcaccgtc tttcattgcc atacggaatt ccggatgagc attcatcagg cgggcaagaa 5460
   tgtgaataaa ggccggataa aacttgtgct tatttttctt tacggtcttt aaaaaggccg 5520
   taatatccag ctgaacggtc tggttatagg tacattgagc aactgactga aatgcctcaa 5580
   aatgttcttt acgatgccat tgggatatat caacggtggt atatccagtg atttttttct 5640
   ccattttagc ttccttagct cctgaaaatc tcgataactc aaaaaatacg cccggtagtg 5700
   atcttatttc attatggtga aagttggaac ctcttacgtg ccgatcaacg tctcattttc 5760
   gccaaaagtt ggcccagggc ttcccggtat caacagggac accaggattt atttattctg 5820
   cgaagtgatc ttccgtcaca ggtatttatt cggcgcaaag tgcgtcgggt gatgctgcca 5880
   acttactgat ttagtgtatg atggtgtttt tgaggtgctc cagtggcttc tgtttctatc 5940
   agctgtccct cctgttcagc tactgacggg gtggtgcgta acggcaaaag caccgccgga 6000
   catcagcgct agcggagtgt atacactccg ctatcgctac gtgactgggt catggctgcg 6060
   ccccgacacc cgccaacacc cgctgacgcg ccctgacggg cttgtctgct cccggcatcc 6120
   gcttacagac aagctgtgac cgtctccggg agctgcatgt gtcagaggtt ttcaccgtca 6180
   tcaccgaaac gcgcgaggca gctggcacga caggtttccc gactggaaaa gcgaattggt 6240
   ataatgccct cgtaataatt ttgtttaact ttaagaagga gatataccca tgacacagag 6300
   ggcccaccat caccatcacc attccatggc tagcggcggc ggaagttccg gtagtgagag 6360
   ttgtgtagcg gtgagagaag atttcgctga cgaagaagat tttgtgaaag ctggtggttc 6420
   tgagattcta tttgttcaaa tgcagcagaa caaagatatg gatgaacagt ctaagcttgt 6480
   tgataagttg cctcctatat caattggtga tggtgctttg gatctagtgg ttattggttg 6540
   tggtcctgct ggtttagcct tggctgcaga atcagctaag cttggattaa aagttggact 6600
   cattggtcca gatcttcctt ttactaacaa ttacggtgtt tgggaagatg aattcaatga 6660
   tcttgggctg caaaaatgta ttgagcatgt ttggagagag actattgtgt atctggatga 6720
   tgacaagcct attaccattg gccgtgctta tggaagagtt agtcgacgtt tgctccatga 6780
   ggagcttttg aggaggtgtg tcgagtcagg tgtctcgtac cttagctcga aagttgacag 6840
   cataacagaa gcttctgatg gccttagact tgttgcttgt gacgacaata acgtcattcc 6900
   ctgcaggctt gccactgttg cttctggagc agcttcggga aagctcttgc aatacgaagt 6960
   tggtggacct agagtctgtg tgcaaactgc atacggcgtg gaggttgagg tggaaaatag 7020
   tccatatgat ccagatcaaa tggttttcat ggattacaga gattatacta acgagaaagt 7080
   tcggagctta gaagctgagt atccaacgtt tctgtacgcc atgcctatga caaagtcaag 7140
   actcttcttc gaggagacat gtttggcctc aaaagatgtc atgccctttg atttgctaaa 7200
   aacgaagctc atgttaagat tagatacact cggaattcga attctaaaga cttacgaaga 7260
   ggagtggtcc tatatcccag ttggtggttc cttgccaaac accgaacaaa agaatctcgc 7320
   ctttggtgct gccgctagca tggtacatcc cgcaacaggc tattcagttg tgagatcttt 7380
   gtctgaagct ccaaaatatg catcagtcat cgcagagata ctaagagaag agactaccaa 7440
   acagatcaac agtaatattt caagacaagc ttgggatact ttatggccac cagaaaggaa 7500
   aagacagaga gcattctttc tctttggtct tgaggctata gttcaattcg ataccgaagg 7560
   cattagaagc ttcttccgta ctttcttccg ccttccaaaa tggatgtggc aagggtttct 7620
   aggatcaaca ttaacatcag gagatctcgt tctcttttct ttatacatgt tcgtcatttc 7680
   accaaacaat ttgagaaaag gtctcatcaa tcatctcatc tctgatccaa ccggagcaac 7740
   catgataaaa acctatctca aagtatgagg atccggctgc taacaaagcc cgttctgttt 7800
   aagaacggga ttttttgctg aaaggaggaa ctatatccgg ccggattact ggcttactat 7860
   gttggcactg atgagggtgt cagtgaagtg cttcatgtgg caggagaaaa aaggctgcac 7920
   cggtgcgtca gcagaatatg tgatacagga tatattccgc ttcctcgctc actgactcgc 7980
   tacgctcggt cgttcgactg cggcgagcgg aaatggctta cgaacggggc ggagatttcc 8040
   tggaagatgc caggaagata cttaacaggg aagtgagagg gccgcggcaa agccgttttt 8100
   ccataggctc cgcccccctg acaagcatca cgaaatctga cgctcaaatc agtggtggcg 8160
   aaacccgaca ggactataaa gataccaggc gtttccccct ggcggctccc tcgtgcgctc 8220
   tcctgttcct gcctttcggt ttaccggtgt cattccgctg ttatggccgc gtttgtctca 8280
   ttccacgcct gacactcagt tccgggtagg cagttcgctc caagctggac tgtatgcacg 8340
   aaccccccgt tcagtccgac cgctgcgcct tatccggtaa ctatcgtctt gagtccaacc 8400
   cggaaagaca tgcaaaagca ccactggcag cagccactgg taattgattt agaggagtta 8460
   gtcttgaagt catgcgccgg ttaaggctaa actgaaagga caagttttgg tgactgcgct 8520
   cctccaagcc agttacctcg gttcaaagag ttggtagctc agagaacctt cgaaaaaccg 8580
   ccctgcaagg cggttttttc gttttcagag caagagatta cgcgcagacc aaaacgatct 8640
   caagaagatc atcttattaa tcagataaa 8669
<210> 33
   <211> 8651
   <212> DNA
   <213> Artifizielle Sequenz
<220>
   <223> Plasmid pGT1518
<400> 33
   ttgtaattct catgtttgac agcttatcat cgataagctt taatgcggta gttaacgcag 60
   tcaggaacct tgcaatgagc agtttcgatg cccatgacct tgacctcgac aaatttccgg 120
   aggtcgtgcg agatcgtttg acgcagttcc tcgatgctca agagctaaca attgctgata 180
   tcggcgctcc tgtcacagat gctgtggccc atcttcgcag tttcgtgctc aatggaggaa 240
   agcgaatccg tcctctttat gcgtgggctg gtttcctggc ggcgcaaggc cataagaatt 300
   cttctgaaaa acttgagtcc gtccttgacg ccgcagcgag tctcgaattc atccaggctt 360
   gtgccttgat tcatgacgat attatcgatt cttctgatac ccggcgcgga gcccctacag 420
   ttcaccgggc tgtggaagct gatcaccgcg ccaataattt cgaaggcgat cctgagcact 480
   ttggcgtttc agtctcgatt ttggctggcg atatggcatt ggtgtgggca gaagacatgc 540
   tgcaggattc cggtttgagt gcagaggcat tggcccgcac gagggatgct tggcgtggca 600
   tgcgtactga ggttattggc ggccagctgc ttgatattta tcttgagtcg cacgccaacg 660
   agtcggtgga gcttgcggat tctgtcaacc gcttcaaaac ggccgcttac acgattgcgc 720
   gcccattgca cctgggcgcc tccattgctg gcggttcgcc gcagcttatc gacgcgctcc 780
   tccactacgg ccacgacatc ggcattgcat tccagttgag ggatgatctg cttggtgtgt 840
   ttggtgatcc tgctatcacc ggcaaaccag ctggagacga tatccgtgaa ggcaagcgca 900
   ctgttcttct tgcgctcgct ctacaacgcg ctgataagca atctcctgaa gctgcaacgg 960
   ccattcgcgc aggtgttgga aaggtgactt caccagaaga tattgctgtc attacagagc 1020
   atattcgagc tactggtgct gaagaagaag ttgagcagcg aatttcccag ctgactgaat 1080
   ccggtttggc tcacctcgat gatgtagaca tccctgatga ggtgcgcgca cagttgcggg 1140
   cactggctat ccgctcaacc gaacgtcgga tgtaataccg cccttttggg ttcaagcagt 1200
   acataacgat ggaaccacat tacaggagta gtgatgaatg aaggacgagc gccttgttca 1260
   gcgtaagaac gatcatctgg atatcgttct cgacccccgt cgcgccgtaa ctcaggctag 1320
   cgcaggtttt gagcgctggc gctttaccca ctgcgccctg ccagagctga attttagcga 1380
   catcacgctg gaaaccacct tcctgaatcg gcagctacag gctccgctgc tgatcagctc 1440
   catgaccggc ggcgttgagc gctcgcgcca tatcaaccgc cacctcgccg aggcggcgca 1500
   ggtgctaaaa attgcgatgg gggtgggctc ccagcgcgtc gccattgaga gcgacgcggg 1560
   cttagggctg gataaaaccc tgcggcagct ggctccggac gtgccgctgc tggcgaacct 1620
   cggcgcggcg cagctgaccg gcagaaaagg tattgattac gcccgacggg ccgtggagat 1680
   gatcgaggcg gatgcgctga ttgtgcacct aaacccgctg caggaggcgc tacagcccgg 1740
   cggcgatcgc gactggcgcg gacggctggc ggctattgaa actctggtcc gcgagctgcc 1800
   cgttccgctg gtggtgaaag aggtgggagc cggtatctcc cgaaccgtgg ccgggcagct 1860
   gatcgatgcc ggcgttaccg tgattgacgt cgcgggcgcg ggcggcacca gctgggccgc 1920
   cgttgaaggc gagcgggcgg ccaccgagca gcagcgcagc gtggccaacg tctttgccga 1980
   ctgggggatc cccaccgctg aggcgctggt tgacattgcc gaggcctggc cgcagatgcc 2040
   ccttattgcc tcgggcggga ttaaaaacgg cgtcgacgcg gcgaaagcgc tgcggctcgg 2100
   cgcgtgcatg gtagggcagg ccgccgccgt gctcggcagc gcaggcgtct ccacggagaa 2160
   ggtgatcgat cacttcaacg tgattattga gcagctgcgg gtggcctgct tctgcaccgg 2220
   cagccgcagc ctgagcgatc taaagcaggc tgatatccgc tatgttcgtg atacgccata 2280
   aggaggtaca accatgaaga aaaccgttgt gattggcgca ggctttggtg gcctggcgct 2340
   ggcgattcgc ctgcaggcgg cagggatccc aaccgtactg ctggagcagc gggacaagcc 2400
   cggcggtcgg gcctacgtct ggcatgacca gggctttacc tttgacgccg ggccgacggt 2460
   gatcaccgat cctaccgcgc ttgaggcgct gttcaccctg gccggcaggc gcatggagga 2520
   ttacgtcagg ctgctgccgg taaaaccctt ctaccgactc tgctgggagt ccgggaagac 2580
   cctcgactat gctaacgaca gcgccgagct tgaggcgcag attacccagt tcaacccccg 2640
   cgacgtcgag ggctaccggc gctttctggc ttactcccag gcggtattcc aggagggata 2700
   tttgcgcctc ggcagcgtgc cgttcctctc ttttcgcgac atgctgcgcg ccgggccgca 2760
   gctgcttaag ctccaggcgt ggcagagcgt ctaccagtcg gtttcgcgct ttattgagga 2820
   tgagcatctg cggcaggcct tctcgttcca ctccctgctg gtaggcggca accccttcac 2880
   cacctcgtcc atctacaccc tgatccacgc ccttgagcgg gagtgggggg tctggttccc 2940
   tgagggcggc accggggcgc tggtgaacgg catggtgaag ctgtttaccg atctgggcgg 3000
   ggagatcgaa ctcaacgccc gggtcgaaga gctggtggtg gccgataacc gcgtaagcca 3060
   ggtccggctg gcggatggtc ggatctttga caccgacgcc gtagcctcga acgctgacgt 3120
   ggtgaacacc tataaaaagc tgctcggcca ccatccggtg gggcagaagc gggcggcagc 3180
   gctggagcgc aagagcatga gcaactcgct gtttgtgctc tacttcggcc tgaaccagcc 3240
   tcattcccag ctggcgcacc ataccatctg ttttggtccc cgctaccggg agctgatcga 3300
   cgagatcttt accggcagcg cgctggcgga tgacttctcg ctctacctgc actcgccctg 3360
   cgtgaccgat ccctcgctcg cgcctcccgg ctgcgccagc ttctacgtgc tggccccggt 3420
   gccgcatctt ggcaacgcgc cgctggactg ggcgcaggag gggccgaagc tgcgcgaccg 3480
   catctttgac taccttgaag agcgctatat gcccggcctg cgtagccagc tggtgaccca 3540
   gcggatcttt accccggcag acttccacga cacgctggat gcgcatctgg gatcggcctt 3600
   ctccatcgag ccgctgctga cccaaagcgc ctggttccgc ccgcacaacc gcgacagcga 3660
   cattgccaac ctctacctgg tgggcgcagg tactcaccct ggggcgggca ttcctggcgt 3720
   agtggcctcg gcgaaagcca ccgccagcct gatgattgag gatctgcaat gagccaaccg 3780
   ccgctgcttg accacgccac gcagaccatg gccaacggct cgaaaagttt tgccaccgct 3840
   gcgaagctgt tcgacccggc cacccgccgt agcgtgctga tgctctacac ctggtgccgc 3900
   cactgcgatg acgtcattga cgaccagacc cacggcttcg ccagcgaggc cgcggcggag 3960
   gaggaggcca cccagcgcct ggcccggctg cgcacgctga ccctggcggc gtttgaaggg 4020
   gccgagatgc aggatccggc cttcgctgcc tttcaggagg tggcgctgac ccacggtatt 4080
   acgccccgca tggcgctcga tcacctcgac ggctttgcga tggacgtggc tcagacccgc 4140
   tatgtcacct ttgaggatac gctgcgctac tgctatcacg tggcgggcgt ggtgggtctg 4200
   atgatggcca gggtgatggg cgtgcgggat gagcgggtgc tggatcgcgc ctgcgatctg 4260
   gggctggcct tccagctgac gaatatcgcc cgggatatta ttgacgatgc ggctattgac 4320
   cgctgctatc tgcccgccga gtggctgcag gatgccgggc tgaccccgga gaactatgcc 4380
   gcgcgggaga atcgggccgc gctggcgcgg gtggcggagc ggcttattga tgccgcagag 4440
   ccgtactaca tctcctccca ggccgggcta cacgatctgc cgccgcgctg cgcctgggcg 4500
   atcgccaccg cccgcagcgt ctaccgggag atcggtatta aggtaaaagc ggcgggaggc 4560
   agcgcctggg atcgccgcca gcacaccagc aaaggtgaaa aaattgccat gctgatggcg 4620
   gcaccggggc aggttattcg ggcgaagacg acgagggtga cgccgcgtcc ggccggtctt 4680
   tggcagcgtc ccgtttagac cgactccaaa cgagtcggtt tttttgcgct tgtttcggcg 4740
   tgggtatggt ggcaggcccc gtggccgggg gactgttggg cgccatctcc ttggggtcga 4800
   atttgctttc gaatttctgc cattcatccg cttattatca cttattcagg cgtagcaacc 4860
   aggcgtttaa gggcaccaat aactgcctta aaaaaattac gccccgccct gccactcatc 4920
   gcagtactgt tgtaattcat taagcattct gccgacatgg aagccatcac agacggcatg 4980
   atgaacctga atcgccagcg gcatcagcac cttgtcgcct tgcgtataat atttgcccat 5040
   ggtgaaaacg ggggcgaaga agttgtccat attggccacg tttaaatcaa aactggtgaa 5100
   actcacccag ggattggctg agacgaaaaa catattctca ataaaccctt tagggaaata 5160
   ggccaggttt tcaccgtaac acgccacatc ttgcgaatat atgtgtagaa actgccggaa 5220
   atcgtcgtgg tattcactcc agagcgatga aaacgtttca gtttgctcat ggaaaacggt 5280
   gtaacaaggg tgaacactat cccatatcac cagctcaccg tctttcattg ccatacggaa 5340
   ttccggatga gcattcatca ggcgggcaag aatgtgaata aaggccggat aaaacttgtg 5400
   cttatttttc tttacggtct ttaaaaaggc cgtaatatcc agctgaacgg tctggttata 5460
   ggtacattga gcaactgact gaaatgcctc aaaatgttct ttacgatgcc attgggatat 5520
   atcaacggtg gtatatccag tgattttttt ctccatttta gcttccttag ctcctgaaaa 5580
   tctcgataac tcaaaaaata cgcccggtag tgatcttatt tcattatggt gaaagttgga 5640
   acctcttacg tgccgatcaa cgtctcattt tcgccaaaag ttggcccagg gcttcccggt 5700
   atcaacaggg acaccaggat ttatttattc tgcgaagtga tcttccgtca caggtattta 5760
   ttcggcgcaa agtgcgtcgg gtgatgctgc caacttactg atttagtgta tgatggtgtt 5820
   tttgaggtgc tccagtggct tctgtttcta tcagctgtcc ctcctgttca gctactgacg 5880
   gggtggtgcg taacggcaaa agcaccgccg gacatcagcg ctagcggagt gtatacactc 5940
   cgctatcgct acgtgactgg gtcatggctg cgccccgaca cccgccaaca cccgctgacg 6000
   cgccctgacg ggcttgtctg ctcccggcat ccgcttacag acaagctgtg accgtctccg 6060
   ggagctgcat gtgtcagagg ttttcaccgt catcaccgaa acgcgcgagg cagctggcac 6120
   gacaggtttc ccgactggaa aagcgaattg gtataatgcc ctcgtaataa ttttgtttaa 6180
   ctttaagaag gagatatacc catgacacag agggcccacc atcaccatca ccattccatg 6240
   gctagcggcg gcggaagttc cggtagtgag agttgtgtag cggtgagaga agatttcgct 6300
   gacgaagaag attttgtgaa agctggtggt tctgagattc tatttgttca aatgcagcag 6360
   aacaaagata tggatgaaca gtctaagctt gttgataagt tgcctcctat atcaattggt 6420
   gatggtgctt tggatctagt ggttattggt tgtggtcctg ctggtttagc cttggctgca 6480
   gaatcagcta agcttggatt aaaagttgga ctcattggtc cagatcttcc ttttactaac 6540
   aattacggtg tttgggaaga tgaattcaat gatcttgggc tgcaaaaatg tattgagcat 6600
   gtttggagag agactattgt gtatctggat gatgacaagc ctattaccat tggccgtgct 6660
   tatggaagag ttagtcgacg tttgctccat gaggagcttt tgaggaggtg tgtcgagtca 6720
   ggtgtctcgt accttagctc gaaagttgac agcataacag aagcttctga tggccttaga 6780
   cttgttgctt gtgacgacaa taacgtcatt ccctgcaggc ttgccactgt tgcttctgga 6840
   gcagcttcgg gaaagctctt gcaatacgaa gttggtggac ctagagtctg tgtgcaaact 6900
   gcatacggcg tggaggttga ggtggaaaat agtccatatg atccagatca aatggttttc 6960
   atggattaca gagattatac taacgagaaa gttcggagct tagaagctga gtatccaacg 7020
   tttctgtacg ccatgcctat gacaaagtca agactcttct tcgaggagac atgtttggcc 7080
   tcaaaagatg tcatgccctt tgatttgcta aaaacgaagc tcatgttaag attagataca 7140
   ctcggaattc gaattctaaa gacttacgaa gaggagtggt cctatatccc agttggtggt 7200
   tccttgccaa acaccgaaca aaagaatctc gcctttggtg ctgccgctag catggtacat 7260
   cccgcaacag gctattcagt tgtgagatct ttgtctgaag ctccaaaata tgcatcagtc 7320
   atcgcagaga tactaagaga agagactacc aaacagatca acagtaatat ttcaagacaa 7380
   gcttgggata ctttatggcc accagaaagg aaaagacaga gagcattctt tctctttggt 7440
   cttgaggcta tagttcaatt cgataccgaa ggcattagaa gcttcttccg tactttcttc 7500
   cgccttccaa aatggatgtg gcaagggttt ctaggatcaa cattaacatc aggagatctc 7560
   gttctctttt ctttatacat gttcgtcatt tcaccaaaca atttgagaaa aggtctcatc 7620
   aatcatctca tctctgatcc aaccggagca accatgataa aaacctatct caaagtatga 7680
   ggatccggct gctaacaaag cccgttctgt ttaagaacgg gattttttgc tgaaaggagg 7740
   aactatatcc ggccggatta ctggcttact atgttggcac tgatgagggt gtcagtgaag 7800
   tgcttcatgt ggcaggagaa aaaaggctgc accggtgcgt cagcagaata tgtgatacag 7860
   gatatattcc gcttcctcgc tcactgactc gctacgctcg gtcgttcgac tgcggcgagc 7920
   ggaaatggct tacgaacggg gcggagattt cctggaagat gccaggaaga tacttaacag 7980
   ggaagtgaga gggccgcggc aaagccgttt ttccataggc tccgcccccc tgacaagcat 8040
   cacgaaatct gacgctcaaa tcagtggtgg cgaaacccga caggactata aagataccag 8100
   gcgtttcccc ctggcggctc cctcgtgcgc tctcctgttc ctgcctttcg gtttaccggt 8160
   gtcattccgc tgttatggcc gcgtttgtct cattccacgc ctgacactca gttccgggta 8220
   ggcagttcgc tccaagctgg actgtatgca cgaacccccc gttcagtccg accgctgcgc 8280
   cttatccggt aactatcgtc ttgagtccaa cccggaaaga catgcaaaag caccactggc 8340
   agcagccact ggtaattgat ttagaggagt tagtcttgaa gtcatgcgcc ggttaaggct 8400
   aaactgaaag gacaagtttt ggtgactgcg ctcctccaag ccagttacct cggttcaaag 8460
   agttggtagc tcagagaacc ttcgaaaaac cgccctgcaa ggcggttttt tcgttttcag 8520
   agcaagagat tacgcgcaga ccaaaacgat ctcaagaaga tcatcttatt aatcagataa 8580
   aatatttcta gatttcagtg caatttatct cttcaaatgt agcacctgaa gtcagcccca 8640
   tacgatataa g 8651
<210> 34
   <211> 8399
   <212> DNA
   <213> Artifizielle Sequenz
<220>
   <223> Plasmid pGT1543
<400> 34
   gctggcacga caggtttccc gactggaaag cgaattgtga tttagcttat taaattcttg 60
   acaggggggc ttgtggtata atgtatgcgt aataattttg taacgcagtg aggaaccttg 120
   caatgagcag tttcgatgcc catgaccttg acctcgacaa atttccggag gtcgtgcgag 180
   atcgtttgac gcagttcctc gatgctcaag agctaacaat tgctgatatc ggcgctcctg 240
   tcacagatgc tgtggcccat cttcgcagtt tcgtgctcaa tggaggaaag cgaatccgtc 300
   ctctttatgc gtgggctggt ttcctggcgg cgcaaggcca taagaattct tctgaaaaac 360
   ttgagtccgt ccttgacgcc gcagcgagtc tcgaattcat ccaggcttgt gccttgattc 420
   atgacgatat tatcgattct tctgataccc ggcgcggagc ccctacagtt caccgggctg 480
   tggaagctga tcaccgcgcc aataatttcg aaggcgatcc tgagcacttt ggcgtttcag 540
   tctcgatttt ggctggcgat atggcattgg tgtgggcaga agacatgctg caggattccg 600
   gtttgagtgc agaggcattg gcccgcacga gggatgcttg gcgtggcatg cgtactgagg 660
   ttattggcgg ccagctgctt gatatttatc ttgagtcgca cgccaacgag tcggtggagc 720
   ttgcggattc tgtcaaccgc ttcaaaacgg ccgcttacac gattgcgcgc ccattgcacc 780
   tgggcgcctc cattgctggc ggttcgccgc agcttatcga cgcgctcctc cactacggcc 840
   acgacatcgg cattgcattc cagttgaggg atgatctgct tggtgtgttt ggtgatcctg 900
   ctatcaccgg caaaccagct ggagacgata tccgtgaagg caagcgcact gttcttcttg 960
   cgctcgctct acaacgcgct gataagcaat ctcctgaagc tgcaacggcc attcgcgcag 1020
   gtgttggaaa ggtgacttca ccagaagata ttgctgtcat tacagagcat attcgagcta 1080
   ctggtgctga agaagaagtt gagcagcgaa tttcccagct gactgaatcc ggtttggctc 1140
   acctcgatga tgtagacatc cctgatgagg tgcgcgcaca gttgcgggca ctggctatcc 1200
   gctcaaccga acgtcggatg taataccgcc cttttgggtt caagcagtac ataacgatgg 1260
   aaccacatta caggagtagt gatgaatgaa ggacgagcgc cttgttcagc gtaagaacga 1320
   tcatctggat atcgttctcg acccccgtcg cgccgtaact caggctagcg caggttttga 1380
   gcgctggcgc tttacccact gcgccctgcc agagctgaat tttagcgaca tcacgctgga 1440
   aaccaccttc ctgaatcggc agctacaggc tccgctgctg atcagctcca tgaccggcgg 1500
   cgttgagcgc tcgcgccata tcaaccgcca cctcgccgag gcggcgcagg tgctaaaaat 1560
   tgcgatgggg gtgggctccc agcgcgtcgc cattgagagc gacgcgggct tagggctgga 1620
   taaaaccctg cggcagctgg ctccggacgt gccgctgctg gcgaacctcg gcgcggcgca 1680
   gctgaccggc agaaaaggta ttgattacgc ccgacgggcc gtggagatga tcgaggcgga 1740
   tgcgctgatt gtgcacctaa acccgctgca ggaggcgcta cagcccggcg gcgatcgcga 1800
   ctggcgcgga cggctggcgg ctattgaaac tctggtccgc gagctgcccg ttccgctggt 1860
   ggtgaaagag gtgggagccg gtatctcccg aaccgtggcc gggcagctga tcgatgccgg 1920
   cgttaccgtg attgacgtcg cgggcgcggg cggcaccagc tgggccgccg ttgaaggcga 1980
   gcgggcggcc accgagcagc agcgcagcgt ggccaacgtc tttgccgact gggggatccc 2040
   caccgctgag gcgctggttg acattgccga ggcctggccg cagatgcccc ttattgcctc 2100
   gggcgggatt aaaaacggcg tcgacgcggc gaaagcgctg cggctcggcg cgtgcatggt 2160
   agggcaggcc gccgccgtgc tcggcagcgc aggcgtctcc acggagaagg tgatcgatca 2220
   cttcaacgtg attattgagc agctgcgggt ggcctgcttc tgcaccggca gccgcagcct 2280
   gagcgatcta aagcaggctg atatccgcta tgttcgtgat acgccataag gaggtacaac 2340
   catgaagaaa accgttgtga ttggcgcagg ctttggtggc ctggcgctgg cgattcgcct 2400
   gcaggcggca gggatcccaa ccgtactgct ggagcagcgg gacaagcccg gcggtcgggc 2460
   ctacgtctgg catgaccagg gctttacctt tgacgccggg ccgacggtga tcaccgatcc 2520
   taccgcgctt gaggcgctgt tcaccctggc cggcaggcgc atggaggatt acgtcaggct 2580
   gctgccggta aaacccttct accgactctg ctgggagtcc gggaagaccc tcgactatgc 2640
   taacgacagc gccgagcttg aggcgcagat tacccagttc aacccccgcg acgtcgaggg 2700
   ctaccggcgc tttctggctt actcccaggc ggtattccag gagggatatt tgcgcctcgg 2760
   cagcgtgccg ttcctctctt ttcgcgacat gctgcgcgcc gggccgcagc tgcttaagct 2820
   ccaggcgtgg cagagcgtct accagtcggt ttcgcgcttt attgaggatg agcatctgcg 2880
   gcaggccttc tcgttccact ccctgctggt aggcggcaac cccttcacca cctcgtccat 2940
   ctacaccctg atccacgccc ttgagcggga gtggggggtc tggttccctg agggcggcac 3000
   cggggcgctg gtgaacggca tggtgaagct gtttaccgat ctgggcgggg agatcgaact 3060
   caacgcccgg gtcgaagagc tggtggtggc cgataaccgc gtaagccagg tccggctggc 3120
   ggatggtcgg atctttgaca ccgacgccgt agcctcgaac gctgacgtgg tgaacaccta 3180
   taaaaagctg ctcggccacc atccggtggg gcagaagcgg gcggcagcgc tggagcgcaa 3240
   gagcatgagc aactcgctgt ttgtgctcta cttcggcctg aaccagcctc attcccagct 3300
   ggcgcaccat accatctgtt ttggtccccg ctaccgggag ctgatcgacg agatctttac 3360
   cggcagcgcg ctggcggatg acttctcgct ctacctgcac tcgccctgcg tgaccgatcc 3420
   ctcgctcgcg cctcccggct gcgccagctt ctacgtgctg gccccggtgc cgcatcttgg 3480
   caacgcgccg ctggactggg cgcaggaggg gccgaagctg cgcgaccgca tctttgacta 3540
   ccttgaagag cgctatatgc ccggcctgcg tagccagctg gtgacccagc ggatctttac 3600
   cccggcagac ttccacgaca cgctggatgc gcatctggga tcggccttct ccatcgagcc 3660
   gctgctgacc caaagcgcct ggttccgccc gcacaaccgc gacagcgaca ttgccaacct 3720
   ctacctggtg ggcgcaggta ctcaccctgg ggcgggcatt cctggcgtag tggcctcggc 3780
   gaaagccacc gccagcctga tgattgagga tctgcaatga gccaaccgcc gctgcttgac 3840
   cacgccacgc agaccatggc caacggctcg aaaagttttg ccaccgctgc gaagctgttc 3900
   gacccggcca cccgccgtag cgtgctgatg ctctacacct ggtgccgcca ctgcgatgac 3960
   gtcattgacg accagaccca cggcttcgcc agcgaggccg cggcggagga ggaggccacc 4020
   cagcgcctgg cccggctgcg cacgctgacc ctggcggcgt ttgaaggggc cgagatgcag 4080
   gatccggcct tcgctgcctt tcaggaggtg gcgctgaccc acggtattac gccccgcatg 4140
   gcgctcgatc acctcgacgg ctttgcgatg gacgtggctc agacccgcta tgtcaccttt 4200
   gaggatacgc tgcgctactg ctatcacgtg gcgggcgtgg tgggtctgat gatggccagg 4260
   gtgatgggcg tgcgggatga gcgggtgctg gatcgcgcct gcgatctggg gctggccttc 4320
   cagctgacga atatcgcccg ggatattatt gacgatgcgg ctattgaccg ctgctatctg 4380
   cccgccgagt ggctgcagga tgccgggctg accccggaga actatgccgc gcgggagaat 4440
   cgggccgcgc tggcgcgggt ggcggagcgg cttattgatg ccgcagagcc gtactacatc 4500
   tcctcccagg ccgggctaca cgatctgccg ccgcgctgcg cctgggcgat cgccaccgcc 4560
   cgcagcgtct accgggagat cggtattaag gtaaaagcgg cgggaggcag cgcctgggat 4620
   cgccgccagc acaccagcaa aggtgaaaaa attgccatgc tgatggcggc accggggcag 4680
   gttattcggg cgaagacgac gagggtgacg ccgcgtccgg ccggtctttg gcagcgtccc 4740
   gtttagaccg actccaaacg agtcggtttt tttgcgcttg tttcggcgtg ggtatggtgg 4800
   caggccccgt ggccggggga ctgttgggcg ccatctcctt ggggtcgaat ttgctttcga 4860
   atttctgcca ttcatccgct tattatcact tattcaggcg tagcaaccag gcgtttaagg 4920
   gcaccaataa ctgccttaaa aaaattacgc cccgccctgc cactcatcgc agtactgttg 4980
   taattcatta agcattctgc cgacatggaa gccatcacag acggcatgat gaacctgaat 5040
   cgccagcggc atcagcacct tgtcgccttg cgtataatat ttgcccatgg tgaaaacggg 5100
   ggcgaagaag ttgtccatat tggccacgtt taaatcaaaa ctggtgaaac tcacccaggg 5160
   attggctgag acgaaaaaca tattctcaat aaacccttta gggaaatagg ccaggttttc 5220
   accgtaacac gccacatctt gcgaatatat gtgtagaaac tgccggaaat cgtcgtggta 5280
   ttcactccag agcgatgaaa acgtttcagt ttgctcatgg aaaacggtgt aacaagggtg 5340
   aacactatcc catatcacca gctcaccgtc tttcattgcc atacggaatt ccggatgagc 5400
   attcatcagg cgggcaagaa tgtgaataaa ggccggataa aacttgtgct tatttttctt 5460
   tacggtcttt aaaaaggccg taatatccag ctgaacggtc tggttatagg tacattgagc 5520
   aactgactga aatgcctcaa aatgttcttt acgatgccat tgggatatat caacggtggt 5580
   atatccagtg atttttttct ccattttagc ttccttagct cctgaaaatc tcgataactc 5640
   aaaaaatacg cccggtagtg atcttatttc attatggtga aagttggaac ctcttacgtg 5700
   ccgatcaacg tctcattttc gccaaaagtt ggcccagggc ttcccggtat caacagggac 5760
   accaggattt atttattctg cgaagtgatc ttccgtcaca ggtttcccga ctggaaaagc 5820
   gaattggtat aatgccctcg taataatttt gtttaacttt aagaaggaga tatacccatg 5880
   acacagaggg cccaccatca ccatcaccat tccatggcta gcggcggcgg aagttccggt 5940
   agtgagagtt gtgtagcggt gagagaagat ttcgctgacg aagaagattt tgtgaaagct 6000
   ggtggttctg agattctatt tgttcaaatg cagcagaaca aagatatgga tgaacagtct 6060
   aagcttgttg ataagttgcc tcctatatca attggtgatg gtgctttgga tctagtggtt 6120
   attggttgtg gtcctgctgg tttagccttg gctgcagaat cagctaagct tggattaaaa 6180
   gttggactca ttggtccaga tcttcctttt actaacaatt acggtgtttg ggaagatgaa 6240
   ttcaatgatc ttgggctgca aaaatgtatt gagcatgttt ggagagagac tattgtgtat 6300
   ctggatgatg acaagcctat taccattggc cgtgcttatg gaagagttag tcgacgtttg 6360
   ctccatgagg agcttttgag gaggtgtgtc gagtcaggtg tctcgtacct tagctcgaaa 6420
   gttgacagca taacagaagc ttctgatggc cttagacttg ttgcttgtga cgacaataac 6480
   gtcattccct gcaggcttgc cactgttgct tctggagcag cttcgggaaa gctcttgcaa 6540
   tacgaagttg gtggacctag agtctgtgtg caaactgcat acggcgtgga ggttgaggtg 6600
   gaaaatagtc catatgatcc agatcaaatg gttttcatgg attacagaga ttatactaac 6660
   gagaaagttc ggagcttaga agctgagtat ccaacgtttc tgtacgccat gcctatgaca 6720
   aagtcaagac tcttcttcga ggagacatgt ttggcctcaa aagatgtcat gccctttgat 6780
   ttgctaaaaa cgaagctcat gttaagatta gatacactcg gaattcgaat tctaaagact 6840
   tacgaagagg agtggtccta tatcccagtt ggtggttcct tgccaaacac cgaacaaaag 6900
   aatctcgcct ttggtgctgc cgctagcatg gtacatcccg caacaggcta ttcagttgtg 6960
   agatctttgt ctgaagctcc aaaatatgca tcagtcatcg cagagatact aagagaagag 7020
   actaccaaac agatcaacag taatatttca agacaagctt gggatacttt atggccacca 7080
   gaaaggaaaa gacagagagc attctttctc tttggtcttg aggctatagt tcaattcgat 7140
   accgaaggca ttagaagctt cttccgtact ttcttccgcc ttccaaaatg gatgtggcaa 7200
   gggtttctag gatcaacatt aacatcagga gatctcgttc tcttttcttt atacatgttc 7260
   gtcatttcac caaacaattt gagaaaaggt ctcatcaatc atctcatctc tgatccaacc 7320
   ggagcaacca tgataaaaac ctatctcaaa gtatgaggat ccggctgcta acaaagcccg 7380
   ttctgtttaa gaacgggatt ttttgctgaa aggaggaact atatccggcc ggcttcatgt     7440
   ggcaggagaa aaaaggctgc accggtgcgt cagcagaata tgtgatacag gatatattcc     7500
   gcttcctcgc tcactgactc gctacgctcg gtcgttcgac tgcggcgagc ggaaatggct     7560
   tacgaacggg gcggagattt cctggaagat gccaggaaga tacttaacag ggaagtgaga     7620
   gggccgcggc aaagccgttt ttccataggc tccgcccccc tgacaagcaa tcacgaatct     7680
   gacgctcaaa tcagtggtgg cgaaacccga caggactata aagataccag gcgtttcccc     7740
   tggcggctcc ctcgtgcgct ctcctgttcc tgcctttcgg tttaccggtg tcattccgct     7800
   gttatggccg cgtttgtctc attccacgcc tgacactcag ttccgggtag gcagttcgct     7860
   ccaagctgga ctgtatgcac gaaccccccg ttcagtccga ccgctgcgcc ttatccggta     7920
   actatcgtct tgagtccaac ccggaaagac atgcaaaagc accactggca gcagccactg     7980
   gtaattgatt tagaggagtt agtcttgaag tcatgcgccg gttaaggcta aactgaaagg     8040
   acaagttttg gtgactgcgc tcctccaagc cagttacctc ggttcaaaga gttggtagct     8100
   cagagaacct tcgaaaaacc gccctgcaag gcggtttttt cgttttcaga gcaagagatt     8160
   acgcgcagac caaaacgatc tcaagaagat catcttatta atcagataaa atatttctag     8220
   tacactccgc tatcgctacg tgactgggtc atggctgcgc cccgacaccc gccaacaccc     8280
   gctgacgcgc cctgacgggc ttgtctgctc ccggcatccg cttacagaca agctgtgacc     8340
   gtctccggga gctgcatgtg tcagaggttt tcaccgtcat caccgaaacg cgcgaggca      8399
<210> 35
   <211> 8397
   <212> DNA
   <213> Artifizielle Sequenz
<220>
   <223> Plasmid pGT1544
<400> 35
   gctggcacga caggtttccc gactggaaag cgaattgtga tagaaaacaa ataattcttg      60
   acatcttcat atggtataat tggaaggtaa taattttgta acgcagtcag gaaccttgca     120
   atgagcagtt tcgatgccca tgaccttgac ctcgacaaat ttccggaggt cgtgcgagat     180
   cgtttgacgc agttcctcga tgctcaagag ctaacaattg ctgatatcgg cgctcctgtc     240
   acagatgctg tggcccatct tcgcagtttc gtgctcaatg gaggaaagcg aatccgtcct     300
   ctttatgcgt gggctggttt cctggcggcg caaggccata agaattcttc tgaaaaactt     360
   gagtccgtcc ttgacgccgc agcgagtctc gaattcatcc aggcttgtgc cttgattcat     420
   gacgatatta tcgattcttc tgatacccgg cgcggagccc ctacagttca ccgggctgtg     480
   gaagctgatc accgcgccaa taatttcgaa ggcgatcctg agcactttgg cgtttcagtc     540
   tcgattttgg ctggcgatat ggcattggtg tgggcagaag acatgctgca ggattccggt      600
   ttgagtgcag aggcattggc ccgcacgagg gatgcttggc gtggcatgcg tactgaggtt      660
   attggcggcc agctgcttga tatttatctt gagtcgcacg ccaacgagtc ggtggagctt      720
   gcggattctg tcaaccgctt caaaacggcc gcttacacga ttgcgcgccc attgcacctg      780
   ggcgcctcca ttgctggcgg ttcgccgcag cttatcgacg cgctcctcca ctacggccac      840
   gacatcggca ttgcattcca gttgagggat gatctgcttg gtgtgtttgg tgatcctgct      900
   atcaccggca aaccagctgg agacgatatc cgtgaaggca agcgcactgt tcttcttgcg      960
   ctcgctctac aacgcgctga taagcaatct cctgaagctg caacggccat tcgcgcaggt     1020
   gttggaaagg tgacttcacc agaagatatt gctgtcatta cagagcatat tcgagctact     1080
   ggtgctgaag aagaagttga gcagcgaatt tcccagctga ctgaatccgg tttggctcac     1140
   ctcgatgatg tagacatccc tgatgaggtg cgcgcacagt tgcgggcact ggctatccgc     1200
   tcaaccgaac gtcggatgta ataccgccct tttgggttca agcagtacat aacgatggaa     1260
   ccacattaca ggagtagtga tgaatgaagg acgagcgcct tgttcagcgt aagaacgatc     1320
   atctggatat cgttctcgac ccccgtcgcg ccgtaactca ggctagcgca ggttttgagc     1380
   gctggcgctt tacccactgc gccctgccag agctgaattt tagcgacatc acgctggaaa     1440
   ccaccttcct gaatcggcag ctacaggctc cgctgctgat cagctccatg accggcggcg     1500
   ttgagcgctc gcgccatatc aaccgccacc tcgccgaggc ggcgcaggtg ctaaaaattg     1560
   cgatgggggt gggctcccag cgcgtcgcca ttgagagcga cgcgggctta gggctggata     1620
   aaaccctgcg gcagctggct ccggacgtgc cgctgctggc gaacctcggc gcggcgcagc     1680
   tgaccggcag aaaaggtatt gattacgccc gacgggccgt ggagatgatc gaggcggatg     1740
   cgctgattgt gcacctaaac ccgctgcagg aggcgctaca gcccggcggc gatcgcgact     1800
   ggcgcggacg gctggcggct attgaaactc tggtccgcga gctgcccgtt ccgctggtgg     1860
   tgaaagaggt gggagccggt atctcccgaa ccgtggccgg gcagctgatc gatgccggcg     1920
   ttaccgtgat tgacgtcgcg ggcgcgggcg gcaccagctg ggccgccgtt gaaggcgagc     1980
   gggcggccac cgagcagcag cgcagcgtgg ccaacgtctt tgccgactgg gggatcccca     2040
   ccgctgaggc gctggttgac attgccgagg cctggccgca gatgcccctt attgcctcgg     2100
   gcgggattaa aaacggcgtc gacgcggcga aagcgctgcg gctcggcgcg tgcatggtag     2160
   ggcaggccgc cgccgtgctc ggcagcgcag gcgtctccac ggagaaggtg atcgatcact     2220
   tcaacgtgat tattgagcag ctgcgggtgg cctgcttctg caccggcagc cgcagcctga     2280
   gcgatctaaa gcaggctgat atccgctatg ttcgtgatac gccataagga ggtacaacca     2340
   tgaagaaaac cgttgtgatt ggcgcaggct ttggtggcct ggcgctggcg attcgcctgc     2400
   aggcggcagg gatcccaacc gtactgctgg agcagcggga caagcccggc ggtcgggcct     2460
   acgtctggca tgaccagggc tttacctttg acgccgggcc gacggtgatc accgatccta 2520
   ccgcgcttga ggcgctgttc accctggccg gcaggcgcat ggaggattac gtcaggctgc 2580
   tgccggtaaa acccttctac cgactctgct gggagtccgg gaagaccctc gactatgcta 2640
   acgacagcgc cgagcttgag gcgcagatta cccagttcaa cccccgcgac gtcgagggct 2700
   accggcgctt tctggcttac tcccaggcgg tattccagga gggatatttg cgcctcggca 2760
   gcgtgccgtt cctctctttt cgcgacatgc tgcgcgccgg gccgcagctg cttaagctcc 2820
   aggcgtggca gagcgtctac cagtcggttt cgcgctttat tgaggatgag catctgcggc 2880
   aggccttctc gttccactcc ctgctggtag gcggcaaccc cttcaccacc tcgtccatct 2940
   acaccctgat ccacgccctt gagcgggagt ggggggtctg gttccctgag ggcggcaccg 3000
   gggcgctggt gaacggcatg gtgaagctgt ttaccgatct gggcggggag atcgaactca 3060
   acgcccgggt cgaagagctg gtggtggccg ataaccgcgt aagccaggtc cggctggcgg 3120
   atggtcggat ctttgacacc gacgccgtag cctcgaacgc tgacgtggtg aacacctata 3180
   aaaagctgct cggccaccat ccggtggggc agaagcgggc ggcagcgctg gagcgcaaga 3240
   gcatgagcaa ctcgctgttt gtgctctact tcggcctgaa ccagcctcat tcccagctgg 3300
   cgcaccatac catctgtttt ggtccccgct accgggagct gatcgacgag atctttaccg 3360
   gcagcgcgct ggcggatgac ttctcgctct acctgcactc gccctgcgtg accgatccct 3420
   cgctcgcgcc tcccggctgc gccagcttct acgtgctggc cccggtgccg catcttggca 3480
   acgcgccgct ggactgggcg caggaggggc cgaagctgcg cgaccgcatc tttgactacc 3540
   ttgaagagcg ctatatgccc ggcctgcgta gccagctggt gacccagcgg atctttaccc 3600
   cggcagactt ccacgacacg ctggatgcgc atctgggatc ggccttctcc atcgagccgc 3660
   tgctgaccca aagcgcctgg ttccgcccgc acaaccgcga cagcgacatt gccaacctct 3720
   acctggtggg cgcaggtact caccctgggg cgggcattcc tggcgtagtg gcctcggcga 3780
   aagccaccgc cagcctgatg attgaggatc tgcaatgagc caaccgccgc tgcttgacca 3840
   cgccacgcag accatggcca acggctcgaa aagttttgcc accgctgcga agctgttcga 3900
   cccggccacc cgccgtagcg tgctgatgct ctacacctgg tgccgccact gcgatgacgt 3960
   cattgacgac cagacccacg gcttcgccag cgaggccgcg gcggaggagg aggccaccca 4020
   gcgcctggcc cggctgcgca cgctgaccct ggcggcgttt gaaggggccg agatgcagga 4080
   tccggccttc gctgcctttc aggaggtggc gctgacccac ggtattacgc cccgcatggc 4140
   gctcgatcac ctcgacggct ttgcgatgga cgtggctcag acccgctatg tcacctttga 4200
   ggatacgctg cgctactgct atcacgtggc gggcgtggtg ggtctgatga tggccagggt 4260
   gatgggcgtg cgggatgagc gggtgctgga tcgcgcctgc gatctggggc tggccttcca 4320
   gctgacgaat atcgcccggg atattattga cgatgcggct attgaccgct gctatctgcc 4380
   cgccgagtgg ctgcaggatg ccgggctgac cccggagaac tatgccgcgc gggagaatcg 4440
   ggccgcgctg gcgcgggtgg cggagcggct tattgatgcc gcagagccgt actacatctc 4500
   ctcccaggcc gggctacacg atctgccgcc gcgctgcgcc tgggcgatcg ccaccgcccg 4560
   cagcgtctac cgggagatcg gtattaaggt aaaagcggcg ggaggcagcg cctgggatcg 4620
   ccgccagcac accagcaaag gtgaaaaaat tgccatgctg atggcggcac cggggcaggt 4680
   tattcgggcg aagacgacga gggtgacgcc gcgtccggcc ggtctttggc agcgtcccgt 4740
   ttagaccgac tccaaacgag tcggtttttt tgcgcttgtt tcggcgtggg tatggtggca 4800
   ggccccgtgg ccgggggact gttgggcgcc atctccttgg ggtcgaattt gctttcgaat 4860
   ttctgccatt catccgctta ttatcactta ttcaggcgta gcaaccaggc gtttaagggc 4920
   accaataact gccttaaaaa aattacgccc cgccctgcca ctcatcgcag tactgttgta 4980
   attcattaag cattctgccg acatggaagc catcacagac ggcatgatga acctgaatcg 5040
   ccagcggcat cagcaccttg tcgccttgcg tataatattt gcccatggtg aaaacggggg 5100
   cgaagaagtt gtccatattg gccacgttta aatcaaaact ggtgaaactc acccagggat 5160
   tggctgagac gaaaaacata ttctcaataa accctttagg gaaataggcc aggttttcac 5220
   cgtaacacgc cacatcttgc gaatatatgt gtagaaactg ccggaaatcg tcgtggtatt 5280
   cactccagag cgatgaaaac gtttcagttt gctcatggaa aacggtgtaa caagggtgaa 5340
   cactatccca tatcaccagc tcaccgtctt tcattgccat acggaattcc ggatgagcat 5400
   tcatcaggcg ggcaagaatg tgaataaagg ccggataaaa cttgtgctta tttttcttta 5460
   cggtctttaa aaaggccgta atatccagct gaacggtctg gttataggta cattgagcaa 5520
   ctgactgaaa tgcctcaaaa tgttctttac gatgccattg ggatatatca acggtggtat 5580
   atccagtgat ttttttctcc attttagctt ccttagctcc tgaaaatctc gataactcaa 5640
   aaaatacgcc cggtagtgat cttatttcat tatggtgaaa gttggaacct cttacgtgcc 5700
   gatcaacgtc tcattttcgc caaaagttgg cccagggctt cccggtatca acagggacac 5760
   caggatttat ttattctgcg aagtgatctt ccgtcacagg tttcccgact ggaaaagcga 5820
   attggtataa tgccctcgta ataattttgt ttaactttaa gaaggagata tacccatgac 5880
   acagagggcc caccatcacc atcaccattc catggctagc ggcggcggaa gttccggtag 5940
   tgagagttgt gtagcggtga gagaagattt cgctgacgaa gaagattttg tgaaagctgg 6000
   tggttctgag attctatttg ttcaaatgca gcagaacaaa gatatggatg aacagtctaa 6060
   gcttgttgat aagttgcctc ctatatcaat tggtgatggt gctttggatc tagtggttat 6120
   tggttgtggt cctgctggtt tagccttggc tgcagaatca gctaagcttg gattaaaagt 6180
   tggactcatt ggtccagatc ttccttttac taacaattac ggtgtttggg aagatgaatt 6240
   caatgatctt gggctgcaaa aatgtattga gcatgtttgg agagagacta ttgtgtatct 6300
   ggatgatgac aagcctatta ccattggccg tgcttatgga agagttagtc gacgtttgct 6360
   ccatgaggag cttttgagga ggtgtgtcga gtcaggtgtc tcgtacctta gctcgaaagt 6420
   tgacagcata acagaagctt ctgatggcct tagacttgtt gcttgtgacg acaataacgt 6480
   cattccctgc aggcttgcca ctgttgcttc tggagcagct tcgggaaagc tcttgcaata 6540
   cgaagttggt ggacctagag tctgtgtgca aactgcatac ggcgtggagg ttgaggtgga 6600
   aaatagtcca tatgatccag atcaaatggt tttcatggat tacagagatt atactaacga 6660
   gaaagttcgg agcttagaag ctgagtatcc aacgtttctg tacgccatgc ctatgacaaa 6720
   gtcaagactc ttcttcgagg agacatgttt ggcctcaaaa gatgtcatgc cctttgattt 6780
   gctaaaaacg aagctcatgt taagattaga tacactcgga attcgaattc taaagactta 6840
   cgaagaggag tggtcctata tcccagttgg tggttccttg ccaaacaccg aacaaaagaa 6900
   tctcgccttt ggtgctgccg ctagcatggt acatcccgca acaggctatt cagttgtgag 6960
   atctttgtct gaagctccaa aatatgcatc agtcatcgca gagatactaa gagaagagac 7020
   taccaaacag atcaacagta atatttcaag acaagcttgg gatactttat ggccaccaga 7080
   aaggaaaaga cagagagcat tctttctctt tggtcttgag gctatagttc aattcgatac 7140
   cgaaggcatt agaagcttct tccgtacttt cttccgcctt ccaaaatgga tgtggcaagg 7200
   gtttctagga tcaacattaa catcaggaga tctcgttctc ttttctttat acatgttcgt 7260
   catttcacca aacaatttga gaaaaggtct catcaatcat ctcatctctg atccaaccgg 7320
   agcaaccatg ataaaaacct atctcaaagt atgaggatcc ggctgctaac aaagcccgtt 7380
   ctgtttaaga acgggatttt ttgctgaaag gaggaactat atccggccgg cttcatgtgg 7440
   caggagaaaa aaggctgcac cggtgcgtca gcagaatatg tgatacagga tatattccgc 7500
   ttcctcgctc actgactcgc tacgctcggt cgttcgactg cggcgagcgg aaatggctta 7560
   cgaacggggc ggagatttcc tggaagatgc caggaagata cttaacaggg aagtgagagg 7620
   gccgcggcaa agccgttttt ccataggctc cgcccccctg acaagcaatc acgaatctga 7680
   cgctcaaatc agtggtggcg aaacccgaca ggactataaa gataccaggc gtttcccctg 7740
   gcggctccct cgtgcgctct cctgttcctg cctttcggtt taccggtgtc attccgctgt 7800
   tatggccgcg tttgtctcat tccacgcctg acactcagtt ccgggtaggc agttcgctcc 7860
   aagctggact gtatgcacga accccccgtt cagtccgacc gctgcgcctt atccggtaac 7920
   tatcgtcttg agtccaaccc ggaaagacat gcaaaagcac cactggcagc agccactggt 7980
   aattgattta gaggagttag tcttgaagtc atgcgccggt taaggctaaa ctgaaaggac 8040
   aagttttggt gactgcgctc ctccaagcca gttacctcgg ttcaaagagt tggtagctca 8100
   gagaaccttc gaaaaaccgc cctgcaaggc ggttttttcg ttttcagagc aagagattac      8160
   gcgcagacca aaacgatctc aagaagatca tcttattaat cagataaaat atttctagta      8220
   cactccgcta tcgctacgtg actgggtcat ggctgcgccc cgacacccgc caacacccgc      8280
   tgacgcgccc tgacgggctt gtctgctccc ggcatccgct tacagacaag ctgtgaccgt      8340
   ctccgggagc tgcatgtgtc agaggttttc accgtcatca ccgaaacgcg cgaggca          8397
<210> 36
   <211> 8402
   <212> DNA
   <213> Artifizielle Sequenz
<220>
   <223> Plasmid pGT1546
<400> 36
   gctggcacga caggtttccc gactggaaag cgaattgtga ttaaacggaa ttctttcttg 60
   acatgttttg tcgttatggt ataatttttt agtaataatt ttgtaacgca gtcaggaacc 120
   ttgcaatgag cagtttcgat gcccatgacc ttgacctcga caaatttccg gaggtcgtgc 180
   gagatcgttt gacgcagttc ctcgatgctc aagagctaac aattgctgat atcggcgctc 240
   ctgtcacaga tgctgtggcc catcttcgca gtttcgtgct caatggagga aagcgaatcc 300
   gtcctcttta tgcgtgggct ggtttcctgg cggcgcaagg ccataagaat tcttctgaaa 360
   aacttgagtc cgtccttgac gccgcagcga gtctcgaatt catccaggct tgtgccttga 420
   ttcatgacga tattatcgat tcttctgata cccggcgcgg agcccctaca gttcaccggg 480
   ctgtggaagc tgatcaccgc gccaataatt tcgaaggcga tcctgagcac tttggcgttt 540
   cagtctcgat tttggctggc gatatggcat tggtgtgggc agaagacatg ctgcaggatt 600
   ccggtttgag tgcagaggca ttggcccgca cgagggatgc ttggcgtggc atgcgtactg 660
   aggttattgg cggccagctg cttgatattt atcttgagtc gcacgccaac gagtcggtgg 720
   agcttgcgga ttctgtcaac cgcttcaaaa cggccgctta cacgattgcg cgcccattgc 780
   acctgggcgc ctccattgct ggcggttcgc cgcagcttat cgacgcgctc ctccactacg 840
   gccacgacat cggcattgca ttccagttga gggatgatct gcttggtgtg tttggtgatc 900
   ctgctatcac cggcaaacca gctggagacg atatccgtga aggcaagcgc actgttcttc 960
   ttgcgctcgc tctacaacgc gctgataagc aatctcctga agctgcaacg gccattcgcg 1020
   caggtgttgg aaaggtgact tcaccagaag atattgctgt cattacagag catattcgag 1080
   ctactggtgc tgaagaagaa gttgagcagc gaatttccca gctgactgaa tccggtttgg 1140
   ctcacctcga tgatgtagac atccctgatg aggtgcgcgc acagttgcgg gcactggcta 1200
   tccgctcaac cgaacgtcgg atgtaatacc gcccttttgg gttcaagcag tacataacga 1260
   tggaaccaca ttacaggagt agtgatgaat gaaggacgag cgccttgttc agcgtaagaa 1320
   cgatcatctg gatatcgttc tcgacccccg tcgcgccgta actcaggcta gcgcaggttt 1380
   tgagcgctgg cgctttaccc actgcgccct gccagagctg aattttagcg acatcacgct 1440
   ggaaaccacc ttcctgaatc ggcagctaca ggctccgctg ctgatcagct ccatgaccgg 1500
   cggcgttgag cgctcgcgcc atatcaaccg ccacctcgcc gaggcggcgc aggtgctaaa 1560
   aattgcgatg ggggtgggct cccagcgcgt cgccattgag agcgacgcgg gcttagggct 1620
   ggataaaacc ctgcggcagc tggctccgga cgtgccgctg ctggcgaacc tcggcgcggc 1680
   gcagctgacc ggcagaaaag gtattgatta cgcccgacgg gccgtggaga tgatcgaggc 1740
   ggatgcgctg attgtgcacc taaacccgct gcaggaggcg ctacagcccg gcggcgatcg 1800
   cgactggcgc ggacggctgg cggctattga aactctggtc cgcgagctgc ccgttccgct 1860
   ggtggtgaaa gaggtgggag ccggtatctc ccgaaccgtg gccgggcagc tgatcgatgc 1920
   cggcgttacc gtgattgacg tcgcgggcgc gggcggcacc agctgggccg ccgttgaagg 1980
   cgagcgggcg gccaccgagc agcagcgcag cgtggccaac gtctttgccg actgggggat 2040
   ccccaccgct gaggcgctgg ttgacattgc cgaggcctgg ccgcagatgc cccttattgc 2100
   ctcgggcggg attaaaaacg gcgtcgacgc ggcgaaagcg ctgcggctcg gcgcgtgcat 2160
   ggtagggcag gccgccgccg tgctcggcag cgcaggcgtc tccacggaga aggtgatcga 2220
   tcacttcaac gtgattattg agcagctgcg ggtggcctgc ttctgcaccg gcagccgcag 2280
   cctgagcgat ctaaagcagg ctgatatccg ctatgttcgt gatacgccat aaggaggtac 2340
   aaccatgaag aaaaccgttg tgattggcgc aggctttggt ggcctggcgc tggcgattcg 2400
   cctgcaggcg gcagggatcc caaccgtact gctggagcag cgggacaagc ccggcggtcg 2460
   ggcctacgtc tggcatgacc agggctttac ctttgacgcc gggccgacgg tgatcaccga 2520
   tcctaccgcg cttgaggcgc tgttcaccct ggccggcagg cgcatggagg attacgtcag 2580
   gctgctgccg gtaaaaccct tctaccgact ctgctgggag tccgggaaga ccctcgacta 2640
   tgctaacgac agcgccgagc ttgaggcgca gattacccag ttcaaccccc gcgacgtcga 2700
   gggctaccgg cgctttctgg cttactccca ggcggtattc caggagggat atttgcgcct 2760
   cggcagcgtg ccgttcctct cttttcgcga catgctgcgc gccgggccgc agctgcttaa 2820
   gctccaggcg tggcagagcg tctaccagtc ggtttcgcgc tttattgagg atgagcatct 2880
   gcggcaggcc ttctcgttcc actccctgct ggtaggcggc aaccccttca ccacctcgtc 2940
   catctacacc ctgatccacg cccttgagcg ggagtggggg gtctggttcc ctgagggcgg 3000
   caccggggcg ctggtgaacg gcatggtgaa gctgtttacc gatctgggcg gggagatcga 3060
   actcaacgcc cgggtcgaag agctggtggt ggccgataac cgcgtaagcc aggtccggct 3120
   ggcggatggt cggatctttg acaccgacgc cgtagcctcg aacgctgacg tggtgaacac 3180
   ctataaaaag ctgctcggcc accatccggt ggggcagaag cgggcggcag cgctggagcg 3240
   caagagcatg agcaactcgc tgtttgtgct ctacttcggc ctgaaccagc ctcattccca 3300
   gctggcgcac cataccatct gttttggtcc ccgctaccgg gagctgatcg acgagatctt 3360
   taccggcagc gcgctggcgg atgacttctc gctctacctg cactcgccct gcgtgaccga 3420
   tccctcgctc gcgcctcccg gctgcgccag cttctacgtg ctggccccgg tgccgcatct 3480
   tggcaacgcg ccgctggact gggcgcagga ggggccgaag ctgcgcgacc gcatctttga 3540
   ctaccttgaa gagcgctata tgcccggcct gcgtagccag ctggtgaccc agcggatctt 3600
   taccccggca gacttccacg acacgctgga tgcgcatctg ggatcggcct tctccatcga 3660
   gccgctgctg acccaaagcg cctggttccg cccgcacaac cgcgacagcg acattgccaa 3720
   cctctacctg gtgggcgcag gtactcaccc tggggcgggc attcctggcg tagtggcctc 3780
   ggcgaaagcc accgccagcc tgatgattga ggatctgcaa tgagccaacc gccgctgctt 3840
   gaccacgcca cgcagaccat ggccaacggc tcgaaaagtt ttgccaccgc tgcgaagctg 3900
   ttcgacccgg ccacccgccg tagcgtgctg atgctctaca cctggtgccg ccactgcgat 3960
   gacgtcattg acgaccagac ccacggcttc gccagcgagg ccgcggcgga ggaggaggcc 4020
   acccagcgcc tggcccggct gcgcacgctg accctggcgg cgtttgaagg ggccgagatg 4080
   caggatccgg ccttcgctgc ctttcaggag gtggcgctga cccacggtat tacgccccgc 4140
   atggcgctcg atcacctcga cggctttgcg atggacgtgg ctcagacccg ctatgtcacc 4200
   tttgaggata cgctgcgcta ctgctatcac gtggcgggcg tggtgggtct gatgatggcc 4260
   agggtgatgg gcgtgcggga tgagcgggtg ctggatcgcg cctgcgatct ggggctggcc 4320
   ttccagctga cgaatatcgc ccgggatatt attgacgatg cggctattga ccgctgctat 4380
   ctgcccgccg agtggctgca ggatgccggg ctgaccccgg agaactatgc cgcgcgggag 4440
   aatcgggccg cgctggcgcg ggtggcggag cggcttattg atgccgcaga gccgtactac 4500
   atctcctccc aggccgggct acacgatctg ccgccgcgct gcgcctgggc gatcgccacc 4560
   gcccgcagcg tctaccggga gatcggtatt aaggtaaaag cggcgggagg cagcgcctgg 4620
   gatcgccgcc agcacaccag caaaggtgaa aaaattgcca tgctgatggc ggcaccgggg 4680
   caggttattc gggcgaagac gacgagggtg acgccgcgtc cggccggtct ttggcagcgt 4740
   cccgtttaga ccgactccaa acgagtcggt ttttttgcgc ttgtttcggc gtgggtatgg 4800
   tggcaggccc cgtggccggg ggactgttgg gcgccatctc cttggggtcg aatttgcttt 4860
   cgaatttctg ccattcatcc gcttattatc acttattcag gcgtagcaac caggcgttta 4920
   agggcaccaa taactgcctt aaaaaaatta cgccccgccc tgccactcat cgcagtactg 4980
   ttgtaattca ttaagcattc tgccgacatg gaagccatca cagacggcat gatgaacctg 5040
   aatcgccagc ggcatcagca ccttgtcgcc ttgcgtataa tatttgccca tggtgaaaac 5100
   gggggcgaag aagttgtcca tattggccac gtttaaatca aaactggtga aactcaccca 5160
   gggattggct gagacgaaaa acatattctc aataaaccct ttagggaaat aggccaggtt 5220
   ttcaccgtaa cacgccacat cttgcgaata tatgtgtaga aactgccgga aatcgtcgtg 5280
   gtattcactc cagagcgatg aaaacgtttc agtttgctca tggaaaacgg tgtaacaagg 5340
   gtgaacacta tcccatatca ccagctcacc gtctttcatt gccatacgga attccggatg 5400
   agcattcatc aggcgggcaa gaatgtgaat aaaggccgga taaaacttgt gcttattttt 5460
   ctttacggtc tttaaaaagg ccgtaatatc cagctgaacg gtctggttat aggtacattg 5520
   agcaactgac tgaaatgcct caaaatgttc tttacgatgc cattgggata tatcaacggt 5580
   ggtatatcca gtgatttttt tctccatttt agcttcctta gctcctgaaa atctcgataa 5640
   ctcaaaaaat acgcccggta gtgatcttat ttcattatgg tgaaagttgg aacctcttac 5700
   gtgccgatca acgtctcatt ttcgccaaaa gttggcccag ggcttcccgg tatcaacagg 5760
   gacaccagga tttatttatt ctgcgaagtg atcttccgtc acaggtttcc cgactggaaa 5820
   agcgaattgg tataatgccc tcgtaataat tttgtttaac tttaagaagg agatataccc 5880
   atgacacaga gggcccacca tcaccatcac cattccatgg ctagcggcgg cggaagttcc 5940
   ggtagtgaga gttgtgtagc ggtgagagaa gatttcgctg acgaagaaga ttttgtgaaa 6000
   gctggtggtt ctgagattct atttgttcaa atgcagcaga acaaagatat ggatgaacag 6060
   tctaagcttg ttgataagtt gcctcctata tcaattggtg atggtgcttt ggatctagtg 6120
   gttattggtt gtggtcctgc tggtttagcc ttggctgcag aatcagctaa gcttggatta 6180
   aaagttggac tcattggtcc agatcttcct tttactaaca attacggtgt ttgggaagat 6240
   gaattcaatg atcttgggct gcaaaaatgt attgagcatg tttggagaga gactattgtg 6300
   tatctggatg atgacaagcc tattaccatt ggccgtgctt atggaagagt tagtcgacgt 6360
   ttgctccatg aggagctttt gaggaggtgt gtcgagtcag gtgtctcgta ccttagctcg 6420
   aaagttgaca gcataacaga agcttctgat ggccttagac ttgttgcttg tgacgacaat 6480
   aacgtcattc cctgcaggct tgccactgtt gcttctggag cagcttcggg aaagctcttg 6540
   caatacgaag ttggtggacc tagagtctgt gtgcaaactg catacggcgt ggaggttgag 6600
   gtggaaaata gtccatatga tccagatcaa atggttttca tggattacag agattatact 6660
   aacgagaaag ttcggagctt agaagctgag tatccaacgt ttctgtacgc catgcctatg 6720
   acaaagtcaa gactcttctt cgaggagaca tgtttggcct caaaagatgt catgcccttt 6780
   gatttgctaa aaacgaagct catgttaaga ttagatacac tcggaattcg aattctaaag 6840
   acttacgaag aggagtggtc ctatatccca gttggtggtt ccttgccaaa caccgaacaa 6900
   aagaatctcg cctttggtgc tgccgctagc atggtacatc ccgcaacagg ctattcagtt 6960
   gtgagatctt tgtctgaagc tccaaaatat gcatcagtca tcgcagagat actaagagaa 7020
   gagactacca aacagatcaa cagtaatatt tcaagacaag cttgggatac tttatggcca 7080
   ccagaaagga aaagacagag agcattcttt ctctttggtc ttgaggctat agttcaattc 7140
   gataccgaag gcattagaag cttcttccgt actttcttcc gccttccaaa atggatgtgg 7200
   caagggtttc taggatcaac attaacatca ggagatctcg ttctcttttc tttatacatg 7260
   ttcgtcattt caccaaacaa tttgagaaaa ggtctcatca atcatctcat ctctgatcca 7320
   accggagcaa ccatgataaa aacctatctc aaagtatgag gatccggctg ctaacaaagc 7380
   ccgttctgtt taagaacggg attttttgct gaaaggagga actatatccg gccggcttca 7440
   tgtggcagga gaaaaaaggc tgcaccggtg cgtcagcaga atatgtgata caggatatat 7500
   tccgcttcct cgctcactga ctcgctacgc tcggtcgttc gactgcggcg agcggaaatg 7560
   gcttacgaac ggggcggaga tttcctggaa gatgccagga agatacttaa cagggaagtg 7620
   agagggccgc ggcaaagccg tttttccata ggctccgccc ccctgacaag caatcacgaa 7680
   tctgacgctc aaatcagtgg tggcgaaacc cgacaggact ataaagatac caggcgtttc 7740
   ccctggcggc tccctcgtgc gctctcctgt tcctgccttt cggtttaccg gtgtcattcc 7800
   gctgttatgg ccgcgtttgt ctcattccac gcctgacact cagttccggg taggcagttc 7860
   gctccaagct ggactgtatg cacgaacccc ccgttcagtc cgaccgctgc gccttatccg 7920
   gtaactatcg tcttgagtcc aacccggaaa gacatgcaaa agcaccactg gcagcagcca 7980
   ctggtaattg atttagagga gttagtcttg aagtcatgcg ccggttaagg ctaaactgaa 8040
   aggacaagtt ttggtgactg cgctcctcca agccagttac ctcggttcaa agagttggta 8100
   gctcagagaa ccttcgaaaa accgccctgc aaggcggttt tttcgttttc agagcaagag 8160
   attacgcgca gaccaaaacg atctcaagaa gatcatctta ttaatcagat aaaatatttc 8220
   tagtacactc cgctatcgct acgtgactgg gtcatggctg cgccccgaca cccgccaaca 8280
   cccgctgacg cgccctgacg ggcttgtctg ctcccggcat ccgcttacag acaagctgtg 8340
   accgtctccg ggagctgcat gtgtcagagg ttttcaccgt catcaccgaa acgcgcgagg 8400
   ca 8402
<210> 37
   <211> 6022
   <212> DNA
   <213> Artifizielle Sequenz
<220>
   <223> Plasmid pGT1454
<400> 37
   acccagtttc atcattccat tttattttgc gagcgagcgc acacttgtga attatctcaa      60
   tagcagtgtg aaataacata attgagcaac tgaaagggag tgcccaatat tacgacatca     120
   tccatcacgc ggagttacct gattatgaca cagagggccc accatcacca tcaccattcc     180
   atggcggaga aactcagtga tggcagcagc atcatctcag tccatcctag accctccaag     240
   ggtttctcct cgaagcttct cgatcttctc gagagacttg ttgtcaagct catgcacgat     300
   gcttctctcc ctctccacta cctctcaggc aacttcgctc ccatccgtga tgaaactcct     360
   cccgtcaagg atctccccgt ccatggattt cttcccgaat gcttgaatgg tgaatttgtg     420
   agggttggtc caaaccccaa gtttgatgct gtcgctggat atcactggtt tgatggagat     480
   gggatgattc atggggtacg catcaaagat gggaaagcta cttatgtttc tcgatatgtt     540
   aagacatcac gtcttaagca ggaagagttc ttcggagctg ccaaattcat gaagattggt     600
   gaccttaagg ggtttttcgg attgctaatg gtcaatgtcc aacagctgag aacgaagctc     660
   aaaatattgg acaacactta tggaaatgga actgccaata cagcactcgt atatcaccat     720
   ggaaaacttc tagcattaca ggaggcagat aagccgtacg tcatcaaagt tttggaagat     780
   ggagacctgc aaactcttgg tataatagat tatgacaaga gattgaccca ctccttcact     840
   gctcacccaa aagttgaccc ggttacgggt gaaatgttta cattcggcta ttcgcatacg     900
   ccaccttatc tcacatacag agttatctcg aaagatggca ttatgcatga cccagtccca     960
   attactatat cagagcctat catgatgcat gattttgcta ttactgagac ttatgcaatc    1020
   ttcatggatc ttcctatgca cttcaggcca aaggaaatgg tgaaagagaa gaaaatgata    1080
   tactcatttg atcccacaaa aaaggctcgt tttggtgttc ttccacgcta tgccaaggat    1140
   gaacttatga ttagatggtt tgagcttccc aactgcttta ttttccacaa cgccaatgct    1200
   tgggaagaag aggatgaagt cgtcctcatc acttgtcgtc ttgagaatcc agatcttgac    1260
   atggtcagtg ggaaagtgaa agaaaaactc gaaaattttg gcaacgaact gtacgaaatg    1320
   agattcaaca tgaaaacggg ctcagcttct caaaaaaaac tatccgcatc tgcggttgat    1380
   ttccccagaa tcaatgagtg ctacaccgga aagaaacaga gatacgtata tggaacaatt    1440
   ctggacagta tcgcaaaggt taccggaatc atcaagtttg atctgcatgc agaagctgag    1500
   acagggaaaa gaatgctgga agtaggaggt aatatcaaag gaatatatga cctgggagaa    1560
   ggcagatatg gttcagaggc tatctatgtt ccgcgtgaga cagcagaaga agacgacggt    1620
   tacttgatat tctttgttca tgatgaaaac acagggaaat catgcgtgac tgtgatagac    1680
   gcaaaaacaa tgtcggctga accggtggca gtggtggagc tgccgcacag ggtcccatat    1740
   ggcttccatg ccttgtttgt tacagaggaa caactccagg aacaaactct tatataagga    1800
   tccggctgct aacaaagccc gaaaggaagc tgagttggct gctgccaccg ctgagcaata    1860
   actagcataa ccccttgggg cctctaaacg ggtcttgagg ggttttttgc tgaaaggagg    1920
   aactatatcc ggccggatat ccacaggacg ggtgtggtcg ccatgatcgc gtagtcgata    1980
   gtggctccaa gtagcgaagc gagcaggact gggcggcggc caaagcggtc ggacagtgct 2040
   ccgagaacgg gtgcgcatag aaattgcatc aacgcatata gcgctagcag cacgccatag 2100
   tgactggcga tgctgtcgga atggacgata tcccgcaaga ggcccggcag taccggcata 2160
   accaagccta tgcctacagc atccagggtg acggtgccga ggatgacgat gagcgcattg 2220
   ttagatttca tacacggtgc ctgactgcgt tagcaattta actgtgataa actaccgcat 2280
   taaagcttat cgatgataag ctgtcaaaca tgagaattct tgaagacgaa agggcctcgt 2340
   gatacgccta tttttatagg ttaatgtcat gcatgagaca ataaccctga taaatgcttc 2400
   aataatattg aaaaaggaag agtatgagta ttcaacattt ccgtgtcgcc cttattccct 2460
   tttttgcggc attttgcctt cctgtttttg ctcacccaga aacgctggtg aaagtaaaag 2520
   atgctgaaga tcagttgggt gcacgagtgg gttacatcga actggatctc aacagcggta 2580
   agatccttga gagttttcgc cccgaagaac gttttccaat gatgagcact tttaaagttc 2640
   tgctatgtgg cgcggtatta tcccgtgttg acgccgggca agagcaactc ggtcgccgca 2700
   tacactattc tcagaatgac ttggttgacg cgtcaccagt cacagaaaag catcttacgg 2760
   atggcatgac agtaagagaa ttatgcagtg ctgccataac catgagtgat aacactgcgg 2820
   ccaacttact tctgacaacg atcggaggac cgaaggagct aaccgctttt ttgcacaaca 2880
   tgggggatca tgtaactcgc cttgatcgtt gggaaccgga gctgaatgaa gccataccaa 2940
   acgacgagcg tgacaccacg atgcctgcag caatggcaac aacgttgcgc aaactattaa 3000
   ctggcgaact acttactcta gcttcccggc aacaattaat agactggatg gaggcggata 3060
   aagttgcagg accacttctg cgctcggccc ttccggctgg ctggtttatt gctgataaat 3120
   ctggagccgg tgagcgtggg tctcgcggta tcattgcagc actggggcca gatggtaagc 3180
   cctcccgtat cgtagttatc tacacgacgg ggagtcaggc aactatggat gaacgaaata 3240
   gacagatcgc tgagataggt gcctcactga ttaagcattg gtaactgtca gaccaagttt 3300
   actcatatat actttagatt gatttaaaac ttcattttta atttaaaagg atctaggtga 3360
   agatcctttt tgataatctc atgcatgacc aaaatccctt aacgtgagtt ttcgttccac 3420
   tgagcgtcag accccgtaga aaagatcaaa ggatcttctt gagatccttt ttttctgcgc 3480
   gtaatctgct gcttgcaaac aaaaaaacca ccgctaccag cggtggtttg tttgccggat 3540
   caagagctac caactctttt tccgaaggta actggcttca gcagagcgca gataccaaat 3600
   actgtccttc tagtgtagcc gtagttaggc caccacttca agaactctgt agcaccgcct 3660
   acatacctcg ctctgctaat cctgttacca gtggctgctg ccagtggcga taagtcgtgt 3720
   cttaccgggt tggactcaag acgatagtta ccggataagg cgcagcggtc gggctgaacg 3780
   gggggttcgt gcacacagcc cagcttggag cgaacgacct acaccgaact gagataccta 3840
   cagcgtgagc tatgagaaag cgccacgctt cccgaaggga gaaaggcgga caggtatccg 3900
   gtaagcggca gggtcggaac aggagagcgc acgagggagc ttccaggggg aaacgcctgg 3960
   tatctttata gtcctgtcgg gtttcgccac ctctgacttg agcgtcgatt tttgtgatgc 4020
   tcgtcagggg ggcggagcct atggaaaaac gccagcaacg cggccttttt acggttcctg 4080
   gccttttgct ggccttttgc tcacatgttc tttcctgcgt tatcccctga ttctgtggat 4140
   aaccgtatta ccgcctttga gtgagctgat accgctcgcc gcagccgaac gaccgagcgc 4200
   agcgagtcag tgagcgagga agcggaagag cgcctgatgc ggtattttct ccttacgcat 4260
   ctgtgcggta tttcacaccg catatatggt gcactctcag tacaatctgc tctgatgccg 4320
   catagttaag ccagtataca ctccgctatc gctacgtgac tgggtcatgg ctgcgccccg 4380
   acacccgcca acacccgctg acgcgccctg acgggcttgt ctgctcccgg catccgctta 4440
   cagacaagct gtgaccgtct ccgggagctg catgtgtcag aggttttcac cgtcatcacc 4500
   gaaacgcgcg aggcagctgg cacgacaggt ttcccgactg ggttggtgcg ttttatcatg 4560
   cctggcgggt aggtcggata aggcgttcac gccgcatccg acaaccacgc agcgttacct 4620
   gatgtgacgc cgacaattct catcatcgct acaacatgac ctcgctattt acatcgcgat 4680
   actcttttgg cgtcgtgtca tatgcttttt taaaaacaga gtagaaatat tgcagcgatg 4740
   gataaccgca catttgcgat atctcattga tcgacaaggt ggttgaaatc agcagactgc 4800
   gcgctttctc cagcttctcg gcatgaatca tggcatggat ggtttcaccc acctcttctt 4860
   taaaacgctt ctcaagattg gagcgcgaga tcccgaccgc atccagtacc tgatccactt 4920
   taatcccttt acaggcgtga ttacgaatgt aatgcatggc ctgaataacg gcgggatcgg 4980
   tcagcgagcg ataatctgtt gagcgccgtt caatgacgcg aactggtggg accaaaattc 5040
   gctgtagcgg catttcttct ttatctaata atcgatgcaa cagttttgcc gcctgatagc 5100
   ccatttgccg cgcgccctga gcgaccgaag aaagggcgac acgcgacaga tagcgggtca 5160
   gttcttcgtt atcgatgcca atcacgcata atttttccgg tacgggaata tgtagatgtt 5220
   cacatacttg cagaatatgc cgcgctcggg cgtcagtaac ggcaataatc ccggtttgcg 5280
   gtggtagcgt ttgtagccag tctgccagcc gattttgcgc gtgttgccag ttctctggcg 5340
   cggtttctaa cccctgataa accactccgc gatacttttc ttcggcgaca agctgacgaa 5400
   atgcatattc gcgctcagtg gcccaacgtt tgccgcttga ttccggaaga ccataaaaag 5460
   caaagcggtt aacgcctttc tcttttaaat gcaaaaatgc gctttcaacc agcgcatagt 5520
   tatcggtggc aatgtaatga acgggtgggt aactttctgc aaggtgatac gagccgccaa 5580
   ccccaacaat ggggacgtcg acatcagcca gcgcttgctc gatctgtttg tcgtcgaagt 5640
   cggcaatgac gccatctcct aaccagtcct tgattttatc aatgcgggcg cggaaatctt 5700
   cttcaatgaa aatatcccat tccgattgtg acgcctgtaa atattcccct acgccttcta 5760
   ctacctgccg gtcataggct ttattggcat tgaacagtaa tgtgatgcgg tgacgtttag 5820
   taaacatggt tcttttcctg ctgaatcatg ctaagtaaca atcaccgcga taaacgtaac 5880
   caatttttag caactaaaca ggggaaaaca attacagatt tttatctttc gattacgatt 5940
   tttggtttat ttcttgattt atgaccgaga tcttactttt gttgcgcaat tgtacttatt 6000
   gcatttttct cttcgaggaa tt 6022
<210> 38
   <211> 6022
   <212> DNA
   <213> Artifizielle Sequenz
<220>
   <223> Plasmid pGT1455
<400> 38
   acccagtttc atcattccat tttattttgc gagcgagcgc acacttgtga attatctcaa      60
   tagcagtgtg aaataacata attgagcaac tgaaagggag tgccctatat tacgacatca     120
   tccatcacgc ggagttacct gattatgaca cagagggccc accatcacca tcaccattcc     180
   atggcggaga aactcagtga tggcagcagc atcatctcag tccatcctag accctccaag     240
   ggtttctcct cgaagcttct cgatcttctc gagagacttg ttgtcaagct catgcacgat     300
   gcttctctcc ctctccacta cctctcaggc aacttcgctc ccatccgtga tgaaactcct     360
   cccgtcaagg atctccccgt ccatggattt cttcccgaat gcttgaatgg tgaatttgtg     420
   agggttggtc caaaccccaa gtttgatgct gtcgctggat atcactggtt tgatggagat     480
   gggatgattc atggggtacg catcaaagat gggaaagcta cttatgtttc tcgatatgtt     540
   aagacatcac gtcttaagca ggaagagttc ttcggagctg ccaaattcat gaagattggt     600
   gaccttaagg ggtttttcgg attgctaatg gtcaatgtcc aacagctgag aacgaagctc     660
   aaaatattgg acaacactta tggaaatgga actgccaata cagcactcgt atatcaccat     720
   ggaaaacttc tagcattaca ggaggcagat aagccgtacg tcatcaaagt tttggaagat     780
   ggagacctgc aaactcttgg tataatagat tatgacaaga gattgaccca ctccttcact     840
   gctcacccaa aagttgaccc ggttacgggt gaaatgttta cattcggcta ttcgcatacg     900
   ccaccttatc tcacatacag agttatctcg aaagatggca ttatgcatga cccagtccca     960
   attactatat cagagcctat catgatgcat gattttgcta ttactgagac ttatgcaatc    1020
   ttcatggatc ttcctatgca cttcaggcca aaggaaatgg tgaaagagaa gaaaatgata    1080
   tactcatttg atcccacaaa aaaggctcgt tttggtgttc ttccacgcta tgccaaggat    1140
   gaacttatga ttagatggtt tgagcttccc aactgcttta ttttccacaa cgccaatgct    1200
   tgggaagaag aggatgaagt cgtcctcatc acttgtcgtc ttgagaatcc agatcttgac    1260
   atggtcagtg ggaaagtgaa agaaaaactc gaaaattttg gcaacgaact gtacgaaatg    1320
   agattcaaca tgaaaacggg ctcagcttct caaaaaaaac tatccgcatc tgcggttgat 1380
   ttccccagaa tcaatgagtg ctacaccgga aagaaacaga gatacgtata tggaacaatt 1440
   ctggacagta tcgcaaaggt taccggaatc atcaagtttg atctgcatgc agaagctgag 1500
   acagggaaaa gaatgctgga agtaggaggt aatatcaaag gaatatatga cctgggagaa 1560
   ggcagatatg gttcagaggc tatctatgtt ccgcgtgaga cagcagaaga agacgacggt 1620
   tacttgatat tctttgttca tgatgaaaac acagggaaat catgcgtgac tgtgatagac 1680
   gcaaaaacaa tgtcggctga accggtggca gtggtggagc tgccgcacag ggtcccatat 1740
   ggcttccatg ccttgtttgt tacagaggaa caactccagg aacaaactct tatataagga 1800
   tccggctgct aacaaagccc gaaaggaagc tgagttggct gctgccaccg ctgagcaata 1860
   actagcataa ccccttgggg cctctaaacg ggtcttgagg ggttttttgc tgaaaggagg 1920
   aactatatcc ggccggatat ccacaggacg ggtgtggtcg ccatgatcgc gtagtcgata 1980
   gtggctccaa gtagcgaagc gagcaggact gggcggcggc caaagcggtc ggacagtgct 2040
   ccgagaacgg gtgcgcatag aaattgcatc aacgcatata gcgctagcag cacgccatag 2100
   tgactggcga tgctgtcgga atggacgata tcccgcaaga ggcccggcag taccggcata 2160
   accaagccta tgcctacagc atccagggtg acggtgccga ggatgacgat gagcgcattg 2220
   ttagatttca tacacggtgc ctgactgcgt tagcaattta actgtgataa actaccgcat 2280
   taaagcttat cgatgataag ctgtcaaaca tgagaattct tgaagacgaa agggcctcgt 2340
   gatacgccta tttttatagg ttaatgtcat gcatgagaca ataaccctga taaatgcttc 2400
   aataatattg aaaaaggaag agtatgagta ttcaacattt ccgtgtcgcc cttattccct 2460
   tttttgcggc attttgcctt cctgtttttg ctcacccaga aacgctggtg aaagtaaaag 2520
   atgctgaaga tcagttgggt gcacgagtgg gttacatcga actggatctc aacagcggta 2580
   agatccttga gagttttcgc cccgaagaac gttttccaat gatgagcact tttaaagttc 2640
   tgctatgtgg cgcggtatta tcccgtgttg acgccgggca agagcaactc ggtcgccgca 2700
   tacactattc tcagaatgac ttggttgacg cgtcaccagt cacagaaaag catcttacgg 2760
   atggcatgac agtaagagaa ttatgcagtg ctgccataac catgagtgat aacactgcgg 2820
   ccaacttact tctgacaacg atcggaggac cgaaggagct aaccgctttt ttgcacaaca 2880
   tgggggatca tgtaactcgc cttgatcgtt gggaaccgga gctgaatgaa gccataccaa 2940
   acgacgagcg tgacaccacg atgcctgcag caatggcaac aacgttgcgc aaactattaa 3000
   ctggcgaact acttactcta gcttcccggc aacaattaat agactggatg gaggcggata 3060
   aagttgcagg accacttctg cgctcggccc ttccggctgg ctggtttatt gctgataaat 3120
   ctggagccgg tgagcgtggg tctcgcggta tcattgcagc actggggcca gatggtaagc 3180
   cctcccgtat cgtagttatc tacacgacgg ggagtcaggc aactatggat gaacgaaata 3240
   gacagatcgc tgagataggt gcctcactga ttaagcattg gtaactgtca gaccaagttt 3300
   actcatatat actttagatt gatttaaaac ttcattttta atttaaaagg atctaggtga 3360
   agatcctttt tgataatctc atgcatgacc aaaatccctt aacgtgagtt ttcgttccac 3420
   tgagcgtcag accccgtaga aaagatcaaa ggatcttctt gagatccttt ttttctgcgc 3480
   gtaatctgct gcttgcaaac aaaaaaacca ccgctaccag cggtggtttg tttgccggat 3540
   caagagctac caactctttt tccgaaggta actggcttca gcagagcgca gataccaaat 3600
   actgtccttc tagtgtagcc gtagttaggc caccacttca agaactctgt agcaccgcct 3660
   acatacctcg ctctgctaat cctgttacca gtggctgctg ccagtggcga taagtcgtgt 3720
   cttaccgggt tggactcaag acgatagtta ccggataagg cgcagcggtc gggctgaacg 3780
   gggggttcgt gcacacagcc cagcttggag cgaacgacct acaccgaact gagataccta 3840
   cagcgtgagc tatgagaaag cgccacgctt cccgaaggga gaaaggcgga caggtatccg 3900
   gtaagcggca gggtcggaac aggagagcgc acgagggagc ttccaggggg aaacgcctgg 3960
   tatctttata gtcctgtcgg gtttcgccac ctctgacttg agcgtcgatt tttgtgatgc 4020
   tcgtcagggg ggcggagcct atggaaaaac gccagcaacg cggccttttt acggttcctg 4080
   gccttttgct ggccttttgc tcacatgttc tttcctgcgt tatcccctga ttctgtggat 4140
   aaccgtatta ccgcctttga gtgagctgat accgctcgcc gcagccgaac gaccgagcgc 4200
   agcgagtcag tgagcgagga agcggaagag cgcctgatgc ggtattttct ccttacgcat 4260
   ctgtgcggta tttcacaccg catatatggt gcactctcag tacaatctgc tctgatgccg 4320
   catagttaag ccagtataca ctccgctatc gctacgtgac tgggtcatgg ctgcgccccg 4380
   acacccgcca acacccgctg acgcgccctg acgggcttgt ctgctcccgg catccgctta 4440
   cagacaagct gtgaccgtct ccgggagctg catgtgtcag aggttttcac cgtcatcacc 4500
   gaaacgcgcg aggcagctgg cacgacaggt ttcccgactg ggttggtgcg ttttatcatg 4560
   cctggcgggt aggtcggata aggcgttcac gccgcatccg acaaccacgc agcgttacct 4620
   gatgtgacgc cgacaattct catcatcgct acaacatgac ctcgctattt acatcgcgat 4680
   actcttttgg cgtcgtgtca tatgcttttt taaaaacaga gtagaaatat tgcagcgatg 4740
   gataaccgca catttgcgat atctcattga tcgacaaggt ggttgaaatc agcagactgc 4800
   gcgctttctc cagcttctcg gcatgaatca tggcatggat ggtttcaccc acctcttctt 4860
   taaaacgctt ctcaagattg gagcgcgaga tcccgaccgc atccagtacc tgatccactt 4920
   taatcccttt acaggcgtga ttacgaatgt aatgcatggc ctgaataacg gcgggatcgg 4980
   tcagcgagcg ataatctgtt gagcgccgtt caatgacgcg aactggtggg accaaaattc 5040
   gctgtagcgg catttcttct ttatctaata atcgatgcaa cagttttgcc gcctgatagc 5100
   ccatttgccg cgcgccctga gcgaccgaag aaagggcgac acgcgacaga tagcgggtca     5160
   gttcttcgtt atcgatgcca atcacgcata atttttccgg tacgggaata tgtagatgtt     5220
   cacatacttg cagaatatgc cgcgctcggg cgtcagtaac ggcaataatc ccggtttgcg     5280
   gtggtagcgt ttgtagccag tctgccagcc gattttgcgc gtgttgccag ttctctggcg     5340
   cggtttctaa cccctgataa accactccgc gatacttttc ttcggcgaca agctgacgaa     5400
   atgcatattc gcgctcagtg gcccaacgtt tgccgcttga ttccggaaga ccataaaaag     5460
   caaagcggtt aacgcctttc tcttttaaat gcaaaaatgc gctttcaacc agcgcatagt     5520
   tatcggtggc aatgtaatga acgggtgggt aactttctgc aaggtgatac gagccgccaa     5580
   ccccaacaat ggggacgtcg acatcagcca gcgcttgctc gatctgtttg tcgtcgaagt     5640
   cggcaatgac gccatctcct aaccagtcct tgattttatc aatgcgggcg cggaaatctt     5700
   cttcaatgaa aatatcccat tccgattgtg acgcctgtaa atattcccct acgccttcta     5760
   ctacctgccg gtcataggct ttattggcat tgaacagtaa tgtgatgcgg tgacgtttag     5820
   taaacatggt tcttttcctg ctgaatcatg ctaagtaaca atcaccgcga taaacgtaac     5880
   caatttttag caactaaaca ggggaaaaca attacagatt tttatctttc gattacgatt     5940
   tttggtttat ttcttgattt atgaccgaga tcttactttt gttgcgcaat tgtacttatt     6000
   gcatttttct cttcgaggaa tt                                              6022
<210> 39
   <211> 4506
   <212> DNA
   <213> Artifizielle Sequenz
<220>
   <223> Plasmid pGT1557
<400> 39
   aaagcgaatt gtgattctag aaaaaaactt cttgacatgt ttggtataat aagagggtaa      60
   taattttgtt taactttaag aaggagatat acccatgaca cagagggccc accatcacca     120
   tcaccattcc atgggtatgc agggtgaaga tgcacagcgt accggtaata ttgttgcagt     180
   taaaccgaaa ccgagccagg gtctgaccag caaagcaatt gattggctgg aatggctgtt     240
   tgtgaaaatg atgcatgata gcaaacagcc gctgcattat ctgagcggta attttgcacc     300
   ggttgatgaa acccctccgc tgaaagatct gccggttacc ggtcatctgc cggaatgtct     360
   gaatggtgaa tttgttcgtg ttggtccgaa tccgaaattt gcaagcattg caggttatca     420
   ttggtttgat ggtgatggta tgattcatgg catgcgcatt aaagatggta aagcaaccta     480
   tgttagccgt tatgttcaga ccagccgtct gaaacaagag gaattctttg gtcgtgccat     540
   gttcatgaaa atcggtgatc tgaaaggtat gtttggtctg ctgatggtta atatgcagat     600
   gctgcgtgca aaactgaaag ttctggatat tagctatggt attggcaccg caaataccgc      660
   actggtttat catcatggta aactgctggc actgagcgaa gcagataaac cgtatgcaat      720
   taaagtgctg gaagatggtg atctgcagac cattggcctg ctggattatg ataaacgtct      780
   ggcacatagc tttaccgcac atccgaaagt tgatccgttt accggtgaga tgtttacctt      840
   tggttatagc catacccctc cgtatgttac ctatcgtgtt attagcaaag atggtgcaat      900
   gaatgatccg gttccgatta ccgttagcgg tccgatcatg atgcacgatt ttgcaattac      960
   cgaaaactac gccatcttta tggatctgcc gctgtatttc aaaccgaaag aaatggtgaa     1020
   agacaagaaa ttcatcttta gcttcgatgc cacccagaaa gcacgttttg gtattctgcc     1080
   tcgttatgcc aagaatgagc tgctgattaa atggtttgaa ctgccgaact gcttcatctt     1140
   tcataatgca aatgcatggg aagagggtga tgaagttgtt ctgattacct gtcgtctgga     1200
   aaatccggat ctggatatgg tgaatagcac cgttaaagaa cgtctggaca actttaagaa     1260
   cgagctgtat gaaatgcgct tcaatctgca gaatggtctg gcaagccaga aaaaactgag     1320
   cgttagcgca gttgattttc cgcgtgttaa tgaaagctat accacccgta aacagcgtta     1380
   tgtttatggc accaccctgg ataagattgc caaagttacc ggcatcatca aattcgatct     1440
   gcatgccgaa ccggaaaccg gtaaagagaa gctggaactg ggtggtaatg tgaaaggcat     1500
   ttttgatctg ggtccgggtc gttttggttc agaagcagtt tttgttccgc gtcatccggg     1560
   tattaccagc gaagaggatg atggttatct gatcttcttt gtgcacgatg aaaacaccgg     1620
   caaaagcgca gttaatgtta ttgatgcaaa aaccatgagc cctgatccgg tggcagttgt     1680
   ggaactgcct aaacgtgttc cgtatggttt tcatgcattt tttgttaccg aagatcagct     1740
   gcaagaacag gccaaagttt aaggatccgg ctgctaacaa agcccgaaag gaagctgagt     1800
   tggctgctgc caccgctgag caataactag cataacccct tggggcctct aaacgggtct     1860
   tgaggggttt tttgctgaaa ggaggaacta tatccggccg gatatccaca ggacgggtgt     1920
   ggtcgccatg atcgcgtagt cgatagtggc tccaagtagc gaagcgagca ggactgggcg     1980
   gcggccaaag cggtcggaca gtgctccgag aacgggtgcg catagaaatt gcatcaacgc     2040
   atatagcgct agcagcacgc catagtgact ggcgatgctg tcggaatgga cgatatcccg     2100
   caagaggccc ggcagtaccg gcataaccaa gcctatgcct acagcatcca gggtgacggt     2160
   gccgaggatg acgatgagcg cattgttaga tttcatacac ggtgcctgac tgcgttagca     2220
   atttaactgt gataaactac cgcattaaag cttatcgatg ataagctgtc aaacatgaga     2280
   attcttgaag acgaaagggc ctcgtgatac gcctattttt ataggttaat gtcatgcatg     2340
   agacaataac cctgataaat gcttcaataa tattgaaaaa ggaagagtat gagtattcaa     2400
   catttccgtg tcgcccttat tccctttttt gcggcatttt gccttcctgt ttttgctcac     2460
   ccagaaacgc tggtgaaagt aaaagatgct gaagatcagt tgggtgcacg agtgggttac     2520
   atcgaactgg atctcaacag cggtaagatc cttgagagtt ttcgccccga agaacgtttt 2580
   ccaatgatga gcacttttaa agttctgcta tgtggcgcgg tattatcccg tgttgacgcc 2640
   gggcaagagc aactcggtcg ccgcatacac tattctcaga atgacttggt tgacgcgtca 2700
   ccagtcacag aaaagcatct tacggatggc atgacagtaa gagaattatg cagtgctgcc 2760
   ataaccatga gtgataacac tgcggccaac ttacttctga caacgatcgg aggaccgaag 2820
   gagctaaccg cttttttgca caacatgggg gatcatgtaa ctcgccttga tcgttgggaa 2880
   ccggagctga atgaagccat accaaacgac gagcgtgaca ccacgatgcc tgcagcaatg 2940
   gcaacaacgt tgcgcaaact attaactggc gaactactta ctctagcttc ccggcaacaa 3000
   ttaatagact ggatggaggc ggataaagtt gcaggaccac ttctgcgctc ggcccttccg 3060
   gctggctggt ttattgctga taaatctgga gccggtgagc gtgggtctcg cggtatcatt 3120
   gcagcactgg ggccagatgg taagccctcc cgtatcgtag ttatctacac gacggggagt 3180
   caggcaacta tggatgaacg aaatagacag atcgctgaga taggtgcctc actgattaag 3240
   cattggtaac tgtcagacca agtttactca tatatacttt agattgattt aaaacttcat 3300
   ttttaattta aaaggatcta ggtgaagatc ctttttgata atctcatgca tgaccaaaat 3360
   cccttaacgt gagttttcgt tccactgagc gtcagacccc gtagaaaaga tcaaaggatc 3420
   ttcttgagat cctttttttc tgcgcgtaat ctgctgcttg caaacaaaaa aaccaccgct 3480
   accagcggtg gtttgtttgc cggatcaaga gctaccaact ctttttccga aggtaactgg 3540
   cttcagcaga gcgcagatac caaatactgt ccttctagtg tagccgtagt taggccacca 3600
   cttcaagaac tctgtagcac cgcctacata cctcgctctg ctaatcctgt taccagtggc 3660
   tgctgccagt ggcgataagt cgtgtcttac cgggttggac tcaagacgat agttaccgga 3720
   taaggcgcag cggtcgggct gaacgggggg ttcgtgcaca cagcccagct tggagcgaac 3780
   gacctacacc gaactgagat acctacagcg tgagctatga gaaagcgcca cgcttcccga 3840
   agggagaaag gcggacaggt atccggtaag cggcagggtc ggaacaggag agcgcacgag 3900
   ggagcttcca gggggaaacg cctggtatct ttatagtcct gtcgggtttc gccacctctg 3960
   acttgagcgt cgatttttgt gatgctcgtc aggggggcgg agcctatgga aaaacgccag 4020
   caacgcggcc tttttacggt tcctggcctt ttgctggcct tttgctcaca tgttctttcc 4080
   tgcgttatcc cctgattctg tggataaccg tattaccgcc tttgagtgag ctgataccgc 4140
   tcgccgcagc cgaacgaccg agcgcagcga gtcagtgagc gaggaagcgg aagagcgcct 4200
   gatgcggtat tttctcctta cgcatctgtg cggtatttca caccgcatat atggtgcact 4260
   ctcagtacaa tctgctctga tgccgcatag ttaagccagt atacactccg ctatcgctac 4320
   gtgactgggt catggctgcg ccccgacacc cgccaacacc cgctgacgcg ccctgacggg 4380
   cttgtctgct cccggcatcc gcttacagac aagctgtgac cgtctccggg agctgcatgt    4440
   gtcagaggtt ttcaccgtca tcaccgaaac gcgcgaggca gctggcacga caggtttccc    4500
   gactgg                                                               4506
<210> 40
   <211> 4507
   <212> DNA
   <213> Artifizielle Sequenz
<220>
   <223> Plasmid pGT1560
<400> 40
   aaagcgaatt gtgattgaaa tcaaaatttt cttgactaat tatggtataa tttactggta      60
   ataattttgt ttaactttaa gaaggagata tacccatgac acagagggcc caccatcacc     120
   atcaccattc catgggtatg cagggtgaag atgcacagcg taccggtaat attgttgcag     180
   ttaaaccgaa accgagccag ggtctgacca gcaaagcaat tgattggctg gaatggctgt     240
   ttgtgaaaat gatgcatgat agcaaacagc cgctgcatta tctgagcggt aattttgcac     300
   cggttgatga aacccctccg ctgaaagatc tgccggttac cggtcatctg ccggaatgtc     360
   tgaatggtga atttgttcgt gttggtccga atccgaaatt tgcaagcatt gcaggttatc     420
   attggtttga tggtgatggt atgattcatg gcatgcgcat taaagatggt aaagcaacct     480
   atgttagccg ttatgttcag accagccgtc tgaaacaaga ggaattcttt ggtcgtgcca     540
   tgttcatgaa aatcggtgat ctgaaaggta tgtttggtct gctgatggtt aatatgcaga     600
   tgctgcgtgc aaaactgaaa gttctggata ttagctatgg tattggcacc gcaaataccg     660
   cactggttta tcatcatggt aaactgctgg cactgagcga agcagataaa ccgtatgcaa     720
   ttaaagtgct ggaagatggt gatctgcaga ccattggcct gctggattat gataaacgtc     780
   tggcacatag ctttaccgca catccgaaag ttgatccgtt taccggtgag atgtttacct     840
   ttggttatag ccatacccct ccgtatgtta cctatcgtgt tattagcaaa gatggtgcaa     900
   tgaatgatcc ggttccgatt accgttagcg gtccgatcat gatgcacgat tttgcaatta     960
   ccgaaaacta cgccatcttt atggatctgc cgctgtattt caaaccgaaa gaaatggtga    1020
   aagacaagaa attcatcttt agcttcgatg ccacccagaa agcacgtttt ggtattctgc    1080
   ctcgttatgc caagaatgag ctgctgatta aatggtttga actgccgaac tgcttcatct    1140
   ttcataatgc aaatgcatgg gaagagggtg atgaagttgt tctgattacc tgtcgtctgg    1200
   aaaatccgga tctggatatg gtgaatagca ccgttaaaga acgtctggac aactttaaga    1260
   acgagctgta tgaaatgcgc ttcaatctgc agaatggtct ggcaagccag aaaaaactga    1320
   gcgttagcgc agttgatttt ccgcgtgtta atgaaagcta taccacccgt aaacagcgtt    1380
   atgtttatgg caccaccctg gataagattg ccaaagttac cggcatcatc aaattcgatc    1440
   tgcatgccga accggaaacc ggtaaagaga agctggaact gggtggtaat gtgaaaggca 1500
   tttttgatct gggtccgggt cgttttggtt cagaagcagt ttttgttccg cgtcatccgg 1560
   gtattaccag cgaagaggat gatggttatc tgatcttctt tgtgcacgat gaaaacaccg 1620
   gcaaaagcgc agttaatgtt attgatgcaa aaaccatgag ccctgatccg gtggcagttg 1680
   tggaactgcc taaacgtgtt ccgtatggtt ttcatgcatt ttttgttacc gaagatcagc 1740
   tgcaagaaca ggccaaagtt taaggatccg gctgctaaca aagcccgaaa ggaagctgag 1800
   ttggctgctg ccaccgctga gcaataacta gcataacccc ttggggcctc taaacgggtc 1860
   ttgaggggtt ttttgctgaa aggaggaact atatccggcc ggatatccac aggacgggtg 1920
   tggtcgccat gatcgcgtag tcgatagtgg ctccaagtag cgaagcgagc aggactgggc 1980
   ggcggccaaa gcggtcggac agtgctccga gaacgggtgc gcatagaaat tgcatcaacg 2040
   catatagcgc tagcagcacg ccatagtgac tggcgatgct gtcggaatgg acgatatccc 2100
   gcaagaggcc cggcagtacc ggcataacca agcctatgcc tacagcatcc agggtgacgg 2160
   tgccgaggat gacgatgagc gcattgttag atttcataca cggtgcctga ctgcgttagc 2220
   aatttaactg tgataaacta ccgcattaaa gcttatcgat gataagctgt caaacatgag 2280
   aattcttgaa gacgaaaggg cctcgtgata cgcctatttt tataggttaa tgtcatgcat 2340
   gagacaataa ccctgataaa tgcttcaata atattgaaaa aggaagagta tgagtattca 2400
   acatttccgt gtcgccctta ttcccttttt tgcggcattt tgccttcctg tttttgctca 2460
   cccagaaacg ctggtgaaag taaaagatgc tgaagatcag ttgggtgcac gagtgggtta 2520
   catcgaactg gatctcaaca gcggtaagat ccttgagagt tttcgccccg aagaacgttt 2580
   tccaatgatg agcactttta aagttctgct atgtggcgcg gtattatccc gtgttgacgc 2640
   cgggcaagag caactcggtc gccgcataca ctattctcag aatgacttgg ttgacgcgtc 2700
   accagtcaca gaaaagcatc ttacggatgg catgacagta agagaattat gcagtgctgc 2760
   cataaccatg agtgataaca ctgcggccaa cttacttctg acaacgatcg gaggaccgaa 2820
   ggagctaacc gcttttttgc acaacatggg ggatcatgta actcgccttg atcgttggga 2880
   accggagctg aatgaagcca taccaaacga cgagcgtgac accacgatgc ctgcagcaat 2940
   ggcaacaacg ttgcgcaaac tattaactgg cgaactactt actctagctt cccggcaaca 3000
   attaatagac tggatggagg cggataaagt tgcaggacca cttctgcgct cggcccttcc 3060
   ggctggctgg tttattgctg ataaatctgg agccggtgag cgtgggtctc gcggtatcat 3120
   tgcagcactg gggccagatg gtaagccctc ccgtatcgta gttatctaca cgacggggag 3180
   tcaggcaact atggatgaac gaaatagaca gatcgctgag ataggtgcct cactgattaa 3240
   gcattggtaa ctgtcagacc aagtttactc atatatactt tagattgatt taaaacttca 3300
   tttttaattt aaaaggatct aggtgaagat cctttttgat aatctcatgc atgaccaaaa 3360
   tcccttaacg tgagttttcg ttccactgag cgtcagaccc cgtagaaaag atcaaaggat 3420
   cttcttgaga tccttttttt ctgcgcgtaa tctgctgctt gcaaacaaaa aaaccaccgc 3480
   taccagcggt ggtttgtttg ccggatcaag agctaccaac tctttttccg aaggtaactg 3540
   gcttcagcag agcgcagata ccaaatactg tccttctagt gtagccgtag ttaggccacc 3600
   acttcaagaa ctctgtagca ccgcctacat acctcgctct gctaatcctg ttaccagtgg 3660
   ctgctgccag tggcgataag tcgtgtctta ccgggttgga ctcaagacga tagttaccgg 3720
   ataaggcgca gcggtcgggc tgaacggggg gttcgtgcac acagcccagc ttggagcgaa 3780
   cgacctacac cgaactgaga tacctacagc gtgagctatg agaaagcgcc acgcttcccg 3840
   aagggagaaa ggcggacagg tatccggtaa gcggcagggt cggaacagga gagcgcacga 3900
   gggagcttcc agggggaaac gcctggtatc tttatagtcc tgtcgggttt cgccacctct 3960
   gacttgagcg tcgatttttg tgatgctcgt caggggggcg gagcctatgg aaaaacgcca 4020
   gcaacgcggc ctttttacgg ttcctggcct tttgctggcc ttttgctcac atgttctttc 4080
   ctgcgttatc ccctgattct gtggataacc gtattaccgc ctttgagtga gctgataccg 4140
   ctcgccgcag ccgaacgacc gagcgcagcg agtcagtgag cgaggaagcg gaagagcgcc 4200
   tgatgcggta ttttctcctt acgcatctgt gcggtatttc acaccgcata tatggtgcac 4260
   tctcagtaca atctgctctg atgccgcata gttaagccag tatacactcc gctatcgcta 4320
   cgtgactggg tcatggctgc gccccgacac ccgccaacac ccgctgacgc gccctgacgg 4380
   gcttgtctgc tcccggcatc cgcttacaga caagctgtga ccgtctccgg gagctgcatg 4440
   tgtcagaggt tttcaccgtc atcaccgaaa cgcgcgaggc agctggcacg acaggtttcc 4500
   cgactgg 4507
<210> 41
   <211> 6037
   <212> DNA
   <213> Artifizielle Sequenz
<220>
   <223> Plasmid pGT1584
<400> 41
   acccagtttc atcattccat tttattttgc gagcgagcgc acacttgtga attatctcaa        60
   tagcagtgtg aaataacata attgagcaac tgaaagggag tgcccaatat tacgacatca       120
   tccatcacgc ggagttacct gattatgaca cagagggccc accatcacca tcaccattcc       180
   atgggtatgc agggtgaaga tgcacagcgt accggtaata ttgttgcagt taaaccgaaa       240
   ccgagccagg gtctgaccag caaagcaatt gattggctgg aatggctgtt tgtgaaaatg       300
   atgcatgata gcaaacagcc gctgcattat ctgagcggta attttgcacc ggttgatgaa       360
   acccctccgc tgaaagatct gccggttacc ggtcatctgc cggaatgtct gaatggtgaa 420
   tttgttcgtg ttggtccgaa tccgaaattt gcaagcattg caggttatca ttggtttgat 480
   ggtgatggta tgattcatgg catgcgcatt aaagatggta aagcaaccta tgttagccgt 540
   tatgttcaga ccagccgtct gaaacaagag gaattctttg gtcgtgccat gttcatgaaa 600
   atcggtgatc tgaaaggtat gtttggtctg ctgatggtta atatgcagat gctgcgtgca 660
   aaactgaaag ttctggatat tagctatggt attggcaccg caaataccgc actggtttat 720
   catcatggta aactgctggc actgagcgaa gcagataaac cgtatgcaat taaagtgctg 780
   gaagatggtg atctgcagac cattggcctg ctggattatg ataaacgtct ggcacatagc 840
   tttaccgcac atccgaaagt tgatccgttt accggtgaga tgtttacctt tggttatagc 900
   catacccctc cgtatgttac ctatcgtgtt attagcaaag atggtgcaat gaatgatccg 960
   gttccgatta ccgttagcgg tccgatcatg atgcacgatt ttgcaattac cgaaaactac 1020
   gccatcttta tggatctgcc gctgtatttc aaaccgaaag aaatggtgaa agacaagaaa 1080
   ttcatcttta gcttcgatgc cacccagaaa gcacgttttg gtattctgcc tcgttatgcc 1140
   aagaatgagc tgctgattaa atggtttgaa ctgccgaact gcttcatctt tcataatgca 1200
   aatgcatggg aagagggtga tgaagttgtt ctgattacct gtcgtctgga aaatccggat 1260
   ctggatatgg tgaatagcac cgttaaagaa cgtctggaca actttaagaa cgagctgtat 1320
   gaaatgcgct tcaatctgca gaatggtctg gcaagccaga aaaaactgag cgttagcgca 1380
   gttgattttc cgcgtgttaa tgaaagctat accacccgta aacagcgtta tgtttatggc 1440
   accaccctgg ataagattgc caaagttacc ggcatcatca aattcgatct gcatgccgaa 1500
   ccggaaaccg gtaaagagaa gctggaactg ggtggtaatg tgaaaggcat ttttgatctg 1560
   ggtccgggtc gttttggttc agaagcagtt tttgttccgc gtcatccggg tattaccagc 1620
   gaagaggatg atggttatct gatcttcttt gtgcacgatg aaaacaccgg caaaagcgca 1680
   gttaatgtta ttgatgcaaa aaccatgagc cctgatccgg tggcagttgt ggaactgcct 1740
   aaacgtgttc cgtatggttt tcatgcattt tttgttaccg aagatcagct gcaagaacag 1800
   gccaaagttt aaggatccgg ctgctaacaa agcccgaaag gaagctgagt tggctgctgc 1860
   caccgctgag caataactag cataacccct tggggcctct aaacgggtct tgaggggttt 1920
   tttgctgaaa ggaggaacta tatccggccg gatatccaca ggacgggtgt ggtcgccatg 1980
   atcgcgtagt cgatagtggc tccaagtagc gaagcgagca ggactgggcg gcggccaaag 2040
   cggtcggaca gtgctccgag aacgggtgcg catagaaatt gcatcaacgc atatagcgct 2100
   agcagcacgc catagtgact ggcgatgctg tcggaatgga cgatatcccg caagaggccc 2160
   ggcagtaccg gcataaccaa gcctatgcct acagcatcca gggtgacggt gccgaggatg 2220
   acgatgagcg cattgttaga tttcatacac ggtgcctgac tgcgttagca atttaactgt 2280
   gataaactac cgcattaaag cttatcgatg ataagctgtc aaacatgaga attcttgaag 2340
   acgaaagggc ctcgtgatac gcctattttt ataggttaat gtcatgcatg agacaataac 2400
   cctgataaat gcttcaataa tattgaaaaa ggaagagtat gagtattcaa catttccgtg 2460
   tcgcccttat tccctttttt gcggcatttt gccttcctgt ttttgctcac ccagaaacgc 2520
   tggtgaaagt aaaagatgct gaagatcagt tgggtgcacg agtgggttac atcgaactgg 2580
   atctcaacag cggtaagatc cttgagagtt ttcgccccga agaacgtttt ccaatgatga 2640
   gcacttttaa agttctgcta tgtggcgcgg tattatcccg tgttgacgcc gggcaagagc 2700
   aactcggtcg ccgcatacac tattctcaga atgacttggt tgacgcgtca ccagtcacag 2760
   aaaagcatct tacggatggc atgacagtaa gagaattatg cagtgctgcc ataaccatga 2820
   gtgataacac tgcggccaac ttacttctga caacgatcgg aggaccgaag gagctaaccg 2880
   cttttttgca caacatgggg gatcatgtaa ctcgccttga tcgttgggaa ccggagctga 2940
   atgaagccat accaaacgac gagcgtgaca ccacgatgcc tgcagcaatg gcaacaacgt 3000
   tgcgcaaact attaactggc gaactactta ctctagcttc ccggcaacaa ttaatagact 3060
   ggatggaggc ggataaagtt gcaggaccac ttctgcgctc ggcccttccg gctggctggt 3120
   ttattgctga taaatctgga gccggtgagc gtgggtctcg cggtatcatt gcagcactgg 3180
   ggccagatgg taagccctcc cgtatcgtag ttatctacac gacggggagt caggcaacta 3240
   tggatgaacg aaatagacag atcgctgaga taggtgcctc actgattaag cattggtaac 3300
   tgtcagacca agtttactca tatatacttt agattgattt aaaacttcat ttttaattta 3360
   aaaggatcta ggtgaagatc ctttttgata atctcatgca tgaccaaaat cccttaacgt 3420
   gagttttcgt tccactgagc gtcagacccc gtagaaaaga tcaaaggatc ttcttgagat 3480
   cctttttttc tgcgcgtaat ctgctgcttg caaacaaaaa aaccaccgct accagcggtg 3540
   gtttgtttgc cggatcaaga gctaccaact ctttttccga aggtaactgg cttcagcaga 3600
   gcgcagatac caaatactgt ccttctagtg tagccgtagt taggccacca cttcaagaac 3660
   tctgtagcac cgcctacata cctcgctctg ctaatcctgt taccagtggc tgctgccagt 3720
   ggcgataagt cgtgtcttac cgggttggac tcaagacgat agttaccgga taaggcgcag 3780
   cggtcgggct gaacgggggg ttcgtgcaca cagcccagct tggagcgaac gacctacacc 3840
   gaactgagat acctacagcg tgagctatga gaaagcgcca cgcttcccga agggagaaag 3900
   gcggacaggt atccggtaag cggcagggtc ggaacaggag agcgcacgag ggagcttcca 3960
   gggggaaacg cctggtatct ttatagtcct gtcgggtttc gccacctctg acttgagcgt 4020
   cgatttttgt gatgctcgtc aggggggcgg agcctatgga aaaacgccag caacgcggcc 4080
   tttttacggt tcctggcctt ttgctggcct tttgctcaca tgttctttcc tgcgttatcc 4140
   cctgattctg tggataaccg tattaccgcc tttgagtgag ctgataccgc tcgccgcagc 4200
   cgaacgaccg agcgcagcga gtcagtgagc gaggaagcgg aagagcgcct gatgcggtat 4260
   tttctcctta cgcatctgtg cggtatttca caccgcatat atggtgcact ctcagtacaa 4320
   tctgctctga tgccgcatag ttaagccagt atacactccg ctatcgctac gtgactgggt 4380
   catggctgcg ccccgacacc cgccaacacc cgctgacgcg ccctgacggg cttgtctgct 4440
   cccggcatcc gcttacagac aagctgtgac cgtctccggg agctgcatgt gtcagaggtt 4500
   ttcaccgtca tcaccgaaac gcgcgaggca gctggcacga caggtttccc gactgggttg 4560
   gtgcgtttta tcatgcctgg cgggtaggtc ggataaggcg ttcacgccgc atccgacaac 4620
   cacgcagcgt tacctgatgt gacgccgaca attctcatca tcgctacaac atgacctcgc 4680
   tatttacatc gcgatactct tttggcgtcg tgtcatatgc ttttttaaaa acagagtaga 4740
   aatattgcag cgatggataa ccgcacattt gcgatatctc attgatcgac aaggtggttg 4800
   aaatcagcag actgcgcgct ttctccagct tctcggcatg aatcatggca tggatggttt 4860
   cacccacctc ttctttaaaa cgcttctcaa gattggagcg cgagatcccg accgcatcca 4920
   gtacctgatc cactttaatc cctttacagg cgtgattacg aatgtaatgc atggcctgaa 4980
   taacggcggg atcggtcagc gagcgataat ctgttgagcg ccgttcaatg acgcgaactg 5040
   gtgggaccaa aattcgctgt agcggcattt cttctttatc taataatcga tgcaacagtt 5100
   ttgccgcctg atagcccatt tgccgcgcgc cctgagcgac cgaagaaagg gcgacacgcg 5160
   acagatagcg ggtcagttct tcgttatcga tgccaatcac gcataatttt tccggtacgg 5220
   gaatatgtag atgttcacat acttgcagaa tatgccgcgc tcgggcgtca gtaacggcaa 5280
   taatcccggt ttgcggtggt agcgtttgta gccagtctgc cagccgattt tgcgcgtgtt 5340
   gccagttctc tggcgcggtt tctaacccct gataaaccac tccgcgatac ttttcttcgg 5400
   cgacaagctg acgaaatgca tattcgcgct cagtggccca acgtttgccg cttgattccg 5460
   gaagaccata aaaagcaaag cggttaacgc ctttctcttt taaatgcaaa aatgcgcttt 5520
   caaccagcgc atagttatcg gtggcaatgt aatgaacggg tgggtaactt tctgcaaggt 5580
   gatacgagcc gccaacccca acaatgggga cgtcgacatc agccagcgct tgctcgatct 5640
   gtttgtcgtc gaagtcggca atgacgccat ctcctaacca gtccttgatt ttatcaatgc 5700
   gggcgcggaa atcttcttca atgaaaatat cccattccga ttgtgacgcc tgtaaatatt 5760
   cccctacgcc ttctactacc tgccggtcat aggctttatt ggcattgaac agtaatgtga 5820
   tgcggtgacg tttagtaaac atggttcttt tcctgctgaa tcatgctaag taacaatcac 5880
   cgcgataaac gtaaccaatt tttagcaact aaacagggga aaacaattac agatttttat 5940
   ctttcgatta cgatttttgg tttatttctt gatttatgac cgagatctta cttttgttgc 6000
   gcaattgtac ttattgcatt tttctcttcg aggaatt 6037
<210> 42
   <211> 6037
   <212> DNA
   <213> Artifizielle Sequenz
<220>
   <223> Plasmid pGT1586
<400> 42
   acccagtttc atcattccat tttattttgc gagcgagcgc acacttgtga attatctcaa 60
   tagcagtgtg aaataacata attgagcaac tgaaagggag tgccctatat tacgacatca 120
   tccatcacgc ggagttacct gattatgaca cagagggccc accatcacca tcaccattcc 180
   atgggtatgc agggtgaaga tgcacagcgt accggtaata ttgttgcagt taaaccgaaa 240
   ccgagccagg gtctgaccag caaagcaatt gattggctgg aatggctgtt tgtgaaaatg 300
   atgcatgata gcaaacagcc gctgcattat ctgagcggta attttgcacc ggttgatgaa 360
   acccctccgc tgaaagatct gccggttacc ggtcatctgc cggaatgtct gaatggtgaa 420
   tttgttcgtg ttggtccgaa tccgaaattt gcaagcattg caggttatca ttggtttgat 480
   ggtgatggta tgattcatgg catgcgcatt aaagatggta aagcaaccta tgttagccgt 540
   tatgttcaga ccagccgtct gaaacaagag gaattctttg gtcgtgccat gttcatgaaa 600
   atcggtgatc tgaaaggtat gtttggtctg ctgatggtta atatgcagat gctgcgtgca 660
   aaactgaaag ttctggatat tagctatggt attggcaccg caaataccgc actggtttat 720
   catcatggta aactgctggc actgagcgaa gcagataaac cgtatgcaat taaagtgctg 780
   gaagatggtg atctgcagac cattggcctg ctggattatg ataaacgtct ggcacatagc 840
   tttaccgcac atccgaaagt tgatccgttt accggtgaga tgtttacctt tggttatagc 900
   catacccctc cgtatgttac ctatcgtgtt attagcaaag atggtgcaat gaatgatccg 960
   gttccgatta ccgttagcgg tccgatcatg atgcacgatt ttgcaattac cgaaaactac 1020
   gccatcttta tggatctgcc gctgtatttc aaaccgaaag aaatggtgaa agacaagaaa 1080
   ttcatcttta gcttcgatgc cacccagaaa gcacgttttg gtattctgcc tcgttatgcc 1140
   aagaatgagc tgctgattaa atggtttgaa ctgccgaact gcttcatctt tcataatgca 1200
   aatgcatggg aagagggtga tgaagttgtt ctgattacct gtcgtctgga aaatccggat 1260
   ctggatatgg tgaatagcac cgttaaagaa cgtctggaca actttaagaa cgagctgtat 1320
   gaaatgcgct tcaatctgca gaatggtctg gcaagccaga aaaaactgag cgttagcgca 1380
   gttgattttc cgcgtgttaa tgaaagctat accacccgta aacagcgtta tgtttatggc 1440
   accaccctgg ataagattgc caaagttacc ggcatcatca aattcgatct gcatgccgaa 1500
   ccggaaaccg gtaaagagaa gctggaactg ggtggtaatg tgaaaggcat ttttgatctg 1560
   ggtccgggtc gttttggttc agaagcagtt tttgttccgc gtcatccggg tattaccagc 1620
   gaagaggatg atggttatct gatcttcttt gtgcacgatg aaaacaccgg caaaagcgca 1680
   gttaatgtta ttgatgcaaa aaccatgagc cctgatccgg tggcagttgt ggaactgcct 1740
   aaacgtgttc cgtatggttt tcatgcattt tttgttaccg aagatcagct gcaagaacag 1800
   gccaaagttt aaggatccgg ctgctaacaa agcccgaaag gaagctgagt tggctgctgc 1860
   caccgctgag caataactag cataacccct tggggcctct aaacgggtct tgaggggttt 1920
   tttgctgaaa ggaggaacta tatccggccg gatatccaca ggacgggtgt ggtcgccatg 1980
   atcgcgtagt cgatagtggc tccaagtagc gaagcgagca ggactgggcg gcggccaaag 2040
   cggtcggaca gtgctccgag aacgggtgcg catagaaatt gcatcaacgc atatagcgct 2100
   agcagcacgc catagtgact ggcgatgctg tcggaatgga cgatatcccg caagaggccc 2160
   ggcagtaccg gcataaccaa gcctatgcct acagcatcca gggtgacggt gccgaggatg 2220
   acgatgagcg cattgttaga tttcatacac ggtgcctgac tgcgttagca atttaactgt 2280
   gataaactac cgcattaaag cttatcgatg ataagctgtc aaacatgaga attcttgaag 2340
   acgaaagggc ctcgtgatac gcctattttt ataggttaat gtcatgcatg agacaataac 2400
   cctgataaat gcttcaataa tattgaaaaa ggaagagtat gagtattcaa catttccgtg 2460
   tcgcccttat tccctttttt gcggcatttt gccttcctgt ttttgctcac ccagaaacgc 2520
   tggtgaaagt aaaagatgct gaagatcagt tgggtgcacg agtgggttac atcgaactgg 2580
   atctcaacag cggtaagatc cttgagagtt ttcgccccga agaacgtttt ccaatgatga 2640
   gcacttttaa agttctgcta tgtggcgcgg tattatcccg tgttgacgcc gggcaagagc 2700
   aactcggtcg ccgcatacac tattctcaga atgacttggt tgacgcgtca ccagtcacag 2760
   aaaagcatct tacggatggc atgacagtaa gagaattatg cagtgctgcc ataaccatga 2820
   gtgataacac tgcggccaac ttacttctga caacgatcgg aggaccgaag gagctaaccg 2880
   cttttttgca caacatgggg gatcatgtaa ctcgccttga tcgttgggaa ccggagctga 2940
   atgaagccat accaaacgac gagcgtgaca ccacgatgcc tgcagcaatg gcaacaacgt 3000
   tgcgcaaact attaactggc gaactactta ctctagcttc ccggcaacaa ttaatagact 3060
   ggatggaggc ggataaagtt gcaggaccac ttctgcgctc ggcccttccg gctggctggt 3120
   ttattgctga taaatctgga gccggtgagc gtgggtctcg cggtatcatt gcagcactgg 3180
   ggccagatgg taagccctcc cgtatcgtag ttatctacac gacggggagt caggcaacta 3240
   tggatgaacg aaatagacag atcgctgaga taggtgcctc actgattaag cattggtaac 3300
   tgtcagacca agtttactca tatatacttt agattgattt aaaacttcat ttttaattta 3360
   aaaggatcta ggtgaagatc ctttttgata atctcatgca tgaccaaaat cccttaacgt 3420
   gagttttcgt tccactgagc gtcagacccc gtagaaaaga tcaaaggatc ttcttgagat 3480
   cctttttttc tgcgcgtaat ctgctgcttg caaacaaaaa aaccaccgct accagcggtg 3540
   gtttgtttgc cggatcaaga gctaccaact ctttttccga aggtaactgg cttcagcaga 3600
   gcgcagatac caaatactgt ccttctagtg tagccgtagt taggccacca cttcaagaac 3660
   tctgtagcac cgcctacata cctcgctctg ctaatcctgt taccagtggc tgctgccagt 3720
   ggcgataagt cgtgtcttac cgggttggac tcaagacgat agttaccgga taaggcgcag 3780
   cggtcgggct gaacgggggg ttcgtgcaca cagcccagct tggagcgaac gacctacacc 3840
   gaactgagat acctacagcg tgagctatga gaaagcgcca cgcttcccga agggagaaag 3900
   gcggacaggt atccggtaag cggcagggtc ggaacaggag agcgcacgag ggagcttcca 3960
   gggggaaacg cctggtatct ttatagtcct gtcgggtttc gccacctctg acttgagcgt 4020
   cgatttttgt gatgctcgtc aggggggcgg agcctatgga aaaacgccag caacgcggcc 4080
   tttttacggt tcctggcctt ttgctggcct tttgctcaca tgttctttcc tgcgttatcc 4140
   cctgattctg tggataaccg tattaccgcc tttgagtgag ctgataccgc tcgccgcagc 4200
   cgaacgaccg agcgcagcga gtcagtgagc gaggaagcgg aagagcgcct gatgcggtat 4260
   tttctcctta cgcatctgtg cggtatttca caccgcatat atggtgcact ctcagtacaa 4320
   tctgctctga tgccgcatag ttaagccagt atacactccg ctatcgctac gtgactgggt 4380
   catggctgcg ccccgacacc cgccaacacc cgctgacgcg ccctgacggg cttgtctgct 4440
   cccggcatcc gcttacagac aagctgtgac cgtctccggg agctgcatgt gtcagaggtt 4500
   ttcaccgtca tcaccgaaac gcgcgaggca gctggcacga caggtttccc gactgggttg 4560
   gtgcgtttta tcatgcctgg cgggtaggtc ggataaggcg ttcacgccgc atccgacaac 4620
   cacgcagcgt tacctgatgt gacgccgaca attctcatca tcgctacaac atgacctcgc 4680
   tatttacatc gcgatactct tttggcgtcg tgtcatatgc ttttttaaaa acagagtaga 4740
   aatattgcag cgatggataa ccgcacattt gcgatatctc attgatcgac aaggtggttg 4800
   aaatcagcag actgcgcgct ttctccagct tctcggcatg aatcatggca tggatggttt 4860
   cacccacctc ttctttaaaa cgcttctcaa gattggagcg cgagatcccg accgcatcca 4920
   gtacctgatc cactttaatc cctttacagg cgtgattacg aatgtaatgc atggcctgaa 4980
   taacggcggg atcggtcagc gagcgataat ctgttgagcg ccgttcaatg acgcgaactg 5040
   gtgggaccaa aattcgctgt agcggcattt cttctttatc taataatcga tgcaacagtt 5100
   ttgccgcctg atagcccatt tgccgcgcgc cctgagcgac cgaagaaagg gcgacacgcg 5160
   acagatagcg ggtcagttct tcgttatcga tgccaatcac gcataatttt tccggtacgg 5220
   gaatatgtag atgttcacat acttgcagaa tatgccgcgc tcgggcgtca gtaacggcaa 5280
   taatcccggt ttgcggtggt agcgtttgta gccagtctgc cagccgattt tgcgcgtgtt 5340
   gccagttctc tggcgcggtt tctaacccct gataaaccac tccgcgatac ttttcttcgg     5400
   cgacaagctg acgaaatgca tattcgcgct cagtggccca acgtttgccg cttgattccg     5460
   gaagaccata aaaagcaaag cggttaacgc ctttctcttt taaatgcaaa aatgcgcttt     5520
   caaccagcgc atagttatcg gtggcaatgt aatgaacggg tgggtaactt tctgcaaggt     5580
   gatacgagcc gccaacccca acaatgggga cgtcgacatc agccagcgct tgctcgatct     5640
   gtttgtcgtc gaagtcggca atgacgccat ctcctaacca gtccttgatt ttatcaatgc     5700
   gggcgcggaa atcttcttca atgaaaatat cccattccga ttgtgacgcc tgtaaatatt     5760
   cccctacgcc ttctactacc tgccggtcat aggctttatt ggcattgaac agtaatgtga     5820
   tgcggtgacg tttagtaaac atggttcttt tcctgctgaa tcatgctaag taacaatcac     5880
   cgcgataaac gtaaccaatt tttagcaact aaacagggga aaacaattac agatttttat     5940
   ctttcgatta cgatttttgg tttatttctt gatttatgac cgagatctta cttttgttgc     6000
   gcaattgtac ttattgcatt tttctcttcg aggaatt                              6037
<210> 43
   <211> 10307
   <212> DNA
   <213> Artifizielle Sequenz
<220>
   <223> Plasmid pGT1574
<400> 43
   ttgtaattct catgtttgac agcttatcat cgataagctt taatgcggta gttaacgcag      60
   tcaggaacct tgcaatgagc agtttcgatg cccatgacct tgacctcgac aaatttccgg     120
   aggtcgtgcg agatcgtttg acgcagttcc tcgatgctca agagctaaca attgctgata     180
   tcggcgctcc tgtcacagat gctgtggccc atcttcgcag tttcgtgctc aatggaggaa     240
   agcgaatccg tcctctttat gcgtgggctg gtttcctggc ggcgcaaggc cataagaatt     300
   cttctgaaaa acttgagtcc gtccttgacg ccgcagcgag tctcgaattc atccaggctt     360
   gtgccttgat tcatgacgat attatcgatt cttctgatac ccggcgcgga gcccctacag     420
   ttcaccgggc tgtggaagct gatcaccgcg ccaataattt cgaaggcgat cctgagcact     480
   ttggcgtttc agtctcgatt ttggctggcg atatggcatt ggtgtgggca gaagacatgc     540
   tgcaggattc cggtttgagt gcagaggcat tggcccgcac gagggatgct tggcgtggca     600
   tgcgtactga ggttattggc ggccagctgc ttgatattta tcttgagtcg cacgccaacg     660
   agtcggtgga gcttgcggat tctgtcaacc gcttcaaaac ggccgcttac acgattgcgc     720
   gcccattgca cctgggcgcc tccattgctg gcggttcgcc gcagcttatc gacgcgctcc     780
   tccactacgg ccacgacatc ggcattgcat tccagttgag ggatgatctg cttggtgtgt     840
   ttggtgatcc tgctatcacc ggcaaaccag ctggagacga tatccgtgaa ggcaagcgca 900
   ctgttcttct tgcgctcgct ctacaacgcg ctgataagca atctcctgaa gctgcaacgg 960
   ccattcgcgc aggtgttgga aaggtgactt caccagaaga tattgctgtc attacagagc 1020
   atattcgagc tactggtgct gaagaagaag ttgagcagcg aatttcccag ctgactgaat 1080
   ccggtttggc tcacctcgat gatgtagaca tccctgatga ggtgcgcgca cagttgcggg 1140
   cactggctat ccgctcaacc gaacgtcgga tgtaataccg cccttttggg ttcaagcagt 1200
   acataacgat ggaaccacat tacaggagta gtgatgaatg aaggacgagc gccttgttca 1260
   gcgtaagaac gatcatctgg atatcgttct cgacccccgt cgcgccgtaa ctcaggctag 1320
   cgcaggtttt gagcgctggc gctttaccca ctgcgccctg ccagagctga attttagcga 1380
   catcacgctg gaaaccacct tcctgaatcg gcagctacag gctccgctgc tgatcagctc 1440
   catgaccggc ggcgttgagc gctcgcgcca tatcaaccgc cacctcgccg aggcggcgca 1500
   ggtgctaaaa attgcgatgg gggtgggctc ccagcgcgtc gccattgaga gcgacgcggg 1560
   cttagggctg gataaaaccc tgcggcagct ggctccggac gtgccgctgc tggcgaacct 1620
   cggcgcggcg cagctgaccg gcagaaaagg tattgattac gcccgacggg ccgtggagat 1680
   gatcgaggcg gatgcgctga ttgtgcacct aaacccgctg caggaggcgc tacagcccgg 1740
   cggcgatcgc gactggcgcg gacggctggc ggctattgaa actctggtcc gcgagctgcc 1800
   cgttccgctg gtggtgaaag aggtgggagc cggtatctcc cgaaccgtgg ccgggcagct 1860
   gatcgatgcc ggcgttaccg tgattgacgt cgcgggcgcg ggcggcacca gctgggccgc 1920
   cgttgaaggc gagcgggcgg ccaccgagca gcagcgcagc gtggccaacg tctttgccga 1980
   ctgggggatc cccaccgctg aggcgctggt tgacattgcc gaggcctggc cgcagatgcc 2040
   ccttattgcc tcgggcggga ttaaaaacgg cgtcgacgcg gcgaaagcgc tgcggctcgg 2100
   cgcgtgcatg gtagggcagg ccgccgccgt gctcggcagc gcaggcgtct ccacggagaa 2160
   ggtgatcgat cacttcaacg tgattattga gcagctgcgg gtggcctgct tctgcaccgg 2220
   cagccgcagc ctgagcgatc taaagcaggc tgatatccgc tatgttcgtg atacgccata 2280
   aggaggtaca accatgaaga aaaccgttgt gattggcgca ggctttggtg gcctggcgct 2340
   ggcgattcgc ctgcaggcgg cagggatccc aaccgtactg ctggagcagc gggacaagcc 2400
   cggcggtcgg gcctacgtct ggcatgacca gggctttacc tttgacgccg ggccgacggt 2460
   gatcaccgat cctaccgcgc ttgaggcgct gttcaccctg gccggcaggc gcatggagga 2520
   ttacgtcagg ctgctgccgg taaaaccctt ctaccgactc tgctgggagt ccgggaagac 2580
   cctcgactat gctaacgaca gcgccgagct tgaggcgcag attacccagt tcaacccccg 2640
   cgacgtcgag ggctaccggc gctttctggc ttactcccag gcggtattcc aggagggata 2700
   tttgcgcctc ggcagcgtgc cgttcctctc ttttcgcgac atgctgcgcg ccgggccgca 2760
   gctgcttaag ctccaggcgt ggcagagcgt ctaccagtcg gtttcgcgct ttattgagga 2820
   tgagcatctg cggcaggcct tctcgttcca ctccctgctg gtaggcggca accccttcac 2880
   cacctcgtcc atctacaccc tgatccacgc ccttgagcgg gagtgggggg tctggttccc 2940
   tgagggcggc accggggcgc tggtgaacgg catggtgaag ctgtttaccg atctgggcgg 3000
   ggagatcgaa ctcaacgccc gggtcgaaga gctggtggtg gccgataacc gcgtaagcca 3060
   ggtccggctg gcggatggtc ggatctttga caccgacgcc gtagcctcga acgctgacgt 3120
   ggtgaacacc tataaaaagc tgctcggcca ccatccggtg gggcagaagc gggcggcagc 3180
   gctggagcgc aagagcatga gcaactcgct gtttgtgctc tacttcggcc tgaaccagcc 3240
   tcattcccag ctggcgcacc ataccatctg ttttggtccc cgctaccggg agctgatcga 3300
   cgagatcttt accggcagcg cgctggcgga tgacttctcg ctctacctgc actcgccctg 3360
   cgtgaccgat ccctcgctcg cgcctcccgg ctgcgccagc ttctacgtgc tggccccggt 3420
   gccgcatctt ggcaacgcgc cgctggactg ggcgcaggag gggccgaagc tgcgcgaccg 3480
   catctttgac taccttgaag agcgctatat gcccggcctg cgtagccagc tggtgaccca 3540
   gcggatcttt accccggcag acttccacga cacgctggat gcgcatctgg gatcggcctt 3600
   ctccatcgag ccgctgctga cccaaagcgc ctggttccgc ccgcacaacc gcgacagcga 3660
   cattgccaac ctctacctgg tgggcgcagg tactcaccct ggggcgggca ttcctggcgt 3720
   agtggcctcg gcgaaagcca ccgccagcct gatgattgag gatctgcaat gagccaaccg 3780
   ccgctgcttg accacgccac gcagaccatg gccaacggct cgaaaagttt tgccaccgct 3840
   gcgaagctgt tcgacccggc cacccgccgt agcgtgctga tgctctacac ctggtgccgc 3900
   cactgcgatg acgtcattga cgaccagacc cacggcttcg ccagcgaggc cgcggcggag 3960
   gaggaggcca cccagcgcct ggcccggctg cgcacgctga ccctggcggc gtttgaaggg 4020
   gccgagatgc aggatccggc cttcgctgcc tttcaggagg tggcgctgac ccacggtatt 4080
   acgccccgca tggcgctcga tcacctcgac ggctttgcga tggacgtggc tcagacccgc 4140
   tatgtcacct ttgaggatac gctgcgctac tgctatcacg tggcgggcgt ggtgggtctg 4200
   atgatggcca gggtgatggg cgtgcgggat gagcgggtgc tggatcgcgc ctgcgatctg 4260
   gggctggcct tccagctgac gaatatcgcc cgggatatta ttgacgatgc ggctattgac 4320
   cgctgctatc tgcccgccga gtggctgcag gatgccgggc tgaccccgga gaactatgcc 4380
   gcgcgggaga atcgggccgc gctggcgcgg gtggcggagc ggcttattga tgccgcagag 4440
   ccgtactaca tctcctccca ggccgggcta cacgatctgc cgccgcgctg cgcctgggcg 4500
   atcgccaccg cccgcagcgt ctaccgggag atcggtatta aggtaaaagc ggcgggaggc 4560
   agcgcctggg atcgccgcca gcacaccagc aaaggtgaaa aaattgccat gctgatggcg 4620
   gcaccggggc aggttattcg ggcgaagacg acgagggtga cgccgcgtcc ggccggtctt 4680
   tggcagcgtc ccgtttagac cgactccaaa cgagtcggtt tttttgcgct tgtttcggcg 4740
   tgggtatggt ggcaggcccc gtggccgggg gactgttggg cgccatctcc ttggggtcga 4800
   atttgctttc gaatttctgc cattcatccg cttattatca cttattcagg cgtagcaacc 4860
   aggcgtttaa gggcaccaat aactgcctta aaaaaattac gccccgccct gccactcatc 4920
   gcagtactgt tgtaattcat taagcattct gccgacatgg aagccatcac agacggcatg 4980
   atgaacctga atcgccagcg gcatcagcac cttgtcgcct tgcgtataat atttgcccat 5040
   ggtgaaaacg ggggcgaaga agttgtccat attggccacg tttaaatcaa aactggtgaa 5100
   actcacccag ggattggctg agacgaaaaa catattctca ataaaccctt tagggaaata 5160
   ggccaggttt tcaccgtaac acgccacatc ttgcgaatat atgtgtagaa actgccggaa 5220
   atcgtcgtgg tattcactcc agagcgatga aaacgtttca gtttgctcat ggaaaacggt 5280
   gtaacaaggg tgaacactat cccatatcac cagctcaccg tctttcattg ccatacggaa 5340
   ttccggatga gcattcatca ggcgggcaag aatgtgaata aaggccggat aaaacttgtg 5400
   cttatttttc tttacggtct ttaaaaaggc cgtaatatcc agctgaacgg tctggttata 5460
   ggtacattga gcaactgact gaaatgcctc aaaatgttct ttacgatgcc attgggatat 5520
   atcaacggtg gtatatccag tgattttttt ctccatttta gcttccttag ctcctgaaaa 5580
   tctcgataac tcaaaaaata cgcccggtag tgatcttatt tcattatggt gaaagttgga 5640
   acctcttacg tgccgatcaa cgtctcattt tcgccaaaag ttggcccagg gcttcccggt 5700
   atcaacaggg acaccaggat ttatttattc tgcgaagtga tcttccgtca caggtattta 5760
   ttcggcgcaa agtgcgtcgg gtgatgctgc caacttactg atttagtgta tgatggtgtt 5820
   tttgaggtgc tccagtggct tctgtttcta tcagctgtcc ctcctgttca gctactgacg 5880
   gggtggtgcg taacggcaaa agcaccgccg gacatcagcg ctagcggagt gtatacactc 5940
   cgctatcgct acgtgactgg gtcatggctg cgccccgaca cccgccaaca cccgctgacg 6000
   cgccctgacg ggcttgtctg ctcccggcat ccgcttacag acaagctgtg accgtctccg 6060
   ggagctgcat gtgtcagagg ttttcaccgt catcaccgaa acgcgcgagg cagctggcac 6120
   gacaggtttc ccgactggaa agcgaattgg gtataatgcc ctcgtaataa ttttgtttaa 6180
   ctttaagaag gagatatacc catgacacag agggcccacc atcaccatca ccattccatg 6240
   gctagcggcg gcggaagttc cggtagtgag agttgtgtag cggtgagaga agatttcgct 6300
   gacgaagaag attttgtgaa agctggtggt tctgagattc tatttgttca aatgcagcag 6360
   aacaaagata tggatgaaca gtctaagctt gttgataagt tgcctcctat atcaattggt 6420
   gatggtgctt tggatctagt ggttattggt tgtggtcctg ctggtttagc cttggctgca 6480
   gaatcagcta agcttggatt aaaagttgga ctcattggtc cagatcttcc ttttactaac 6540
   aattacggtg tttgggaaga tgaattcaat gatcttgggc tgcaaaaatg tattgagcat 6600
   gtttggagag agactattgt gtatctggat gatgacaagc ctattaccat tggccgtgct 6660
   tatggaagag ttagtcgacg tttgctccat gaggagcttt tgaggaggtg tgtcgagtca 6720
   ggtgtctcgt accttagctc gaaagttgac agcataacag aagcttctga tggccttaga 6780
   cttgttgctt gtgacgacaa taacgtcatt ccctgcaggc ttgccactgt tgcttctgga 6840
   gcagcttcgg gaaagctctt gcaatacgaa gttggtggac ctagagtctg tgtgcaaact 6900
   gcatacggcg tggaggttga ggtggaaaat agtccatatg atccagatca aatggttttc 6960
   atggattaca gagattatac taacgagaaa gttcggagct tagaagctga gtatccaacg 7020
   tttctgtacg ccatgcctat gacaaagtca agactcttct tcgaggagac atgtttggcc 7080
   tcaaaagatg tcatgccctt tgatttgcta aaaacgaagc tcatgttaag attagataca 7140
   ctcggaattc gaattctaaa gacttacgaa gaggagtggt cctatatccc agttggtggt 7200
   tccttgccaa acaccgaaca aaagaatctc gcctttggtg ctgccgctag catggtacat 7260
   cccgcaacag gctattcagt tgtgagatct ttgtctgaag ctccaaaata tgcatcagtc 7320
   atcgcagaga tactaagaga agagactacc aaacagatca acagtaatat ttcaagacaa 7380
   gcttgggata ctttatggcc accagaaagg aaaagacaga gagcattctt tctctttggt 7440
   cttgaggcta tagttcaatt cgataccgaa ggcattagaa gcttcttccg tactttcttc 7500
   cgccttccaa aatggatgtg gcaagggttt ctaggatcaa cattaacatc aggagatctc 7560
   gttctctttt ctttatacat gttcgtcatt tcaccaaaca atttgagaaa aggtctcatc 7620
   aatcatctca tctctgatcc aaccggagca accatgataa aaacctatct caaagtataa 7680
   ggaggatctt actccatggc ccaccatcac catcaccatt ccgcggagaa actcagtgat 7740
   ggcagcagca tcatctcagt ccatcctaga ccctccaagg gtttctcctc gaagcttctc 7800
   gatcttctcg agagacttgt tgtcaagctc atgcacgatg cttctctccc tctccactac 7860
   ctctcaggca acttcgctcc catccgtgat gaaactcctc ccgtcaagga tctccccgtc 7920
   catggatttc ttcccgaatg cttgaatggt gaatttgtga gggttggtcc aaaccccaag 7980
   tttgatgctg tcgctggata tcactggttt gatggagatg ggatgattca tggggtacgc 8040
   atcaaagatg ggaaagctac ttatgtttct cgatatgtta agacatcacg tcttaagcag 8100
   gaagagttct tcggagctgc caaattcatg aagattggtg accttaaggg gtttttcgga 8160
   ttgctaatgg tcaatgtcca acagctgaga acgaagctca aaatattgga caacacttat 8220
   ggaaatggaa ctgccaatac agcactcgta tatcaccatg gaaaacttct agcattacag 8280
   gaggcagata agccgtacgt catcaaagtt ttggaagatg gagacctgca aactcttggt 8340
   ataatagatt atgacaagag attgacccac tccttcactg ctcacccaaa agttgacccg 8400
   gttacgggtg aaatgtttac attcggctat tcgcatacgc caccttatct cacatacaga     8460
   gttatctcga aagatggcat tatgcatgac ccagtcccaa ttactatatc agagcctatc     8520
   atgatgcatg attttgctat tactgagact tatgcaatct tcatggatct tcctatgcac     8580
   ttcaggccaa aggaaatggt gaaagagaag aaaatgatat actcatttga tcccacaaaa     8640
   aaggctcgtt ttggtgttct tccacgctat gccaaggatg aacttatgat tagatggttt     8700
   gagcttccca actgctttat tttccacaac gccaatgctt gggaagaaga ggatgaagtc     8760
   gtcctcatca cttgtcgtct tgagaatcca gatcttgaca tggtcagtgg gaaagtgaaa     8820
   gaaaaactcg aaaattttgg caacgaactg tacgaaatga gattcaacat gaaaacgggc     8880
   tcagcttctc aaaaaaaact atccgcatct gcggttgatt tccccagaat caatgagtgc     8940
   tacaccggaa agaaacagag atacgtatat ggaacaattc tggacagtat cgcaaaggtt     9000
   accggaatca tcaagtttga tctgcatgca gaagctgaga cagggaaaag aatgctggaa     9060
   gtaggaggta atatcaaagg aatatatgac ctgggagaag gcagatatgg ttcagaggct     9120
   atctatgttc cgcgtgagac agcagaagaa gacgacggtt acttgatatt ctttgttcat     9180
   gatgaaaaca cagggaaatc atgcgtgact gtgatagacg caaaaacaat gtcggctgaa     9240
   ccggtggcag tggtggagct gccgcacagg gtcccatatg gcttccatgc cttgtttgtt     9300
   acagaggaac aactccagga acaaactctt atataaggat ccggctgcta acaaagcccg     9360
   ttctgtttaa gaacgggatt ttttgctgaa aggaggaact atatccggcc ggattactgg     9420
   cttactatgt tggcactgat gagggtgtca gtgaagtgct tcatgtggca ggagaaaaaa     9480
   ggctgcaccg gtgcgtcagc agaatatgtg atacaggata tattccgctt cctcgctcac     9540
   tgactcgcta cgctcggtcg ttcgactgcg gcgagcggaa atggcttacg aacggggcgg     9600
   agatttcctg gaagatgcca ggaagatact taacagggaa gtgagagggc cgcggcaaag     9660
   ccgtttttcc ataggctccg cccccctgac aagcatcacg aaatctgacg ctcaaatcag     9720
   tggtggcgaa acccgacagg actataaaga taccaggcgt ttccccctgg cggctccctc     9780
   gtgcgctctc ctgttcctgc ctttcggttt accggtgtca ttccgctgtt atggccgcgt     9840
   ttgtctcatt ccacgcctga cactcagttc cgggtaggca gttcgctcca agctggactg     9900
   tatgcacgaa ccccccgttc agtccgaccg ctgcgcctta tccggtaact atcgtcttga     9960
   gtccaacccg gaaagacatg caaaagcacc actggcagca gccactggta attgatttag    10020
   aggagttagt cttgaagtca tgcgccggtt aaggctaaac tgaaaggaca agttttggtg    10080
   actgcgctcc tccaagccag ttacctcggt tcaaagagtt ggtagctcag agaaccttcg    10140
   aaaaaccgcc ctgcaaggcg gttttttcgt tttcagagca agagattacg cgcagaccaa    10200
   aacgatctca agaagatcat cttattaatc agataaaata tttctagatt tcagtgcaat    10260
   ttatctcttc aaatgtagca cctgaagtca gccccatacg atataag                  10307
<210> 44
   <211> 10325
   <212> DNA
   <213> Artifizielle Sequenz
<220>
   <223> Plasmid pGT1575
<400> 44
   ttgtaattct catgtttgac agcttatcat cgataagctt taatgcggta gttaacgcag      60
   tcaggaacct tgcaatgagc agtttcgatg cccatgacct tgacctcgac aaatttccgg     120
   aggtcgtgcg agatcgtttg acgcagttcc tcgatgctca agagctaaca attgctgata     180
   tcggcgctcc tgtcacagat gctgtggccc atcttcgcag tttcgtgctc aatggaggaa     240
   agcgaatccg tcctctttat gcgtgggctg gtttcctggc ggcgcaaggc cataagaatt     300
   cttctgaaaa acttgagtcc gtccttgacg ccgcagcgag tctcgaattc atccaggctt     360
   gtgccttgat tcatgacgat attatcgatt cttctgatac ccggcgcgga gcccctacag     420
   ttcaccgggc tgtggaagct gatcaccgcg ccaataattt cgaaggcgat cctgagcact     480
   ttggcgtttc agtctcgatt ttggctggcg atatggcatt ggtgtgggca gaagacatgc     540
   tgcaggattc cggtttgagt gcagaggcat tggcccgcac gagggatgct tggcgtggca     600
   tgcgtactga ggttattggc ggccagctgc ttgatattta tcttgagtcg cacgccaacg     660
   agtcggtgga gcttgcggat tctgtcaacc gcttcaaaac ggccgcttac acgattgcgc     720
   gcccattgca cctgggcgcc tccattgctg gcggttcgcc gcagcttatc gacgcgctcc     780
   tccactacgg ccacgacatc ggcattgcat tccagttgag ggatgatctg cttggtgtgt     840
   ttggtgatcc tgctatcacc ggcaaaccag ctggagacga tatccgtgaa ggcaagcgca     900
   ctgttcttct tgcgctcgct ctacaacgcg ctgataagca atctcctgaa gctgcaacgg     960
   ccattcgcgc aggtgttgga aaggtgactt caccagaaga tattgctgtc attacagagc    1020
   atattcgagc tactggtgct gaagaagaag ttgagcagcg aatttcccag ctgactgaat    1080
   ccggtttggc tcacctcgat gatgtagaca tccctgatga ggtgcgcgca cagttgcggg    1140
   cactggctat ccgctcaacc gaacgtcgga tgtaataccg cccttttggg ttcaagcagt    1200
   acataacgat ggaaccacat tacaggagta gtgatgaatg aaggacgagc gccttgttca    1260
   gcgtaagaac gatcatctgg atatcgttct cgacccccgt cgcgccgtaa ctcaggctag    1320
   cgcaggtttt gagcgctggc gctttaccca ctgcgccctg ccagagctga attttagcga    1380
   catcacgctg gaaaccacct tcctgaatcg gcagctacag gctccgctgc tgatcagctc    1440
   catgaccggc ggcgttgagc gctcgcgcca tatcaaccgc cacctcgccg aggcggcgca    1500
   ggtgctaaaa attgcgatgg gggtgggctc ccagcgcgtc gccattgaga gcgacgcggg    1560
   cttagggctg gataaaaccc tgcggcagct ggctccggac gtgccgctgc tggcgaacct 1620
   cggcgcggcg cagctgaccg gcagaaaagg tattgattac gcccgacggg ccgtggagat 1680
   gatcgaggcg gatgcgctga ttgtgcacct aaacccgctg caggaggcgc tacagcccgg 1740
   cggcgatcgc gactggcgcg gacggctggc ggctattgaa actctggtcc gcgagctgcc 1800
   cgttccgctg gtggtgaaag aggtgggagc cggtatctcc cgaaccgtgg ccgggcagct 1860
   gatcgatgcc ggcgttaccg tgattgacgt cgcgggcgcg ggcggcacca gctgggccgc 1920
   cgttgaaggc gagcgggcgg ccaccgagca gcagcgcagc gtggccaacg tctttgccga 1980
   ctgggggatc cccaccgctg aggcgctggt tgacattgcc gaggcctggc cgcagatgcc 2040
   ccttattgcc tcgggcggga ttaaaaacgg cgtcgacgcg gcgaaagcgc tgcggctcgg 2100
   cgcgtgcatg gtagggcagg ccgccgccgt gctcggcagc gcaggcgtct ccacggagaa 2160
   ggtgatcgat cacttcaacg tgattattga gcagctgcgg gtggcctgct tctgcaccgg 2220
   cagccgcagc ctgagcgatc taaagcaggc tgatatccgc tatgttcgtg atacgccata 2280
   aggaggtaca accatgaaga aaaccgttgt gattggcgca ggctttggtg gcctggcgct 2340
   ggcgattcgc ctgcaggcgg cagggatccc aaccgtactg ctggagcagc gggacaagcc 2400
   cggcggtcgg gcctacgtct ggcatgacca gggctttacc tttgacgccg ggccgacggt 2460
   gatcaccgat cctaccgcgc ttgaggcgct gttcaccctg gccggcaggc gcatggagga 2520
   ttacgtcagg ctgctgccgg taaaaccctt ctaccgactc tgctgggagt ccgggaagac 2580
   cctcgactat gctaacgaca gcgccgagct tgaggcgcag attacccagt tcaacccccg 2640
   cgacgtcgag ggctaccggc gctttctggc ttactcccag gcggtattcc aggagggata 2700
   tttgcgcctc ggcagcgtgc cgttcctctc ttttcgcgac atgctgcgcg ccgggccgca 2760
   gctgcttaag ctccaggcgt ggcagagcgt ctaccagtcg gtttcgcgct ttattgagga 2820
   tgagcatctg cggcaggcct tctcgttcca ctccctgctg gtaggcggca accccttcac 2880
   cacctcgtcc atctacaccc tgatccacgc ccttgagcgg gagtgggggg tctggttccc 2940
   tgagggcggc accggggcgc tggtgaacgg catggtgaag ctgtttaccg atctgggcgg 3000
   ggagatcgaa ctcaacgccc gggtcgaaga gctggtggtg gccgataacc gcgtaagcca 3060
   ggtccggctg gcggatggtc ggatctttga caccgacgcc gtagcctcga acgctgacgt 3120
   ggtgaacacc tataaaaagc tgctcggcca ccatccggtg gggcagaagc gggcggcagc 3180
   gctggagcgc aagagcatga gcaactcgct gtttgtgctc tacttcggcc tgaaccagcc 3240
   tcattcccag ctggcgcacc ataccatctg ttttggtccc cgctaccggg agctgatcga 3300
   cgagatcttt accggcagcg cgctggcgga tgacttctcg ctctacctgc actcgccctg 3360
   cgtgaccgat ccctcgctcg cgcctcccgg ctgcgccagc ttctacgtgc tggccccggt 3420
   gccgcatctt ggcaacgcgc cgctggactg ggcgcaggag gggccgaagc tgcgcgaccg 3480
   catctttgac taccttgaag agcgctatat gcccggcctg cgtagccagc tggtgaccca 3540
   gcggatcttt accccggcag acttccacga cacgctggat gcgcatctgg gatcggcctt 3600
   ctccatcgag ccgctgctga cccaaagcgc ctggttccgc ccgcacaacc gcgacagcga 3660
   cattgccaac ctctacctgg tgggcgcagg tactcaccct ggggcgggca ttcctggcgt 3720
   agtggcctcg gcgaaagcca ccgccagcct gatgattgag gatctgcaat gagccaaccg 3780
   ccgctgcttg accacgccac gcagaccatg gccaacggct cgaaaagttt tgccaccgct 3840
   gcgaagctgt tcgacccggc cacccgccgt agcgtgctga tgctctacac ctggtgccgc 3900
   cactgcgatg acgtcattga cgaccagacc cacggcttcg ccagcgaggc cgcggcggag 3960
   gaggaggcca cccagcgcct ggcccggctg cgcacgctga ccctggcggc gtttgaaggg 4020
   gccgagatgc aggatccggc cttcgctgcc tttcaggagg tggcgctgac ccacggtatt 4080
   acgccccgca tggcgctcga tcacctcgac ggctttgcga tggacgtggc tcagacccgc 4140
   tatgtcacct ttgaggatac gctgcgctac tgctatcacg tggcgggcgt ggtgggtctg 4200
   atgatggcca gggtgatggg cgtgcgggat gagcgggtgc tggatcgcgc ctgcgatctg 4260
   gggctggcct tccagctgac gaatatcgcc cgggatatta ttgacgatgc ggctattgac 4320
   cgctgctatc tgcccgccga gtggctgcag gatgccgggc tgaccccgga gaactatgcc 4380
   gcgcgggaga atcgggccgc gctggcgcgg gtggcggagc ggcttattga tgccgcagag 4440
   ccgtactaca tctcctccca ggccgggcta cacgatctgc cgccgcgctg cgcctgggcg 4500
   atcgccaccg cccgcagcgt ctaccgggag atcggtatta aggtaaaagc ggcgggaggc 4560
   agcgcctggg atcgccgcca gcacaccagc aaaggtgaaa aaattgccat gctgatggcg 4620
   gcaccggggc aggttattcg ggcgaagacg acgagggtga cgccgcgtcc ggccggtctt 4680
   tggcagcgtc ccgtttagac cgactccaaa cgagtcggtt tttttgcgct tgtttcggcg 4740
   tgggtatggt ggcaggcccc gtggccgggg gactgttggg cgccatctcc ttggggtcga 4800
   atttgctttc gaatttctgc cattcatccg cttattatca cttattcagg cgtagcaacc 4860
   aggcgtttaa gggcaccaat aactgcctta aaaaaattac gccccgccct gccactcatc 4920
   gcagtactgt tgtaattcat taagcattct gccgacatgg aagccatcac agacggcatg 4980
   atgaacctga atcgccagcg gcatcagcac cttgtcgcct tgcgtataat atttgcccat 5040
   ggtgaaaacg ggggcgaaga agttgtccat attggccacg tttaaatcaa aactggtgaa 5100
   actcacccag ggattggctg agacgaaaaa catattctca ataaaccctt tagggaaata 5160
   ggccaggttt tcaccgtaac acgccacatc ttgcgaatat atgtgtagaa actgccggaa 5220
   atcgtcgtgg tattcactcc agagcgatga aaacgtttca gtttgctcat ggaaaacggt 5280
   gtaacaaggg tgaacactat cccatatcac cagctcaccg tctttcattg ccatacggaa 5340
   ttccggatga gcattcatca ggcgggcaag aatgtgaata aaggccggat aaaacttgtg 5400
   cttatttttc tttacggtct ttaaaaaggc cgtaatatcc agctgaacgg tctggttata 5460
   ggtacattga gcaactgact gaaatgcctc aaaatgttct ttacgatgcc attgggatat 5520
   atcaacggtg gtatatccag tgattttttt ctccatttta gcttccttag ctcctgaaaa 5580
   tctcgataac tcaaaaaata cgcccggtag tgatcttatt tcattatggt gaaagttgga 5640
   acctcttacg tgccgatcaa cgtctcattt tcgccaaaag ttggcccagg gcttcccggt 5700
   atcaacaggg acaccaggat ttatttattc tgcgaagtga tcttccgtca caggtattta 5760
   ttcggcgcaa agtgcgtcgg gtgatgctgc caacttactg atttagtgta tgatggtgtt 5820
   tttgaggtgc tccagtggct tctgtttcta tcagctgtcc ctcctgttca gctactgacg 5880
   gggtggtgcg taacggcaaa agcaccgccg gacatcagcg ctagcggagt gtatacactc 5940
   cgctatcgct acgtgactgg gtcatggctg cgccccgaca cccgccaaca cccgctgacg 6000
   cgccctgacg ggcttgtctg ctcccggcat ccgcttacag acaagctgtg accgtctccg 6060
   ggagctgcat gtgtcagagg ttttcaccgt catcaccgaa acgcgcgagg cagctggcac 6120
   gacaggtttc ccgactggaa aagcgaattg gtataatgcc ctcgtaataa ttttgtttaa 6180
   ctttaagaag gagatatacc catgacacag agggcccacc atcaccatca ccattccatg 6240
   gctagcggcg gcggaagttc cggtagtgag agttgtgtag cggtgagaga agatttcgct 6300
   gacgaagaag attttgtgaa agctggtggt tctgagattc tatttgttca aatgcagcag 6360
   aacaaagata tggatgaaca gtctaagctt gttgataagt tgcctcctat atcaattggt 6420
   gatggtgctt tggatctagt ggttattggt tgtggtcctg ctggtttagc cttggctgca 6480
   gaatcagcta agcttggatt aaaagttgga ctcattggtc cagatcttcc ttttactaac 6540
   aattacggtg tttgggaaga tgaattcaat gatcttgggc tgcaaaaatg tattgagcat 6600
   gtttggagag agactattgt gtatctggat gatgacaagc ctattaccat tggccgtgct 6660
   tatggaagag ttagtcgacg tttgctccat gaggagcttt tgaggaggtg tgtcgagtca 6720
   ggtgtctcgt accttagctc gaaagttgac agcataacag aagcttctga tggccttaga 6780
   cttgttgctt gtgacgacaa taacgtcatt ccctgcaggc ttgccactgt tgcttctgga 6840
   gcagcttcgg gaaagctctt gcaatacgaa gttggtggac ctagagtctg tgtgcaaact 6900
   gcatacggcg tggaggttga ggtggaaaat agtccatatg atccagatca aatggttttc 6960
   atggattaca gagattatac taacgagaaa gttcggagct tagaagctga gtatccaacg 7020
   tttctgtacg ccatgcctat gacaaagtca agactcttct tcgaggagac atgtttggcc 7080
   tcaaaagatg tcatgccctt tgatttgcta aaaacgaagc tcatgttaag attagataca 7140
   ctcggaattc gaattctaaa gacttacgaa gaggagtggt cctatatccc agttggtggt 7200
   tccttgccaa acaccgaaca aaagaatctc gcctttggtg ctgccgctag catggtacat 7260
   cccgcaacag gctattcagt tgtgagatct ttgtctgaag ctccaaaata tgcatcagtc 7320
   atcgcagaga tactaagaga agagactacc aaacagatca acagtaatat ttcaagacaa 7380
   gcttgggata ctttatggcc accagaaagg aaaagacaga gagcattctt tctctttggt 7440
   cttgaggcta tagttcaatt cgataccgaa ggcattagaa gcttcttccg tactttcttc 7500
   cgccttccaa aatggatgtg gcaagggttt ctaggatcaa cattaacatc aggagatctc 7560
   gttctctttt ctttatacat gttcgtcatt tcaccaaaca atttgagaaa aggtctcatc 7620
   aatcatctca tctctgatcc aaccggagca accatgataa aaacctatct caaagtataa 7680
   ggaggatctt actccatggc ccaccatcac catcaccatt ccatgggtat gcagggtgaa 7740
   gatgcacagc gtaccggtaa tattgttgca gttaaaccga aaccgagcca gggtctgacc 7800
   agcaaagcaa ttgattggct ggaatggctg tttgtgaaaa tgatgcatga tagcaaacag 7860
   ccgctgcatt atctgagcgg taattttgca ccggttgatg aaacccctcc gctgaaagat 7920
   ctgccggtta ccggtcatct gccggaatgt ctgaatggtg aatttgttcg tgttggtccg 7980
   aatccgaaat ttgcaagcat tgcaggttat cattggtttg atggtgatgg tatgattcat 8040
   ggcatgcgca ttaaagatgg taaagcaacc tatgttagcc gttatgttca gaccagccgt 8100
   ctgaaacaag aggaattctt tggtcgtgcc atgttcatga aaatcggtga tctgaaaggt 8160
   atgtttggtc tgctgatggt taatatgcag atgctgcgtg caaaactgaa agttctggat 8220
   attagctatg gtattggcac cgcaaatacc gcactggttt atcatcatgg taaactgctg 8280
   gcactgagcg aagcagataa accgtatgca attaaagtgc tggaagatgg tgatctgcag 8340
   accattggcc tgctggatta tgataaacgt ctggcacata gctttaccgc acatccgaaa 8400
   gttgatccgt ttaccggtga gatgtttacc tttggttata gccatacccc tccgtatgtt 8460
   acctatcgtg ttattagcaa agatggtgca atgaatgatc cggttccgat taccgttagc 8520
   ggtccgatca tgatgcacga ttttgcaatt accgaaaact acgccatctt tatggatctg 8580
   ccgctgtatt tcaaaccgaa agaaatggtg aaagacaaga aattcatctt tagcttcgat 8640
   gccacccaga aagcacgttt tggtattctg cctcgttatg ccaagaatga gctgctgatt 8700
   aaatggtttg aactgccgaa ctgcttcatc tttcataatg caaatgcatg ggaagagggt 8760
   gatgaagttg ttctgattac ctgtcgtctg gaaaatccgg atctggatat ggtgaatagc 8820
   accgttaaag aacgtctgga caactttaag aacgagctgt atgaaatgcg cttcaatctg 8880
   cagaatggtc tggcaagcca gaaaaaactg agcgttagcg cagttgattt tccgcgtgtt 8940
   aatgaaagct ataccacccg taaacagcgt tatgtttatg gcaccaccct ggataagatt 9000
   gccaaagtta ccggcatcat caaattcgat ctgcatgccg aaccggaaac cggtaaagag 9060
   aagctggaac tgggtggtaa tgtgaaaggc atttttgatc tgggtccggg tcgttttggt 9120
   tcagaagcag tttttgttcc gcgtcatccg ggtattacca gcgaagagga tgatggttat 9180
   ctgatcttct ttgtgcacga tgaaaacacc ggcaaaagcg cagttaatgt tattgatgca 9240
   aaaaccatga gccctgatcc ggtggcagtt gtggaactgc ctaaacgtgt tccgtatggt 9300
   tttcatgcat tttttgttac cgaagatcag ctgcaagaac aggccaaagt ttaaggatcc 9360
   ggctgctaac aaagcccgtt ctgtttaaga acgggatttt ttgctgaaag gaggaactat 9420
   atccggccgg attactggct tactatgttg gcactgatga gggtgtcagt gaagtgcttc 9480
   atgtggcagg agaaaaaagg ctgcaccggt gcgtcagcag aatatgtgat acaggatata 9540
   ttccgcttcc tcgctcactg actcgctacg ctcggtcgtt cgactgcggc gagcggaaat 9600
   ggcttacgaa cggggcggag atttcctgga agatgccagg aagatactta acagggaagt 9660
   gagagggccg cggcaaagcc gtttttccat aggctccgcc cccctgacaa gcatcacgaa 9720
   atctgacgct caaatcagtg gtggcgaaac ccgacaggac tataaagata ccaggcgttt 9780
   ccccctggcg gctccctcgt gcgctctcct gttcctgcct ttcggtttac cggtgtcatt 9840
   ccgctgttat ggccgcgttt gtctcattcc acgcctgaca ctcagttccg ggtaggcagt 9900
   tcgctccaag ctggactgta tgcacgaacc ccccgttcag tccgaccgct gcgccttatc 9960
   cggtaactat cgtcttgagt ccaacccgga aagacatgca aaagcaccac tggcagcagc 10020
   cactggtaat tgatttagag gagttagtct tgaagtcatg cgccggttaa ggctaaactg 10080
   aaaggacaag ttttggtgac tgcgctcctc caagccagtt acctcggttc aaagagttgg 10140
   tagctcagag aaccttcgaa aaaccgccct gcaaggcggt tttttcgttt tcagagcaag 10200
   agattacgcg cagaccaaaa cgatctcaag aagatcatct tattaatcag ataaaatatt 10260
   tctagatttc agtgcaattt atctcttcaa atgtagcacc tgaagtcagc cccatacgat 10320
   ataag 10325
<210> 45
   <211> 5183
   <212> DNA
   <213> Artifizielle Sequenz
<220>
   <223> Plasmid pGT1534
<400> 45
   aaagcgaatt gtgattgaaa tcaaaatttt cttgactaat tatggtataa tttactggta      60
   ataattttgt ttaactttaa gaaggagata taccatgggc gatcttttat caatacagga     120
   cccgtcgttt ttaaaaaaca tgtccattga tgaattagag aaattaagtg atgaaatccg     180
   tcagttttta attacaagtt tatccgcttc cggcggccac atcggcccaa acttaggtgt     240
   cgtagagctt actgttgccc tgcataagga atttaacagc ccgaaagaca aatttttatg     300
   ggatgtaggc catcagtcgt atgtccataa gctgctgaca ggacgcggaa aagaatttgc     360
   gacgcttcgc cagtacaaag ggctttgcgg atttccaaag cggagtgaaa gcgagcacga      420
   tgtttgggaa accgggcaca gctcgacttc tctgtcaggc gcgatgggaa tggcagctgc      480
   ccgtgatatt aaaggaacgg atgaatatat tattccgatc attggtgacg gcgcgctgac      540
   cggcggtatg gcgctcgaag cccttaacca catcggcgac gagaaaaaag acatgattgt      600
   catccttaat gataatgaaa tgagtattgc gccaaacgtc ggtgccattc actctatgct      660
   cggacggctc cgcactgcgg ggaaatacca gtgggtcaaa gatgagcttg aatacttatt      720
   taaaaagatt ccggcagttg ggggcaagct tgccgccacg gcggaacggg tcaaagacag       780
   cctgaaatac atgctcgtct ccggaatgtt tttcgaggag ctcggtttta cgtatttggg      840
   cccagtggac ggacattctt atcatgagct gattgagaat cttcaatacg ccaaaaaaac      900
   gaaaggccct gttcttctgc acgtcattac gaaaaaaggg aaggggtaca aaccggctga      960
   gaccgatacg attgggacat ggcatggtac cggaccatat aaaattaata ccggtgactt     1020
   tgtaaagccg aaagccgcag ctccttcgtg gagcggtctt gtcagcggaa ctgtgcagcg     1080
   aatggcgcgc gaggacggac gcattgtagc cattacgccg gctatgcctg tcggttcaaa     1140
   gcttgaaggc ttcgcaaagg aattccctga ccggatgttc gacgtaggaa tcgcagaaca     1200
   gcatgccgca acaatggctg cagctatggc aatgcagggt atgaagccgt ttttggcgat      1260
   ttactcaacc ttcctgcaaa gggcatatga ccaagttgtt catgacatct gccgccaaaa     1320
   cgctaatgtg tttattggaa ttgaccgtgc tggactcgtt ggcgctgatg gagagacaca     1380
   tcaaggcgtg tttgatattg cgtttatgcg ccacattcca aacatggtct taatgatgcc     1440
   gaaagacgaa aatgaaggcc agcacatggt tcatacagca cttagctatg acgaaggccc     1500
   gatagcaatg cgttttccgc gcggaaacgg actcggcgta aaaatggatg aacagttgaa     1560
   aacgattccg atcggtacgt gggaggtgct gcgtccaggg aacgatgctg tcatcttaac     1620
   attcggcaca acaatcgaaa tggcgattga agcagccgaa gagctgcaga aagaaggcct     1680
   ttccgtgcgc gttgtgaatg cgcgttttat taagccgatt gatgaaaaga tgatgaagag     1740
   tatcctaaaa gaaggcttgc caattttaac aattgaagaa gcggtcttag aaggcggttt     1800
   cggaagctcg attttagaat tcgctcatga tcaaggtgaa tatcatactc cgattgacag     1860
   aatgggtata cctgatcggt ttattgaaca cggaagtgta acagcgcttc ttgaggaaat     1920
   tggactgaca aaacagcagg tggcaaatcg tattagatta ctgatgccac caaagacaca     1980
   caaaggaatt ggatcataag gatccggctg ctaacaaagc ccgaaaggaa gctgagttgg     2040
   ctgctgccac cgctgagcaa taactagcat aaccccttgg ggcctctaaa cgggtcttga     2100
   ggggtttttt gctgaaagga ggaactatat ccggccggat atccacagga cgggtgtggt     2160
   cgccatgatc gcgtagtcga tattattaga ccagcttatg gattgtttcc atatcggctg     2220
   cctccagcag ggtaccctgt tccacacgcg cccaccattc catcagaaag ttgtccacga 2280
   ccaagcggaa ccaaggtgac agtttcacac cttcctctcc ggcatccgcc ttgcgcagca 2340
   gctctttcag ctgatcacga ttgacatact ttacatccgc cacttcatcc ggattcggca 2400
   gcagtttcac atcgcgcacg ataaacagca aataatctac ctcatgctcg ccccatttac 2460
   cgtcggatgg cgccttgtac agcatacggc ccagcgggat aaactcatcc actggaatgt 2520
   cctccgcagg aatgcccagt tcatccagga gtttacgctg cgcggcattg cgcacaccca 2580
   ggtagttttc ttgaattaac tcagactcgc ggtacaaggg gtggctgcag caggtgtttg 2640
   tccagaccag ggggaaggtc accttggttt cactgcgttg ctgtaaaagt aattcatatt 2700
   tgctattaaa cagaaacact gaaaacgcac ggtgcaagag gttcaacgac tcaatctttt 2760
   cgacaagatg gcaattatat ttcgactcat gtccaatcac aacgtcgttt tcgtcaacga 2820
   gaatacattc atcttcaaac ataagacggc gctgaacagc atccatgctg gcatcgtcgg 2880
   ctacggctgc catgaattta acctcctgat aaactaccgc attaaagctt atcgatgata 2940
   agctgtcaaa catgagaatt cttgaagacg aaagggcctc gtgatacgcc tatttttata 3000
   ggttaatgtc atgcatgaga caataaccct gataaatgct tcaataatat tgaaaaagga 3060
   agagtatgag tattcaacat ttccgtgtcg cccttattcc cttttttgcg gcattttgcc 3120
   ttcctgtttt tgctcaccca gaaacgctgg tgaaagtaaa agatgctgaa gatcagttgg 3180
   gtgcacgagt gggttacatc gaactggatc tcaacagcgg taagatcctt gagagttttc 3240
   gccccgaaga acgttttcca atgatgagca cttttaaagt tctgctatgt ggcgcggtat 3300
   tatcccgtgt tgacgccggg caagagcaac tcggtcgccg catacactat tctcagaatg 3360
   acttggttga cgcgtcacca gtcacagaaa agcatcttac ggatggcatg acagtaagag 3420
   aattatgcag tgctgccata accatgagtg ataacactgc ggccaactta cttctgacaa 3480
   cgatcggagg accgaaggag ctaaccgctt ttttgcacaa catgggggat catgtaactc 3540
   gccttgatcg ttgggaaccg gagctgaatg aagccatacc aaacgacgag cgtgacacca 3600
   cgatgcctgc agcaatggca acaacgttgc gcaaactatt aactggcgaa ctacttactc 3660
   tagcttcccg gcaacaatta atagactgga tggaggcgga taaagttgca ggaccacttc 3720
   tgcgctcggc ccttccggct ggctggttta ttgctgataa atctggagcc ggtgagcgtg 3780
   ggtctcgcgg tatcattgca gcactggggc cagatggtaa gccctcccgt atcgtagtta 3840
   tctacacgac ggggagtcag gcaactatgg atgaacgaaa tagacagatc gctgagatag 3900
   gtgcctcact gattaagcat tggtaactgt cagaccaagt ttactcatat atactttaga 3960
   ttgatttaaa acttcatttt taatttaaaa ggatctaggt gaagatcctt tttgataatc 4020
   tcatgcatga ccaaaatccc ttaacgtgag ttttcgttcc actgagcgtc agaccccgta 4080
   gaaaagatca aaggatcttc ttgagatcct ttttttctgc gcgtaatctg ctgcttgcaa 4140
   acaaaaaaac caccgctacc agcggtggtt tgtttgccgg atcaagagct accaactctt 4200
   tttccgaagg taactggctt cagcagagcg cagataccaa atactgtcct tctagtgtag 4260
   ccgtagttag gccaccactt caagaactct gtagcaccgc ctacatacct cgctctgcta 4320
   atcctgttac cagtggctgc tgccagtggc gataagtcgt gtcttaccgg gttggactca 4380
   agacgatagt taccggataa ggcgcagcgg tcgggctgaa cggggggttc gtgcacacag 4440
   cccagcttgg agcgaacgac ctacaccgaa ctgagatacc tacagcgtga gctatgagaa 4500
   agcgccacgc ttcccgaagg gagaaaggcg gacaggtatc cggtaagcgg cagggtcgga 4560
   acaggagagc gcacgaggga gcttccaggg ggaaacgcct ggtatcttta tagtcctgtc 4620
   gggtttcgcc acctctgact tgagcgtcga tttttgtgat gctcgtcagg ggggcggagc 4680
   ctatggaaaa acgccagcaa cgcggccttt ttacggttcc tggccttttg ctggcctttt 4740
   gctcacatgt tctttcctgc gttatcccct gattctgtgg ataaccgtat taccgccttt 4800
   gagtgagctg ataccgctcg ccgcagccga acgaccgagc gcagcgagtc agtgagcgag 4860
   gaagcggaag agcgcctgat gcggtatttt ctccttacgc atctgtgcgg tatttcacac 4920
   cgcatatatg gtgcactctc agtacaatct gctctgatgc cgcatagtta agccagtata 4980
   cactccgcta tcgctacgtg actgggtcat ggctgcgccc cgacacccgc caacacccgc 5040
   tgacgcgccc tgacgggctt gtctgctccc ggcatccgct tacagacaag ctgtgaccgt 5100
   ctccgggagc tgcatgtgtc agaggttttc accgtcatca ccgaaacgcg cgaggcagct 5160
   ggcacgacag gtttcccgac tgg 5183
<210> 46
   <211> 6337
   <212> DNA
   <213> Artifizielle Sequenz
<220>
   <223> Plasmid pGT1579
<400> 46
   aaagcgaatt gtgattgaaa tcaaaatttt cttgactaat tatggtataa tttactggta      60
   ataattttgt ttaactttaa gaaggagata taccatgggc gatcttttat caatacagga     120
   cccgtcgttt ttaaaaaaca tgtccattga tgaattagag aaattaagtg atgaaatccg     180
   tcagttttta attacaagtt tatccgcttc cggcggccac atcggcccaa acttaggtgt     240
   cgtagagctt actgttgccc tgcataagga atttaacagc ccgaaagaca aatttttatg     300
   ggatgtaggc catcagtcgt atgtccataa gctgctgaca ggacgcggaa aagaatttgc     360
   gacgcttcgc cagtacaaag ggctttgcgg atttccaaag cggagtgaaa gcgagcacga     420
   tgtttgggaa accgggcaca gctcgacttc tctgtcaggc gcgatgggaa tggcagctgc     480
   ccgtgatatt aaaggaacgg atgaatatat tattccgatc attggtgacg gcgcgctgac 540
   cggcggtatg gcgctcgaag cccttaacca catcggcgac gagaaaaaag acatgattgt 600
   catccttaat gataatgaaa tgagtattgc gccaaacgtc ggtgccattc actctatgct 660
   cggacggctc cgcactgcgg ggaaatacca gtgggtcaaa gatgagcttg aatacttatt 720
   taaaaagatt ccggcagttg ggggcaagct tgccgccacg gcggaacggg tcaaagacag 780
   cctgaaatac atgctcgtct ccggaatgtt tttcgaggag ctcggtttta cgtatttggg 840
   cccagtggac ggacattctt atcatgagct gattgagaat cttcaatacg ccaaaaaaac 900
   gaaaggccct gttcttctgc acgtcattac gaaaaaaggg aaggggtaca aaccggctga 960
   gaccgatacg attgggacat ggcatggtac cggaccatat aaaattaata ccggtgactt 1020
   tgtaaagccg aaagccgcag ctccttcgtg gagcggtctt gtcagcggaa ctgtgcagcg 1080
   aatggcgcgc gaggacggac gcattgtagc cattacgccg gctatgcctg tcggttcaaa 1140
   gcttgaaggc ttcgcaaagg aattccctga ccggatgttc gacgtaggaa tcgcagaaca 1200
   gcatgccgca acaatggctg cagctatggc aatgcagggt atgaagccgt ttttggcgat 1260
   ttactcaacc ttcctgcaaa gggcatatga ccaagttgtt catgacatct gccgccaaaa 1320
   cgctaatgtg tttattggaa ttgaccgtgc tggactcgtt ggcgctgatg gagagacaca 1380
   tcaaggcgtg tttgatattg cgtttatgcg ccacattcca aacatggtct taatgatgcc 1440
   gaaagacgaa aatgaaggcc agcacatggt tcatacagca cttagctatg acgaaggccc 1500
   gatagcaatg cgttttccgc gcggaaacgg actcggcgta aaaatggatg aacagttgaa 1560
   aacgattccg atcggtacgt gggaggtgct gcgtccaggg aacgatgctg tcatcttaac 1620
   attcggcaca acaatcgaaa tggcgattga agcagccgaa gagctgcaga aagaaggcct 1680
   ttccgtgcgc gttgtgaatg cgcgttttat taagccgatt gatgaaaaga tgatgaagag 1740
   tatcctaaaa gaaggcttgc caattttaac aattgaagaa gcggtcttag aaggcggttt 1800
   cggaagctcg attttagaat tcgctcatga tcaaggtgaa tatcatactc cgattgacag 1860
   aatgggtata cctgatcggt ttattgaaca cggaagtgta acagcgcttc ttgaggaaat 1920
   tggactgaca aaacagcagg tggcaaatcg tattagatta ctgatgccac caaagacaca 1980
   caaaggaatt ggatcataag gatccggctg ctaacaaagc ccgaaaggaa gctgagttgg 2040
   ctgctgccac cgctgagcaa taactagcat aaccccttgg ggcctctaaa cgggtcttga 2100
   ggggtttttt gctgaaagga ggaactatat ccggccggat atccacagga cgggtgtggt 2160
   cgccatgatc gcgtagtcga aaaaacccgc cagaatagcg ggtagattat tatttttcaa 2220
   cctgctgaac gtcaattcga cgcgcttcgt ccagctgact ggctttcgca cgaatgcgtg 2280
   cttccagctg gtcgatcata tcgttgttgt ccagacggtc tttgcgcacg ccatcttcat 2340
   agaggccgct tttcttgttg ccgccggtga cgccgagtgt agaaaccagc gcctcacctg 2400
   ggccattcac cacgcagccg ataatcgaaa cgtccatcgg agtgatgata tcttccaggc 2460
   gttgctccag cgcgttaacc gtaccgataa catcaaattc ctgacgcgaa caggtcgggc 2520
   aggcgatgaa gttgatccct cgcgaacgga tacgcagcga tttcaaaata tcgaaaccga 2580
   ctttgatctc ttcgaccgga tcggccgcca gcgatacgcg cagcgtgtcg ccgatgcctt 2640
   cagacagcag cagacctaaa ccaatggcgg attttactgc cccgctgcgc gcaccaccgg 2700
   cttcggtgat ccccagatgc aacggctgat cgatctgttt tgccagcaaa cgataagact 2760
   caacagcgag gaagacgtca gacgctttca cgctgacttt gaactgatcg aagttcaggc 2820
   gatcgagatg atcaacatga cgcatggcag attccagcaa cgcctgcggc gtcggttcgc 2880
   catacttttc ttgcagatct ttttccagcg atccggcgtt aacgccaata cggatcggaa 2940
   tgtttttatc gcgcgcacag tcaaccacca tgcgaatacg ctcttcatta ccgatattgc 3000
   cagggttaat acgcagacaa tcgacgccgt attccgctac tttcagcgca atgcgatagt 3060
   cgaagtggat gtcagccacc agcggcacgt taacctgctg tttgatgagt ttgaacgctt 3120
   ctgccgcgtc catcgtcggt acggatacac ggacgatatc agcgccaacg cgttccagcg 3180
   ccttgatttg attgaccgtt gcttcgacgt ctgtcgtacg cgtattggtc atggactgta 3240
   cggcgatggg agcaccatcg ccaatcggca cattcccaac gtaaatacgt gttgattttc 3300
   tacgttgaat tggagcctgg ttatgcatta gtaatctcct tagaccagct tatggattgt 3360
   ttccatatcg gctgcctcca gcagggtacc ctgttccaca cgcgcccacc attccatcag 3420
   aaagttgtcc acgaccaagc ggaaccaagg tgacagtttc acaccttcct ctccggcatc 3480
   cgccttgcgc agcagctctt tcagctgatc acgattgaca tactttacat ccgccacttc 3540
   atccggattc ggcagcagtt tcacatcgcg cacgataaac agcaaataat ctacctcatg 3600
   ctcgccccat ttaccgtcgg atggcgcctt gtacagcata cggcccagcg ggataaactc 3660
   atccactgga atgtcctccg caggaatgcc cagttcatcc aggagtttac gctgcgcggc 3720
   attgcgcaca cccaggtagt tttcttgaat taactcagac tcgcggtaca aggggtggct 3780
   gcagcaggtg tttgtccaga ccagggggaa ggtcaccttg gtttcactgc gttgctgtaa 3840
   aagtaattca tatttgctat taaacagaaa cactgaaaac gcacggtgca agaggttcaa 3900
   cgactcaatc ttttcgacaa gatggcaatt atatttcgac tcatgtccaa tcacaacgtc 3960
   gttttcgtca acgagaatac attcatcttc aaacataaga cggcgctgaa cagcatccat 4020
   gctggcatcg tcggctacgg ctgccatgaa tttaacctcc tgataaacta ccgcattaaa 4080
   gcttatcgat gataagctgt caaacatgag aattcttgaa gacgaaaggg cctcgtgata 4140
   cgcctatttt tataggttaa tgtcatgcat gagacaataa ccctgataaa tgcttcaata 4200
   atattgaaaa aggaagagta tgagtattca acatttccgt gtcgccctta ttcccttttt 4260
   tgcggcattt tgccttcctg tttttgctca cccagaaacg ctggtgaaag taaaagatgc 4320
   tgaagatcag ttgggtgcac gagtgggtta catcgaactg gatctcaaca gcggtaagat 4380
   ccttgagagt tttcgccccg aagaacgttt tccaatgatg agcactttta aagttctgct 4440
   atgtggcgcg gtattatccc gtgttgacgc cgggcaagag caactcggtc gccgcataca 4500
   ctattctcag aatgacttgg ttgacgcgtc accagtcaca gaaaagcatc ttacggatgg 4560
   catgacagta agagaattat gcagtgctgc cataaccatg agtgataaca ctgcggccaa 4620
   cttacttctg acaacgatcg gaggaccgaa ggagctaacc gcttttttgc acaacatggg 4680
   ggatcatgta actcgccttg atcgttggga accggagctg aatgaagcca taccaaacga 4740
   cgagcgtgac accacgatgc ctgcagcaat ggcaacaacg ttgcgcaaac tattaactgg 4800
   cgaactactt actctagctt cccggcaaca attaatagac tggatggagg cggataaagt 4860
   tgcaggacca cttctgcgct cggcccttcc ggctggctgg tttattgctg ataaatctgg 4920
   agccggtgag cgtgggtctc gcggtatcat tgcagcactg gggccagatg gtaagccctc 4980
   ccgtatcgta gttatctaca cgacggggag tcaggcaact atggatgaac gaaatagaca 5040
   gatcgctgag ataggtgcct cactgattaa gcattggtaa ctgtcagacc aagtttactc 5100
   atatatactt tagattgatt taaaacttca tttttaattt aaaaggatct aggtgaagat 5160
   cctttttgat aatctcatgc atgaccaaaa tcccttaacg tgagttttcg ttccactgag 5220
   cgtcagaccc cgtagaaaag atcaaaggat cttcttgaga tccttttttt ctgcgcgtaa 5280
   tctgctgctt gcaaacaaaa aaaccaccgc taccagcggt ggtttgtttg ccggatcaag 5340
   agctaccaac tctttttccg aaggtaactg gcttcagcag agcgcagata ccaaatactg 5400
   tccttctagt gtagccgtag ttaggccacc acttcaagaa ctctgtagca ccgcctacat 5460
   acctcgctct gctaatcctg ttaccagtgg ctgctgccag tggcgataag tcgtgtctta 5520
   ccgggttgga ctcaagacga tagttaccgg ataaggcgca gcggtcgggc tgaacggggg 5580
   gttcgtgcac acagcccagc ttggagcgaa cgacctacac cgaactgaga tacctacagc 5640
   gtgagctatg agaaagcgcc acgcttcccg aagggagaaa ggcggacagg tatccggtaa 5700
   gcggcagggt cggaacagga gagcgcacga gggagcttcc agggggaaac gcctggtatc 5760
   tttatagtcc tgtcgggttt cgccacctct gacttgagcg tcgatttttg tgatgctcgt 5820
   caggggggcg gagcctatgg aaaaacgcca gcaacgcggc ctttttacgg ttcctggcct 5880
   tttgctggcc ttttgctcac atgttctttc ctgcgttatc ccctgattct gtggataacc 5940
   gtattaccgc ctttgagtga gctgataccg ctcgccgcag ccgaacgacc gagcgcagcg 6000
   agtcagtgag cgaggaagcg gaagagcgcc tgatgcggta ttttctcctt acgcatctgt 6060
   gcggtatttc acaccgcata tatggtgcac tctcagtaca atctgctctg atgccgcata 6120
   gttaagccag tatacactcc gctatcgcta cgtgactggg tcatggctgc gccccgacac 6180
   ccgccaacac ccgctgacgc gccctgacgg gcttgtctgc tcccggcatc cgcttacaga 6240
   caagctgtga ccgtctccgg gagctgcatg tgtcagaggt tttcaccgtc atcaccgaaa 6300
   cgcgcgaggc agctggcacg acaggtttcc cgactgg 6337
<210> 47
   <211> 6378
   <212> DNA
   <213> Artifizielle Sequenz
<220>
   <223> Plasmid pGT1582
<400> 47
   aaagcgaatt gtgattgaaa tcaaaatttt cttgactaat tatggtataa tttactggta      60
   atacgataaa tcagcccggg aattaacatg gcaaccactc atttggatgt ttgcgccgtg     120
   gttccggcgg ccggatttgg ccgtcgaatg caaacggaat gtcctaagca atatctctca     180
   atcggtaatc aaaccattct tgaacactcg gtgcatgcgc tgctggcgca tccccgggtg     240
   aaacgtgtcg tcattgccat aagtcctggc gatagccgtt ttgcacaact tcctctggcg     300
   aatcatccgc aaatcaccgt tgtagatggc ggtgatgagc gtgccgattc cgtgctggca     360
   ggtctgaaag ccgctggcga cgcgcagtgg gtattggtgc atgacgccgc tcgtccttgt     420
   ttgcatcagg atgacctcgc gcgattgttg gcgttgagcg aaaccagccg cacggggggg     480
   atcctcgccg caccagtgcg cgatactatg aaacgtgccg aaccgggcaa aaatgccatt     540
   gctcataccg ttgatcgcaa cggcttatgg cacgcgctga cgccgcaatt tttccctcgt     600
   gagctgttac atgactgtct gacgcgcgct ctaaatgaag gcgcgactat taccgacgaa     660
   gcctcggcgc tggaatattg cggattccat cctcagttgg tcgaaggccg tgcggataac     720
   attaaagtca cgcgcccgga agatttggca ctggccgagt tttacctcac ccgaaccatc     780
   catcaggaga atacataatg cgaattggac acggttttga cgtacatgcc tttggcggtg     840
   aaggcccaat tatcattggt ggcgtacgca ttccttacga aaaaggattg ctggcgcatt     900
   ctgatggcga cgtggcgctc catgcgttga ccgatgcatt gcttggcgcg gcggcgctgg     960
   gggatatcgg caagctgttc ccggataccg atccggcatt taaaggtgcc gatagccgcg    1020
   agctgctacg cgaagcctgg cgtcgtattc aggcgaaggg ttataccctt ggcaacgtcg    1080
   atgtcactat catcgctcag gcaccgaaga tgttgccgca cattccacaa atgcgcgtgt    1140
   ttattgccga agatctcggc tgccatatgg atgatgttaa cgtgaaagcc actactacgg    1200
   aaaaactggg atttaccgga cgtggggaag ggattgcctg tgaagcggtg gcgctactca    1260
   ttaaggcaac aaaataagga ggatatacca tgggcgatct tttatcaata caggacccgt    1320
   cgtttttaaa aaacatgtcc attgatgaat tagagaaatt aagtgatgaa atccgtcagt    1380
   ttttaattac aagtttatcc gcttccggcg gccacatcgg cccaaactta ggtgtcgtag 1440
   agcttactgt tgccctgcat aaggaattta acagcccgaa agacaaattt ttatgggatg 1500
   taggccatca gtcgtatgtc cataagctgc tgacaggacg cggaaaagaa tttgcgacgc 1560
   ttcgccagta caaagggctt tgcggatttc caaagcggag tgaaagcgag cacgatgttt 1620
   gggaaaccgg gcacagctcg acttctctgt caggcgcgat gggaatggca gctgcccgtg 1680
   atattaaagg aacggatgaa tatattattc cgatcattgg tgacggcgcg ctgaccggcg 1740
   gtatggcgct cgaagccctt aaccacatcg gcgacgagaa aaaagacatg attgtcatcc 1800
   ttaatgataa tgaaatgagt attgcgccaa acgtcggtgc cattcactct atgctcggac 1860
   ggctccgcac tgcggggaaa taccagtggg tcaaagatga gcttgaatac ttatttaaaa 1920
   agattccggc agttgggggc aagcttgccg ccacggcgga acgggtcaaa gacagcctga 1980
   aatacatgct cgtctccgga atgtttttcg aggagctcgg ttttacgtat ttgggcccag 2040
   tggacggaca ttcttatcat gagctgattg agaatcttca atacgccaaa aaaacgaaag 2100
   gccctgttct tctgcacgtc attacgaaaa aagggaaggg gtacaaaccg gctgagaccg 2160
   atacgattgg gacatggcat ggtaccggac catataaaat taataccggt gactttgtaa 2220
   agccgaaagc cgcagctcct tcgtggagcg gtcttgtcag cggaactgtg cagcgaatgg 2280
   cgcgcgagga cggacgcatt gtagccatta cgccggctat gcctgtcggt tcaaagcttg 2340
   aaggcttcgc aaaggaattc cctgaccgga tgttcgacgt aggaatcgca gaacagcatg 2400
   ccgcaacaat ggctgcagct atggcaatgc agggtatgaa gccgtttttg gcgatttact 2460
   caaccttcct gcaaagggca tatgaccaag ttgttcatga catctgccgc caaaacgcta 2520
   atgtgtttat tggaattgac cgtgctggac tcgttggcgc tgatggagag acacatcaag 2580
   gcgtgtttga tattgcgttt atgcgccaca ttccaaacat ggtcttaatg atgccgaaag 2640
   acgaaaatga aggccagcac atggttcata cagcacttag ctatgacgaa ggcccgatag 2700
   caatgcgttt tccgcgcgga aacggactcg gcgtaaaaat ggatgaacag ttgaaaacga 2760
   ttccgatcgg tacgtgggag gtgctgcgtc cagggaacga tgctgtcatc ttaacattcg 2820
   gcacaacaat cgaaatggcg attgaagcag ccgaagagct gcagaaagaa ggcctttccg 2880
   tgcgcgttgt gaatgcgcgt tttattaagc cgattgatga aaagatgatg aagagtatcc 2940
   taaaagaagg cttgccaatt ttaacaattg aagaagcggt cttagaaggc ggtttcggaa 3000
   gctcgatttt agaattcgct catgatcaag gtgaatatca tactccgatt gacagaatgg 3060
   gtatacctga tcggtttatt gaacacggaa gtgtaacagc gcttcttgag gaaattggac 3120
   tgacaaaaca gcaggtggca aatcgtatta gattactgat gccaccaaag acacacaaag 3180
   gaattggatc ataaggatcc ggctgctaac aaagcccgaa aggaagctga gttggctgct 3240
   gccaccgctg agcaataact agcataaccc cttggggcct ctaaacgggt cttgaggggt 3300
   tttttgctga aaggaggaac tatatccggc cggatatcca caggacgggt gtggtcgcca     3360
   tgatcgcgta gtcgatatta ttagaccagc ttatggattg tttccatatc ggctgcctcc     3420
   agcagggtac cctgttccac acgcgcccac cattccatca gaaagttgtc cacgaccaag     3480
   cggaaccaag gtgacagttt cacaccttcc tctccggcat ccgccttgcg cagcagctct     3540
   ttcagctgat cacgattgac atactttaca tccgccactt catccggatt cggcagcagt     3600
   ttcacatcgc gcacgataaa cagcaaataa tctacctcat gctcgcccca tttaccgtcg     3660
   gatggcgcct tgtacagcat acggcccagc gggataaact catccactgg aatgtcctcc     3720
   gcaggaatgc ccagttcatc caggagttta cgctgcgcgg cattgcgcac acccaggtag     3780
   ttttcttgaa ttaactcaga ctcgcggtac aaggggtggc tgcagcaggt gtttgtccag     3840
   accaggggga aggtcacctt ggtttcactg cgttgctgta aaagtaattc atatttgcta     3900
   ttaaacagaa acactgaaaa cgcacggtgc aagaggttca acgactcaat cttttcgaca     3960
   agatggcaat tatatttcga ctcatgtcca atcacaacgt cgttttcgtc aacgagaata     4020
   cattcatctt caaacataag acggcgctga acagcatcca tgctggcatc gtcggctacg     4080
   gctgccatga atttaacctc ctgataaact accgcattaa agcttatcga tgataagctg     4140
   tcaaacatga gaattcttga agacgaaagg gcctcgtgat acgcctattt ttataggtta     4200
   atgtcatgca tgagacaata accctgataa atgcttcaat aatattgaaa aaggaagagt     4260
   atgagtattc aacatttccg tgtcgccctt attccctttt ttgcggcatt ttgccttcct     4320
   gtttttgctc acccagaaac gctggtgaaa gtaaaagatg ctgaagatca gttgggtgca     4380
   cgagtgggtt acatcgaact ggatctcaac agcggtaaga tccttgagag ttttcgcccc     4440
   gaagaacgtt ttccaatgat gagcactttt aaagttctgc tatgtggcgc ggtattatcc     4500
   cgtgttgacg ccgggcaaga gcaactcggt cgccgcatac actattctca gaatgacttg     4560
   gttgacgcgt caccagtcac agaaaagcat cttacggatg gcatgacagt aagagaatta     4620
   tgcagtgctg ccataaccat gagtgataac actgcggcca acttacttct gacaacgatc     4680
   ggaggaccga aggagctaac cgcttttttg cacaacatgg gggatcatgt aactcgcctt     4740
   gatcgttggg aaccggagct gaatgaagcc ataccaaacg acgagcgtga caccacgatg     4800
   cctgcagcaa tggcaacaac gttgcgcaaa ctattaactg gcgaactact tactctagct     4860
   tcccggcaac aattaataga ctggatggag gcggataaag ttgcaggacc acttctgcgc     4920
   tcggcccttc cggctggctg gtttattgct gataaatctg gagccggtga gcgtgggtct     4980
   cgcggtatca ttgcagcact ggggccagat ggtaagccct cccgtatcgt agttatctac     5040
   acgacgggga gtcaggcaac tatggatgaa cgaaatagac agatcgctga gataggtgcc     5100
   tcactgatta agcattggta actgtcagac caagtttact catatatact ttagattgat     5160
   ttaaaacttc atttttaatt taaaaggatc taggtgaaga tcctttttga taatctcatg     5220
   catgaccaaa atcccttaac gtgagttttc gttccactga gcgtcagacc ccgtagaaaa     5280
   gatcaaagga tcttcttgag atcctttttt tctgcgcgta atctgctgct tgcaaacaaa     5340
   aaaaccaccg ctaccagcgg tggtttgttt gccggatcaa gagctaccaa ctctttttcc     5400
   gaaggtaact ggcttcagca gagcgcagat accaaatact gtccttctag tgtagccgta     5460
   gttaggccac cacttcaaga actctgtagc accgcctaca tacctcgctc tgctaatcct     5520
   gttaccagtg gctgctgcca gtggcgataa gtcgtgtctt accgggttgg actcaagacg     5580
   atagttaccg gataaggcgc agcggtcggg ctgaacgggg ggttcgtgca cacagcccag     5640
   cttggagcga acgacctaca ccgaactgag atacctacag cgtgagctat gagaaagcgc     5700
   cacgcttccc gaagggagaa aggcggacag gtatccggta agcggcaggg tcggaacagg     5760
   agagcgcacg agggagcttc cagggggaaa cgcctggtat ctttatagtc ctgtcgggtt     5820
   tcgccacctc tgacttgagc gtcgattttt gtgatgctcg tcaggggggc ggagcctatg     5880
   gaaaaacgcc agcaacgcgg cctttttacg gttcctggcc ttttgctggc cttttgctca     5940
   catgttcttt cctgcgttat cccctgattc tgtggataac cgtattaccg cctttgagtg     6000
   agctgatacc gctcgccgca gccgaacgac cgagcgcagc gagtcagtga gcgaggaagc     6060
   ggaagagcgc ctgatgcggt attttctcct tacgcatctg tgcggtattt cacaccgcat     6120
   atatggtgca ctctcagtac aatctgctct gatgccgcat agttaagcca gtatacactc     6180
   cgctatcgct acgtgactgg gtcatggctg cgccccgaca cccgccaaca cccgctgacg     6240
   cgccctgacg ggcttgtctg ctcccggcat ccgcttacag acaagctgtg accgtctccg     6300
   ggagctgcat gtgtcagagg ttttcaccgt catcaccgaa acgcgcgagg cagctggcac     6360
   gacaggtttc ccgactgg                                                   6378